# FASCICULE DE BREVET EUROPEEN

(11) **EP 2 382 205 B1**
(45) Date de publication et mention de la délivrance du brevet: **23.04.2014**
(21) Numéro de dépôt: 09805789.6
(22) Date de dépôt: 24.12.2009
(51) Int. Cl.: C07D 401/14, C07D 409/14, A61K 31/4439, G01N 33/68, C07D 401/04

(54) **DÉRIVÉS DE 2-PYRIDIN-2-YL-PYRAZOL-3(2H)-ONE, LEUR PRÉPARATION ET LEUR APPLICATION EN THÉRAPEUTIQUE COMME ACTIVATEURS DE HIF**
DERIVATE VON 2-PYRIDIN-2-YLPYRAZOL-3(2H)-ON, DEREN HERSTELLUNG UND DEREN THERAPEUTISCHE VERWENDUNG ALS HIF-AKTIVATOREN
DERIVATIVES OF 2-PYRIDIN-2-YL-PYRAZOL-3(2H)-ONE, PREPARATION AND THERAPEUTIC USE THEREOF AS HIF ACTIVATORS

(30) Priorité: 29.12.2008 FR 0807474; 28.08.2009 FR 0904092
(43) Date de publication de la demande: 02.11.2011
(73) Titulaire: SANOFI, 75008 Paris (FR)
(72) Inventeur: ALTENBURGER, Jean-Michel, F-75013 Paris (FR); FOSSEY, Valérie, F-75013 Paris (FR); ILLIANO, Stéphane, F-75013 Paris (FR); MANETTE, Géraldine, F-75013 Paris (FR)
(74) Mandataire: Rauline, Mathilde
(86) Numéro de dépôt international: PCT/FR2009/052691
(87) Numéro de publication internationale: WO 2010/076524

(56) Documents cités:
- EP-A- 0 469 357
- WO-A-2006/114213
- WO-A-2008/067871
- DATABASE CA [Online] CHEMICAL ABSTRACTS SERVICE, COLUMBUS, OHIO, US; RN: 5193-04-4 1966, BUECHI, J ET AL: "Synthesis and pharmacological properties of certain pyridylpyrazol-5-ones" XP002530755 extrait de STN Database accession no. 1966:51993 & HELVETICA CHIMICA ACTA , 49(1), 272-80 CODEN: HCACAV; ISSN: 0018-019X, 1966,
- EGLEN R M ET AL: "BETA GALACTOSIDASE ENZYME FRAGMENT COMPLEMENTATION AS A NOVEL TECHNOLOGY FOR HIGH THROUGHPUT SCREENING" COMBINATORIAL CHEMISTRY AND HIGH THROUGHPUT SCREENING, BENTHAM SCIENCE PUBLISHERS, NL, vol. 6, no. 4, 1 juin 2003 (2003-06-01), pages 381-387, XP008053948 ISSN: 1386-2073
- EGLEN RICHARD M: "Enzyme fragment complementation: A flexible high throughput screening assay technology" ASSAY AND DRUG DEVELOPMENT TECHNOLOGIES, US LNKD- DOI:10.1089/154065802761001356, vol. 1, no. 1, 1 novembre 2002 (2002-11-01), pages 97-104, XP002503059 ISSN: 1540-658X

## Description

La présente invention a pour objet des nouveaux dérivés de dihydropyrazolones substituées, leur préparation et leur application en thérapeutique en tant qu'activateurs du facteur de transcription HIF.

Le facteur induit par l'hypoxie (HIF pour « Hypoxia Inducible Factor ») (HIF1α) est un facteur de transcription exprimé de façon constitutive dans tous les tissus. Cette protéine a été découverte en 1994 par Gregg Semenza lors de l'étude des séquences régulatrices du gène de l'EPO. Il a identifié une séquence située en position 3' non-codante dans le promoteur de l'EPO, qui porte le nom d'« hypoxia response element » (HRE) et qui est un site de fixation de la protéine HIF1α permettant l'activation transcriptionnelle de l'EPO. Par la suite, la séquence HRE a aussi été localisée sur plus de 70 autres gènes, tels que le VEGF (vascular endothelial growth factor) ou Glut1 (glucose transporter 1). Le complexe transcriptionnel HIF-1 est à minima un hétérodimère constitué de la protéine HIF1α ou HIF2α et d'un autre facteur de transcrition ARNT (anciennement nommé HIF1β). ARNT est exprimé de façon constitutive et stable dans les cellules et l'essentiel de la régulation du complexe transcriptionnel est lié à la quantité de HIF1α présent dans les cellules et qui est donc le facteur limitant.

Dans des conditions normales en oxygène la protéine HIF1α est rapidement dégradée (demi-vie de 5 minutes). Cette dégradation est consécutive à l'hydroxylation de HIF1α ou de HIF2α respectivement sur les Prolines 402 et 563 et les Prolines 405 et 531 pour les formes humaines par les HIF Prolyl Hydroxylase (HIF_PHDs ou EGLNs). Cette hydroxylation permet la liaison de la protéine de Von Hippell Lindau (pVHL) associée à une ubiquitine ligase, ce qui va entrainer la dégradation de HIF1α ou HIF2α par le système ubiquitine protéasome. Lorsque la cellule ou le tissu sont soumis à une hypoxie/ischémie, HIF1α ou HIF2α ne sont plus dégradés par le système ubiquitin-protéasome et peuvent alors s'associer avec les autres facteurs de transcription du complexe HIF pour basculer dans le noyau et activer leurs gènes cibles.

Bien que l'hypoxie soit la principale cause de l'activation des protéines HIF1-α et HIF2α d'autres inducteurs, tels que l'insuline, les facteurs de croissance peuvent aussi jouer un rôle dans leur stabilisation notamment via sa phosphorylation sur les Serine 641 et 643.

WO 2006/114213 et WO 2008/067871 décrivent des dérivés du pyrazole et leurs utilisation pour le traitement de maladie cardiovasculaires et de troubles hématologiques.

Un screening phénotypique visant à mesurer la stabilisation de la protéine HIF1α et/ou HIF2α a donc été établi pour identifier les composés de la présente invention.

Les composés selon la présente invention répondent à la formule (I) suivante: 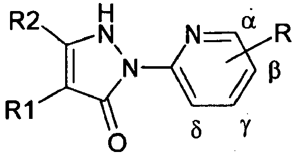 dans laquelle
**R** représente un groupe -SO₂-NR3R4 ou un groupe -SO₂-R4 ; **R3, R4** et **R5** étant tels que définis ci-dessous ;
**R1** représente un groupe hétérocycloalkyle ne contenant pas d'atome d'azote, un groupe -W-(C3-C6)cycloalkyle, un groupe W-aryle, un groupe W-hétéroaryle, un groupe W-hétérocycloalkyle, un groupe -W-COOR5 ou un groupe -W-CONR5R6,
   (i) lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes - (C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkylène-O-(C1-C5)alkyle, les groupes -O-(C1-C5)alkylène-NR5R6, les groupes -SO₂-(C1-C5)alkyle, les groupes -NR5R6 et les groupes - CO₂R5, et
   (ii) étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
**R2** représente un atome d'hydrogène, un groupe -(C1-C5)alkyle, un groupe -(C1-C5)alkylène-O-(C1-C5)alkyle, un groupe -halogéno(C1-C5)alkyle, un groupe -W-COOR5, un groupe -W-C(O)NHR5 ou un groupe W-C(O)-NR5R6 ; **W, R5** et **R6** étant tels que définis ci-dessous;
étant entendu que :
o n représente 0, 1 ou 2 ;
o **W est**
   (i) un groupe -(C1-C5)alkylène-, éventuellement substitué par un groupe choisi parmi les groupes -(CH₂)n-CO₂R5 et les groupes -(CH₂)n-(CO)NR5R6, avec n tel que défini ci-dessus et **R5** et **R6** tels que définis ci-dessous ;ou
   (ii) un groupe -(C3-C6)cycloalkylène-,
o **R3** et **R4**
   (i) Identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe -W-CHOH-CH₂OH, un groupe -W-CO₂R5, un groupe -W-NR5R6 ou un groupe -W-O-(CH₂)n-aryle;
      lesdits groupes -(C3-C6)cycloalkyle et hétérocycloalkyle étant éventuellement substitués
      o sur au moins un atome de carbone par au moins un groupe choisi parmi -(C1-C5)alkyle, un groupe -(C1-C5)alcoxy, une fonction hydroxy, un groupe -W-NR5R6 et un groupe -W-CO₂R5, dans le cas des groupes -(C3-C6)cycloalkyle et hétérocycloalkyle et/ou
      o sur au moins un hétéroatome choisi parmi l'azote par au moins un groupe choisi parmi -(C1-C5)alkyle dans le cas d'un groupe hétérocycloalkyle,
      avec **W** et **n** tels que définis précédemment et **R5** et **R6** tels que définis ci-dessous et étant entendu que lorsque **R3** et **R4** sont identiques, ils ne peuvent pas être un atome d'hydrogène;
   (ii) ou bien **R3** et **R4** forment ensemble avec l'atome d'azote qui les porte un groupe hétérocycloalkyle, éventuellement substitué sur au moins un atome de carbone et/ou, le cas échéant, sur au moins un hétéroatome; par au moins un substituant choisi parmi les groupes -(C1-C5)alkyle et les groupes -CH₂-aryle ;
o **R5** et R6, identiques ou différents, représentent, indépendamment l'un de l'autre, un atome d'hydrogène, un groupe -(C1-C5)alkyle ou un groupe (C1-C5) halogénoalkyle.

Les composés de formule (I) peuvent exister à l'état de bases ou de sels, les composés de formule (I) ayant été dans ce cas salifiés par des acides ou des bases, notamment des acides ou des bases pharmaceutiquement acceptables. On parle alors de sels d'addition qui font partie de l'invention.
Ces sels sont avantageusement préparés avec des acides pharmaceutiquement acceptables, mais les sels d'autres acides utiles, par exemple, pour la purification ou l'isolement des composés de formule (I), font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Les différentes formes tautomériques des composés de formule (I) font également partie de l'invention.

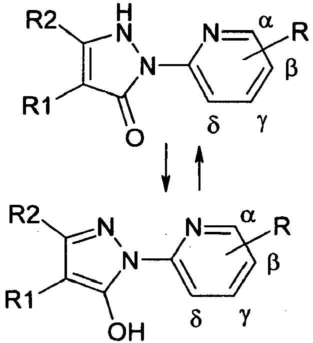

De plus, la demande décrit un procédé de test homogène de mesure directe par complémentation beta galactosidase de la quantité de la protéine HIF1-alpha dans le noyau des cellules, de préférence des cellules HEK, après traitement des dites cellules par un ou des composés à tester consistant à :
(a) Ensemencer, de préférence en plaques 384 puits, lesdites cellules dans un milieu de culture approprié, de préférence du sérum de veau foetal à 1% (abrégé SVF);
(b) Ajouter le ou les composés à tester à une concentration appropriée dans un solvant approprié aux cellules précédemment ensemencées dans ledit milieu de culture, de préférence les composés à tester sont dilués dans le SVF à 0,1%;
(c) Incuber lesdites cellules ainsi préparées dans un incubateur à environ 37°C, avantageusement pendant environ 6h;
(d) Lyser les cellules avec un tampon de lyse contenant un substrat chemiluminescent de la beta galactosidase ;
(e) Incuber à l'abri de la lumière avant lecture et mesure de la luminescence qui est fonction de l'activité de la beta galactosidase.

Les composés selon l'invention ont subi un test de screening selon le test tel que défini ci-dessus.

Dans le cadre de la présente invention, et sauf mention différente dans le texte, on entend par :
- un atome d'halogène : un atome de fluor, de chlore, de brome ou d'iode ;
- un groupe alkyle : un groupe aliphatique saturé, linéaire ou ramifié, pouvant comporter 1, 2, 3, 4 ou 5 atomes de carbone (abrégé par *-(C1-C5)alkyle).* A titre d'exemples, on peut citer comme (i) groupe *-C1alkyle,* le groupe méthyle, comme (ii) groupe *-C2alkyle,* le groupe éthyle, comme (iii) groupe *-C3alkyle,* le groupe propyle, isopropyle, comme (iv) groupe *-C4alkyle,* le groupe butyle, isobutyle, tertbutyle, comme (v) groupe *-C5alkyle* le groupe pentyle, isopentyle;
- un groupe alkylène : un groupe alkyle tel que précédemment défini, divalent saturé, linéaire ou ramifié, pouvant comporter 1, 2, 3, 4 ou 5 atomes de carbone (abrégé *-(C1-C5)alkylène-).* A titre d'exemple, on peut citer les radicaux méthylène (ou -CH₂-), éthylène (ou -CH₂-CH₂-), propylène (-CH₂-CH₂CH₂- ou -C(CH₃)₂-) ;
- un groupe cycloalkyle : un groupe alkyle cyclique pouvant comporter 3, 4, 5 ou 6 atomes de carbone, abrégé également *-(C3-C6)cycloalkyle.* A titre d'exemples, on peut citer les groupes cyclopropyle, cyclobutyle, cyclopentyle, cyclohexyle ;
- un groupe cycloalkylène : un groupe cycloalkyle, tel que précédemment défini, divalent saturé, pouvant comporter 3, 4, 5 ou 6 atomes de carbone et qui est alors abrégé *-(C3-C6)cycloalkylène-.* A titre d'exemple, on peut citer les radicaux - cyclopropylène-, -cyclobutylène-, -cyclopentylène-, -cyclohexylène- ;
- un groupe alcoxy : un radical *-O-alkyle* où le groupe alkyle est tel que précédemment défini. A titre d'exemples, on peut citer les groupes *-O-(C1-C5)alkyle* ou - *(C1-C5)alcoxy,* et en particulier comme (i) groupe *-O-C1alkyle,* le groupe -Ométhyle, comme (ii) groupe *-O-C2alkyle,* le groupe -Oéthyle, comme (iii) groupe *-O-C3alkyle,* le groupe -Opropyle, -Oisopropyle, comme (iv) groupe *-O-C4alkyle,* le groupe -Obutyle, - Oisobutyle, -Otertbutyle, comme (v) groupe *-O-C5alkyle* le groupe -Opentyle, - Oisopentyle, -Oneopentyle;
- un groupe alcoxy-alkyle : un radical de formule *-alkylène-O-alkyle,* où les groupes alkyle et alkylène, comprenant le même nombre de carbone ou ne comprenant pas le même nombre de carbone, sont tels que définis précédemment. A titre d'exmples, on peut citer les groupes *-(C1-C5)alkylène-O-(C1-C5)alkyle,* avec -(C1-C5)alkylène- et - (C1-C5)alkyle tels que définis ci-dessus ;
- un groupe alcoxy-alcoxy : un radical de formule *-O-alkylène-O-alkyle,* où les groupes alkylènes et alkyles, comprenant le même nombre de carbone ou ne comprenant pas le même nombre de carbone, sont tels que définis précédemment ;
- un groupe halogénoalkyle : un groupe alkyle tel que défini ci-dessus substitué par 1, 2, 3, 4 ou 5 atomes d'halogène, tels que définis précédemment. On citera par exemple les groupes *-halogéno(C1-C5)alkyle,* avec (C1-C5)alkyle tel que défini ci-dessus, comme par exemple le groupe trifluorométhyle (abrégé -CF₃), le groupe -CH₂-CF₃;
- un groupe aryle : un groupe aromatique cyclique comprenant 5 ou 6 atomes de carbone. A titre d'exemples de groupes aryles, on peut citer le groupe phényle ;
- un groupe hétéroaryle : un groupe aromatique cyclique comprenant 2, 3, 4 ou 5 atomes de carbone et comprenant 1 à 3 hétéroatomes, qui peu(ven)t être choisi(s) parmi l'atome d'azote, l'atome d'oxygène et l'atome de soufre, indépendemment l'un de l'autre, de manière à être identiques ou différents, lorsqu'ils sont au nombre de 2 ou indépendemment les uns des autres, de manière à être identiques ou différents, lorsqu'ils sont au nombre de 3. On peut citer les groupes pyridyle, pyrrole, furanyle ;
- un hétérocycloalkyle : un groupe alkyle cyclique éventuellement ponté comprenant 4, 5, 6 ou 7 atomes de carbone et comprenant 1, 2 ou 3 hétéroatomes choisi(s) parmi l'oxygène, l'azote et le soufre. On peut notamment citer les groupes pipéridinyle, pipérazinyle, pyrrolidinyle, hexaméthylèneimino, morpholinyle, 1,1-dioxydotetrahydrothiényle;
- les lettres α, β, γ et δ autour de la pyridine des composés de formule (I) permettent d'identifier les positions des différents atomes de carbone.

Parmi les composés décrits dans la présente invention, on peut citer un premier groupe de composés répondant à la formule (I) dans laquelle :
**R** représente un groupe -SO₂-NR3R4, ou un groupe -SO₂-R4 ; **R3, R4** et **R5** étant tels que définis ci-dessous ;
Et/ou
**R1** représente un groupe hétérocycloalkyle ne contenant pas d'atome d'azote, un groupe -W-(C3-C6)cycloalkyle, un groupe -W-aryle, un groupe -W-hétéroaryle, un groupe -W-hétérocycloalkyle, un groupe -W-COOR5 ou un groupe -W-CONR5R6,
   (i) lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes -(C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkylène-O-(C1-C5)alkyle, les groupes -O-(C1-C5)alkylène-NR5R6, les groupes -SO₂-(C1-C5)alkyle, les groupes -NR5R6 et les groupes - CO₂R5, et
   (ii) étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
   et/ou
**R2** représente un atome d'hydrogène, un groupe -(C1-C5)alkyle, un groupe -(C1-C5)alkylène-O-(C1-C5)alkyle, un groupe -halogéno(C1-C5)alkyle, un groupe -W-COOR5, un groupe -W-C(O)NHR5 ou un groupe -W-C(O)-NR5R6 ; **W, R5** et **R6** étant tels que définis ci-dessous;
   et/ou
n représente 0, 1 ou 2 ;
   et/ou
**W** est
   (i) un groupe -(C1-C5)alkylène-, éventuellement substitué par un groupe choisi parmi les groupes -(CH₂)n-CO₂R5 et les groupes -(CH₂)n-(CO)NR5R6, avec n tel que défini ci-dessus et **R5** et **R6** tels que définis ci-dessous ;ou
   (ii) un groupe -(C3-C6)cycloalkylène-,
   et/ou
**R3** et **R4**
   (i) Identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe -W-CHOH-CH₂OH, un groupe -W-CO₂R5, un groupe -W-NR5R6 ou un groupe -W-O-(CH₂)n-aryle;
      lesdits groupes -(C3-C6)cycloalkyle et hétérocycloalkyle étant éventuellement substitués
      o sur au moins un atome de carbone par au moins un groupe choisi parmi -(C1-C5)alkyle, un groupe -(C1-C5)alcoxy, une fonction hydroxy, un groupe -W-NR5R6 et un groupe -W-CO₂R5, dans le cas des groupes -(C3-C6)cycloalkyle et hétérocycloalkyle eUou
      o sur au moins un hétéroatome choisi parmi l'azote par au moins un groupe choisi parmi -(C1-C5)alkyle, dans le cas d'un groupe hétérocycloalkyle,
      avec **W** et **n** tels que définis précédemment et **R5** et **R6** tels que définis ci-dessous et étant entendu que lorsque **R3** et **R4** sont identiques, ils ne peuvent pas être un atome d'hydrogène;
   (ii) ou bien **R3** et **R4** forment ensemble avec l'atome d'azote qui les porte un groupe hétérocycloalkyle, éventuellement substitué sur au moins un atome de carbone et/ou, le cas échéant, sur au moins un hétéroatome, par au moins un substituant choisi parmi les groupes -(C1-C5)alkyle et les groupes -CH₂-aryle ;
   et/ou
**R5** et **R6**, identiques ou différents, représentent, indépendamment l'un de l'autre, un atome d'hydrogène, un groupe -(C1-C5)alkyle ou un groupe (C1-C5) halogénoalkyle; à l'exclusion des composés déjà mentionnés ci-dessus en tant que tels.

Un deuxième sous-groupe de composés de l'invention est constitué par les composés de formule (I) dans laquelle **R** représente un groupe -SO₂-NR3R4 avec **R3, R5** et **R4** tel que définis précédemment.

Un troisième sous-groupe de composés de l'invention est constitué par les composés de formule (I) dans laquelle **R** représente un groupe -SO₂-R4 avec **R4** et **R5** tels que définis ci-dessus.

Un quatrième sous-groupe de composés de l'invention est constitué par les composés de formule (I) dans laquelle R est un substituant de l'atome situé en position β de la pyridine.

Un cinquième sous-groupe de composés de l'invention est constitué par les composés de formule (I) dans laquelle **R1** représente un groupe hétérocycloalkyle ne contenant pas d'atome d'azote, un groupe -W-(C3-C6)cycloalkyle, un groupe -W-aryle, un groupe -W-hétéroaryle, un groupe -W-hétérocycloalkyle, un groupe -W-COOR5 ou un groupe -W-CONR5R6,
lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes -(C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkylène-O-(C1-C5)alkyle, les groupes -O-(C1-C5)alkylène-NR5R6, les groupes -SO₂-(C1-C5)alkyle, les groupes - NR5R6 et les groupes - CO₂R5, et
étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
où **W** est un groupe (C1-C5)alkylène ou un groupe (C3-C6)cycloalkylène et où **R5** et **R6**, identiques ou différents, représentent, indépendamment l'un de l'autre, un atome d'hydrogène, un groupe -(C1-C5)alkyle ou un groupe (C1-C5) halogénoalkyle. Avantageusement, le groupe hétérocycloalkyle représente un groupe pipéridinyle, le groupe aryle représente un groupe phényle et le groupe hétéroaryle représente un groupe pyridine.

Un sixième sous-groupe de composés de l'invention est constitué par les composés de formule (I) dans laquelle **R2** représente un atome d'hydrogène, un groupe -(C1-C5)alkyle, un groupe -(C1-C5)alkylène-O-(C1-C5)alkyle, un groupe -halogéno(C1-C5)alkyle, un groupe -W-COOR5, un groupe -W-C(O)NHR5 ou un groupe W-C(O)-NR5R6 ; où **W, R5** et **R6** sont tels que définis ci-dessus.

Un septième sous-groupe de composés de l'invention est constitué par les composés de formule (I) dans laquelle **R** représente un groupe -SO₂-NR3R4, avantageusement situé en position β de la pyridine, et dans lequelle **R3** et **R4**, identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe -W-CHOH-CH₂OH, un groupe -W-CO₂R5, un groupe -W-NR5R6 ou un groupe -W-O-(CH₂)n-aryle;
étant entendu que :
lorsque **R3** et **R4** sont identiques, ils ne peuvent pas être un atome d'hydrogène;
   et que
lorsque **R3** et/ou **R4** sont choisis parmi lesdits groupes -(C3-C6)cycloalkyle et hétérocycloalkyle, ces derniers peuvent éventuellement être substitués
   o sur au moins un atome de carbone, par au moins un groupe choisi parmi -(C1-C5)alkyle, un groupe -(C1-C5)alcoxy, une fonction hydroxy, un groupe -W-NR5R6 et un groupe -W-CO₂R5 dans le cas des groupes -(C3-C6)cycloalkyle et hétérocycloalkyle, et/ou
   o sur au moins un hétéroatome choisi parmi l'azote par au moins un groupe choisi parmi -(C1-C5)alkyle, dans le cas d'un groupe hétérocycloalkyle,
où **W, R5** et **R6** sont tels que définis ci-dessus et où n représente 0, 1 ou 2.

Avantageusement, le groupe hétérocycloalkyle représente un groupe pipéridinyle, le groupe aryle représente un groupe phényle et le groupe hétéroaryle représente un groupe pyridine.

Un huitième sous-groupe de composés de l'invention est constitué par les composés de formule (I) dans laquelle **R** représente un groupe -SO₂-NR3R4, avantageusement situé en position β de la pyridine, et dans lequelle **R3** et **R4** forment ensemble avec l'atome d'azote qui les porte un groupe hétérocycloalkyle, éventuellement substitué sur au moins un atome de carbone et/ou, le cas échéant, sur au moins un hétéroatome, par au moins un substituant choisi parmi les groupes -(C1-C5)alkyle et les groupes -CH₂-aryle. Avantageusement, ledit groupe hétérocycloalkyle représente un groupe choisi parmi un groupe pipéridinyle, pipérazinyle, morpholinyle, pyrrolidinyle, hexaméthylèneimino, et le groupe aryle représente un groupe phényle.

Un neuvième sous-groupe de composés de l'invention est constitué par les composés de formule (I) dans laquelle le ou le(s) groupe(s) **R**, **R1** et/ou **R2** comprend ou comprennent le ou les groupe(s) **R5** et/ou **R6**,
**R5** ou **R6** est un atome d'hydrogène, un groupe ₋(C1-C5)alkyle ou un groupe (C1-C5) halogénoalkyle, ou
**R5** et **R6**, identiques ou différents, représentent, indépendamment l'un de l'autre, un atome d'hydrogène, un groupe -(C1-C5)alkyle ou un groupe (C1-C5) halogénoalkyle. Avantageusement, R5 et/ou R6 sont choisis parmi les groupes (C1-C5)alkyle.

Un dixième sous-groupe de composés de l'invention est constitué par les composés de formule (I) dans laquelle **R** représente un groupe -SO₂-NR3R4 avec R3 et R4 tels que définis ci-dessus et/ou **R2** représente un atome d'hydrogène ou un groupe -(C1-C5)alkyle, avantageusement un méthyle et/ou **R1** représente un groupe -W-aryle ou un groupe -W-hétéroaryle, avec avantageusement ledit W représentant un -CH₂-, ledit aryle représentant un phényle et ledit hétéroaryle représentant une pyridine.

Les sous-groupes définis ci-dessus pris séparément ou en combinaison font également partie de l'invention.

Parmi les composés de formule (I) objets de l'invention, on peut notamment citer les composés suivants :
- 4-(2-chlorobenzyl)-5-methyl-2-[5-(trifluoromethyl)pyridin-2-yl]-1,2-dihydro-3H-pyrazol-3-one ;
- Acide 6-[4-(2-chlorobenzyl)-3-methyl-5-oxo-2,5-dihydro-1H-pyrazol-1-yl]pyridine-3-carboxylique ;
- 4-(2-chlorobenzyl)-5-methyl-2-(pyridin-2-yl)-1,2-dihydro-3H-pyrazol-3-one ;
- Trifluoroacétate de 4-(2-chlorobenzyl)-5-methyl-2-{5-[(4-methylpiperazin-1-yl)sulfonyl]pyridin-2-yl}-1,2-dihydro-3H-pyrazol-3-one ;
- (+/-)4-(2-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
- 6-[4-(2-chlorobenzyl)-3-methyl-5-oxo-2,5-dihydro-1H-pyrazol-1-yl]-N,N-diethylpyridine-3-sulfonamide ;
- 6-[4-(2-chlorobenzyl)-3-methyl-5-oxo-2,5-dihydro-1H-pyrazol-1-yl]-N-ethyl-N-phenylpyridine-3-sulfonamide ;
- 6-[4-(2-chlorobenzyl)-3-methyl-5-oxo-2,5-dihydro-1H-pyrazol-1-yl]-N,N-di(propan-2-yl)pyridine-3-sulfonamide ;
- 4-(2-chlorobenzyl)-5-methyl-2-[5-(piperidin-1-ylsulfonyl)pyridin-2-yl]-1,2-dihydro-3H-pyrazol-3-one ;
- N,N-diethyl-6-[4-(2-fluorobenzyl)-3-methyl-5-oxo-2,5-dihydro-1H-pyrazol-1-yl]pyridine-3-sulfonamide ;
- 4-(2-fluorobenzyl)-5-methyl-2-(pyridin-2-yl)-1,2-dihydro-3H-pyrazol-3-one ;
- 4-(2-fluorobenzyl)-5-methyl-2-[5-(trifluoromethyl)pyridin-2-yl]-1,2-dihydro-3H-pyrazol-3-one ;
- Trifluoroacétate de 4-(2-fluorobenzyl)-5-methyl-2-{5-[(4-methylpiperazin-1-yl)sulfonyl]pyridin-2-yl}-1,2-dihydro-3H-pyrazol-3-one ;
- 2-(5-{[(3R,5S)-3,5-dimethylpiperidin-1-yl]sulfonyl}pyridin-2-yl)-4-(2-fluorobenzyl)-5-methyl-1,2-dihydro-3H-pyrazol-3-one ;
- N-ethyl-6-[4-(2-fluorobenzyl)-3-methyl-5-oxo-2,5-dihydro-1H-pyrazol-1-yl]-N-phenylpyridine-3-sulfonamide ;
- 4-(2-fluorobenzyl)-5-methyl-2-[5-(piperidin-1-ylsulfonyl)pyridin-2-yl]-1,2-dihydro-3H-pyrazol-3-one ;
- 4-(2,4-dichlorobenzyl)-5-methyl-2-(pyridin-2-yl)-1,2-dihydro-3H-pyrazol-3-one ;
- 4-(2,4-dichlorobenzyl)-5-methyl-2-[5-(trifluoromethyl)pyridin-2-yl]-1,2-dihydro-3H-pyrazol-3-one ;
- Trifluoroacétate de 4-(2,4-dichlorobenzyl)-5-methyl-2-{5-[(4-methylpiperazin-1-yl)sulfonyl]pyridin-2-yl}-1,2-dihydro-3H-pyrazol-3-one ;
- 4-(2,4-dichlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
- 6-[4-(2,4-dichlorobenzyl)-3-methyl-5-oxo-2,5-dihydro-1H-pyrazol-1-yl]-N-ethyl-N-phenylpyridine-3-sulfonamide ;
- 6-[4-(2,4-dichlorobenzyl)-3-methyl-5-oxo-2,5-dihydro-1H-pyrazol-1-yl]-N,N-di(propan-2-yl)pyridine-3-sulfonamide ;
- 4-(2,4-dichlorobenzyl)-5-methyl-2-[5-(piperidin-1-ylsulfonyl)pyridin-2-yl]-1,2-dihydro-3H-pyrazol-3-one ;
- 4-(2-chloro-6-fluorobenzyl)-5-methyl-2-[5-(trifluoromethyl)pyridin-2-yl]-1,2-dihydro-3H-pyrazol-3-one ;
- Trifluoroacétate de 4-(2-chloro-6-fluorobenzyl)-5-methyl-2-{5-[(4-methylpiperazin-1-yl)sulfonyl]pyridin-2-yl}-1,2-dihydro-3H-pyrazol-3-one ;
- 4-(2-chloro-6-fluorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
- 6-[4-(2-chloro-6-fluorobenzyl)-3-methyl-5-oxo-2,5-dihydro-1H-pyrazol-1-yl]-N,N-diethylpyridine-3-sulfonamide ;
- 6-[4-(2-chloro-6-fluorobenzyl)-3-methyl-5-oxo-2,5-dihydro-1H-pyrazol-1-yl]-N-ethyl-N-phenylpyridine-3-sulfonamide ;
- 6-[4-(2-chloro-6-fluorobenzyl)-3-methyl-5-oxo-2,5-dihydro-1H-pyrazol-1-yl]-N,N-di(propan-2-yl)pyridine-3-sulfonamide ;
- 4-(2-chloro-6-fluorobenzyl)-5-methyl-2-[5-(piperidin-1-ylsulfonyl)pyridin-2-yl]-1,2-dihydro-3H-pyrazol-3-one ;
- methyl6-[4-(2-chlorobenzyl)-3-methyl-5-oxo-2,5-dihydro-1H-pyrazol-1-yl]pyridine-3-carboxylate ;
- 4-(4-chlorobenzyl)-5-methyl-2-(pyridin-2-yl)-1,2-dihydro-3H-pyrazol-3-one ;
- 4-(4-chlorobenzyl)-5-methyl-2-{5-[(4-methylpiperazin-1-yl)sulfonyl]pyridin-2-yl}-1,2-dihydro-3H-pyrazol-3-one ;
- 4-(4-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
- 6-[4-(4-chlorobenzyl)-3-methyl-5-oxo-2,5-dihydro-1H-pyrazol-1-yl]-N,N-diethylpyridine-3-sulfonamide ;
- 6-[4-(4-chlorobenzyl)-3-methyl-5-oxo-2,5-dihydro-1H-pyrazol-1-yl]-N-ethyl-N-phenylpyridine-3-sulfonamide ;
- 6-[4-(4-chlorobenzyl)-3-methyl-5-oxo-2,5-dihydro-1H-pyrazol-1-yl]-N,N-di(propan-2-yl)pyridine-3-sulfonamide ;
- 4-(4-chlorobenzyl)-5-methyl-2-[5-(piperidin-1-ylsulfonyl)pyridin-2-yl]-1,2-dihydro-3H-pyrazol-3-one ;
- 6-[4-(2-fluorobenzyl)-3-methyl-5-oxo-2,5-dihydro-1H-pyrazol-1-yl]-N,N-di(propan-2-yl)pyridine-3-sulfonamide ;
- 4-(1,1-dioxidotetrahydrothiophen-3-yl)-5-methyl-2-[5-(piperidin-1-ylsulfonyl)pyridin-2-yl]-1,2-dihydro-3H-pyrazol-3-one;
- 6-[4-(1,1-dioxidotetrahydrothiophen-3-yl)-3-methyl-5-oxo-2,5-dihydro-1H-pyrazol-1-yl]-N-ethyl-N-phenylpyridine-3-sulfonamide ;
- 6-[4-(1,1-dioxidotetrahydrothiophen-3-yl)-3-methyl-5-oxo-2,5-dihydro-1H-pyrazol-1-yl]-N,N-di(propan-2-yl)pyridine-3-sulfonamide ;
- 2-{[5-methyl-3-oxo-2-(pyridin-2-yl)-2,3-dihydro-1H-pyrazol-4-yl]methyl}benzonitrile ;
- 4-{[5-methyl-3-oxo-2-(pyridin-2-yl)-2,3-dihydro-1H-pyrazol-4-yl]methyl}benzonitrile ;
- N-ethyl-6-{4-[4-(methoxymethyl)benzyl]-3-methyl-5-oxo-2,5-dihydro-1H-pyrazol-1-yl}-N-phenylpyridine-3-sulfonamide ;
- 4-[3-(methoxymethyl)benzyl]-5-methyl-2-(pyridin-2-yl)-1,2-dihydro-3H-pyrazol-3-one ;
- N-ethyl-6-{4-[3-(methoxymethyl)benzyl]-3-methyl-5-oxo-2,5-dihydro-1H-pyrazol-1-yl}-N-phenylpyridine-3-sulfonamide ;
- 6-[4-(3-cyanobenzyl)-3-methyl-5-oxo-2,5-dihydro-1H-pyrazol-1-yl]-N-ethyl-N-phenylpyridine-3-sulfonamide ;
- 6-[4-(4-cyanobenzyl)-3-methyl-5-oxo-2,5-dihydro-1H-pyrazol-1-yl]-N-ethyl-N-phenylpyridine-3-sulfonamide ;
- 5-methyl-2-(pyridin-2-yl)-4-(pyridin-4-ylmethyl)-1,2-dihydro-3H-pyrazol-3-one ;
- 3-{[5-methyl-3-oxo-2-(pyridin-2-yl)-2,3-dihydro-1H-pyrazol-4-yl]methyl}benzonitrile ;
- 4-benzyl-2-(5-bromopyridin-2-yl)-5-methyl-1,2-dihydro-3H-pyrazol-3-one ;
- 6-[4-(2-cyanobenzyl)-3-methyl-5-oxo-2,5-dihydro-1H-pyrazol-1-yl]-N-ethyl-N-phenylpyridine-3-sulfonamide ;
- N-ethyl-6-{4-[3-(2-methoxyethoxy)benzyl]-3-methyl-5-oxo-2,5-dihydro-1H-pyrazol-1-yl}-N-phenylpyridine-3-sulfonamide ;
- 4-(3,5-dimethoxybenzyl)-5-methyl-2-(pyridin-2-yl)-1,2-dihydro-3H-pyrazol-3-one ;
- 5-methyl-4-[4-(methylsulfonyl)benzyl]-2-(pyridin-2-yl)-1,2-dihydro-3H-pyrazol-3-one ;
- N-ethyl-6-{3-methyl-4-[4-(methylsulfonyl)benzyl]-5-oxo-2,5-dihydro-1H-pyrazol-1-yl}-N-phenylpyridine-3-sulfonamide ;
- 4-[3-(2-methoxyethoxy)benzyl]-5-methyl-2-(pyridin-2-yl)-1,2-dihydro-3H-pyrazol-3-one ;
- 4-benzyl-5-methyl-2-(pyridin-2-yl)-1,2-dihydro-3H-pyrazol-3-one ;
- N-ethyl-6-[3-methyl-5-oxo-4-(pyridin-4-ylmethyl)-2,5-dihydro-1H-pyrazol-1-yl]-N-phenylpyridine-3-sulfonamide ;
- N-ethyl-6-[3-methyl-5-oxo-4-(pyridin-2-ylmethyl)-2,5-dihydro-1H-pyrazol-1-yl]-N-phenylpyridine-3-sulfonamide ;
- 5-methyl-2-(pyridin-2-yl)-4-(pyridin-3-ylmethyl)-1,2-dihydro-3H-pyrazol-3-one ;
- 6-(4-benzyl-3-methyl-5-oxo-2,5-dihydro-1H-pyrazol-1-yl)-N-ethyl-N-phenylpyridine-3-sulfonamide ;
- N-ethyl-6-[3-methyl-5-oxo-4-(pyridin-3-ylmethyl)-2,5-dihydro-1H-pyrazol-1-yl]-N-phenylpyridine-3-sulfonamide ;
- 4-(2,5-dimethoxybenzyl)-5-methyl-2-(pyridin-2-yl)-1,2-dihydro-3H-pyrazol-3-one ;
- 6-[4-(2,5-dimethoxybenzyl)-3-methyl-5-oxo-2,5-dihydro-1H-pyrazol-1-yl]-N-ethyl-N-phenylpyridine-3-sulfonamide ;
- N-ethyl-6-{3-methyl-4-[(1-methylpiperidin-4-yl)methyl]-5-oxo-2,5-dihydro-1H-pyrazol-1-yl}-N-phenylpyridine-3-sulfonamide ;
- N-ethyl-6-{3-methyl-5-oxo-4-[4-(trifluoromethyl)benzyl]-2,5-dihydro-1H-pyrazol-1-yl}-N-phenylpyridine-3-sulfonamide ;
- N-ethyl-6-{3-methyl-5-oxo-4-[3-(trifluoromethyl)benzyl]-2,5-dihydro-1H-pyrazol-1-yl}-N-phenylpyridine-3-sulfonamide ;
- 5-methyl-2-(pyridin-2-yl)-4-[3-(trifluoromethyl)benzyl]-1,2-dihydro-3H-pyrazol-3-one ;
- 5-methyl-2-(pyridin-2-yl)-4-[4-(trifluoromethyl)benzyl]-1,2-dihydro-3H-pyrazol-3-one ;
- 6-[4-(3,5-dimethoxybenzyl)-3-methyl-5-oxo-2,5-dihydro-1H-pyrazol-1-yl]-N-ethyl-N-phenylpyridine-3-sulfonamide ;
- 4-(4-hydroxybenzyl)-5-methyl-2-(pyridin-2-yl)-1,2-dihydro-3H-pyrazol-3-one ;
- N-ethyl-6-[4-(4-hydroxybenzyl)-3-methyl-5-oxo-2,5-dihydro-1H-pyrazol-1-yl]-N-phenylpyridine-3-sulfonamide ;
- 6-(4-{4-[2-(dimethylamino)ethoxy]benzyl}-3-methyl-5-oxo-2,5-dihydro-1H-pyrazol-1-yl)-N-ethyl-N-phenylpyridine-3-sulfonamide ;
- 6-{4-[4-(dimethylamino)benzyl]-3-methyl-5-oxo-2,5-dihydro-1H-pyrazol-1-yl}-N-ethyl-N-phenylpyridine-3-sulfonamide ;
- 5-methyl-2-(pyridin-2-yl)-4-[2-(trifluoromethyl)benzyl]-1,2-dihydro-3H-pyrazol-3-one ;
- 4-[4-(dimethylamino)benzyl]-5-methyl-2-(pyridin-2-yl)-1,2-dihydro-3H-pyrazol-3-one ;
- N-ethyl-6-{3-methyl-5-oxo-4-[2-(trifluoromethyl)benzyl]-2,5-dihydro-1H-pyrazol-1-yl}-N-phenylpyridine-3-sulfonamide ;
- 6-(4-benzyl-3-methyl-5-oxo-2,5-dihydro-1H-pyrazol-1-yl)-N-(3-methylbutyl)pyridine-3-sulfonamide ;
- 4-[3-(dimethylamino)benzyl]-5-methyl-2-(pyridin-2-yl)-1,2-dihydro-3H-pyrazol-3-one ;
- 6-{4-[3-(dimethylamino)benzyl]-3-methyl-5-oxo-2,5-dihydro-1H-pyrazol-1-yl}-N-ethyl-N-phenylpyridine-3-sulfonamide ;
- 4-(4-methoxybenzyl)-5-methyl-2-(pyridin-2-yl)-1,2-dihydro-3H-pyrazol-3-one ;
- N-ethyl-6-[4-(4-methoxybenzyl)-3-methyl-5-oxo-2,5-dihydro-1H-pyrazol-1-yl]-N-phenylpyridine-3-sulfonamide ;
- 4-(2-methoxybenzyl)-5-methyl-2-(pyridin-2-yl)-1,2-dihydro-3H-pyrazol-3-one ;
- 4-(3-methoxybenzyl)-5-methyl-2-(pyridin-2-yl)-1,2-dihydro-3H-pyrazol-3-one ;
- N-ethyl-6-[4-(3-methoxybenzyl)-3-methyl-5-oxo-2,5-dihydro-1H-pyrazol-1-yl]-N-phenylpyridine-3-sulfonamide ;
- 6-(4-{3-[2-(dimethylamino)ethoxy]benzyl}-3-methyl-5-oxo-2,5-dihydro-1H-pyrazol-1-yl)-N-ethyl-N-phenylpyridine-3-sulfonamide ;
- N-ethyl-6-[4-(2-methoxybenzyl)-3-methyl-5-oxo-2,5-dihydro-1H-pyrazol-1-yl]-N-phenylpyridine-3-sulfonamide ;
- 4-benzyl-5-methyl-2-[5-(morpholin-4-ylsulfonyl)pyridin-2-yl]-1,2-dihydro-3H-pyrazol-3-one ;
- N-ethyl-6-[4-(3-hydroxybenzyl)-3-methyl-5-oxo-2,5-dihydro-1H-pyrazol-1-yl]-N-phenylpyridine-3-sulfonamide ;
- 6-(4-benzyl-3-ethyl-5-oxo-2,5-dihydro-1H-pyrazol-1-yl)-N-ethyl-N-phenylpyridine-3-sulfonamide ;
- 4-(3-hydroxybenzyl)-5-methyl-2-(pyridin-2-yl)-1,2-dihydro-3H-pyrazol-3-one ;
- N-ethyl-6-{4-[4-(2-methoxyethoxy)benzyl]-3-methyl-5-oxo-2,5-dihydro-1H-pyrazol-1-yl}-N-phenylpyridine-3-sulfonamide ;
- 4-benzyl-5-ethyl-2-(pyridin-2-yl)-1,2-dihydro-3H-pyrazol-3-one ;
- 4-benzyl-5-methyl-2-[-(pyrrolidin-1-ylsulfonyl)pyridin-2-yl]-1,2-dihydro-3H-pyrazol-3-one ;
- 2-[5-(azepan-1-ylsulfonyl)pyridin-2-yl]-4-benzyl-5-methyl-1,2-dihydro-3H-pyrazol-3-one ;
- N,N-diethyl-6-[3-methyl-5-oxo-4-(pyridin-3-ylmethyl)-2,5-dihydro-1H-pyrazol-1-yl]pyridine-3-sulfonamide ;
- chlorhydrate de N,N-diethyl-6-[3-methyl-5-oxo-4-(pyridin-3-ylmethyl)-2,5-dihydro-1H-pyrazol-1-yl]pyridine-3-sulfonamide ;
- N,N-dimethyl-6-[3-methyl-5-oxo-4-(pyridin-3-ylmethyl)-2,5-dihydro-1H-pyrazol-1-yl]pyridine-3-sulfonamide ;
- Chlorhydrate de N,N-dimethyl-6-[3-methyl-5-oxo-4-(pyridin-3-ylmethyl)-2,5-dihydro-1H-pyrazol-1-yl]pyridine-3-sulfonamide ;
- Chlorhydrate de 6-(4-benzyl-5-oxo-3-propyl-2,5-dihydro-1H-pyrazol-1-yl)-N-ethyl-N-phenylpyridine-3-sulfonamide ;
- Chlorhydrate de 6-[3-methyl-5-oxo-4-(pyridin-3-ylmethyl)-2,5-dihydro-1H-pyrazol-1-yl]-N-(propan-2-yl)pyridine-3-sulfonamide ;
- Chlorhydrate de 5-methyl-4-(pyridin-3-ylmethyl)-2-[5-(pyrrolidin-1-ylsulfonyl)pyridin-2-yl]-1,2-dihydro-3H-pyrazol-3-one ;
- Chlorhydrate de N-tert-butyl-6-[3-methyl-5-oxo-4-(pyridin-3-ylmethyl)-2,5-dihydro-1H-pyrazol-1-yl]pyridine-3-sulfonamide ;
- Chlorhydrate de N-cyclopropyl-6-[3-methyl-5-oxo-4-(pyridin-3-ylmethyl)-2,5-dihydro-1H-pyrazol-1-yl]pyridine-3-sulfonamide ;
- Chlorhydrate de N-cyclopentyl-6-[3-methyl-5-oxo-4-(pyridin-3-ylmethyl)-2,5-dihydro-1H-pyrazol-1-yl]pyridine-3-sulfonamide ;
- N-ethyl-6-[3-methyl-5-oxo-4-(2-phenylethyl)-2,5-dihydro-1H-pyrazol-1-yl]-N-phenylpyridine-3-sulfonamide ;
- 5-methyl-4-(2-phenylethyl)-2-(pyridin-2-yl)-1,2-dihydro-3H-pyrazol-3-one ;
- Chlorhydrate de N-methyl-6-[3-methyl-5-oxo-4-(pyridin-3-ylmethyl)-2,5-dihydro-1H-pyrazol-1-yl]-N-(pyridin-2-yl)pyridine-3-sulfonamide ;
- 2-{5-[(4-benzylpiperidin-1-yl)sulfonyl]pyridin-2-yl}-5-methyl-4-(pyridin-3-ylmethyl)-1,2-dihydro-3H-pyrazol-3-one ;
- N-ethyl-6-[3-methyl-5-oxo-4-(2-phenylpropan-2-yl)-2,5-dihydro-1H-pyrazol-1-yl]-N-phenylpyridine-3-sulfonamide ;
- N-ethyl-6-{4-[(6-methoxypyridin-2-yl)methyl]-3-methyl-5-oxo-2,5-dihydro-1H-pyrazol-1-yl}-N-phenylpyridine-3-sulfonamide ;
- Chlorhydrate de 6-[3-methyl-5-oxo-4-(pyridin-3-ylmethyl)-2,5-dihydro-1H-pyrazol-1-yl]-N-(pyridin-2-yl)pyridine-3-sulfonamide ;
- N-ethyl-6-[3-methyl-5-oxo-4-(1-phenylcyclopropyl)-2,5-dihydro-1H-pyrazol-1-yl]-N-phenylpyridine-3-sulfonamide ;
- Chlorhydrate de N-ethyl-6-{4-[(3-methoxypyridin-2-yl)methyl]-3-methyl-5-oxo-2,5-dihydro-1H-pyrazol-1-yl}-N-phenylpyridine-3-sulfonamide ;
- 6-[4-benzyl-3-(methoxymethyl)-5-oxo-2,5-dihydro-1H-pyrazol-1-yl]-N-ethyl-N-phenylpyridine-3-sulfonamide ;
- 6-[4-benzyl-5-oxo-3-(trifluoromethyl)-2,5-dihydro-1H-pyrazol-1-yl]-N-ethyl-N-phenylpyridine-3-sulfonamide ;
- 1-[5-(azepan-1-ylsulfonyl)pyridin-2-yl]-3-methyl-4-(pyridin-3-ylmethyl)-1H-pyrazol-5-olate ;
- N-ethyl-6-{3-methyl-5-oxo-4-[2-(pyridin-2-yl)ethyl]-2,5-dihydro-1H-pyrazol-1-yl}-N-phenylpyridine-3-sulfonamide ;
- N-ethyl-6-{4-[(5-methoxypyridin-3-yl)methyl]-3-methyl-5-oxo-2,5-dihydro-1H-pyrazol-1-yl}-N-phenylpyridine-3-sulfonamide ;
- N-(2-methoxyethyl)-N-methyl-6-[3-methyl-5-oxo-4-(pyridin-3-ylmethyl)-2,5-dihydro-1H-pyrazol-1-yl]pyridine-3-sulfonamide;
- 6-[4-benzyl-3-(2-methylpropyl)-5-oxo-2,5-dihydro-1H-pyrazol-1-yl]-N-ethyl-N-phenylpyridine-3-sulfonamide ;
- N-ethyl-6-[3-methyl-5-oxo-4-(pyridin-3-ylmethyl)-2,5-dihydro-1H-pyrazol-1-yl]pyridine-3-sulfonamide ;
- 6-[3-methyl-5-oxo-4-(pyridin-3-ylmethyl)-2,5-dihydro-1H-pyrazol-1-yl]-N-phenylpyridine-3-sulfonamide ;
- N-ethyl-6-[3-methyl-5-oxo-4-(3-phenylpropyl)-2,5-dihydro-1H-pyrazol-1-yl]-N-phenylpyridine-3-sulfonamide ;
- 6-(4-benzyl-5-oxo-2,5-dihydro-1H-pyrazol-1-yl)-N-ethyl-N-phenylpyridine-3-sulfonamide ;
- N-cyclopropyl-N-methyl-6-[3-methyl-5-oxo-4-(pyridin-3-ylmethyl)-2,5-dihydro-1H-pyrazol-1-yl]pyridine-3-sulfonamide ;
- N-tert-butyl-6-{4-[(5-methoxypyridin-3-yl)methyl]-3-methyl-5-oxo-2,5-dihydro-1H-pyrazol-1-yl}pyridine-3-sulfonamide ;
- 6-{4-[(5-cyanopyridin-3-yl)methyl]-3-methyl-5-oxo-2,5-dihydro-1H-pyrazol-1-yl}-N-ethyl-N-phenylpyridine-3-sulfonamide ;
- tert-butyl 6-[5-oxo-4-(pyridin-3-ylmethyl)-2,5-dihydro-1H-pyrazol-1-yl]pyridine-3-carboxylate ;
- tert-butyl 6-[3-methyl-5-oxo-4-(pyridin-3-ylmethyl)-2,5-dihydro-1H-pyrazol-1-yl]pyridine-3-carboxylate ;
- N-tert-butyl-6-[5-oxo-4-(pyridin-3-ylmethyl)-2,5-dihydro-1H-pyrazol-1-yl]pyridine-3-sulfonamide ;
- chlorhydrate de N-tert-butyl-6-[5-oxo-4-(pyridin-3-ylmethyl)-2,5-dihydro-1H-pyrazol-1-yl]pyridine-3-sulfonamide ;
- N-ethyl-6-[5-oxo-4-(pyridin-3-ylmethyl)-2,5-dihydro-1H-pyrazol-1-yl]-N-phenylpyridine-3-sulfonamide ;
- 6-(4-benzyl-5-oxo-2,5-dihydro-1H-pyrazol-1-yl)-N-tert-butyl-N-methylpyridine-3-sulfonamide ;
- N-ethyl-6-{4-[(5-methoxypyridin-3-yl)methyl]-5-oxo-2,5-dihydro-1H-pyrazol-1-yl}-N-phenylpyridine-3-sulfonamide ;
- 5-methyl-2-[5-(phenylsulfonyl)pyridin-2-yl]-4-(pyridin-3-ylmethyl)-1,2-dihydro-3H-pyrazol-3-one ;
- N-tert-butyl-6-{4-[(5-methoxypyridin-3-yl)methyl]-3-methyl-5-oxo-2,5-dihydro-1H-pyrazol-1-yl}-N-methylpyridine-3-sulfonamide ;
- 6-{4-[(6-cyanopyridin-3-yl)methyl]-3-methyl-5-oxo-2,5-dihydro-1H-pyrazol-1-yl}-N-ethyl-N-phenylpyridine-3-sulfonamide ;
- Acide (2-{5-[ethyl(phenyl)sulfamoyl]pyridin-2-yl}-3-oxo-2,3-dihydro-1H-pyrazol-4-yl)acetique;
- 2-(2-{5-[ethyl(phenyl)sulfamoyl]pyridin-2-yl}-3-oxo-2,3-dihydro-1H-pyrazol-4-yl)-N,N-dimethylacetamide ;
- methyl (2-{5-[ethyl(phenyl)sulfamoyl]pyridin-2-yl}-3-oxo-2,3-dihydro-1H-pyrazol-4-yl)acetate ;
- ethyl (4-benzyl-1-{5-[ethyl(phenyl)sulfamoyl]pyridin-2-yl}-5-oxo-2,5-dihydro-1H-pyrazol-3-yl)acetate ;
- 2-(2-{5-[ethyl(phenyl)sulfamoyl]pyridin-2-yl}-3-oxo-2,3-dihydro-1H-pyrazol-4-yl)-N-methylacetamide ;
- N-tert-butyl-6-{4-[(5-methoxypyridin-2-yl)methyl]-3-methyl-5-oxo-2,5-dihydro-1H-pyrazol-1-yl}-N-methylpyridine-3-sulfonamide ;
- 2-(4-benzyl-1-{5-[ethyl(phenyl)sulfamoyl]pyridin-2-yl}-5-oxo-2,5-dihydro-1H-pyrazol-3-yl)-N,N-dimethylacetamide ;
- Acide 3-(2-{5-[ethyl(phenyl)sulfamoyl]pyridin-2-yl}-3-oxo-2,3-dihydro-1H-pyrazol-4-yl)propanoique ;
- methyl 3-[(2-{5-[ethyl(phenyl)sulfamoyl]pyridin-2-yl}-5-methyl-3-oxo-2,3-dihydro-1H-pyrazol-4-yl)methyl]benzoate ;
- methyl 3-(2-{5-[ethyl(phenyl)sulfamoyl]pyridin-2-yl}-3-oxo-2,3-dihydro-1H-pyrazol-4-yl)propanoate ;
- methyl {2-[5-(tert-butylsulfamoyl)pyridin-2-yl]-3-oxo-2,3-dihydro-1H-pyrazol-4-yl}(phenyl)acetate ;
- methyl 2-[(2-{5-[ethyl(phenyl)sulfamoyl]pyridin-2-yl}-5-methyl-3-oxo-2,3-dihydro-1H-pyrazol-4-yl)methyl]benzoate ;
- N-cyclopentyl-N-ethyl-6-[5-oxo-4-(pyridin-3-ylmethyl)-2,5-dihydro-1H-pyrazol-1-yl]pyridine-3-sulfonamide ;
- N-cyclopentyl-N-methyl-6-[5-oxo-4-(pyridin-3-ylmethyl)-2,5-dihydro-1H-pyrazol-1-yl]pyridine-3-sulfonamide ;
- 2-[(1-{5-[ethyl(phenyl)sulfamoyl]pyridin-2-yl}-3-methyl-5-oxido-1H-pyrazol-4-yl)methyl]benzoate ;
- 2-[5-(azepan-1-ylsulfonyl)pyridin-2-yl]-4-(pyridin-3-ylmethyl)-1,2-dihydro-3H-pyrazol-3-one ;
- N-cyclopentyl-N-methyl-6-[5-oxo-4-(pyridin-4-ylmethyl)-2,5-dihydro-1H-pyrazol-1-yl]pyridine-3-sulfonamide ;
- N-cyclopentyl-N-ethyl-6-[5-oxo-4-(pyridin-4-ylmethyl)-2,5-dihydro-1H-pyrazol-1-yl]pyridine-3-sulfonamide ;
- methyl 3-(2-{5-[cyclopentyl(methyl)sulfamoyl]pyridin-2-yl}-3-oxo-2,3-dihydro-1H-pyrazol-4-yl)-3-phenylpropanoate ;
- N-(2,2-dimethylpropyl)-N-methyl-6-[5-oxo-4-(pyridin-3-yl-methyl)-2,5-dihydro-1H-pyrazol-1-yl]pyridine-3-sulfonamide ;
- 2,2-dimethylpropyl 6-[5-oxo-4-(pyridin-3-ylmethyl)-2,5-dihydro-1H-pyrazol-1-yl]pyridine-3-carboxylate ;
- methyl N-cyclopentyl-N-({6-[5-oxo-4-(pyridin-3-ylmethyl)-2,5-dihydro-1H-pyrazol-1-yl]pyridin-3-yl}sulfonyl)glycinate ;
- 6-(4-benzyl-5-oxo-2,5-dihydro-1H-pyrazol-1-yl)-N-[2-(benzyloxy)ethyl]-N-cyclopentylpyridine-3-sulfonamide ;
- 6-(4-benzyl-5-oxo-2,5-dihydro-1H-pyrazol-1-yl)-N-[3-(benzyloxy)propyl]-N-cyclopentylpyridine-3-sulfonamide ;
- 6-(4-benzyl-5-oxo-2,5-dihydro-1H-pyrazol-1-yl)-N-cyclopentyl-N-(3-hydroxypropyl)pyridine-3-sulfonamide ;
- 6-(4-benzyl-5-oxo-2,5-dihydro-1H-pyrazol-1-yl)-N-cyclopentyl-N-(2-hydroxyethyl)pyridine-3-sulfonamide ;
- methyl (1S,2R)-2-[methyl({6-[5-oxo-4-(pyridin-3-ylmethyl)-2,5-dihydro-1H-pyrazol-1-yl]pyridin-3-yl}sulfonyl)amino]cyclopentanecarboxylate ;
- 6-(4-benzyl-5-oxo-2,5-dihydro-1H-pyrazol-1-yl)-N-cyclopentyl-N-(2,3-dihydroxypropyl)pyridine-3-sulfonamide ;
- 6-(4-benzyl-5-oxo-2,5-dihydro-1H-pyrazol-1-yl)-N-cyclopentyl-N-[2-(dimethylamino)ethyl]pyridine-3-sulfonamide ;
- 4-benzyl-2-{5-[(4-methylpiperazin-1-yl)sulfonyl]pyridin-2-yl}-1,2-dihydro-3H-pyrazol-3-one ;
- 6-[4-(cyclopentylmethyl)-5-oxo-2,5-dihydro-1H-pyrazol-1-yl]-N-ethyl-N-phenylpyridine-3-sulfonamide ;
- 4-benzyl-2-{5-[(4-methyl-1,4-diazepan-1-yl)sulfonyl]pyridin-2-yl}-1,2-dihydro-3H-pyrazol-3-one ;
- N-(2,2-dimethylpropyl)-6-[5-oxo-4-(pyridin-3-ylmethyl)-2,5-dihydro-1H-pyrazol-1-yl]pyridine-3-sulfonamide ;
- N-(2-methylbutan-2-yl)-6-[5-oxo-4-(pyridin-3-ylmethyl)-2,5-dihydro-1H-pyrazol-1-yl]pyridine-3-sulfonamide ;
- 6-(4-benzyl-5-oxo-2,5-dihydro-1H-pyrazol-1-yl)-N-[(1R,3S)-3-(hydroxymethyl)cyclopentyl]-N-methylpyridine-3-sulfonamide ;
- 6-(4-benzyl-5-oxo-2,5-dihydro-1H-pyrazol-1-yl)-N-methyl-N-(1-methylpyrrolidin-3-yl)pyridine-3-sulfonamide ;
- 6-(4-benzyl-5-oxo-2,5-dihydro-1H-pyrazol-1-yl)-N-(2-methoxy-2-methylpropyl)pyridine-3-sulfonamide ;
- 2,2-dimethylpropyl 6-(4-benzyl-5-oxo-2,5-dihydro-1H-pyrazol-1-yl)pyridine-3-carboxylate ;
- 6-(4-benzyl-5-oxo-2,5-dihydro-1H-pyrazol-1-yl)-N-cyclopentyl-N-[(2R)-2,3-dihydroxypropyl]pyridine-3-sulfonamide ;
- 6-(4-benzyl-5-oxo-2,5-dihydro-1H-pyrazol-1-yl)-N-(2,3-dihydroxypropyl)-N-phenylpyridine-3-sulfonamide ;
- N-cyclopentyl-6-{4-[(4-methoxypyridin-3-yl)methyl]-5-oxo-2,5-dihydro-1H-pyrazol-1-yl}-N-methylpyridine-3-sulfonamide ;
- N-methyl-N-(2-methylbutan-2-yl)-6-[5-oxo-4-(pyridin-3-ylmethyl)-2,5-dihydro-1H-pyrazol-1-yl]pyridine-3-sulfonamide ;
- 3-(2-{5-[cyclopentyl(methyl)sulfamoyl]pyridin-2-yl}-3-oxo-2,3-dihydro-1H-pyrazol-4-yl)-3-phenyl-N-(2,2,2-trifluoroethyl)propanamide ;
- 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide ;
- 6-(4-benzyl-5-oxo-2,5-dihydro-1H-pyrazol-1-yl)-N-cyclopentyl-N[(2S)-2,3-dihydroxypropyl]pyridine-3-sulfonamide ;
- 2,2-dimethylpropyl6-[5-oxo-4-(pyridin-4-ylmethyl)-2,5-dihydro-1H-pyrazol-1-yl]pyridine-3-carboxylate ;
- 6-(4-benzyl-5-oxo-2,5-dihydro-1H-pyrazol-1-y1)-N-[(1S,2S)-2-hydroxycyclopentyl]pyridine-3-sulfonamide ;
- N-tert-butyl-6-[5-oxo-4-(pyridin-4-ylmethyl)-2,5-dihydro-1H-pyrazol-1-yl]pyridine-3-sulfonamide ;
- 4-benzyl-2-(5-{[(3R)-3-(dimethylamino)pyrrolidin-1-yl]sulfonyl}pyridin-2-yl)-1,2-dihydro-3H-pyrazol-3-one ;
- 6-(4-benzyl-5-oxo-2,5-dihydro-1 H-pyrazol-1-yl)-N-[1-(dimethylamino)-2-methylpropan-2-yl]pyridine-3-sulfonamide ;
- 6-(4-benzyl-5-oxo-2,5-dihydro-1H-pyrazol-1-yl)-N-methyl-N-phenylpyridine-3-sulfonamide ;
- 3-{2-[5-(tert-butylsulfamoyl)pyridin-2-yl]-3-oxo-2,3-dihydro-1H-pyrazol-4-yl}-3-(pyridin-3-yl)propanoate de méthyle ;
- 3-{2-[5-(tert-butylsulfamoyl)pyridin-2-yl]-3-oxo-2,3-dihydro-1H-pyrazol-4-yl}-3-phenylpropanoate d'éthyle ;
- 6-(4-benzyl-5-oxo-2,5-dihydro-1H-pyrazol-1-yl)-N-tert-butyl-N-(2,3-dihydroxypropyl)pyridine-3-sulfonamide ;
- 6-(4-benzyl-5-oxo-2,5-dihydro-1H-pyrazol-1-yl)-N-(2-hydroxyethyl)pyridine-3-sulfonamide ;
- Chlorhydrate de N-(2,3-dihydroxypropyl)-6-[5-oxo-4-(pyridin-3-ylmethyl)-2,5-dihydro-1H-pyrazol-1-yl]-N-phenylpyridine-3-sulfonamide ;
- Chlorhydrate de 3-(2-{5-[cyclopentyl(methyl)sulfamoyl]pyridin-2-yl}-3-oxo-2,3-dihydro-1H-pyrazol-4-yl)-3-(pyridin-3-yl) propanoate de méthyle ;
- Chlorhydrate de 6-(4-benzyl-5-oxo-2,5-dihydro-1H-pyrazol-1-yl)-N-cyclopentyl-N-[3-(dimethylamino)propyl]pyridine-3-sulfonamide;
- Chlorhydrate de 6-(4-benzyl-5-oxo-2,5-dihydro-1H-pyrazol-1-yl)-N-cyclopentyl-N-[3-(diméthylamino)propyl]pyridine-3-sulfonamide.

Dans ce qui suit, on entend par groupe protecteur (Pg) un groupe qui permet, d'une part,de protéger une fonction réactive telle qu'un alcool ou une amine pendant une synthèse.Des exemples de groupes protecteurs ainsi que des méthodes de protection et dedéprotection sont données dans « Protective Groups in Organic Synthesis », Greenet al., 3rd Edition (John Wiley & Sons, Inc., New York).

On entend par groupe partant (Lg), dans ce qui suit, un groupement nucléofuge pouvantêtre facilement clivé d'une molécule par rupture d'une liaison hétérolytique, avec départ d'une paire électronique. Ce groupement peut ainsi être remplacé facilement par un groupement nucléophile lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon les procédés ci-après.

Le schéma 1 décrit la synthèse des composés de formule (I) pour laquelle **R** peut être un groupe -SO₂-NR3R4, ou un groupe -SO₂-R4 ; **R1** peut être un groupe hétérocycloalkyle ne contenant pas d'atome d'azote, un groupe -W-(C3-C6)cycloalkyle, un groupe -W-aryle, un groupe W-hétéroaryle, un groupe -W-hétérocycloalkyle, un groupe -W-COOR5 ou un groupe -W-CONR5R6, avec **R3, R4** et **R5** tels que définis plus haut; **W** peut être un groupe (C1-C5)alkylène, et n peut être 0, 1 ou 2.

Ces composés sont appelés ci-après composés de formule (Ia).

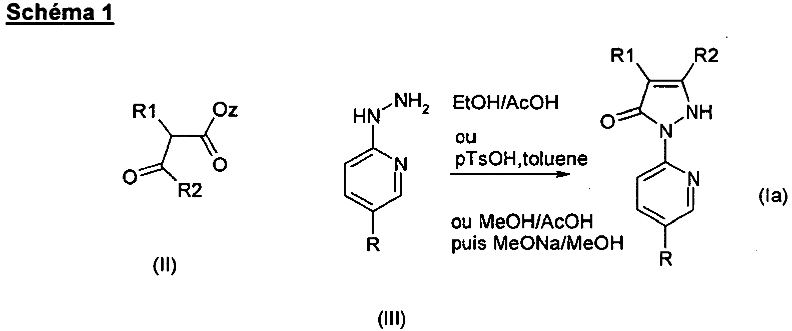

Dans le schéma 1, des composés de formule (II) pour laquelle R1 et R2 sont tels que définis pour les composés de formule (la) et z représente un groupe alkyle tel qu'un groupe méthyle ou éthyle, réagissent avec les composés de formule (III) pour aboutir aux composés de formule (la) de préférence dans un solvant protique tel qu'un mélange éthanol -acide acétique à une température de 80°C ou dans un solvant aprotique tel que le toluène, à une température comprise entre 80 et 110°C, en présence de quantités catalytiques d'acide organique tel que l'acide paratoluène sulfonique. Alternativement, ils peuvent être obtenus de manière séquentielle par réaction dans un mélange méthanol - acide acétique pour obtenir l'hydrazone intermédiaire suivie d'une réaction de cyclisation dans du méthanol en présence de méthylate de sodium de préférence à une température de 40°C.

Les composés (I) obtenus sont optionnellement convertis avec un acide ou une base en leur sels correspondants.

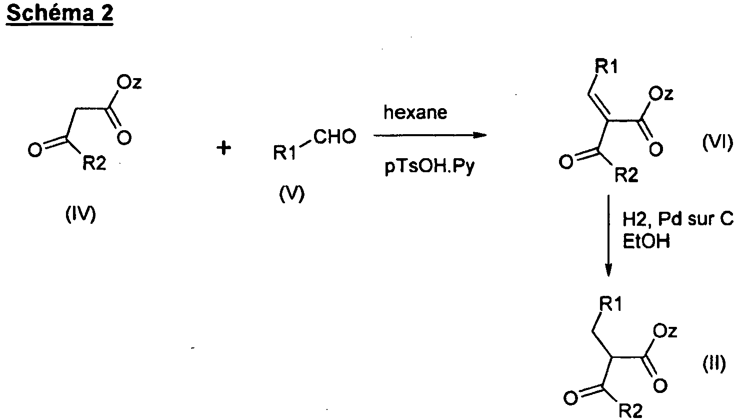

Les composés de formule (II) peuvent être obtenus selon le schéma 2 par une réaction de type Knoevenagel entre un betacétoester de formule (IV) R2COCH₂CO₂z et un aldéhyde de formule (V) R1CHO, pour lequel R1 est défini tel que précédemment, et R2 est un groupe (C1-C5)alkyle, -(C1-C5)alkyl-(C1-C5)alcoxy, halogéno(C1-C5)alkyle, un groupe W-COOR5, dans lequel W est tel que précedémment défini ; z représente un groupe alkyle tel qu'un groupe méthyle ou éthyle ,dans un solvant apolaire, de préférence du n-hexane, en présence de sel de pyridinium paratoluensulfonate en quantité catalytique pour conduire au composé de formule (VI), suivie d'une étape d'hydrogénation dans un solvant protique polaire, préférentiellement de l'éthanol en présence d'un catalyseur tel que du Palladium sur Charbon.

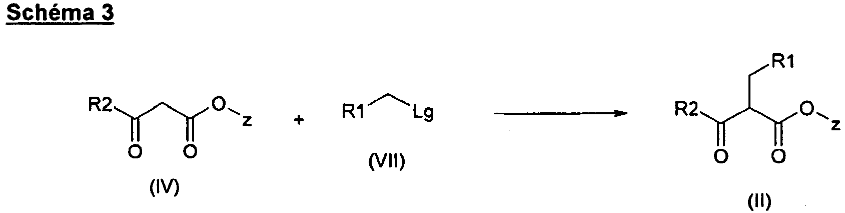

Alternativement les composés de formule (II) peuvent être obtenus selon le schéma 3 de manière séquentielle par déprotonation du betacétoester (IV) de formule R2COCH₂CO₂z par une base organique de type alcoolate, préférentiellement du méthylate de sodium dans un solvant alcoolique, ou une base inorganique telle que la potasse ou une base plus forte telle que l'hydrure de sodium, suivie de l'addition d'un électrophile de formule (VII) R1-CH₂-Lg, où R1 est tel que défini précédemment et R2 est un groupe (C1-C5)alkyle, -(C1-C5)alkyl-(C1-C5)alcoxy, halogéno(C1-C5)alkyle, un groupe -W-COOR5, dans lequel W est tel que précédemment défini et z est un groupe alkyle tel qu'un groupe méthyle ou éthyle et Lg est tel que décrit précédemment.

La synthèse des composés de formule (II) pour laquelle R1 est de type W-Aryle ou W-Heteroaryle avec W représentant un groupe alkylène ramifié de formule -CH(alkyle)(alkyle) est décrite dans le schéma 4. Ces composés sont appelés ci-après composés de formule (IIa).

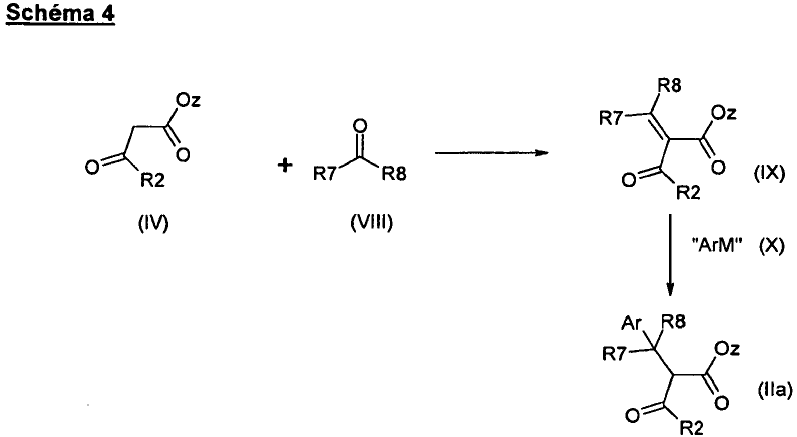

Les composés de formule (IX) sont obtenus par condensation d'une cétone de formule (VIII) R7COR8, avec R7 et R8 pouvant être indépendamment ou ensemble un groupe alkyle de C1 à C4, sur un betacétoester de formule (IV) R2-CH₂-CO₂z où R2 est défini tel que précédemment et z est un groupe alkyle tel qu'un groupe méthyle ou éthyle, en présence d'un acide de Lewis de préférence du chlorure de zinc dans un solvant tel que l'anhydride acétique à une température comprise entre 40°C et 80°C .Les composés de formule (IIa) sont ensuite obtenus par addition 1,4 d'un composé organométallique de formule (X) Aryle-Métal (« ArM »), de préférence un composé organomagnésien de type Aryle-MgX, avec X représentant un atome d'halogène tel qu'un atome de brome ou de chlore, en présence d'une quantité catalytique d'iodure de cuivre sur le composé de formule (IX) dans un solvant anhydre, de préférence de l'éther éthylique.

La synthèse des composés de formule (II) pour laquelle R2 est un hydrogène est décrite dans le schéma 5. Ces composés sont appelés ci-après composés de formule (IIb).

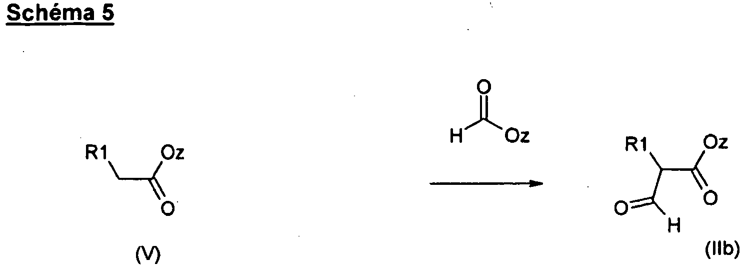

Les composés de formule (IIb) sont obtenus par formylation de l'ester (V) de formule R1-CH₂-CO₂z où R1 est défini tel que précédemment et z est un groupe alkyle tel qu'un groupe méthyle ou éthyle. L'étape de formylation consiste à faire réagir préférentiellement le formiate de méthyle ou d'éthyle sur l'ester (V) en présence, soit de sodium métallique dans un solvant aprotique anhydre tel que l'éther éthylique, à une température comprise entre 0°C et 30°C, soit en présence d'un acide de Lewis tel que le tétrachlorure de titane, d'une base organique comme la tributylamine en présence de quantités catalytiques de triméthylsilyltrifluorométhanesulfonate dans un solvant aprotique tel que le toluène à une température comprise entre 50 °C et 60°C.

Lorsqu'ils ne sont pas commerciaux, la synthèse des composés de formule (III) pour laquelle R représente un groupe halogénoalkyle ou -CO₂R5 avec R5 tel que décrit précédemment est décrite dans le schéma 6. Ces composés sont appelés ci-après composés de formule (IIIa).

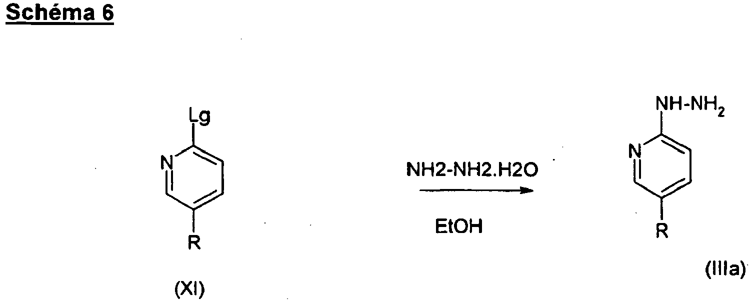

Les composés de formule (IIIa) sont obtenus à partir du composé XI, avec Lg et R tels que définis précédemment, par addition d'hydrazine hydrate, de préférence dans un solvant protique tel que l'EtOH à une température comprise entre 60 et 80°C.

La synthèse des composés de formule (III) pour laquelle R représente un groupe - SO₂NR3R4 et R3 et R4 sont tels que décrits précédemment est décrite dans le schéma 7. Ces composés sont appelés ci-après composés de formule (IIIb).

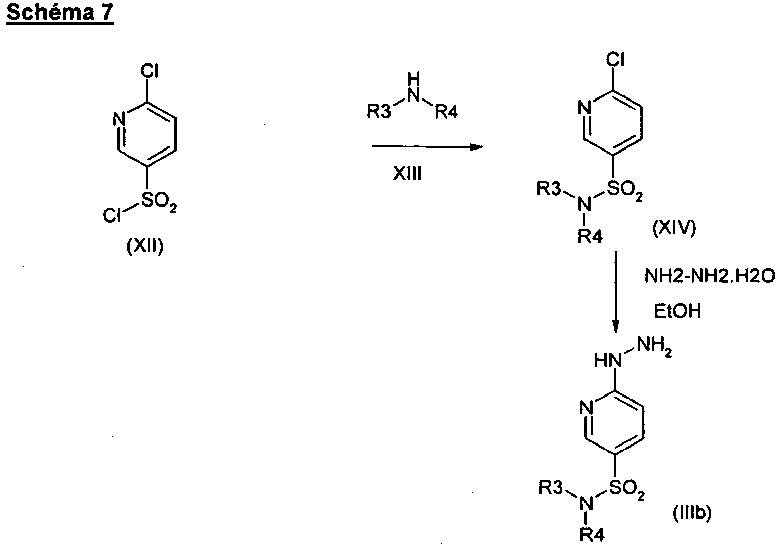

Les composés de formule (IIIb) sont obtenus à partir du chlorure de 2-chloro-5-sulfonylpyridine de formule (XII), par réaction sur une amine de formule (XIII) R3NHR4 avec R3 et R4 tels que définis précédemment, en présence d'une base organique, de préférence la triéthylamine, dans un solvant polaire, de préférence le dichlorométhane. Le composé obtenu de formule (XIV) est ensuite traité par de l'hydrazine hydrate dans un solvant protique tel que l'éthanol à 70°C pour aboutir aux composés désirés.

La synthèse des composés de formule (III) pour laquelle R est un groupe -alkyle, ou alcoxy est décrite dans le schéma 8. Ces composés sont appelés ci-après composés de formule (IIIc). Les composés de formule (IIIc) sont obtenus à partir du composé de formule (XV), avec Lg et R tels que définis précédemment. La fonction hydrazine est introduite par une réaction de couplage entre la benzophénone hydrazone de formule (XVI) et le composé de formule (XV) en présence d'une quantité catalytique de palladium pour aboutir à l'intermédiaire de formule (XVII), dont la fonction hydrazine est libérée par traitement acide tel que l'acide chlorhydrique, dans un mélange binaire de solvants non miscibles tels que le toluène et l'eau à une température de 100°C.

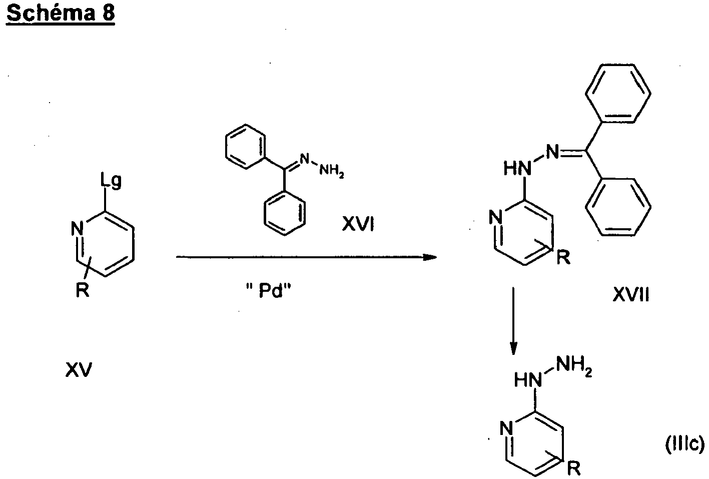

Le schéma 9 décrit la synthèse des composés (I) pour laquelle R1 est un groupe W-aryle, -W-hétéroaryle, lesdits groupes étant substitués par un ou plusieurs groupes - NR5R6 et où W est un groupe (C1-C5)alkylène. Ces composés sont appelés ci-après composés de formule (Ib).

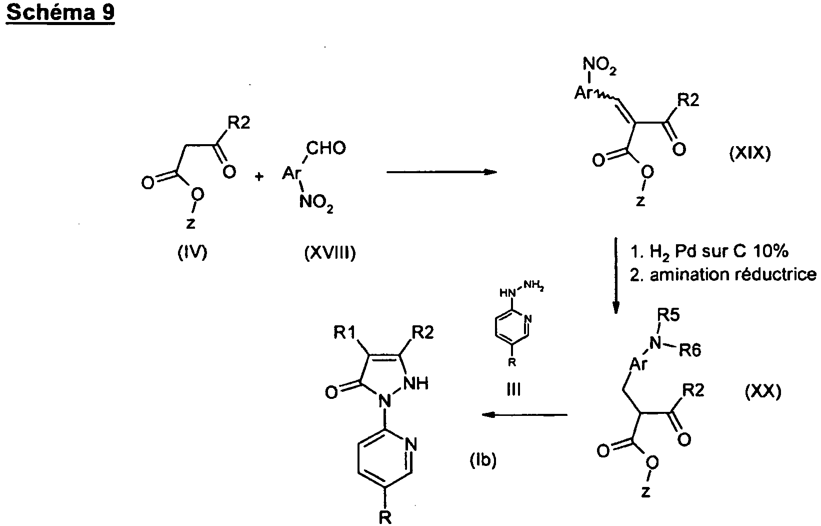

Le composé de formule (XIX) est obtenu par réaction d'un composé betacétoester de formule (IV) R2COCH₂CO₂z, R2 étant un groupe (C1-C5)alkyle, -(C1-C5)alkyl-(C1-C5)alcoxy, halogéno(C1-C5)alkyle et z représentant un groupe alkyle tel qu'un groupe méthyle ou éthyle, et d'un composé benzaldéhyde de formule (XVIII) substitué par un ou plusieurs groupes nitro. Le composé de formule (XX) est ensuite obtenu par hydrogénation totale en présence de catalyseur tel que le palladium sur charbon dans un solvant protique tel que l'éthanol, suivi préférentiellement d'une amination réductrice, dans des conditions telles que, lorsque R5 et R6 sont un groupe méthyle, de l'acide formique en présence d'un agent de réduction tel que le triacétoxyborohydrure de sodium dans un solvant protique tel que l'acide acétique. Le composé (XX) est ensuite mis en réaction comme décrit dans le schéma 1, avec le composé de formule (III) pour conduire aux composés désirés de formule (Ib).

Le schéma 10 décrit la synthèse des composés (I) pour laquelle R1 est un groupe -W-aryle, avec W est un groupe (C1-C5)alkylène; ledit groupe aryle étant substitué par un ou plusieurs groupes, -O-(C1-C5)alkyl-O-(C1-C5)alkyl-(C1-C5)alcoxy, -O-(C1-C5)alkyl-NR5R6. Ces composés sont appelés ci-après composés de formule (Ic).

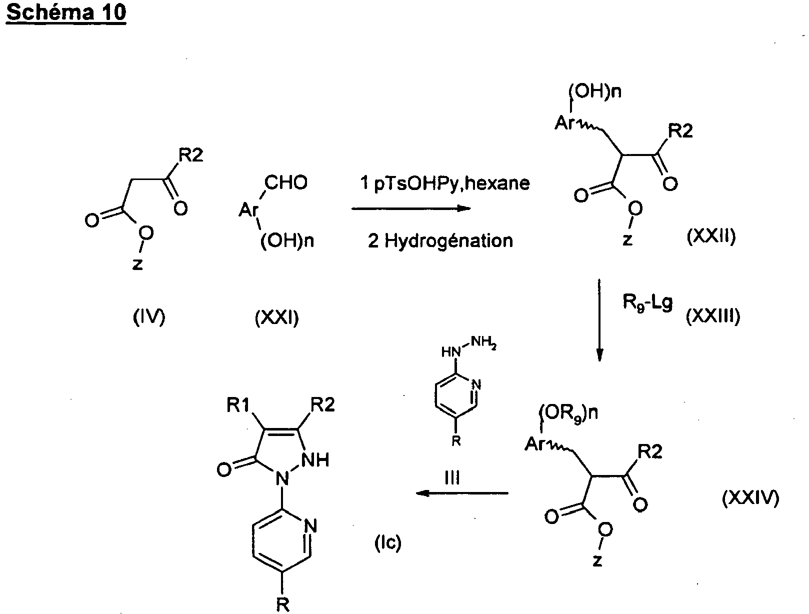

Le composé de formule (XXII) est obtenu par réaction d'un composé betacétoester de formule (IV) R2COCH₂CO₂z avec R2 est tel que décrit précédemment et z représente un groupe alkyle tel qu'un groupe méthyle ou éthyle et d'un composé benzaldéhyde de formule (XXI), suivie d'une étape d'hydrogénation totale en présence de palladium' sur charbon de préférence dans un solvant tel que l'éthanol. Le composé de formule (XXIV) est ensuite obtenu par une alkylation avec un composé de formule (XXIII) R₉-Lg, avec R₉ représentant un groupe (C1-C5)alkylène, -(C1-C5)alkyl-(C1-C5)alcoxy, -(C1-C5)alkyl-NR5R6 et Lg tel que défini précédemment, en présence d'une base préférentiellement inorganique telle que le carbonate de potassium et dans un solvant polaire tel que le DMF; puis il est mis en réaction comme décrit dans le schéma 1 avec le composé de formule (III) pour conduire aux composés désirés de formule (Ic).

Dans les exemples suivants, les composés de départ, les intermédiaires et les réactifs, quand leur mode de préparation n'est pas décrit sont disponibles dans le commerce ou décrits dans la littérature, ou bien peuvent être préparées selon des méthodes qui sont connues de l'homme de métier.

Les exemples suivants illustrent la préparation de certains composés conformes à l'invention. Les numéros des composés exemplifiés renvoient à ceux du tableau donné plus loin qui illustre les structures chimiques et les propriétés physiques de quelques composés selon l'invention.

Les abréviations et les formules suivantes sont utilisées :
- AcOEt: Acétate d'éthyle
- DCM: Dichlorométhane
- DMF: Diméthylformamide
- DMSO: Diméthyle sulfoxide
- DME: 1,2 diméthoxyéthane
- EtOH: Ethanol
- tBuOH: Tert-butanol
- Et₂O: Ether diéthylique
- MeOH: Méthanol
- iPrOH: Isopropanol
- AcOH: Acide acétique
- CH₃CN: Acétonitrile
- Et₂O: Diéthyléther
- THF: Tétrahydrofurane
- h: Heure(s)
- HCl: Acide chlorhydrique
- H₂SO₄: Acide sulfurique
- K₂CO₃: Carbonate de potassium
- KOH: Hydroxyde de potassium
- NH₄Cl: Chlorure d'ammonium
- NaHCO₃: Hydrogénocarbonate de sodium
- Na₂SO₄: Sulfate de sodium
- Cs₂CO₃: Carbonate de césium
- TEA: Triéthylamine
- TFA: Acide trifluoroacétique
- THF: Tétrahydrofurane
- T.A.: Temperature ambiante
- ZnCl₂: Chlorure de zinc
- PPTS: Pyridinium paratoluenesulfonique acide
- anh.: anhydre
- Pd -c: Palladium sur charbon
- Cu I: Iodure de cuivre
- MeCN: Acétonitrile
- NaI: Iodure de sodium
- DIEA: Diisopropyléthylamine
- TBTU: O-(Benzotriazol-1-yl)-N,N,N',N'-tetramethyluronium tétrafluoroborate
- DCC: N,N-Dicyclohexylacarbodiimide
- NMM: 4-méthylmorpholine
- NMO: 4-methylmorpholine N-oxide
- OsO4: Tétroxide d'osmium
- Pd(OAc)₂: Palladium acétate
- P(OTol)₃: Tri(o-tolyl)phosphine
- pTsOH: acide paratoluénesulfonique
- Tr: temps de rétention
- T: temps
- T°C: température en °C
- Min: minute
- PF: point de fusion

Les spectres de résonnance magnétique du proton (RMN1H), tels que décrits ci-dessous, sont enregistrés à 400MHz dans du DMSO-d6, en utilisant le pic du DMSO-d5 comme référence. Les déplacements chimiques δ sont exprimés en partie par million (ppm). Les signaux observés sont exprimés ainsi : s = singulet ; sl = singulet large; d = doublet ; dd = doublet de doublet ; dt = doublet de triplet ; t = triplet ; m = massif ; H = proton.

Les spectres de masse sont obtenus dans les conditions de couplage LC/MS suivantes :
**Méthode 1 :** Colonne Jsphere33x2 mm ; 4µM;
   Eluants : A = H2O +0.05%TFA ; B = CH₃CN +0.05 %TFA ;
   T0 :98%A ; T1,0 à T5,0min :95%B ;
**Méthode 2:** Colonne : Acquity BEH C18 (50x2,1 mm ; 1,7 µM) ;
   Eluants : A = H2O +0.05% TFA ; B = CH₃CN +0.035TFA ;
   T0 :98%A ; T1,6 à T2,1min :100%B ; T2,5 à T3min: 98%A ;Débit 1.0mL/min- T°C=40°C, injection 2 µL.
**Méthode 3:** Colonne Kromasil C18 (50x2,1mm ; 3,5µm) ;
   Eluants : A CH3CO₂NH4+ 3%CH₃CN ; B= CH₃CN ;
   T0 :100%A ; T5,5 à T7min : 100% B ;T7,1 à T10min :100% B ; Débit 0.8mL/min-T°=40°C - Injection 5µL.

### Exemple 1: N-éthyl-6-[3-méthyl-4-(1-methyl-1-phényléthyl)-5-oxo-2,5-dihydro-1H-pyrazol-1-yl]-N-phénylpyridine-3-sulfonamide (Composé n°77 du tableau I)

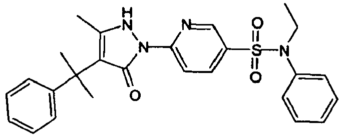

### 1.1. 2-acétyl-3-méthylbut-2-ènoate de méthyle

A un mélange de 9,54 g (70 mmoles) de ZnCl₂ anh., de 53,9 mL (500 mmoles) de méthylacétoacétate, et de 55 mL (750 mmoles) d'acétone, on ajoute 64 mL d'anhydride acétique. Le milieu réactionnel est ensuite chauffé 3 jours à 50°C, puis dilué avec 1 L de DCM et lavé avec de l'eau (3X100 mL). La phase organique est séchée sur Na₂SO₄, filtrée, puis concentrée sous pression réduite. Le résidu obtenu est purifié par chromatographie sur colonne de gel de silice en éluant avec un gradient cyclohexane / AcOEt de 0 à 10 % d'AcOEt. Après concentration sous pression réduite, on obtient 51,5 g de 2-acétyl-3-méthylbut-2-ènoate de méthyle sous forme d'une huile incolore.
Rendement = 70 %
RMN ¹H, CDCl₃, 400MHz, δ (ppm) : 3,79 (s,3H); 2,29 (s,3H); 2,12 (s,3H); 1,97 (s,3H).

### 1.2. 2-acétyl-3-méthyl-3-phénylbutanoate de méthyle

A une suspension de 146 mg (0,8mmoles) de Cul (I) anh. dans 5 mL d'éther anh.est ajouté, à 0°C, sous flux d'argon, 3,6 mL (10,9 mmoles) d'une solution 3M dans l'Et₂O de bromure de phénylmagnésium. Après 1/2h d'agitation à 0°C, 1 g (6,4 mmoles) de 2-acétyl-3-méthylbut-2-ènoate de méthyle est ajouté en une fois,. On laisse revenir à TA et l'agitation est poursuivie 18h. Le mélange réactionnel est ensuite traité avec 100 mL d'une solution saturée en NH₄Cl, décanté, et la phase organique est à nouveau traitée par 100mL d'une solution saturée en NH₄Cl. Les phases aqueuses sont extraites par 4×100 mL de DCM. Les phases organiques sont réunies, séchées sur Na₂SOµ₄ anh. et concentrées sous pression réduite. Après purification par chromatographie sur gel de silice, en éluant avec un mélange de cyclohexane/AcOEt 95:5, on obtient 1 g de 2-acétyl-3-méthyl-3-phénylbutanoate de méthyle sous forme d'une huile incolore.
Rendement= 67%.
RMN ¹H, CDCl₃, 400MHz, δ (ppm) : 7,4-7,2 (m, 5H); 3.9 (s,1H); 3,8 (s,3H); 1,90 (s,3H); 1,6 (s,3H), 1,55 (s,3H).

### 1.3. N-éthyl-6-[3-méthyl-4-(1-méthyl-1-phényléthyl)-5-oxo-2,5-dihydro-1H-pyrazol-1-yl]-N-phénylpyridine-3-sulfonamide

A une solution de 200 mg (0,85 mmoles) de 2-acétyl-3-méthyl-3-phénylbutanoate de méthyle dans 2mL d'un mélange EtOH/AcOH (1 :1), on ajoute 249 mg (0,85 mmoles) de N-éthyl-6-hydrazino-N-phénylpyridine-3-sulfonamide puis on chauffe le mélange réactionnel pendant 2 h à 90°C. Après concentration sous pression réduite, le résidu est repris par 100 mL de DCM, lavé avec 2 X 30 mL d'une solution saturée en NaHCO₃, séché sur Na₂SO₄, filtré, concentré sous pression réduite, puis purifié par chromatographie sur colonne de gel de silice en éluant avec un gradient cyclohexane/AcOEt de 0 à 20% d'AcOEt. Après concentration sous pression réduite, on obtient 312 mg d'une huile jaune qui est concrétisée dans 20 mL de pentane. Le solide obtenu est filtré, puis séché sous vide. On obtient 178 mg de N-éthyl-6-[3-methyl-4-(1-méthyl-1-phényléthyl)-5-oxo-2,5-dihydro-1H-pyrazol-1-yl]-N-phénylpyridine-3-sulfonamide sous forme d'une poudre blanche.
Rendement= 44%
PF(°C) =122
M = C₂₆H₂₈N₄O₃S = 476 ; M+H = 477; Méthode 2: Tr=1,54 min
RMN ¹H, d6-DMSO, 400MHz, δ (ppm) : 12,0 (sl, 1H); 8,55 (sl, 1H); 8,45 (s, 1H); 8,1 (d, 1H); 7,5-7,3 (m,7H); 7,2 (m, 1H); 7,1 (d,2H); 3,65 (q,2H); 1,9 (s,3H); 1,7 (s,6H); 1,0 (t,3H).

### Exemple 2 : N-éthyl-6-[3-methyl-5-oxo-4-(pyridin-3-ylméthyl)-2,5-dihydro-1H-pyrazol-1-yl]-N-phénylpyridine-3-sulfonamide (Composé n°43 du tableau I)

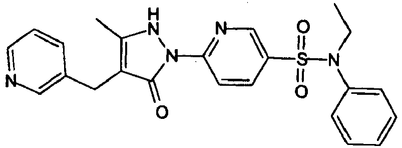

### 2.1. (2E/Z)-3-oxo-2-(pyridin-3-ylméthylidène)butanoate de méthyle

Un mélange de 10 g (86 mmoles) de 3-oxobutanoate de méthyle 9,2 g (86 mmoles) de pyridine-2-carboxaldéhyde et de 70 mg (1,1 mmoles) de PPTS dans 27 mL d'hexane est chauffé à reflux 48h dans un montage de Dean&Stark. Le milieu est ensuite concentré sous pression réduite, repris par 50 mL d'AcOEt, lavé successivement avec de l'eau (2×100 mL), de la saumure (100 mL), séché sur Na₂SO₄, filtré et concentré sous pression réduite. On obtient 17,6 g de (2E/Z)-3-oxo-2-(pyridin-3-ylméthylidène)butanoate de méthyle sous forme d'une huile jaune qui est utilisée telle quelle à l'étape suivante.
Rendement= 99%.
RMN ¹H, CDCl₃, 400MHz, δ (ppm) : 8,7 (s, 1H); 8,5 (d, 1H); 8,00 (d, 1H); 7,6 (s, 1H); 7,35 (dd, 1 H); 3,7 (s,3H); 2,3 (s,3H).

### 2.2. 3-oxo-2-(pyridin-3-ylméthyl)butanoate de méthyle

Dans un appareillage de Parr, un mélange de 14 g (68 mmol) de (2E/Z)-3-oxo-2-(pyridin-3-ylméthylidène) butanoate de méthyle dans 200 mL de MeOH et 2,2 g de Pd/C 10% est hydrogéné 48 h sous 7 bars. Le mélange réactionnel est ensuite filtré sur papier whatman GF/F et concentré sous pression réduite. On obtient ainsi 14 g de 3-oxo-2-(pyridin-3-ylméthyl)butanoate de méthyle sous forme d'une huile jaune foncé utilisée telle quelle à l'étape suivante.
Rendement = 99%.
RMN ¹H, CDCl₃, 400MHz, δ (ppm) : 8,5 (m,2H); 7,28 (d1H); 7,21 (dd1H); 3,78 (t, 1H); 3,71 (s, 3H); 3,16 (dd,2H); 2,23 (s,3H).

### 2.3 Chlorhydrate de N-éthyl-6-[3-méthyl-5-oxo-4-(pyridin-3-ylméthyl)-2,5-dihydro-1H-pyrazol-1-yl]-N-phénylpyridine-3-sulfonamide

Une solution de 300 mg (1,45 mmoles) de 3-oxo-2-(pyridin-3-ylméthyl)butanoate de méthyle et de 423 ring (1,45 mmoles) de N-éthyl-6-hydrazino-N-phénylpyridine-3-sulfonamide dans 4 mL d'un mélange EtOH/AcOH (1 :1) est chauffée pendant 4 h à 85°C. Après retour à TA, le précipité obtenu, est filtré puis lavé successivement avec de l' Et₂O (20 mL) et du pentane (20 mL). On isole 210 mg d'un résidu qui est repris par 40 mL d'eau, 2 mL d'acétonitrile et 0,48 mL d'une solution d'HCl 0,2N avant d'être lyophilisé. On obtient ainsi 220 mg de chlorhydrate de N-éthyl-6-[3-méthyl-5-oxo-4-(pyridin-3-ylméthyl)-2,5-dihydro-1H-pyrazol-1-yl]-N-phénylpyridine-3-sulfonamide sous forme d'un lyophilisat blanc.
Rendement = 33 %
PF(°C) =128
M = C₂₃H₂₃N₅O₃S = 449 ; M+H = 450; Méthode 2: Tr=0,85min
RMN ¹H, d6-DMSO, 400MHz, δ (ppm) : 12,5 (sl, 1H); 8,90 (s, 1H) ; 8,8 (d, 1H); 8,6 (d, 1H); 8,5 (d, 1H); 8,4 (s,1H); 8,2 (dd, 1H); 8,05 (dd, 1H); 7,4 (m,3H); 7,1 (d,2H); 3,8 (s,2H); 3,65 (q,2H); 2,3 (s,3H); 1,0 (t,3H).

### Exemple 3: Chlorhydrate de 6-(4-{3-[2-(diméthylamino)éthoxy]benzyl}-3-méthyl-5-oxo-2,5-dihydro-1H-pyrazol-1-yl)-N-éthyl-N-phénylpyridine-3-sulfonamide (Composé n°56 du tableau I)

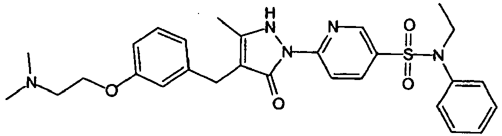

### 3.1. (2E/Z)-2-[(3-hydroxyphényl)méthylidène]-3-oxobutanoate de méthyle.

Selon le procédé décrit dans l'exemple 2.1, on obtient, à partir de 5 g de 3 hydroxybenzaldéhyde et 4,75g de 3-oxobutanoate de méthyle, 2,5 g de (2E/Z)-2-[(3-hydroxyphenyl)méthylidene]-3-oxobutanoate de méthyle sous forme d'une poudre jaune clair.
Rendement = 27%
RMN ¹H (CDCl₃, 400MHz, δ (ppm) : 7,4 (s, 1H); 7,2 (m, 1H); 6,9-6,8 (m,2H); 5,1 (s, 1H); 3,8 (s,3H); 2,4 (s,3H); 2,4(s,3H).

### 3.2. 2-(3-hydroxybenzyl)-3-oxobutanoate de méthyle

Selon le procédé décrit dans l'exemple 2.2, on obtient, à partir de 2,5 g de (2E/Z)-2-[(3-hydroxyphényl)méthylidene]-3-oxobutanoate de méthyle , 2,5 g. de 2-(3-hydroxybenzyl)-3-oxobutanoate de méthyle sous forme d'une cire translucide.
Rendement = 99%
RMN ¹H CDCl₃, 400 MHz, δ (ppm) : 7,15 (t, 1H); 6,7-6,85 (m,3H); 5,6 (s, 1H); 3.7 (s,3H); 3,15 (d,2H); 2.25 (s, 3H).

### 3.3. 2-{3-[2-(diméthylamino)éthoxy]benzyl}-3-oxobutanoate de méthyle

Un mélange de 1,5 g (6,75 mmoles) de méthyl 2-(3-hydroxybenzyl)-3-oxobutanoate de méthyle, de 6,6 g (20,25 mmoles) de Cs₂CO₃ anh, de 0,1 g (0,67 mmoles) de Nal et de 1 g (7,1 mmoles) de chlorhydrate de chlorure de 2-diméthylaminoéthyle dans 20 mL de CH₃CN anh. est chauffé pendant 4 h à 90°C, puis agité une nuit à TA. Le milieu réactionnel est ensuite filtré, puis concentré sous pression réduite. Le résidu obtenu est repris par du DCM (100 mL), lavé avec de la saumure (30 mL), séché sur Na₂SO₄, filtré, concentré sous pression réduite, puis purifié par chromatographie sur colonne de gel de silice en éluant avec un gradient DCM/MeOH de 0 à 10 % de MeOH. Après concentration sous pression réduite, on obtient 409 mg de méthyl 2-{3-[2-(diméthylamino) éthoxy]benzyl}-3-oxobutanoate sous forme d'une cire marron.
Rendement = 19,5 %
RMN ¹H, CDCl₃, 400MHz, δ (ppm) :7,2 (t, 1H); 6,7-6,8 (m,3H); 4,1 (t,2H); 3,8 (t, 1H); 3.7 (s,3H); 3,15 (d,2H); 2,8 (t,2H); 2.4 (s, 6H); 2,3 (s,3H).

### 3.4. Chlorhydrate de 6-(4-{3-[2-(diméthylamino)éthoxy]benzyl}-3-méthyl-5-oxo-2,5-dihydro-1H-pyrazol-1-yl)-N-éthyl-N-phénylpyridine-3-sulfonamide

Selon le procédé décrit dans l'exemple 2.3, on obtient, à partir de 195 mg de méthyl 2-{3-[2-(diméthylamino)éthoxy]benzyl}-3-oxobutanoate et de 194 mg de N-éthyl-6-hydrazino-N-phénylpyridine-3-sulfonamide, 70 mg de chlorhydrate de 6-(4-{3-[2-(diméthylamino)éthoxy]benzyl}-3-méthyl-5-oxo-2,5-dihydro-1H-pyrazol-1-yl)-N-éthyl-N-phénylpyridine-3-sulfonamide sous forme d'une lyophilisat blanc. Rendement = 20 %
PF(°C) =124
M = C₂₈H₃₃N₅O₄S = 535 ; M+H = 536; Méthode 2: Tr=0,98 min
RMN ¹H, d6-DMSO, 400MHz, δ (ppm) : 12,0 (sl, 1H); 10,0 (sl, 1H); 8,6 (d, 1H); 8,4 (s, 1H); 8,1 (d, 1H); 7,4 (m,3H); 7,25 (t, 1H); 7,15 (d,2H); 6,9 (d,2H); 6,8 (d, 1H); 4,3 (t, 2H); 3,6 (q,2H); 3,5 (s,2H); 3,4 (t,2H); 2,8 (s,6H); 2,2 (s,3H); 1,0 (t,3H).

### Exemple 4: 6-{4-[3-(diméthylamino)benzyl]-3-méthyl-5-oxo-2,5-dihydro-1H-pyazol-1-yl}-N-éthyl-N-phénylpyridine-3-sulfonamide (Composé n°53 du tableau I)

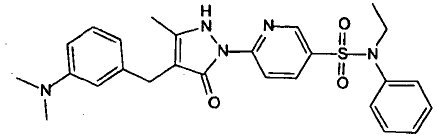

### 4.1. (2Z/E)-2-[(4-nitrophényl)méthylidene]-3-oxobutanoate de méthyle

Selon le procédé décrit dans l'exemple 2.1, on obtient, à partir de 6,18 g de 4 nitrobenzaldéhyde et 4,75 g de 3-oxobutanoate de méthyle, 4,2 g de (2Z/E)-2-[(4-nitrophényl)méthylidenel-3-oxobutanoate de méthyle sous forme d'huile.
Rendement =41 %
RMN ¹HCDCl₃, 400MHz, δ (ppm) : 8,4 (s, 1H); 8,3 (d, 1H); 7,8 (d, 1H); 7,6 (m,2H); 3.9 (s,3H); 2,5 (s,3H).

### 4.2. 2-(4-aminobenzyl)-3-oxobutanoate de méthyle

Selon le procédé décrit dans l'exemple 2.2, on obtient, à partir de 4,2 g de I (2Z/E)-2-[(4-nitrophényl)méthylidene]-3-oxobutanoate de méthyle, 2 g de méthyl 2-(4-aminobenzyl)-3-oxobutanoate de méthyle sous forme d'huile.
RMN ¹H, CDCl₃, 400MHz, δ (ppm) : 7,1 (t,1H); 6,6-6,5 (m,3H); 3.9 (t,1H); 3,8 (s,3H); 3,7 (sl,2H); 3,1 (d,2H); 2,2 (s,3H).
Rendement= 54%

### 4.3 2-[4-(diméthylamino)benzyl]-3-oxobutanoate de méthyle

A un mélange de 1,5 g (6,78 mmoles) de 2-(4-aminobenzyl)-3-oxobutanoate de méthyle, de 40 µl (0,04 mmoles) d'AcOH et de 5,1 mL (67,8 mmoles) d'une solution aqueuse à 37% en formaldéhyde dans 13 mL de CH₃CN, on ajoute à 0°C par petites portions, 4,3 g (20,34 mmoles) de triacétoxyborohydrure de sodium. On laisse ensuite le milieu réactionnel progressivement revenir à TA et l'agitation est poursuivie 12 h. Le mélange réactionnel est versé sur 50 mL d'une solution saturée en NaHCO₃ et 30 g de glace, extrait avec AcOEt (2X100 mL), séché sur Na₂SO₄, filtré, et concentré sous pression réduite, puis purifié par chromatographie sur colonne de gel de silice en éluant avec un gradient cyclohexane / AcOEt de 0 à 50 % AcOEt. Après on obtient 344 mg de méthyl 2-[4-(diméthylamino)benzyl]-3-oxobutanoate sous forme d'une huile jaune.
Rendement : 20%
RMN ¹H, CDCl₃, 400MHz, δ (ppm) : 7,1 (t,1H); 6,65 (d,1H); 6,55 (m,2H); 3.9 (t,1H); 3,8 (s,3H); 3,2 (d,2H); 3,0 (s,6H); 2,2 (s,3H).

### 4.4. 6-{4-[3-(diméthylamino)benzyl]-3-méthyl-5-oxo-2,5-dihydro-1H-pyrazol-1-yl}-N-éthyl-N-phénylpyridine-3-sulfonamide

Selon le procédé décrit dans l'exemple 1.3, on obtient, à partir de 172 mg de 2-[4-(diméthylamino)benzyl]-3-oxobutanoate méthyle et de 202 mg de N-éthyl-6-hydrazino-N-phénylpyridine-3-sulfonamide, 138 mg de 6-{4-[3-(diméthylamino)benzyl]-3-méthyl-5-oxo-2,5-dihydro-1H-pyrazol-1-yl}-N-éthyl-N-phénylpyridine-3-sulfonamide sous forme d'un solide blanc.
Rendement= 41%
PF(°C) =122
M = C₂₈H₂₉N₅O₃S = 491 ; M+H = 492; Méthode 2: Tr=0,96
RMN ¹H, d6-DMSO, 400MHz, δ (ppm) : 12,0 (sl, 1H); 8,7 (sl, 1H); 8,45 (s, 1H); 8,1 (d, 1H); 7,4 (m,3H); 7,2 (d,2H); 7,1 (t,1H); 6,7 (s, 1H); 6,5 (d,2H); 3,7 (q,2H); 3,5 (s,2H); 2,9 (s,6H); 2,15 (s,3H); 1,0 (t, 3H).

### Exemple 5: N,N-diméthyl-6-[3-méthyl-5-oxo-4-(pyridin-3-ylméthyl)-2,5-dihydro-1H-pyrazol-1-yl]pyridine-3-sulfonamide (Composé n°66 du tableau I)

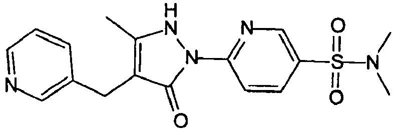

### 5.1. 6-chloro-N,N-diméthylpyridine-3-sulfonamide

A un mélange de 4,7 mL (9,43 mmoles) de diméthylamine (2N dans le THF) et de 2,6 mL (18,86 mmoles) de TEA dans 20 mL de THF, est ajouté, à 0°C, au goutte à goutte une solution de 2 g (9,43 mmoles) de chlorure de 6-chloropyridine-3-sulfonyle (préparé selon le document W09840332) dans 5 mL de THF. Après 40 minutes d'agitation à 0°C, le milieu réactionnel est repris par 40 mL d'AcOEt, lavé avec de l'eau (2 X 40 mL) et de la saumure (40 mL), séché sur Na₂SO₄, filtré et concentré sous pression réduite. On obtient 1,93 g de 6-chloro-N,N-diméthylpyridine-3-sulfonamide sous forme d'un solide marron utilisé tel quel à l'étape suivante.
Rendement = 93%
RMN ¹H CDCl₃, 400MHz, δ (ppm):8,8(s,1H) ; 8,0(d, 1H) ; 7,4 (d, 1H);2,7(s,6H).

### 5.2. 6-hydrazino-N,N-diméthylpyridine-3-sulfonamide

Un mélange de 1,9 g (8,8 mmoles) de 6-chloro-N,N-diméthylpyridine-3-sulfonamide et de 4,6 mL (91,5 mmoles) d' hydrazine monohydrate dans 10 mL d'EtOH est chauffé pendant 2 h à 80°C. Le précipité obtenu, après retour à TA, est filtré puis lavé avec 10 mL d'EtOH et séché sous vide. On obtient 1,62 g de 6-hydrazino-N,N-diméthylpyridine-3-sulfonamide sous forme d'une poudre blanche.
RMN ¹H, d6-DMSO, 400MHz, δ (ppm) : 8,5 (sl,1H); 8,3 (s,1H); 7,7 (d,1H); 6,85 (d,1H); 4,4 (s,2H); 2,6 (s,6H).

### 5.3. N,N-diméthyl-6-[3-méthyl-5-oxo-4-(pyridin-3-ylméthyl)-2,5-dihydro-1H-pyrazol-1-yl]pyridine-3-sulfonamide

Un mélange de 400 mg (1,85 mmoles) de 6-hydrazino-N,N-diméthylpyridine-3-sulfonamide et de 383 mg (1,85 mmoles) de 3-oxo-2-(pyridin-3-ylméthyl)butanoate de méthyle dans 4 mL d'un mélange EtOH/AcOH (1:1), est chauffé 4 h à 80°C puis concentré sous pression réduite. Le résidu obtenu est repris par 20 mL d'AcOEt, lavé successivement avec de l'eau (2 X20 mL), une solution saturée en NaHCO₃ (20 mL), de la saumure (20 mL), séché sur Na₂SO₄, filtré, et concentré sous pression réduite. Le résidu est ensuite concrétisé dans 20 mL d'un mélange Et₂O/pentane (1 :1), filtré et recristallisé dans un mélange cyclohexane /EtOH. On obtient 188 mg de N,N-diméthyl-6-[3-méthyl-5-oxo-4-(pyridin-3-ylméthyl)-2,5-dihydro-1H-pyrazol-1-yl]pyridine-3-sulfonamide sous forme d'un cristaux blancs .
Rendement = 28 %
PF(°C) = 212
M = C₁₇H₁₉N₅O₃S = 373 ; M+H = 374; Méthode 2: Tr=0,58 min
RMN ¹H, d6-DMSO, 400MHz, δ (ppm) : 12 (s),1H); 8,7 (s,1H); 8,65 (d,1H); 8,5 (s,1H); 8,4 (d,1H); 8,35 (d,1H); 7,7 (d,1H); 7,3 (dd,1H); 3,6 (s,2H); 2,7 (s,6H); 2,2 (s,3H).

### Exemple 6 : N-éthyl-6-{4-[3-(méthoxyméthyl)benzyl]-3-méthyl-5-oxo-2,5-dihydro-1H-pyrazol-1-yl}-N-phénylpyridine-3-sulfonamide (Composé n°34 du tableau I)

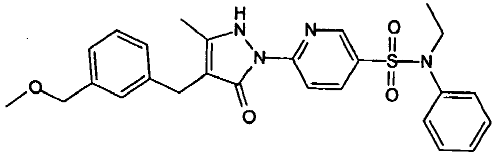

### 6.1. 3-(méthoxyméthyl)benzoate de méthyle

A une solution de 15 g (65,5 mmoles) de 3-(bromométhyl)benzoate de méthyle dans 20 mL de MeOH anh., on ajoute à TA, au goutte à goutte, une solution de méthylate de sodium dans du MeOH, préparée prélablement à partir de 2,25 g (98,2 mmoles) de sodium dans 65 mL de MeOH. Le milieu réactionnel est ensuite chauffé 4h à 65°C puis concentré sous pression réduite, repris par 500 mL de DCM, lavé avec de l'eau (100mL) et de la saumure (100 mL), séché sur Na₂SO₄, filtré et concentré sous pression réduite. On obtient 7,8 g de 3-(méthoxyméthyl)benzoate de méthyle sous forme d'une huile qui est engagée telle quelle à l'étape suivante.
Rendement = 66%
RMN ¹H, CDCl₃, 400MHz, δ (ppm) : 8,1 (s,1H); 8,0 (d,1H); 7,5 (d,1H); 7,4 (t,1H); 4,5 (s,2H); 4,0 (s,3H); 3,4 (s,3H).

### 6.2. 2 [3-(méthoxyméthyl)phényl]méthanol

A une solution de 7,8 g (43,3 mmoles) de 3-(méthoxyméthyl) benzoate de méthyle dans 60 mL d'un mélange THF/dioxane (1:1) est ajouté 0,94 g (43,3 mmoles) de borohydrure de lithium. Le milieu réactionnel est alors chauffé 3 h à 80°C puis agité une nuit à TA. Le milieu réactionnel est repris par 500 mL de AcOEt, lavé avec de l'eau (2X100 mL), séché sur Na₂SO₄, filtré, et concentré sous pression réduite, on obtient 5,9 g de 2 [3-(méthoxymethyl)phényl]méthanol sous forme d'un liquide jaune qui est engagé tel quel à l'étape suivante.
Rendement= 90%
RMN ¹H, CDCl₃, 400MHz, δ (ppm) : 7,4-7,2 (m,4H); 4,7 (s,2H); 4,5 (s,2H); 3,4 (s,3H); 2,4 (sl,1H).

### 6.3. 1-(bromométhyl)-3-(méthoxyméthyl)benzène

A une solution de 5,91 g (38,8 mmoles) de 2 [3-(méthoxyméthyl)phényl]méthanol dans 75 mL d'Et₂O, on ajoute à 0°C au goutte à goutte 9,1 mL (97,1 mmoles) de tribromure de phosphore. On laisse le mélange réactionnel remonter lentement à TA et l'agitation est maintenue 4 h. Le brut réactionnel est alors versé avec précaution sur un mélange de 100 g de glace et 100 mL de MeOH. Après évaporation du MeOH sous pression réduite, la phase aqueuse est extraite avec du DCM (2X200 mL). Les phases organiques sont rassemblées, séchées sur Na₂SO₄, filtrées puis concentrées sous pression réduite. Le résidu obtenu est purifié par chromatographie sur colonne de gel de silice en éluant avec un gradient cyclohexane / AcOEt de 0 à 20% d'AcOEt.

On obtient ainsi 3,25 g de1-(bromométhyl)-3-(méthoxyméthyl)benzène sous forme d'huile.
Rendement= 39%
RMN ¹H CDCl₃, 400MHz, δ (ppm):7,4-7,1(m,4H); 4,45(s,2H);4,35(s,2H); 3,3 (s,3H).

### 6.4. 2-[3-(méthoxyméthyl)benzyl]-3-oxobutanoate de méthyle

A une solution de 0,34 g (15,1 mmoles) de sodium dans 8 mL de MeOH anh., , on ajoute au goutte à goutte à TA et sous argon ,1,6 mL (15,1 mmoles) d' acétoacétate de méthyle. Après ½ h d'agitation, 3,25 g (15,11 mmoles) de 1-(bromométhyl)-3-(méthoxyméthyl) benzène sont ajoutés par un rapide goutte à goutte puis le milieu réactionnel est chauffé 2h30 à 70°C. Le milieu réactionnel est ensuite concentré sous pression réduite, le résidu obtenu est purifié par chromatographie sur colonne de gel de silice en éluant avec un gradient cyclohexane / AcOEt de 0 à 20% d'AcOEt. On obtient 3,2 g de 2-[3-(méthoxyméthyl) benzyl]-3-oxobutanoate de méthyle sous forme d'huile.
Rendement = 85%
RMN ¹H, CDCl₃, 400MHz, δ (ppm) : 7,4-7,1 (m,4H); 4,45 (s,2H); 3,8 (t,1H); 3,7 (t,3H); 3,4 (t,3H); 3,2 (d,2H); 2,2 (s,3H).

### 6.5. N-éthyl-6-{4-[3-(méthoxyméthyl)benzyl]-3-méthyl-5-oxo-2,5-dihydro-1H-pyrazol-1-yl}-N-phénylpyridine-3-sulfonamide

Selon le procédé décrit dans l'exemple 1.3, on obtient, à partir de 300 mg de 2-[3-(méthoxyméthyl)benzyl]-3-oxobutanoate de méthyle et de 350 mg de N-éthyl-6-hydrazino-N-phénylpyridine-3-sulfonamide, 341 mg de N-éthyl-6-{4-[3-(méthoxyméthyl)benzyl]-3-méthyl-5-oxo-2,5-dihydro-1H-pyrazol-1-yl}-N-phénylpyridine-3-sulfonamide sous forme de poudre blanche.
Rendement= 58%
PF(°C) =144
M = C₂₆H₂₈N₄O₄S = 492 ; M+H = 493; Méthode 2: Tr=1,29 min.
RMN ¹H, d6-DMSO, 400MHz, δ (ppm) : 12,0 (sl,1H); 8,7 (sl,1H); 8,45 (s,1H); 8,1 (d,1H); 7,4 (m,3H); 7,3-7,1 (m,6H); 4,35 (s,2H); 3,6 (q,2H); 3,5 (s,2H); 3,3 (s,3H); 2,15 (s,3H); 1,0 (t, 3H).

### Exemple 7 : N-éthyl-6-[3-méthyl-5-oxo-4-(2-phényléthyl)-2,5-dihydro-1H-pyrazol-1-yl]-N-phénylpyridine-3-sulfonamide (Composé n°74 du tableau I)

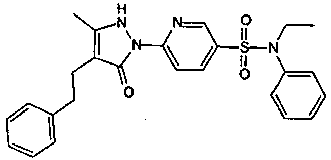

### 7.1. 3-oxo-2-(2-phényléthyl)butanoate de méthyle,

Selon le procédé décrit dans l'exemple 6.4, on obtient, à partir de 5,16 g de (2-bromoéthyl)benzène et de 3,2 g d' acétoacétate de méthyle, 1,9 g de 3-oxo-2-(2-phényléthyl)butanoate de méthyle sous forme d'une huile translucide.
Rendement= 31%.
RMN ¹H, CDCl3, 400MHz, δ (ppm) : 7,2-7,35 (m,5H); 3,75 (s,3H); 3,5 (t, 1 H); 2,5-2,7 (m,2H); 2,2 (s,3H); 2,15-2,3 (m,2H).

### 7.2. N-éthyl-6-[3-méthyl-5-oxo-4-(2-phényléthyl)-2,5-dihydro-1H-pyrazol-1-yl]-N-phénylpyridine-3-sulfonamide

Selon le procédé décrit dans l'exemple 1.3, on obtient, à partir de 264 mg de 3-oxo-2-(2-phényléthyl)butanoate de méthyle et de 351 mg de N-éthyl-6-hydrazino-N-phénylpyridine-3-sulfonamide, 291 mg de N-éthyl-6-[3-méthyl-5-oxo-4-(2-phényléthyl)-2,5-dihydro-1H-pyrazol-1-yl]-N-phénylpyridine-3-sulfonamide, sous forme de poudre blanche.
Rendement= 53%
PF(°C) =158
M = C₂₅H₂₆N₄O₃S = 462 ; M+H = 463; Méthode 2= 1,35 min
RMN ¹H, d6-DMSO, 400MHz, δ (ppm) : 11,8 (sl,1H); 8,6 (sl,1H); 8,45 (s,1H); 8,1 (d,1H); 7,4 (m,3H); 7,3 (m,2H); 7,2 (d,3H); 7,1 (d,2H); 3,6 (q,2H); 2,8 (t,2H); 2,5 (t,2H); 1,9 (s,3H); 1,0 (t, 3H).

### Exemple 8: 6-(4-benzyl-5-oxo-3-propyl-2,5-dihydro-1H-pyrazol-1-yl)-N-éthyl-N-phénylpyridine-3-sulfonamide (Composé n°68 du tableau I)

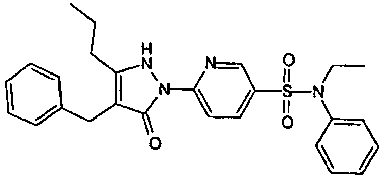

### 8.1. 2-benzyl-3-oxohéxanoate d'éthyle

Selon le procédé décrit dans l'exemple 6.4, on obtient, à partir de 2,2 g de bromure de benzyle et 4 g d'éthylbutyrylacétate, 2,5 g de 2-benzyl-3-oxohéxanoate d'éthyle sous forme d'une huile translucide.
Rendement = 40%
RMN ¹H, CDCl₃, 400MHz, δ (ppm) : 7,15-7,30 (m,5H); 4,2 (q,2H); 3,8 (t,1H); 3,2 (dd, 2H); 2,5-2,3 (m,2H); 1,55 (m, 2H); 1,2 (t,3H); 0,86 (t,3H).

### 8.2. 6-(4-benzyl-5-oxo-3-propyl-2,5-dihydro-1H-pyrazol-1-yl)-N-éthyl-N-phénylpyridine-3-sulfonamide

Selon le procédé décrit dans l'exemple 1.3, on obtient, à partir de 300 mg de 2-benzyl-3-oxohéxanoate d'éthyle et de 353 mg de N-éthyl-6-hydrazino-N-phénylpyridine-3-sulfonamide, 400 mg de 6-(4-benzyl-5-oxo-3-propyl-2,5-dihydro-1H-pyrazol-1-yl)-N-éthyl-N-phénylpyridine-3-sulfonamide sous forme de poudre blanche.
Rendement= 69%
PF(°C) =180
M = C₂₆H₂₈N₄O₃S = 476 ; M+H = 477; Méthode 2: Tr=1,45 min.
RMN ¹H, d6-DMSO, 400MHz, δ (ppm) : 12,0 (sl,1H); 8,6(sl,1H); 8,45 (s,1H); 8,1 (d,1H); 7,45-7,1 (m,10H); 3,65 (q,2H); 3,6 (s,2H); 2,5 (t,2H); 1,5(m,2H);1,0 (t,3H); 0,9 (t,3H).

### Exemple 9 : N-éthyl-6-{4-[3-(2-méthoxyéthoxy)benzyl]-3-méthyl-5-oxo-2,5-dihydro-1H-pyrazol-1-yl}-N-phénylpyridine-3-sulfonamide (Composé n°38 du tableau I)

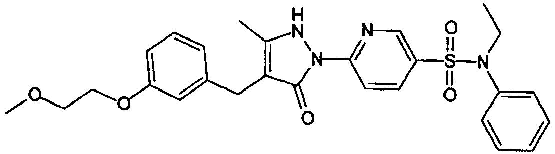

### 9.1. [3-(2-méthoxvéthoxy)phényl]méthanol

Un mélange de 10 g (80,6 mmoles) de 3-(hydroxyméthyl)phénol, de 78,4 g (241,7 mmoles) de Cs₂CO₃ et de 8,4 mL (88,6 mmoles) de bromoéthylméthyléther dans 150 mL de CH₃CN est chauffé 12h à 110°C. Après retour à TA, le milieu est filtré, concentré sous pression réduite, repris par 500 mL de DCM, lavé avec de la saumure (2 X100 mL) séché sur Na₂SO₄, filtré puis concentré sous pression réduite.

On obtient 10,9 g de 3-(2-méthoxyéthoxy)phényl]méthanol sous forme d'huile jaune.
Rendement =75%
RMN ¹H, CDCl₃, 400MHz, δ (ppm) : 7,3 (t,1H); 6,95 (m,2H); 6,85 (d,1H); 4,6 (s,2H); 4,1 (t,2H); 3,8 (t,2H); 3,4 (s,3H); 2,1 (sl,1H)

### 9.2. 1-(bromométhyl)-3-(2-méthoxyéthoxy)benzène

Selon le procédé décrit dans l'exemple 6.3, on obtient, à partir de 10 g de [3-(2-m éthoxyéthoxy)phényl]méthanol, 11,8 g de 1-(bromométhyl)-3-(2-méthoxyéthoxy)benzène, sous forme d'une huile.
Rendement= 87%
RMN ₁H, CDCl3, 400MHz, δ (ppm) : 7,3 (m,1H); 7,0 (m,2H); 6,9 (d,1H ); 4,45 (s,2H); 4,15 (t,2H); 3,75 (t,2H); 3,4 (s,3H)

### 9.3 2-[3-(2-méthoxyéthoxy)benzyl]-3-oxobutanoate de méthyle

Selon le procédé décrit dans l'exemple 6.4, on obtient à partir de 5,6 g de 1-(bromométhyl)-3-(2-méthoxyéthoxy)benzène et de 2,52g d' acétoacétate de méthyle ,3,44 g de 2-[3-(2-méthoxyéthoxy)benzyl]-3-oxobutanoatede méthyle , sous forme d'une huile jaune.
RMN ¹H CDCl₃, 400MHz, δ (ppm) : 7,2 (t, 1H); 6,75(m,3H); 4,15(d,2H); 3,8(t,1H); 3,75(s,3H); 3,45(s,3H); 3,15(d,2H); 2,2(s,3H).

### 9.4. N-éthyl-6-{4-[3-(2-méthoxyéthoxy)benzyl]-3-méthyl-5-oxo-2,5-dihydro-1H-pyrazol-1-yl}-N-phénylpyridine-3-sulfonamide

Selon le procédé décrit dans l'exemple 1.3, on obtient, à partir de 336 mg de méthyl 2-[3-(2-méthoxyéthoxy)benzyl]-3-oxobutanoate et de 351 mg de N-éthyl-6-hydrazino-N-phénylpyridine-3-sulfonamide, 441 mg de N-éthyl-6-{4-[3-(2-méthoxyéthoxy)benzyl]-3-méthyl-5-oxo-2,5-dihydro-1H-pyrazol-1-yl}-N-phénylpyridine-3-sulfonamide sous forme de poudre blanche.
Rendement= 70%
PF(°C) =126
M = C₂₇H₃₀N₄O₅S = 522 ; M+H = 523; Méthode 2 : Tr= 1,27 min.
RMN ¹H, d6-DMSO, 400MHz, δ (ppm) : 12,0 (sl,1H); 8,6 (sl,1H); 8,45 (s,1H); 8,1 (d,1H); 7,4 (m,3H); 7,3-7,1 (m,3H); 6,8 (d,2H); 7,1(d,1H); 4,1 (d,2H); 3,6 (m,4H); 3,5 (s,2H); 3,3 (s,3H); 2,1 (s,3H); 1,0 (t, 3H).

### Exemple 10: N-éthyl-6-[4-(4-méthoxybenzyl)-3-méthyl-5-oxo-2,5-dihydro-1H-pyrazol-1-yl]-N-phénylpyridine-3-sulfonamide (Composé n°54 du tableau I)

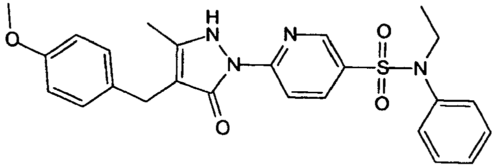

### 10.1. 2-(4-méthoxybenzyl)-3-oxobutanoate de méthyle

Un mélange de 1,34 g (8,6 mmoles) de 1-(chlorométhyl)-4-méthoxybenzène, de 0,93 mL (8,6 mmoles) d' acétoacétate de méthyle , de 0,24 g (0,86 mmoles) de chlorure de tétrabutylammonium et de 6,9 g d'un mélange massique de K₂CO₃/KOH (4:1) dans 5 mL de toluène est chauffé 5 minutes à 110°C dans un micro-ondes. Le milieu réactionnel est repris par 80 mL d'AcOEt, lavé successivement avec de l'eau (2 X20 mL), une solution de NaHCO₃ saturée (20 mL), de la saumure (20 mL), séché sur Na₂SO₄ puis concentré sous pression réduite. Le résidu obtenu est purifié par chromatographie sur colonne de gel de silice en éluant avec un gradient cyclohexane / AcOEt de 0 à 30 % d'AcOEt.

On obtient ainsi 0,93 g de 2-(4-méthoxybenzyl)-3-oxobutanoate de méthyle sous forme d'huile jaune.
Rendement = 48%
RMN ¹H, CDCl3, 400MHz, δ (ppm) : 7,2 (d,1H); 7,1 (d,1H); 6,8 (m,2H); 3,8 (s,3H); 3,75 (t,1H); 3,7 (s,3H); 3,2 (m,2H); 2,2 (s,3H).

### 10.2. N-éthyl-6-[4-(4-méthoxybenzyl)-3-méthyl-5-oxo-2,5-dihydro-1H-pyrazol-1-yl]-N-phénylpyridine-3-sulfonamide

Selon le procédé décrit dans l'exemple 1.3, on obtient, à partir de 300 mg de 2-(4-méthoxybenzyl)-3-oxobutanoate de méthyle et de 371 mg de N-éthyl-6-hydrazino-N-phénylpyridine-3-sulfonamide 338 mg de N-éthyl-6-[4-(4-méthoxybenzyl)-3-méthyl-5-oxo-2,5-dihydro-1H-pyrazol-1-yl]-N-phénylpyridine-3-sulfonamide sous forme d'une poudre blanche.
Rendement= 56 %
PF(°C) =188
M = C₂₅H₂₆N₄O₄S = 478 ; M+H = 479; Méthode 2 : Tr= 1,29 min.
RMN ¹H, d6-DMSO, 400MHz, δ (ppm) : 11,8 (sl,1H); 8,6 (sl,1H); 8,45 (s,1H): 8,1 (d,1H); 7,4 (m,3H); 7,3-7,1 (m,4H); 6,8 (d,2H); 3,75 (s,3H); 3,6 (s,2H); 3,5 (s,2H); 2,1(s,3H); 1,0 (t, 3H).

### Exemple 11: 6-[4-(2-chloro-6-fluorobenzyl)-3-méthyl-5-oxo-2,5-dihydro-1H-pyrazol-1-yl]-N-éthyl-N-phénylpyridine-3-suifonamide (Composé n°20 du tableau I)

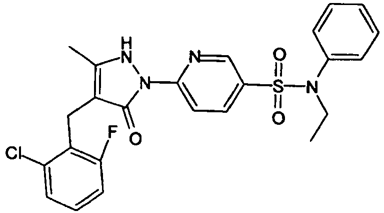

Un mélange de 52 mg (0,2 mmoles) de 2-(2-chloro-6-fluorobenzyl)-3-oxobutanoate de méthyle, de 73 mg (0,25 mmoles) de N-éthyl-6-hydrazino-N-phénylpyridine-3-sulfonamide et de 5 mg de pTsOH dans 2 mL de toluène est chauffé 12 h à 110°C puis concentré sous pression réduite. Le résidu est repris par 2 mL de DMF, filtré et le filtrat est chromatographié sur phase inverse RP18 en éluant avec un gradient H₂O (à 2%TFA) /CH₃CN de 0 à 100% en CH₃CN.

On obtient 86mg de 6-[4-(2-chloro-6-fluorobenzyl)-3-méthyl-5-oxo-2,5-dihydro-1H-pyrazol-1-yl]-N-éthyl-N-phénylpyridine-3-sulfonamide sous forme d'une poudre blanche ; Rendement = 99%
PF(°C) = 206
M = C₂₄H₂₂CIFN₄O₃S =501. M+H =502; Méthode 1
RMN ¹H, d6-DMSO, 400MHz, δ (ppm) : 11,9 (sl,1H); 8,6 (sl,1H); 8,4 (s,1H); 8,0 (d,1H); 7,5-7,3 (m,5H); 7,2-7,0 (m,3H); 3,7 (s,2H); 3,6 (q,2H); 2,1 (s,3H);1,0 (t, 3H).

### Exemple 12: N-éthyl-6-[3-méthyl-5-oxo-4-(1-phénylcyclopropyl)-2,5-dihydro-1H-pyrazol-1-yl]-N-phénylpyridine-3-sulfonamide (Composé n°80 du tableau I)

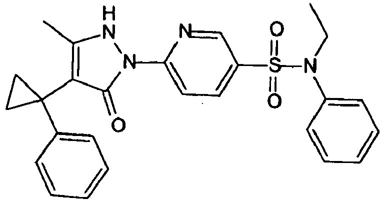

### 12.1. 2-acétyl-3-phénylbut-3-ènoate de méthyle

Une suspension de 3,2 g (27,5 mmoles) de 3-oxobutanoate de méthyle, de 5,6 g (55,0 mmoles) de phénylacétylène et de 700 mg (0,8 mmoles) de *[ReBr(CO)₃(THF)]₂ dans 55 mL de toluène anh. est agité 18 h à 50°C. Après retour à TA, le milieu est concentré sous pression réduite. Le résidu obtenu est repris par 100 mL de DCM, lavé successivement avec de l'eau (100 mL) et de la saumure (100 mL), séché sur Na₂SO₄ puis concentré sous pression réduite et purifié par chromatographie sur colonne de gel de silice en éluant avec un mélange DCM/MeOH 99:1. On obtient 5 g de 2-acétyl-3-phénylbut-3-ènoate de méthyle sous forme d'une huile jaune.
Rendement = 84%
RMN ¹H, CDCl₃, 400MHz, δ (ppm) : 12,6 (s,1H); 7,3-7,2 (m,5H); 5,7 (s,1H); 5,1 (s,1H); 3,55 (s,3H); 1,9 (s,3H).

* :[ReBr(CO)₃(THF)]₂ est fraîchement préparé à partir de 2 g (4,9 mmoles) de ReBr(CO)₅ agité à reflux dans 60 mL de THF anh. pendant 16h. Après concentration sous pression réduite et recristallisation dans 5 mL de n-hexane/THF (1 :1) on obtient 700 mg de [ReBr(CO)₃(THF)]₂ sous forme d'une poudre blanche.
Rendement = 35%.

### 12.2. 3-oxo-2(1-phénylcyclopropyl)butanoate de méthyle

A une solution de 1 g (4,6 mmoles) de 2-acétyl-3-phénylbut-3-ènoate de méthyle dans 16 mL de DCM est ajouté successivement, au goutte à goutte, 20,8 mL (22,9 mmoles) d'une solution de diéthylzinc en solution 1,1M dans le toluène et 3,7 mL (45,8 mmoles) de diiodométhane. Le milieu réactionnel est chauffé 18 h au reflux. Après retour à TA, le mélange réactionnel est traité avec 100 mL d'eau, puis extrait avec du DCM (3X100 mL). Les phases organiques sont rassemblées, lavées successivement avec de l'eau (4X100 mL), de la saumure (100 mL), séchées sur Na₂SO₄, filtrées, puis concentrées sous pression réduite. Le résidu obtenu est purifié par chromatographie sur colonne de gel de silice en éluant avec un mélange cyclohexane/ACOEt 95:5. On obtient 280 mg de 3-oxo-2(1-phénylcyclopropyl)butanoate de méthyle sous forme d'huile jaune.
Rendement =26%.
RMN ¹H, CDCl₃, 400MHz, δ (ppm) : 7,3-6,9 (m,5H); 3,7 (s,1H); 3,6 (s,3H); 2,0 (s,3H); 1,4 (s,2H); 1,25 (s,2H)

### 12.3. N-éthyl-6-[3-méthyl-5-oxo-4-(1-phénylcyclopropryl)-2,5-dihydro-1H-pyrazol-1-yl]-N-phénylpyridine-3-sulfonamide

Selon le procédé décrit dans l'exemple 1.3, on obtient, à partir de 280 mg 3-oxo-2(1-phénylcyclopropyl)butanoate de méthyle et de 352 mg de N-éthyl-6-hydrazino-N-phénylpyridine-3-sulfonamide, 110 mg de N-éthyl-6-[3-méthyl-5-oxo-4-(1-phénylcyclopropyl)-2,5-dihydro-1H-pyrazol-1-yl]-N-phénylpyridine-3-sulfonamide sous forme de poudre blanche.
PF(°C) =146
M = C₂₈H₂₆N₄O₃S =474, M+H=475; Méthode 2: Tr=1,44 min.
RMN ¹H, d6-DMSO, 400MHz, δ (ppm) : 12,0 (sl,1H); 8,6 (sl,1H); 8,4 (s,1H); 8,1 (d,1H); 7,4-7,1 (m,10H); 3,6 (q,2H); 2,2 (s,3H); 1,15 (dd,4H); 1,0 (t, 3H).

### Exemple 14: Chlorhydrate de N-tert-butyl-6-[5-oxo-4-(pyridin-3-ylmethyl)-2,5-dihydro-1H-pyrazol-1-yl]pyridine-3-sulfonamide (Composé n°98B du tableau I)

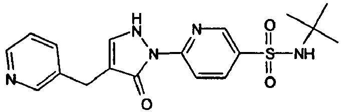

### 14.1. N-tert-butyl-6-chloropyridine-3-sulfonamide

A un mélange de 17.3mL (165 mmoles) de tertbutylamine et de 69 mL (495 mmoles) de TEA dans 330 mL de DCM, est ajouté, à 0°C, par petites portions 35 g (165 mmoles) de chlorure de 6-chloropyridine-3-sulfonyle. Après 2h d'agitation à 0°C, le milieu réactionnel est repris par 600 mL de DCM, lavé avec de l'eau (1 L) ,une solution saturée de NaHCO₃ (1L) et de la saumure (1L), séché sur Na₂SO₄, filtré et concentré sous pression réduite. On obtient 32.4 g de N-tert-butyl-6-chloropyridine-3-sulfonamide sous forme d'un solide blanc .
Rendement = 79%
RMN ¹H CDCl₃, 400MHz, δ (ppm):8,8 (s,1 H) ; 8,0(d, 1H) ; 7,4 (d, 1H);4,5 (sl,1H); 1.2 (s,9H).

### 14.2 2-formyl-3-(pyridin-3-yl)propanoate de méthyle

A une suspension de 2.1 g (90.8 mmoles) de sodium dans 55 mL d'Et₂O anh., on ajoute au goutte à goutte, à 0°C, sous argon, un mélange de 15 g (90.8 mmoles) de 3-(pyridin-3-yl)propanoate de méthyle et de 7.3 mL (57,41 mmoles) de formiate d'éthyle. Le milieu est ensuite agité 12 h à T.A, repris par 200 mL d'eau et extrait avec 100 mL d'Et₂O. La phase aqueuse est acidifiée à pH=5 puis extraite avec 2X 300 mL d'AcOEt . La phase organique est alors séchée sur Na₂SO₄, filtrée, puis concentrée sous pression réduite. Le résidu obtenu est purifié par chromatographie sur colonne de gel de silice en éluant avec un gradient DCM/MeOH de 0 à 10% de MeOH .. On obtient 4.8 g de 2-formyl-3-(pyridin-3-yl)propanoate de méthyle sour forme d'un solide blanc.
Rendement = 28%
RMN ¹H, d6-DMSO, 400MHz, δ (ppm) : 11,0 (sl, 1H); 8,45 (s, 1 H); 8,4 (d, 1 H); 7,7 (d, 1H); 7,5 (d,1H); 7,2 (dd,1H); 3,6 (s,2H); 3,5 (s, 3H)

### 14.3 N-tert-butyl-6-hydrazinylpyridine-3-sulfonamide

Selon le procédé décrit dans l'exemple 5.2, on obtient à partir de 32.4g de N-tert-butyl-6-chloropyridine-3-sulfonamide et de 12.9 mL d'hydrazine monohydrate , 22.6 g de N-tert-butyl-6-hydrazinylpyridine-3-sulfonamide sous forme d'un solide blanc .
Rendement: 71%
RMN ¹H, d6-DMSO, 400MHz, δ (ppm): 8,3 (d,2H) ;7,8 (d, 1H);7,2 (s, 1H); 7,0 (sl,1H); 4,3 (sl,2H); 1.2 (s,9H).

### 14.4. N-tert-butyl-6-[5-oxo-4-(pyridin-3-ylmethyl)-2,5-dihydro-1H-pyrazol-1-yl]pyridine-3-sulfonamide (Composé n°98A du tableau I)

Selon le procédé décrit dans l'exemple 1.3, on obtient , à partir de 12g de N-tert-butyl-6-hydrazinylpyridine-3-sulfonamide et de 9.1 g de 2-formyl-3-(pyridin-3-yl)propanoate de méthyle, 9.92 g de N-tert-butyl-6-[5-oxo-4-(pyridin-3-ylmethyl)-2,5-dihydro-1H-pyrazol-1-yl]pyridine-3-sulfonamide sous forme d'un solide blanc
RMN ¹H, d6-DMSO, 400MHz, δ (ppm) : 8.9 (sl 1H); 8,5 (sl, 2H); 8,4 (s, 1H); 8.3 (d,1H);7,9 (s, 1H); 7,8 (s, 1H); 7,7 (d, 1H); 7,3 (dd,1H); 3,6 (s,2H); 1,,2 (s, 9H) Rendement = 64%
PF(°C) =160
M = C₁₈H₂₁N₅O₃S=387 ; M+H=388; Méthode 2: Tr= 0,58min.
RMN ¹H, d6-DMSO, 400MHz, δ (ppm) : 8,8 (s,1H); 8,3(sl, 3H); 8,4(s, 1H); 8,3 (d, 1H) ;7,9 (s, 1H); 7,7(d, 1 H); 7,4 (t,1H); 3,6 (s,2H);3,4 (sl, 1H); 1,1 (s, 9H)

### 14.5 Chlorhydrate de N-tert-butyl-6-[5-oxo-4-(pyridin-3-ylmethyl)-2,5-dihydro-1H-pyrazol-1-yl]pyridine-3-sulfonamide

A 912 mg de N-tert-butyl-6-[5-oxo-4-(pyridin-3-ylmethyl)-2,5-dihydro-1H-pyrazol-1-yl]pyridine-3-sulfonamide dans 20 mL de DCM on ajoute 1 eq d'HCl (4N dans le dioxane), le milieu est ensuite concentré sous vide. On obtient 1g de chlorhydrate de N-tert-butyl-6-[5-oxo-4-(pyridin-3-ylmethyl)-2,5-dihydro-1H-pyrazol-1-yl]pyridine-3-sulfonamide sous forme d'une poudre blanche.
Rendement = 100%
PF(°C) =140°C
M = C₁₈H₂₁N₅0₃S=387 ; M+H=388; Méthode 2: Tr= 0,58min.
RMN ¹H, d6-DMSO, 400MHz, δ (ppm) : 12, 0 (sl,1H); 8.9 (sl 1H); 8,8 (s,1H); 8,7 (d, 1H); 8,5 (d, 2H); 8,3 (d, 1H); 8.0 (t,1H);7,9 (s, 1H); 7,8 (s, 1H); 3,8 (s,2H); 1,2 (s, 9H)

### Exemple 15: Acide (2-{5-[éthyl(phényl)sulfamoyl]pyridin-2-yl}-3-oxo-2,3-dihydro-1H-pyrazol-4-yl)acétique (composé 104 du tableau I)

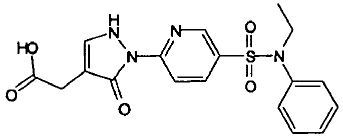

### 15.1 1 2-formylbutanedioate d'éthyle

A une suspension de 1.32 g (57.41 mmoles) de sodium dans 35 mL d'Et₂O anh., on ajoute au goutte à goutte, à 0°C, sous argon, un mélange de 10 g (57,41 mmoles) de butanedioate d'éthyle et de 4.62 mL (57,41 mmoles) de formiate d'éthyle. Le milieu est ensuite agité 12 h à T.A, repris par 100 mL d'eau et extrait avec 100 mL d'Et₂O. La phase aqueuse est acidifiée à pH=5 puis extraite avec 100 mL d'Et₂O. La phase organique est alors séchée sur Na₂SO₄, filtrée, puis concentrée sous pression réduite. Le résidu obtenu est purifié par chromatographie sur colonne de gel de silice sur gel de silice en éluant avec un mélange cyclohexane/ACOEt 7 :3. On obtient 4.3 g de 2-formylbutanediaote d'éthyle sour forme d'une huile incolore.
Rendement : 37%
RMN ¹H, RMN ¹H, CDCl₃, 400MHz, δ (ppm): 10,0 (s, 1H) ; 7,1 (d,1H) ; 4.4-4,2 (m,5H) ; 2,9 (dd, 2H) ; 1,3 (m,6H);

### 15.2 Acide (2-{5-[éthyl(phényl)sulfamoyl]pyridin-2-yl}-3-oxo-2,3-dihydro-1H-pyrazol-4-yl) acétique

Un mélange de 2,17 g (7,42 mmoles) de N-éthyl-6-hydrazino-N-phénylpyridine-3-sulfonamide et de 1,5 g (7,42 mmoles) de 2-formylbutanedioate d'éthyle dans 15 mL d'EtOH est chauffé 5h à 80°C. Le milieu est ensuite concentré sous pression réduite. Le précipité obtenu est trituré dans l'Et₂O, puis filtré et séché à la pompe. Les 4,1 g de poudre beige obtenus sont ajoutés à une solution de 212 mg (9,23 mmoles) de sodium dissous dans 17 mL de MeOH anh. à T.A. et l'agitation est poursuivie 2 h. Le milieu est concentré sous pression réduite, puis solubilisé dans 10 mL d'eau et 4 mL de soude 1 N puis agité 12 h à T.A. Le milieu est ensuite acidifié jusqu'à pH=5 à l'aide d'HCl 1 N puis extrait avec du DCM (2x100mL). Les phases organiques rassemblées sont séchées sur Na₂SO₄, filtrées, puis concentrées sous pression réduite. Le résidu obtenu est concrétisé dans du pentane, filtré et séché sous vide On obtient 2,1g d'acide (2-{5-[éthyl(phényl)sulfamoyl]pyridin-2-yl}-3-oxo-2,3-dihydro-1H-pyrazol-4-yl)acétique sous forme d'une poudre blanche.
Rendement = 58%
PF(°C) =174°C
M = C₁₈H₁₈N₄O₅S =402 ; M+H=403 ; Méthode Tr=1.02 min.
RMN ¹H, d6-DMSO, 400MHz, δ (ppm) :12,0(sl,2H); 8,6 (sl,1H); 8,5 (sl,1H); 8,2 (d,1H); 7,9 (sl, 1H); 7,4 (m,3H); 7,2 (d,2H); 3,7 (q,2H); 3,4(s,2H); 1,0 (t, 3H).

### Exemple 16: (2-{5-[éthyl(phényl)sulfamoyl]pyridin-2-yl}-3-oxo-2,3-dihydro-1H-pyrazol-4-yl)acétate de méthyle (composé 107 du tableau I)

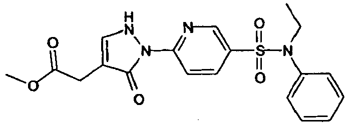

### 16.1 (2-{5-[éthyl(phényl)sulfamoyl]pyridin-2-yl}-3-oxo-2,3-dihvdro-1H-pyrazol-4-yl)acétate de méthyle

Un mélange de 0.5 g (1.24 mmoles) d' acide (2-{5-[éthyl(phényl)sulfamoyl]pyridin-2-yl}-3-oxo-2,3-dihydro-1H-pyrazol-4-yl)acétique et de 0.256 g (1.24 mmoles) de DCC est agité 3h à T.A .Le précipité formé est filtré et le filtrat est concentré sous pression réduite. Le résidu obtenu est purifié par chromatographie sur colonne de gel de silice en éluant avec un mélange DCM/MeOH 9/1. On obtient 0.5 g de 2-{5-[éthyl(phényl)sulfamoyl]pyridin-2-yl}-3-oxo-2,3-dihydro-1H-pyrazol-4-yl)acétate de méthyle sous forme d'une poudre marron.
Rendement= 96 %
PF(°C) =140°C
M = C₁₉H₂₀N₄O₅S =416 ; M+H=417 ; Méthode 2; Tr=1.44 min
RMN ¹H, d6-DMSO, 400MHz, δ (ppm) :12,0 (sl,1H); 8,6 (s,1H); 8,4 (s,1H); 8,2 (d,1H); 7,9 (s, 1H); 7,4 (m,3H); 7,2 (d, 2H); 5,6 (d,0.5H); 3,75 (q,2H); 3.7(s,3H); 3,3 (d,1.5H); 1,0 (t,3H)

### Exemple 17: 2-(2-{5-[éthyl(phényl)sulfamoyl]pyridin-2-yl}-3-oxo-2,3-dihydro-1H-pyrazol-4-yl)-N-méthylacétamide (composé 108 du tableau I)

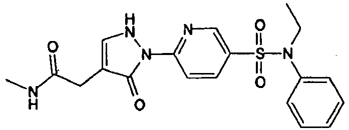

A un mélange de 0.28 g (0.7 mmoles) d'acide (2-{5-[éthyl(phényl)sulfamoyl]pyridin-2-yl}-3-oxo-2,3-dihydro-1H-pyrazol-4-yl)acétique, de 0.61 mL (3.48 mmoles) de DIEA, et de 0.8 mL (1.4 mmoles) de méthylamine(2N en solution dans le THF) dans 2 mL de DCM, est ajouté à 0°C 0.33 g ( 1.04 mmoles ) de TBTU .Après 3h d'agitation en T.A, le milieu est repris par 500 mL de DCM, lavé successivement avec HCl 0.1N (2x40 mL), une solution saturée en NaHCO₃ (2x40mL) et de la saumure (30 mL), séché sur Na₂SO₄ puis concentré sous pression réduite et purifié par chromatographie sur colonne de gel de silice en éluant avec un mélange DCM/MeOH 95:5 On obtient 18 mg de 2-(2-{5-[éthyl(phényl)sulfamoyl]pyridin-2-yl}-3-oxo-2,3-dihydro-1H-pyrazol-4-yl)-N-méthytacétamide sous forme d'une poudre marron.
Rendement = 6 %
PF(°C) =194°C
M = C₁₉H₂₁N₅O₅S =415 ; M+H=416 ; Méthode 2 Tr=0.98
RMN ¹H, d6-DMSO, 400MHz, δ (ppm) : 12,0 (sl,1H); 8,6 (sl,1H); 8,5 (s,1H); 8,2 (d,1H); 7,8 (s, 2H); 7,5 (m,3H);7,2 (d,2H); 3,6 (q,2H); 3.1(s,2H); 2.6 (s,3H); 1,0 (t,3H)

### Exemple 18: (4-benzyl-1-{5-[éthyl(phényl)sulfamoyl]pyridin-2-yl}-5-oxo-2,5-dihydro-1H-pyrazol-3-yl)acetate d'éthyle (composé 106 du tableau I)

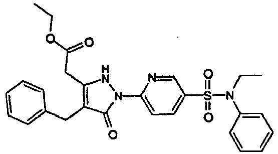

### 18.1 2-benzyl-3-oxopentanedioate de diéthyle

Sous argon,et à TA, 1.7 g (74.18 mmoles) de sodium sont dans 75 mL d'EtOH anh. On ajoute ensuite, au goutte à goutte, et à T.A, 13.5 mL (74.2 mmoles) de 3-oxopentanedioate de diéthyle puis 8.8 mL (74.2 mmoles) de bromure de benzyle. Le milieu est ensuite chauffé 3h au reflux, concentré sous pression réduite et purifié par chromatographie sur colonne de gel de silice en éluant avec un gradient cyclohexane / AcOEt de 0 à 20 % d'AcOEt. 8.29 g de 2-benzyl-3-oxopentanedioate de diéthyle, sous forme d'une huile translucide, sont obtenus.
Rendement = 38%
RMN ¹H, RMN ¹H, CDCl₃, 400MHz, δ (ppm): 7,4-7,2 (m, 5H) ; 4,2 (m,4H) ; 4,1 (t,1H) ; 3,2 (d, 2H) ; 1,2 (m,6H);

### 18.2. 4-benzyl-1-{5-[éthyl(phényl)sulfamoyl]pyridin-2-yl}-5-oxo-2,5-dihydro-1H-pyrazol-3-yl) acétate d'éthyle

Selon le procédé décrit dans l'exemple 1.3, on obtient, à partir de 5 g (17.1 mmoles) de 2-benzyl-3-oxopentanedioate de diéthyle et de 5 g (17.1 mmoles) de N-éthyl-6-hydrazino-N-phénylpyridine-3-sulfonamide, 3 g de 4-benzyl-1-{5-[éthyl(phényl)sulfamoyl] pyridin-2-yl}-5-oxo-2,5-dihydro-1H-pyrazol-3-yl) acétate d'éthyle sous forme d'une poudre blanche.
Rendement= 34%
PF(°C) =156
M = C₂₇H₂₈N₄O₅S =520 ; M+H=521 Méthode 2 : Tr=1.52,
RMN ¹H, d6-DMSO, 400MHz, δ (ppm) : 12.0 (sl,1H); 8,5 (sl,2H); 8,15 (d,1H); 7,4-7,1 (m,10 H); 4,1 (q,2H); 3,8-3,6 (m,6H); 1,2(t,3H); 1,0 (t, 3H).

### Exemple 19: 6-{4-[(5-cyanopyridin-3-yl)méthyl]-3-méthyl-5-oxo-2,5-dihydro-1H-pyrazol-1-yl}-N-éthyl-N-phenylpyridine-3-sulfonamide (composé 97 du tableau I)

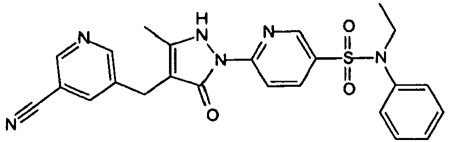

### 19.1 (5-bromopyridin-3-yl)méthanol

A une solution de 12 g (59.4 mmoles) d'acide 5-bromopyridine-3-carboxylique dans 300 mL de THF anh. sous argon, sont ajoutés à -10°C, 6.6 mL de NMM et puis 5.7 mL (59.4 mmoles) de chloroformiate d'éthyle. Après 20 min d'agitation à -10°C, 6.8 g (179.8 mmoles) de borohydrure de sodium sont ajoutés par petites portions. Le milieu est ensuite refroidi à -70°C et 400 mL de MeOH sont ajoutés en 1h30. On laisse ensuite remonter la température à TA et l'agitation est maintenue 12 h. Le milieu est ensuite concentré sous pression réduite, puis purifié par chromatographie sur colonne de gel de silice en éluant avec un mélange DCM/MeOH 98:2. On obtient 8.4 g de (5-bromopyridin-3-yl)méthanol sous forme d'une huile jaune.
Rendement = 75%
RMN ¹H, RMN ¹H, CDCl₃, 400MHz, δ (ppm): 8,5 (s, 1H) ;8,4 (s,1H); 7,9 (1H,s); 4,6 (s,2H) ; 2,8 (sl,1H)

### 19.2 5-(hydroxyméthyl)pyridine-3-carbonitrile

Un mélange de 4.2 g (22.34 mmoles) de (5-bromopyridin-3-yl)méthanol, de 5 g ( 55.84 mmoles) de cyanure de cuivre dans 22 mL de pyridine est chauffé 20 h dans un tube scellé à 160°C. Après retour à TA, le milieu est repris par 10 mL d'ammoniaque concentrée et 30 mL d'une solution saturée en NH₄Cl puis agité 2h. Le milieu est alors extrait avec 200 mL d'un mélange DCM/iPrOH (85:15), séché sur Na₂SO₄ puis concentré sous pression réduite et purifié par chromatographie sur colonne de gel de silice en éluant avec un mélange DCM/MeOH 98:2. On obtient 2,13 g de 5-(hydroxyméthyl)pyridine-3-carbonitrile sous forme d'un solide blanc.
Rendement = 51 %
RMN ¹H, RMN ¹H, CDCl₃, 400MHz, δ (ppm): 8,9 (d, 2H) ;8,0 (s,1H); 4,9 (s,2H) ; 2,3 (sl,1H)

### 19.3 5-(chlorométhyl)pyridine-3-carbonitrile

A 0.2 g (1.49 mmoles) de 5-(hydroxyméthyl)pyridine-3-carbonitrile dans 2 mL de DCM est ajouté 1 mL (4 mmoles) d'HCl 4N dans le dioxane. Le mélange est concentré sous pression réduite puis ajouté à 0.65 mL (8.95 mmoles) de chlorure de thionyle .puis chauffé 3h à 60°C. Après retour à T.A, le milieu est repris par 20 mL de toluène, le précipité formé est filtré puis traité avec dans 30 mL de DCM et 30 mL d'une solution saturée en NaHCO₃. La phase organique est séparée et séchée sur Na₂SO₄ puis concentrée sous pression réduite.161 mg de 5-(chlorométhyl)pyridine-3-carbonitrile sous forme d'huile sont obtenus.
Rendement = 73%
RMN ¹H, RMN ¹H, CDCl₃, 400MHz, δ (ppm): 8,9 (d, 2H) ;8,0 (s,1H); 3.5 (s,2H)

### 19.4 2-[(5-cyanopyridin-3-yl)méthyl]-3-oxobutanoate de méthyle

A une supension de 84 mg (2.11 mmoles) d'hydrure de sodium (60% dans l'huile) dans 3 ml de DME anh., est ajouté sous argon, à 0°C, 0.23 ml (2.11 mmoles) d' acétoacétate de méthyke . Le milieu réactionnel est agité 1/2h à 0°C et 1/2h à T.A puis on ajoute 161 mg (1.06 mmoles) de 5-(chlorométhyl)pyridine-3-carbonitrile dilué dans 1mL de DME et 29 mg (0.11 mmoles) d'iodure de tétrabutylammonium. Le milieu est ensuite chauffé à 65°C pendant 4h. Après retour à TA, le milieu est repris par 10mL d'eau, neutralisé par ajout d'HCl 0,1N, puis extrait à l'AcOEt (2x40 mL), puis séché sur Na₂SO₄ concentré sous pression réduite et purifié par chromatographie sur colonne de gel de silice en éluant avec un mélange cyclohexane /AcOEt (8 :2). On obtient 140 mg de 2-[(5-cyanopyridin-3-yl)méthyl]-3-oxobutanoate de méthyle sous forme d'une huile incolore.
Rendement = 57%
RMN ¹H, RMN ¹H, CDCl₃, 400MHz, δ (ppm): 8,8 (s, 1H) ;8,6 (s,1H); 7,8 (s,1H); 3.7(t,1H) ; 3,65 (s,3H) ; 3,2 (dd,2H); 2,2 (s,3H)

### 19.5 6{4-[(5-cyanopyridin-3-yl)méthyl]-3-méthyl-5-oxo-2,5-dihydro-1H-pyrazol-1-yl}-N-éthyl-N-phénylpyridine-3-sulfonamide

Selon le procédé décrit dans l'exemple 1.3, on obtient, à partir de 125 mg (0.43 mmoles) de 2-[(5-cyanopyridin-3-yl)méthyl]-3-oxobutanoate de méthyle et de 99 mg (0.43 mmoles) de N-éthyl-6-hydrazino-N-phénylpyridine-3-sulfonamide, 103 mg de 6-{4-[(5-cyanopyridin-3-yl)méthyl]-3-méthyl-5-oxo-2,5-dihydro-1H-pyrazol-1-yl}-N-éthyl-N-phenylpyridine-3-sulfonamide sous forme d'une poudre jaune.
Rendement= 51%
PF(°C) =164
M = C₂₄H₂₂N₆O₃S =474 ; M+H = 475; Méthode 2 : Tr= 1.24min.
RMN ¹H, d6-DMSO, 400MHz, δ (ppm) : 12,0 (sl,1H); 8.9 (s,1H); 8,8 (s,1H); 8,6 (sl,1H); 8,4 (s, 1H); 8,2 (s, 1H); 8,15 (d,1H); 7,4 (m,3 H); 7,1 (d, 2H); 3,7 (s, 4 H); 2,2 (s, 3H); 1,0 (t, 3H).

### Exemple 20: 5-méthyl-2-[5-(phénylsulfonyl)pyridin-2-yl]-4-(pyridin-3-ylméthyl)-1,2-dihydro-3H-pyrazol-3-one (composé 197du tableau II)

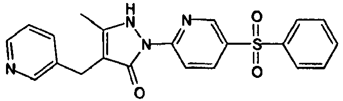

### 20.1 2-chloro-5-(phénylsulfanyl)pyridine

Un mélange de 3.4 g (14.3 mmoles) de 2-chloro-5 iodo- pyridine, de 1.9 g (17.2 mmoles) de thiophénol, de 0.93 g (17.2 mmoles) de méthylate de sodium et de 0.36 g (5.7 mmoles) de cuivre dans 18 mL de MeOH est chauffé 12 h à 80°C. Après retour à T.A, le milieu est repris par 100 mL de NaOH 1N et le MeOH est évaporé sous pression réduite. Le milieu réactionnel est extrait avec de l'AcOEt (2x100mL), la phase organique est lavée avec NaOH 0.1N (2x30 mL), puis séchée sur Na₂SO₄, concentrée sous pression réduite et purifiée par chromatographie sur colonne de gel de silice en éluant avec un mélange cyclohexane /AcOEt (8:2). On obtient 1.90 g de 2-chloro-5-(phénylsulfanyl)pyridine sous forme d'une poudre blanche.
Rendement = 60%
RMN ¹H, RMN ¹H, CDCl₃, 400MHz, δ (ppm): 8,5 (d, 1 H) ;8,3 (s,1H); 7,9 (1 H,s) ; 7.5-7.4 (m,5H)

### 20.2 2-chloro-5-(phénylsulfonyl)pyridine

A une solution de 1.9 g (8.57 mmoles) de 2-chloro-5-(phénylsulfanyl)pyridine dans 40 mL de DCM est ajouté à T.A en 1/4h une suspension de 4.8 g (21.42 mmoles) d'acide 3 -chloro perbenzoïque à 77% dans 20 mL de DCM. Après 1h d'agitation, le précipité formé est filtré, le filtrat est repris par 200 mL de DCM, lavé successivement avec 100 mL de soude 0.2N puis 100 mL d'une solution saturée en thiosulfate de sodium, séché sur Na₂SO₄, concentré sous pression réduite et purifié par chromatographie sur colonne de gel de silice en éluant avec un mélange cyclohexane /AcOEt (8 :2). On obtient 0.67 g de 2-chloro-5-(phénylsulfonyl)pyridine sous forme d'une poudre blanche.
Rendement = 31 %
RMN ¹H, RMN ¹H, CDCl₃, 400MHz, δ (ppm): 8,9 (s, 1H) ;8,1-7,9 (m,3H); 7,6-7,4 (3H,m) ; 7.3 (m,1 H)

### 20.3 2-hydrazinyl-5-(phénylsulfonyl)pyridine

Selon le procédé décrit dans l'exemple 5.2, on obtient, à partir de 0.67g (2.64 mmoles) de 2-chloro-5-(phénylsulfonyl)pyridine, 340 mg de 2-hydrazinyl-5-(phénylsulfonyl)pyridine sous forme d'une poudre blanche.
Rendement= 51 %
RMN ¹H, d6-DMSO, 400MHz, δ (ppm) : 9,0 (s,1H); 8,6 (d,1H); 8,5 (s,1H); 8,4 (m, 2H); 8,0 (d, 1H); 7,8-7,5 (m,3H); 7,3 (m,1H); 3,3(sl, 1H);

### 20.4 5-méthyl-2-[5-(phénylsulfonyl)pyridin-2-yl]-4-(pyridin-3-ylméthyl)-1,2-dihydro-3H-pyrazol-3-one

Selon le procédé décrit dans l'exemple 1.3, on obtient, à partir de 340 mg (1.36 mmoles) de 2-hydrazinyl-5-(phénylsulfonyl)pyridine et de 283 mg (1.36 mmoles) de 3-oxo-2-(pyridin-3-ylméthyl)butanoate de méthyle, 130 mg de 5-méthyl-2-[5-(phénylsulfonyl)pyridin-2-yl]-4-(pyridin-3-ylméthyl)-1,2-dihydro-3H-pyrazol-3-one sous forme d'une poudre blanche .
Rendement=23%
PF(°C) =176
M = C₂₁H₁₈N₄O₃S= 406 ; M+H = 407; Méthode 2 : Tr=0.78 min,.
RMN ¹H, d6-DMSO, 400MHz, δ (ppm) 12,0 (sl,1H); 9,0 (s,1H); 8,6 (d,1H); 8,5 (s,1H); 8,4 (m, 2H); 8,0 (d,2H); 7,8-7,6 (m,4H); 7,3 (m,1H); 3,6 (s, 2H); 2,2 (s, 3H);

### Exemple 21: Chlorhydrate de N-éthyl-6-{4-[(5-méthoxypyridin-3-yl)méthyl]-5-oxo-2,5-dihydro-1H-pyrazol-1-yl}-N-phénylpyridine-3-sulfonamide (composé 101 du tableau I)

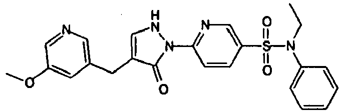

### 21.1 (2E)-3-(5-méthoxypyridin-3-yl)prop-2-énoate de méthyle

A une suspension de 3.12 g (78.1 mmoles) d'hydrure de sodium (60% dans l'huile) dans 30 mL de THF anh. est ajouté, en 45 min, sous argon et à 0°C, 16.4 g (78.1 mmoles) de (diéthoxyphosphoryl)acétate de méthyle dans 10 mL de THF. L'agitation est maintenue 1/2h à 0°C puis 5.1 g (37.2 mmoles) de 5-méthoxypyridine-3-carbaldéhyde dans 20 mL de THF anh. sont additionnés au goutte à goutte à 0°C. Après retour à TA, le mélange réactionnel est traité avec 150 mL d'eau, puis extrait avec de l'AcOEt (3X100 mL). Les phases organiques sont rassemblées, lavées successivement avec de l'eau (2x20 mL), séchées sur Na₂SO₄, filtrées, puis concentrées sous pression réduite. Le résidu obtenu est purifié par chromatographie sur colonne de gel de silice en éluant avec un gradient cyclohexane / AcOEt de 0 à 40 % d'AcOEt. On obtient 1g de 1 (2E)-3-(5-méthoxypyridin-3-yl)prop-2-énoate de méthyle sous forme d'une poudre blanche.
Rendement = 14%.
RMN ¹H, CDCl₃, 400MHz, δ (ppm) : 8,4 (d,1H); 7,7 (d,1H); 7,4 (s, 1H); 6,5 (d,1H); 3,9 (s, 3H); 3,8 (s, 3H)

### 21.2 3-(5-méthoxypyridin-3-yl)propanoate de méthyle

Dans un appareillage de Parr, un mélange de 1 g (5.33 mmoles) de (2E)-3-(5-méthoxypyridin-3-yl)prop-2-énoate de méthyle dans 20 mL de MeOH et 0.1 g de Pd/C 10% est hydrogéné sous 7 bars pendant 5h . Le mélange réactionnel est ensuite filtré sur papier Whatman GF/F et concentré sous pression réduite. On obtient ainsi 1 g de 3-(5-méthoxypyridin-3-yl)propanoate de méthyle sous forme de cire utilisée tel quel dans l'étape suivante.
Rendement = 100%
RMN ¹H, CDCl₃, 400MHz, δ (ppm) :8,3 (s, 1H); 8,2 (s, 1H):; 7,1 (s, 1H); 3,85 (s,3H); 3,6 (s, 3H); 3,0 (t, 2H); 2,7 (t, 3H).

### 21.3 2-formyl-3-(5-méthoxypyridin-3-yl)propanoate de méthyle

Selon le procédé décrit dans l'exemple 14.2, on obtient, à partir de 1.04 g (5.33 mmoles) de 3-(5-méthoxypyridin-3-yl)propanoate de méthyle, 600 mg de 2-formyl-3-(5-méthoxypyridin-3-yl)propanoate de méthyle sous forme d'une cire utilisée telle quelle dans l'étape suivante.
Rendement= 51%

### 21.4 Chlorhydrate de N-éthyl-6-{4-[(5-méthoxypyridin-3-yl)méthyl]-5-oxo-2,5-dihydro-1H-pyrazol-1-yl}-N-phénylpyridine-3-sulfonamide

Selon le procédé décrit dans l'exemple 2.3, on obtient, à partir de 250 mg (1.12 mmoles) de 2-formyl-3-(5-méthoxypyridin-3-yl)propanoate de méthyle et de 327 mg (1.12 mmoles) de N-éthyl-6-hydrazino-N-phénylpyridine-3-sulfonamide, 131 mg de chlorhydrate de N-éthyl-6-{4-[(5-méthoxypyridin-3-yl)méthyl]-5-oxo-2,5-dihydro-1H-pyrazol-1-yl}-N-phénylpyridine-3-sulfonamide sous forme d'un lyophilisat.
Rendement= 25%
PF(°C) =136
M = C₂₃H₂₃N₅O₄S= 465 ; M+H = 466; Méthode 2 : Tr= 0.97 min,.
RMN ¹H, d6-DMSO, 400MHz, δ (ppm) :11,0 (sl, 1H); 8.6 (s,2H); 8,4(d,2H); 8,2 (d,1H); 8,1 (s, 1 H); 7,9 (s,1H); 7,4 (m,3H); 7,1 (d, 2H); 4,0 (s, 3 H); 3,8 (s, 2H); 3,6 (q, 2H); 1,0 (t,3H)

### Exemple 22: {2-[5-(tert-butylsulfamoyl)pyridin-2-yl]-3-oxo-2,3-dihydro-1H-pyrazol-4-yl}(phényl)acétate de méthyle (composé 113 du tableau I)

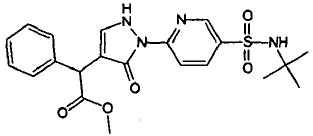

### 22.1. 3-cyano-3-phénylpropanoate d'éthyle

Un mélange de 64 g (257.8 mmoles) de benzylidènepropanedioate de diéthyle, de 17 g (261 mmoles) de cyanure de potassium dans 750 mL d'EtOH et 75 ml d'eau est chauffé 18h à 60°C. Le milieu est ensuite concentré sous pression réduite et repris par 500 mL de saumure puis extrait à l'Et₂O (2x500mL). Les phases organiques sont séchées sur Na₂SO₄, filtrées, puis concentrées sous pression réduite. 43.5 g de 3-cyano-3-phénylpropanoate d'éthyle sous forme d'un solide sont obtenus et engagés tel quel dans l'étape suivante.
Rendement = 83%

### 22.2. Acide 2-phénylbutanedioïque

Un mélange de 43.5 g (214 mmoles) de 3-cyano-3-phénylpropanoate d'éthyle, de 52.2 g (930.4 mmoles) de potasse dans 670 mL d'EtOH est chauffé au reflux pendant 4h. Après retour à T.A, le milieu est concentré sous vide puis traité avec 1 L d'HCl 1 N, il se forme alors un précipité qui est filtré et rincé avec de l'eau (2x50 mL). Le solide obtenu est repris dans un mélange de 200 mL de toluene et 40 mL d'EtOH et concentré sous pression réduite puis séché à la pompe. 37 g d'acide 2-phénylbutanedioïque sous forme d'un solide sont obtenus et engagés tel quel dans l'étape suivante.
Rendement = 89%

### 22.3 2-phénylbutanedioate de diéthyle

Dans un appareillage à Dean-Stark, un mélange de 37 g (190.5 mmoles) d'acide 2-phénylbutanedioïque de 6 mL d'H₂SO₄ conc, de 80 mL de toluène et 80 mL d'EtOH est chauffé au reflux pendant 72h. Après retour à TA, le mélange réactionnel est concentré sous pression réduite puis traité avec 300 mL d'eau, puis extrait avec de l'Et₂O (2x400 mL). Les phases organiques sont rassemblées, séchées sur Na₂SO₄, filtrées, puis concentrées sous pression réduite. Le résidu obtenu est purifié par chromatographie sur colonne de gel de silice en éluant avec un gradient cyclohexane / AcOEt de 0 à 10 % d'AcOEt, 22 g de 2-phénylbutanedioate de diéthyle sous forme d'une huile sont obtenus.
Rendement =46%.
RMN ¹H, CDCl₃, 400MHz, δ (ppm) : 7,5 (m, 5H); 4,2 (m, 5H); 3,0 (dd,2H; 1,0 (t,6H)

### 22.4 2-formyl-3-phénylbutanedioate de diéthyle

Selon le procédé décrit dans l'exemple 14.2, on obtient, à partir de 7.0 g (28 mmoles) de 2-phénylbutanedioate de diéthyle, 7.0 g de 2-formyl-3-phénylbutanedioate de diéthyle sous forme d'une huile.
Rendement= 89%

### 22.5 {2-[5-(tert-butylsulfamoyl)pyridin-2-yl]-3-oxo-2,3-dihydro-1H-pyrazol-4-yl}(phényl)acétate d'éthyle

Selon le procédé décrit dans l'exemple 27.2, on obtient, à partir de 2 g (7.19 mmoles) de 2-formyl-3-phénylbutanedioate de diéthyle et de 1.75 g (7.19 mmoles) de N-tert-butyl-6-hydrazinylpyridine-3-sulfonamide, 3.1 g de {2-[5-(tert-butylsulfamoyl)pyridin-2-yl]-3-oxo-2,3-dihydro-1H-pyrazol-4-yl}(phényl)acétate de méthyle sous forme d'une poudre
Rendement= 93%
RMN ¹H, d6-DMSO, 400MHz, δ (ppm) : 8.6 (s,1H); 8,6 (d,1H); 8,0 (d,1H); 7,7(s, 2H); 7,3 (m , 5H); 4,6 (s, 1 H); 4,1 (q, 2H); 1,0 (m,12H) ;

### 22.6 1-[5-(tert-butylsulfamoyl)pyridin-2-yl]-4-[carboxy(phényl)methyl]-1H-pyrazol-5-olate

Un mélange de 2.8 g (5,8 mmoles de 2-[5-(tert-butylsulfamoyl)pyridin-2-yl]-3-oxo-2,3-dihydro-1H-pyrazol-4-yl}(phényl)acétate d'éthyle, de 5.8 mL (5.8 mmoles) de NaOH 1N, dans 12 mL d'EtOH est agité 5h à T.A . Puis 5.8 mL d'HCl 1N sont ajoutés, et le milieu est extrait au DCM (2x100 mL). Les phases organiques sont séchées sur Na₂SO₄, filtrées, puis concentrées sous pression réduite. On obtient 2 g de -[5-(tert-butylsulfamoyl)pyridin-2-yl]-4-[carboxy(phényl)méthyl]-1H-pyrazol-5-olate sous forme de poudre beige qui sont engagés tel quel dans l'étape suivante.

### 22.7 {2-[5-(tert-butylsulfamoyl)pyridin-2-yl]-3-oxo-2,3-dihydro-1H-pyrazol-4-yl}(phényl) acétate de méthyle

A un mélange de 0.3 g (0.66 mmoles) de 1-[5-(tert-butylsulfamoyl)pyridin-2-yl]-4-[carboxy(phényl)méthyl]-1H-pyrazol-5-olate dans 3 mL de MeOH anh. on ajoute à 0°C, 0.11 mL(0.73 mmoles) de chlorure de thionyle. Après retour à T.A, l'agitation est maintenue 12h. Le milieu est repris par 40 mL de DCM, lavé avec 20 mL d'une solution saturée en NaHCO₃, séché sur Na₂SO₄ puis concentré sous pression réduite. Le résidu obtenu est concrétisé dans un mélange DCM/pentane puis filtré et séché sous vide. On obtient 0.67 g 2-[5-(tert-butylsulfamoyl)pyridin-2-yl]-3-oxo-2,3-dihydro-1H-pyrazol-4-yl}(phényl)acétate de méthyle sous forme d'une poudre beige.
Rendement= 73%
PF(°C) =66
M = C₂₁H₂₄N₄O₅S= 444 ; M+H = 445; Méthode 2 : Tr= 1.21 min,
RMN ¹H, d6-DMSO, 400MHz, δ (ppm) :12,3 (sl,1H); 8,8 (s,1H); 8.6 (sl,1H); 8,3 (d,1H); 7,7 (d, 2H); 7,4 (m,5H); 4,9 (s, 1H); 3,7 (s, 3 H);1,0 (t,9H)

### Exemple 23: Chlorhydrate de N-cyclopentyl-N-({6-[5-oxo-4-(pyridin-3-ylméthyl)-2,5-dihydro-1H-pyrazol-1-yl]pyridin-3-yl}sulfonyl)glycinate de méthyle (composé 128 du tableau I)

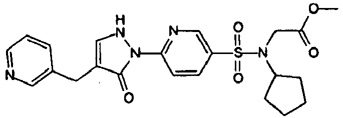

### 23.1. 6-chloro-N-cyclopentylpyridine-3-sulfonamide

Selon le procédé décrit dans l'exemple 14.1, on obtient, à partir de 5 g (23.6 mmoles) de chlorure de 6 chloropyridine -3 sulfonyle et de 2 g (23.6 mmoles) de cyclopentylamine, 5.1 g de 6-chloro-N-cyclopentylpyridine-3-sulfonamide sous forme d'un solide marron.
Rendement= 84%
RMN ¹H, CDCl₃, 400MHz, δ (ppm) : 8,8 (s, 1H); 8,0 (d, 1H); 7,4 (d,1H) ; 4.5 (d,1H); 3,6 (m,1H); 1,8 (m,2H);1,6 (m,4H); 1.3 (m,2H)

### 23.2. N-[(6-chloropyridin-3-yl)sulfonyl]-N-cyclopentylglycinate de méthyle

Un mélange de 2 g (7.67 mmoles) de 6-chloro-N-cyclopentylpyridine-3-sulfonamide, 0.7 mL(7.67 mmoles) de bromoacétate de méthyle et de 1.2 g (8.4 mmoles) de K₂CO₃ dans 15 mL de CH₃CN est chauffé 12 h à 80°C. Après retour à T.A, le milieu est filtré et le filtrat est concentré. Le résidu est repris par 100 mL de DCM, lavé successivement avec 50 mL d'une solution saturée en NaHCO₃, 50 mL d'eau puis la phase organique est séchée sur Na₂SO₄, concentrée sous pression réduite et purifiée par chromatographie sur colonne de gel de silice en éluant avec un mélange heptane/AcOEt 8 :2 . 2.4 g de N-[(6-chloropyridin-3-yl)sulfonyl]-N-cyclopentylglycinate de méthyle sous forme d'huile sont obtenus.
Rendement= 93%
RMN ¹H, CDCl₃, 400MHz, δ (ppm) 9,0 (s, 1H); 8,3 (d, 1H); 7,4 (d,1H) ; 4,1 (s, 2H); 4.,05 (m,1H); 3,8 (s,3H); 1,9 (m,2H);1,6 (m,4H); 1.3 (m,2H)

### 23.3 N-cyclopentyl-N-[(6-hydrazinylpyridin-3-yl)sulfonyl]glycinate de méthyle

Selon le procédé décrit dans l'exemple 5.2, on obtient, à partir de 2.4 g (7.2 mmoles) de N-[(6-chloropyridin-3-yl)sulfonyl]-N-cyclopentylglycinate de méthyle, 2 g de N-cyclopentyl-N-[(6-hydrazinylpyridin-3-yl)sulfonyl]glycinate de méthyle sous forme d'un solide jaune.
Rendement= 85%
RMN 1 H, CDCl3, 400MHz, δ (ppm) 8,6 (s, 1H); 8,0 (d, 1H); 6,8 (d,1H) ; 6,6 (sl,1H); 4.,05 (m,1H); 4,0 (s, 2H); 3,6 (s,3H); 1,8 (m,2H);1,6 (m,4H); 1.2 (m,2H)

### 23.4 Chlorhydrate de N-cyclopentyl-N-({6-[5-oxo-4-(pyridin-3-ylméthyl)-2,5-dihydro-1H-pyrazol-1-yl]pyridin-3-yl}sulfonyl)glycinate de méthyle

Selon le procédé décrit dans l'exemple 2.3, on obtient, à partir de 200 mg (0.61 mmoles) de N-cyclopentyl-N-[(6-hydrazinylpyridin-3-yl)sulfonyl]glycinate de méthyle et de 117 mg (0.61 mmoles) de 3-oxo-2-(pyridin-3-ylméthyl)butanoate de méthyle, 130 mg de chlorhydrate de N-cyclopentyl-N-({6-[5-oxo-4-(pyridin-3-ylméthyl)-2,5-dihydro-1H-pyrazol-1-yl]pyridin-3-yl}sulfonyl)glycinate de méthyle sous forme d'un lyophilisat blanc.
Rendement=34%
PF(°C) =100
M = C₂₂H₂₅N₅O₅S= 471 ; M+H = 472; Méthode 2 : Tr=0.87 min .
RMN ¹H, d6-DMSO, 400MHz, δ (ppm) 8,7 (d,2H); 8,6 (d,1H); 8,4 (d,2H); 8,3 (d,1H); 7,8 (m, 1H); 7,7 (s,1H); 5,0-4,0 (sl,2H); 3,9 (m,1H); 3,8 (s, 2H); 3,6 (s, 2H); 3,4 (s,3H); 1,4-1,0 (m, 8H).

### Exemple 24: 6-[5-oxo-4-(pyridin-3-ylméthyl)-2,5-dihydro-1H-pyrazol-1-yl]pyridine-3-carboxylate de 2,2-diméthylpropyle (composé 198 du tableau II)

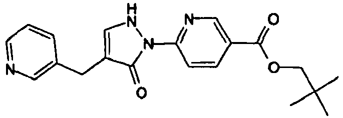

### 24.1. 6-chloropyridine-3-carboxylate de 2,2-diméthylpropyle

A une solution de 10 g (56.8 mmoles) de chlorure de 6-chloropyridine-3-carbonyle dans 100 mL de toluène anh. sont ajoutés sous argon à T.A, 15 g (170.4 mmoles) de 2,2-diméthylpropanol. Le milieu réactionnel est chauffé ensuite 6h à 80°C. Après retour à T.A, le milieu est concentré et le résidu obtenu est repris par 800 mL d'AcOEt, lavé successivement avec de l'eau (2x200mL), une solution saturée en NaHCO₃ (2x200mL) et de la saumure (100mL), séché sur Na₂SO₄, puis concentré sous pression réduite et purifié par chromatographie sur colonne de gel de silice en éluant avec un gradient cyclohexane / AcOEt de 0 à 5% d'AcOEt. On obtient 11.9 g de 6-chloropyridine-3-carboxylate de 2,2-diméthylpropyle sous forme d'une poudre blanche.
Rendement= 92%.
RMN ¹H, CDCl₃, 400MHz, δ (ppm) : 7,5 (m, 5H); 4,2 (m, 5H); 3,0 (dd,2H; 1,0 (t,6H)

### 24.2. 6-hydrazinylpyridine-3-carboxylate de 2,2-diméthylpropyle

Selon le procédé décrit dans l'exemple 5.2, on obtient, à partir de 11,9 g (52.26 mmoles) de 6-chloropyridine-3-carboxylate de 2,2-diméthylpropyle, 4.3 g de 6-hydrazinylpyridine-3-carboxylate de 2,2-diméthylpropyle sous forme d'une poudre blanche.
Rendement= 37%
RMN ¹H, d6-DMSO, 400MHz, δ (ppm) : 8,7 (s, 1H) ; 8,15 (d,1 H) ; 6,9 (d, 1 H) ; 4,0 ( s,2H); 3.5 (sl, 1H); 1,0 (s, 9H).

### 24.3. 6-[5-oxo-4-(pyridin-3-ylméthyl)-2,5-dihydro-1H-pyrazol-1-yl]pyridine-3-carboxylate de 2,2-diméthylpropyle

Selon le procédé décrit dans l'exemple 1.3, on obtient, à partir de 0.3 g (1.34 mmoles) de 6-hydrazinylpyridine-3-carboxylate de 2,2-diméthylpropyle et de 0.26 g (1.34 mmoles) de 3-oxo-2-(pyridin-3-ylméthyl)butanoate de méthyle, 185 mg de 6-[5-oxo-4-(pyridin-3-ylméthyl)-2,5-dihydro-1H-pyrazol-1-yl]pyridine-3-carboxylate de 2,2-diméthylpropyle sous forme d'un solide blanc.
Rendement=38%
PF(°C) =160
M = C₂₀H₂₂N₄O₃= 366 ; M+H = 367; Méthode 2 : Tr=1.01 min .
RMN ¹H, d6-DMSO, 400MHz, δ (ppm) :12,0 (sl, 1H); 8,9 (s,1H); 8,8 (s,1H); 8,7 (d,1H); 8,5 (d,1H); 8,4 (d,1H); 8,3(d,1H); 8,0 (t, 1H) 7,9(s, 1H); 3,9 (s,2H); 3,8 (s, 2 H); 1,0 (s, 9H);

### Exemple 25: 3-(2-{5-[cyclopentyl(méthyl)sulfamoyl]pyridin-2-yl}-3-oxo-2,3-dihydro-1H-pyrazol-4-yl)-3-phénylpropanoate de méthyle (composé 121 du tableau I)

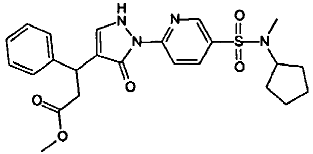

### 25.1. 3-phénylpentanedioate de diéthyle

Un mélange de 6 g (28.8 mmoles) d'acide 3-phénylpentanedioïque et de 7,9 mL (109.5 moles) de chlorure de thionyle est chauffé à 80°C pendant 1h. Le milieu est ensuite concentré et le solide obtenu est ajouté par petites portions à 8 mL d'EtOH à 0°C. Le mileu est ensuite chauffé à 80°C pendant 1/2h. Après retour à T.A, le milieu est concentré et le résidu obtenu est repris par 400 mL de DCM, lavé successivement avec une solution saturée en NaHCO₃ (2x100mL) et de la saumure (100mL), séché sur Na₂SO₄ puis concentré sous pression réduite. On obtient 6.97 g de 3-phénylpentanedioate de diéthyle sous forme d'une poudre qui est engagé tel quel dans l'étape suivante.
Rendement =91.5%.

### 25.2. 2-formyl-3-phénylpentanedioate de diéthyle

Selon le procédé décrit dans l'exemple 14.2, on obtient, à partir de 3 g (11.35 mmoles) de 3-phénylpentanedioate de diéthyle, 0.23 g de 2-formyl-3-phénylpentanedioate de diéthyle sous forme d'une huile jaune.
Rendement= 7%
RMN ¹H, d6-DMSO, 400MHz, δ (ppm) 10.9 (s, 1H); 7,7 (d, 1H); 7,3-7,1 (m,5H) ; 4,5 (t,1H); 4.,0 (q,4H); 3,0 (m, 2H); 1;0 (m,6H)

### 25.3. 3-(2-{5-[cyclopentyl(méthyl)sulfamoyl]pyridin-2-yl}-3-oxo-2,3-dihydro-1H-pyrazol-4-yl)-3-phénylpropanoate

Selon les procédés 22.5 et 22.6 , on obtient, à partir de 0.23 g (0.79 mmoles de 2-formyl-3-phenylpentanedioate de diéthyle et de 0.21 g (0.79mmoles) de N-cyclopentyl-6-hydrazinyl-N-methylpyridine-3-sulfonamide, 0.38 g de 3-(1-{5-[cyclopenty l(méthyl)sulfamoyl] pyridin-2-yl}-5-oxido-1H-pyrazol-4-yl)-3-phénylpropanoate sous forme d'une poudre.
Rendement= 89%

### 25.4. 3-(2-{5-[cyclopentyl(méthyl)sulfamoyl]pyridin-2-yl}-3-oxo-2,3-dihydro-1H-pyrazol-4-yl)-3-phénylpropanoate de méthyle

Selon le procédé décrit dans l'exemple 22.7, on obtient, à partir de 0.38 g (0.78 mmoles de 3-(2-{5-[cyclopentyl(méthyl)sulfamoyl]pyridin-2-yl}-3-oxo-2,3-dihydro-1H-pyrazol-4-yl)-3-phénylpropanoate, 0.34 g de 3-(1-{5-[cyclopentyl(methyl)sulfamoyl]pyridin-2-yl}-5-oxido-1H-pyrazol-4-yl)-3-phénylpropanoate de méthyle.
PF(°C) =80
M = C₂₄H₂₈N₄O₅S= 484 ; M+H = 485 ; Méthode 3 : Tr=4.4 min .
RMN ¹H, d6-DMSO, 400MHz, δ (ppm) : 8,9 (s, 1H); 8,7 (sl, 1H); 8,5 (d,1H); 7,9 (s,1H); 7,4 (d, 1H); 7,3(t,3H); 7,2 (t,2H); 4,4 (t,1H); 4,3 (t, 1H); 3,6 (s, 3 H); 3,2 (dd, 1H);3.1 (dd, 1H); 2,7 (s, 3H); 1,8-1,4 (m, 8H)

### Exemple 26: Chlorhydrate de 6-(4-benzyl-5-oxo-2,5-dihydro-1H-pyrazol-1-yl)-N-cyclopentyl-N-[3-(diméthylamino)propyl]pyridine-3-sulfonamide (composé 123 du tableau I)

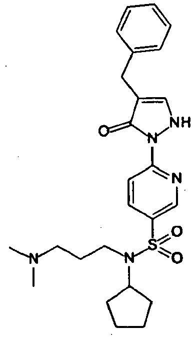

### 26.1. N-[3-(benzyloxy)propyl]-6-chloro-N-cyclopentylpyridine-3-sulfonamide

Un mélange de 1 g (3.84 mmoles) de 6-chloro-N-cyclopentylpyridine-3-sulfonamide, 1.32 g (9.59 mmoles) de K₂CO₃ et de 0.88 mL (4.99 mmoles) de [(3-bromopropoxy)méthyl]benzène dans 8 mL de DMF anh. est chauffé 12 h à 40°C. Après retour à T.A, le milieu est repris par 300 mL de d'AcOEt, lavé successivement avec de l'eau (2x100mL) une solution saturée en NaHCO₃ (100mL) et de la saumure (100mL), séché sur Na₂SO₄ puis concentré sous pression réduite et purifié par chromatographie sur colonne de gel de silice en éluant avec un gradient cyclohexane / AcOEt de 0 à 10 % d'AcOEt. On obtient ainsi 1.65 g de N-[3-(benzyloxy)propyl]-6-chloro-N-cyclopentylpyridine-3-sulfonamide sous forme d'huile.
Rendement = 99%.
RMN ¹H, CDCl₃, 400MHz, δ (ppm) : 8.85 (s, 1H); 8.1 (d,1H); 7,5 (d, 1H); 7,4-7,3 (m, 5H); 4,5 (s, 2H); 4,2 (m,1H); 3,6 (t,2H); 3,2 (dd,2H) ;2,1 (m, 2H); 1,6-1.3 (m.8H)

### 26.2. N-[3-(benzyloxy)propyl]-N-cyclopentyl-6-hydrazinylpyridine-3-sulfonamide

Selon le procédé décrit dans l'exemple 5.2, on obtient, à partir de 1.55 g (3.79 mmoles) de N-[3-(benzyloxy)propyl]-6-chloro-N-cyclopentylpyridine-3-sulfonamide, 1.5 g de N-[3-(benzyloxy)propyl]-N-cyclopentyl-6-hydrazinylpyridine-3-sulfonamide sous forme d'un solide jaune.
Rendement= 90%
RMN ¹H, CDCl₃, 400MHz, δ (ppm) : 8.6 (s, 1 H); 8,4 (sl, 1H); 7,95 (d,1H); 7,85 (d, 1H); 7,5-7,3 (m, 5H); 6,9 (d, 1H); 6,6 (sl, 1H); 4,5 (s, 2H); 4,2 (m,1H); 3,6 (t,2H); 3,2 (dd,2H) ;2,1 (m, 2H); 1,6-1.3 (m,8H)

### 26.3. 6-(4-benzyl-5-oxo-2,5-dihydro-1H-pyrazol-1-yl)-N-[3-(benzyloxy)propyl]-N-cyclopentylpyridine-3-sulfonamide

Selon le procédé décrit dans l'exemple 1.3, on obtient, à partir de 0.19 g de 2-benzyl-3-oxopropanoate de méthyle et de 0.4 g de N-[3-(benzyloxy)propyl]-N-cyclopentyl-6-hydrazinylpyridine-3-sulfonamide, 0.29 g de 6-(4-benzyl-5-oxo-2,5-dihydro-1H-pyrazol-1-yl)-N-[3-(benzyloxy)propyl]-N-cyclopentylpyridine-3-sulfonamide sous forme d'un solide beige.
Rendement= 55%
RMN ¹H, d6-DMSO, 400MHz, δ (ppm) : 12,0 (sl,1H); 8,8 (s,1H); 8,6 (sl,1H); 8,4 (d,1H); 7,7 (s,1H); 7,4-7,1 (m,10H); 4,5 (s, 2H); 4,2 (m, 1H);3,6 (s, 2H); 3.5(t,2H); 3,2 (t, 2H); 2,0 (m,2H); 1,6-1,2 (6H).

### 26.4. 6-(4-benzyl-5-oxo-2,5-dihydro-1H-pyrazol-1-yl)-N-cyclopentyl-N-(3-bydroxypropyl)pyridine-3-sulfonamide

A une solution de 150 mg (0.27 mmoles de 6-(4-benzyl-5-oxo-2,5-dihydro-1H-pyrazol-1-yl)-N-[3-(benzyloxy)propyl]-N-cyclopentylpyridine-3-sulfonamide dans 2 mL DCM, on ajoute à -78 °C, sous argon, au goutte à goutte , 0.82 mL (0.82 mmoles) de tribromure de bore (1M dans le DCM). L'agitation est maintenue 1H à -78°C puis 2mL de MeOH sont additionnés à 0°C. Le milieu est repris par 40 mL de de DCM, lavé successivement avec une solution saturée en NaHCO₃ (30mL) et de la saumure (30 mL), séché sur Na₂SO₄ puis concentré sous pression réduite et purifié par chromatographie sur colonne de gel de silice en éluant avec un mélange DCM/MeOH 9 :1.On obtient ainsi 106 mg de 6-(4-benzyl-5-oxo-2,5-dihydro-1H-pyrazol-1-yl)-N-cyclopentyl-N-(3-hydroxypropyl)pyridine-3-sulfonamide sous forme de poudre..
Rendement= 85%
RMN ¹H, d6-DMSO, 400MHz, δ (ppm) : 8,8 (s,1H); 8,6 (sl,1H); 8,4 (d,1H); 7,7 (s,1H); 7,4 (m,5H); 7,3 (m, 1 H); 4,5 (t, 1H); 4,2 (m, 1H);3,6 (s, 2H); 3.5(q,2H); 3,2 (t, 2H); 1,8 (m,2H); 1,5-1,2 (6H).

### 26.5. Chlorhydrate de 6-(4-benzyl-5-oxo-2,5-dihydro-1H-pyrazol-yl)-N-cyclopentyl-N-[3-(diméthylamino)propyl]pyridine-3-sulfonamide

A une solution de 82 mg (0.18 mmoles) de 6-(4-benzyl-5-oxo-2,5-dihydro-1H-pyrazol-1-yl)-N-cyclopentyl-N-(3-hydroxypropyl)pyridine-3-sulfonamide et de 50µL (0.36 mmoles) de NEt₃ dans 0.5 mL de DCM anh. on ajoute , sous argon, à 0°C, 27µl(0.36 mmoles) de chlorure de mésyle; on laisse progressivement remonter la température jusqu'à T.A et l'agitation est poursuivie 1h. Le milieu est repris par 20 mL de de DCM, lavé successivement avec de l'eau (2x10mL) et de la saumure (20 mL), séché sur Na₂SO₄ puis concentré sous pression réduite. Dans un tube scellé, le résidu obtenu (110 mg d'huile jaune) est repris dans 2mL de DCM puis traité 1 min par un courant de diméthylamine est mise à barboter dans la solution pendant 1 min. Le milieu est ensuite avant d'être chauffé 11h à 60°C. Après retour à T.A, le milieu est concentré, le résidu obtenu est trituré dans un mélange Et₂O/CH₃CN. Le précipité formé est filtré, rincé au pentane séché sous pression réduite puis lyophilisé après ajout d'1eq d'HCl 1N. On obtient ainsi 57 mg de chlorhydrate de 6-(4-benzyl-5-oxo-2,5-dihydro-1H-pyrazol-1-yl)-N-cyclopentyl-N-[3-(diméthylamino) propyl] pyridine-3-sulfonamide sous forme de lyophilisat.
Rendement= 88%
PF(°C) =230
M = C₂₅H₃₃N₅O₃S= 483; M+H = 484 ; Méthode 2 : Tr =1.1 min
RMN ¹H, d6-DMSO, 400MHz, δ (ppm) :12,0 (sl,1H); 10,8 (sl,1H); 8,9 (s,1H); 8,8 (s, 1 H); 8,5 (d,1H); 7,8 (s,1H); 7,4 (m, 5H); 7,3 (m, 1 H); 4,5 (m,1H); 4,2 (m,1H); 3,6 (s, 2H); 3.3(t,2H); 3,2 (q, 2H); 2,7 t(s, 6H) ; 2,0 (m,2H); 1,6-1,3 (6H).

### Exemple 27: 6-(4-benzyl-5-oxo-2,5-dihydro-1H-pyrazol-1-yl)-N-éthyl-N-phénylpyridine-3-sulfonamide (Composé n°93 du tableau I)

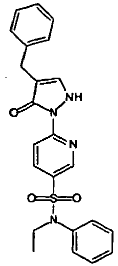

### 27.1. 2-benzyl-3-oxopropanoate de méthyle

A un mélange de 3 g (18,3 mmoles) de 3-phénylpropanoate de méthyle et de 3,57 mL (54,8 mmoles) de formiate de méthyle dans 36 mL de toluène, sous argon sont ajoutés successivement au goutte à goutte 54,8 mL (54,8 mmoles) d'une solution 1 M de TiCl₄ dans le toluène, 0,17 mL (0,91 mmoles) de triméthylsilyltrifluorométhanesulfonate et 19,6 mL (82,2 mmoles) de tributylamine. Le milieu est ensuite chauffé 2h30 à 60°C puis agité 12 h à T.A.

Le milieu réactionnel est hydrolysé avec 200 mL d'eau, extrait avec 200 mL d'Et₂O. La phase organique est séchée sur Na₂SO₄, filtrée et concentrée sous pression réduite. On obtient 3,12 g de 2-benzyl-3-oxopropanoate de méthyle sous forme d'huile utilisée telle quelle à l'étape suivante.
Rendement = 87%

### 27.2. 6-(4-benzyl-5-oxo-2,5-dihydro-1H-pyrazol-1-yl)-N-éthyl-N-phénylpyridine-3-sulfonamide

Un mélange de 0,38 g (1,3 mmoles) de N-éthyl-6-hydrazino-N-phénylpyridine-3-sulfonamide et de 0,25 g (1,3 mmoles) de 2-benzyl-3-oxopropanoate de méthyle dans 2 mL d'un mélange AcOH/MeOH (1 :1) est chauffé 4h à 80°C. Le milieu est ensuite concentré sous pression réduite, le résidu obtenu est concrétisé dans un mélange Et₂O/pentane (1 :1), puis filtré et séché sous pression réduite. A TA, les 440 mg de solide obtenus sont ensuite ajoutés par petites portions à une solution de 22 mg (0,96 mmoles) de sodium dans 1 mL de MeOH puis l'agitation est poursuivie 3h à T.A. agité. Le mélange réactionnel est ensuite concentré sous pression réduite, repris par 10 mL d'eau puis acidifié jusqu'à pH 3-4 par ajout d'AcOH. Le précipité obtenu est alors filtré, lavé au pentane puis recristallisé dans l'EtOH et séché. On obtient ainsi 245 mg de 6-(4-benzyl-5-oxo-2,5-dihydro-1H-pyrazol-1-yl)-N-éthyl-N-phénylpyridine-3-sulfonamide sous forme de poudre blanche.
Rendement = 59%
PF(°C) =180
M = C₂₃H₂₂N₄O₃S = 434 ; M+H = 435; Méthode 2: Tr=1,35 min.
RMN ¹H, d6-DMSO, 400MHz, δ (ppm): 12 (sl,1H); 8,6 (sl,1H); 8,5 (s,1H); 8,1 (d,1H) 7,7 (s,1H); 7,5-7,1 (m,10H); 3,7 (q,2H); 3,6(s,2H); 1,0 (t,3H).

### Exemple 28: 6-(4-benzyl-5-oxo-3-trifluorométhyl-2,5-dihydro-1H-pyrazol-1-yl)-N-éthyl-N-phénylpyridine-3-sulfonamide (Composé n°83 du tableau I)

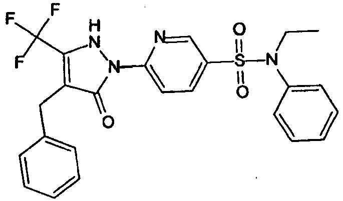

### 28.1. 2-benzyl-4,4,4-trifluoro-3,3-dihydroxybutanoate de méthyle

A une solution de 675 mg (29,4 mmoles) de sodium dans 15 mL de MeOH anh, sous argon, on ajoute, au goutte à goutte, 3,73 mL (29,4 mmoles) de 3,3,3-trifluoropropanoate de méthyle . Après ½ h d'agitation à TA, on ajoute 3,5 mL de bromure de benzyle et le milieu est chauffé 12 h à 70°C. Le mélange réactionnel est ensuite concentré sous pression réduite. Le résidu obtenu est repris par 100 mL d'AcOEt, lavé avec 50 mL de saumure, séché sur Na₂SO₄, filtré et concentré sous pression réduite. Après purification par chromatographie sur colonne de gel de silice en éluant avec un mélange cyclohexane/ACOEt (85:15), on obtient 3,4 g 2-benzyl-4,4,4-trifluoro-3,3-dihydroxybutanoate de méthyle sous forme d'huile utilisée telle quelle à l'étape suivante.
Rendement= 37%.

### 28.2. 6-(4-benzyl-5-oxo-3-trifluorométhyl-2,5-dihydro-1H-pyrazol-1-yl)-N-éthyl-N-phénylpyridine-3-sulfonamide

Un mélange de 1g (3,59 mmoles) de 2-benzyl-4,4,4-trifluoro-3,3-dihydroxybutanoate de méthyle, de 1,05 g (3,59 mmoles) de N-éthyl-6-hydrazino-N-phénylpyridine-3-sulfonamide et de 1 g de tamis moléculaire 4Å dans 8 mL de MeOH est chauffé 12 h à 90°C. A TA, le milieu réactionnel est repris par 30 mL de toluène, chauffé 12h au reflux dans un montage de Dean&Stark. Le milieu réactionnel est ensuite filtré, concentré sous pression réduite, puis le résidu obtenu est repris par 50 mL de DCM, lavé avec une solution d'HCl 1 N (2 X 50 mL), séché sur Na₂SO₄, filtré et concentré sous pression réduite. Le solide ainsi obtenu est recristallisé dans l'EtOH. On obtient 224mg de 6-(4-benzyl-5-oxo-3-trifluorométhyl-2,5-dihydro-1H-pyrazol-1-yl)-N-éthyl-N-phénylpyridine-3-sulfonamide sous forme de cristaux blancs.
Rendement = 12%
PF(°C) =186
M = C₂₄H₂₁F₃N₄O₃S = 502; M+H = 503; Méthode 3: Tr= 4,4min .
RMN ¹H, d6-DMSO, 400MHz, δ (ppm): 12 (sl,1H); 8, 7 (s,1H); 8,25 (d,1H); 8,0 (d,1H); 7,5-7,1 (m,10H); 3,9 (s,2H); 3,7 (q,2H); 1,0 (t,3H).

### Exemple 29: 6-(4-benzyl-5-oxo-2,5-dihydro -1H-pyrazol-1-yl) -N-[(1R,3S)-3-(hydroxyméthyl) cyclopentyl] -N-méthylpyridine-3-sulfonamide (Composé n°138 du tableau I)

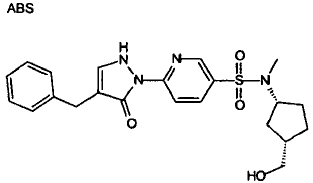

### 29.1. tert-butyl [(1R,3S)-3-(hydroxyméthyl)cyclopentyl)carbamate

A une solution de 2 g (8.7 mmoles) d'acide (1 S,3R)-3- [(tert-butoxycarbonyl)amino] cyclopentanecarboxylique et de 1.33 mL (9.6 mmoles) de NEt3 dans 20mL de THF anh. est ajouté , à -20°C au goutte à goutte 1.2 mL( 9.2 mmoles) d'isobutylchloroformate. Le milieu est agité 45 min à -20°C puis l'insoluble formé est filtré. Une solution de 1g (26.2 mmmoles) de borohydrure de sodium dans un mélange THF/H2O (16mU4mL) est ajouté au filtrat au goutte à goutte à -10°C puis l'agitation est poursuivie en laissant remonter la température jusqu'à T.A. On ajoute ensuite 100 mL d'HCl 0.1N lentement puis le milieu réactionnel est extrait par 2X200 mL d'AcoEt, puis séché sur Na₂SO₄, filtré et concentré sous pression réduite. Après purification par chromatographie sur colonne de gel de silice en éluant avec un mélange DCM/MeOH (95 :5), on obtient 1,4 g sous forme de tert-butyl [(1 R,3S)-3-(hydroxyméthyl)cyclopentyl]carbamate sous forme d'huile.
Rendement= 77%.
RMN ¹H, CDCl₃, 400MHz, δ (ppm) : 4,5 ( sl, 1 H); 3,9 (m,1H); 3,5(d,2H) ;2,1 (m, 2H); 1,8-1,7 (m, 3H); 1,5 (s,2H); 1,4 (s, 9H); 1,0 (m, 1H)

### 29.2. tert-butyl {(1R,3S)-3-[(benzyloxy)methyl]cyclopentyl} carbamate

A une solution de 1.4 g (6.7 mmoles) de tert-butyl [(1 R,3S)-3-(hydroxymethyl)cyclopentyl]carbamate dans 20 mL de THF anh, on ajoute sous argon par petites portions, à T.A, 0.27 g (6.7 mmoles) d'hydrure de sodium à 60% dans l'huile. Après 45 min d'agitation, le milieu est refroidi à 0°C et on ajoute 0.8 mL (6.7 mmoles) de bromure de benzyle. Après 1h d'agitation à T.A, le milieu est hydrolysé par 30mL d'eau et extrait avec 2X100mL d'AcOEt , séché sur Na₂SO₄, concentré sous pression réduite puis purifié par chromatographie sur colonne de gel de silice en éluant avec un mélange DCM / MeOH 99 :1. On obtient 1.53 g de tert-butyl {(1R,3S)-3-[(benzyloxy)méthyl]cyclopentyl} carbamate sous forme d'une huile .
Rendement= 75%

### 29.3. Chlorhydrate de (1R,3S)-3-[(benzyloxy)méthyl]cyclopentanamine

A une solution de 1.52 g ( 5 mmoles) dans 20 mL de DCM, on ajoute à 0°C, 5 mL (20 mmoles) d'une solution d'HCl 4N dans le dioxanne. Le milieu est ensuite agité 12h à T.A puis concentré sous pression réduite. Le résidu obtenu est concrétisé dans 20mL d'Et₂O, filtré et séché sous vide . On obtient 1g de chlorhydrate de (1R,3S)-3-[(benzyloxy)méthyl]cyclopentanamine sous forme d'une poudre
Rendement= 83%

### 29.4. N-{(1R,3S)-3-[(benzyloxy)méthyl]cyclopentyl}-6-chloropyridine-3-sulfonamide

Selon le procédé de 14.1, on obtient , à partir de 1g de_chlorhydrate de (1R,3S)-3-[(benzyloxy)méthyl]cyclopentanamine et de 1 g de chlorure de 6-chloropyridine-3-sulfonyle, 1.2 g de N-{(1R,3S)-3-[(benzyloxy)méthyl]cyclopentyl}-6-chloropyridine-3-sulfonamide sous forme d'une poudre rose.
Rendement= 83%
RMN ¹H, CDCl₃, 400MHz, δ (ppm) : 8,6 (s, 1H); 7,8 (dd,1H); 7,4-7,3 (m,5H); 7,2 (s, 1H); 5,8 (d, 1H); 4,5 ( dd, 2H); 3,7 (m,1H); 3,3(m,2H) ;2,2 (m,1H); 1,9 (m,1H); 1,6-1,5 (m, 4H); 1,2( dd,1H)

### 29.5. N-{(1R,3S)-3-[(benzyloxy)méthyl]cyclopentyl}-6-chloro-N-méthylpyridine-3-sulfonamide

Selon le procédé 23.2, on obtient, à partir de 0.66 g de 4 N-{(1R,3S)-3-[(benzyloxy)méthyl]cyclopentyl}-6-chloropyridine-3-sulfonamide et de 0.22mL d'iodure de méthyle, 0,58 g de N-{(1R,3S)-3-[(benzyloxy)méthyl]cyclopentyl}-6-chloro-N-méthylpyridine-3-sulfonamide sous forme d'huile.
Rendement= 86%

### 29.6. N-{(1R,3S)-3-[(benzyloxy)méthyl]cyclopentyl}-6-hydrazinyl-N-méthylpyridine-3-sulfonamide

Selon le procède 5.2, on obtient à partir de 0.58 g de N-{(1R,3S)-3-[(benzyloxy)méthyl]cyclopentyl}-6-chloro-N-méthylpyridine-3-sulfonamide et de 0.15 mL d'hydrazine hydrate, 0.49 g de N-{(1R,3S)-3-[(benzyloxy)méthyl]cyclopentyl}-6-hydrazinyl-N-méthylpyridine-3-sulfonamide.
Rendement= 86%

### 29.7. 6-(4-benzyl-5-oxo-2,5-dihydro-1H-pyrazol-1-yl)-N-{(1R,3S)-3-[(benzyloxy)méthyl] cyclopentyl}pyridine-3-sulfonamide

Selon le procédé décrit dans l'exemple 1.3, on obtient à partir de 0.495 g de N-{C1 R,3S)-3-[(benzyloxy)méthyl]cyclopentyl}-6-hydrazinyl-N-méthylpyridine-3-sulfonamide et de 0.244 g de 2-benzyl-3-oxopropanoate de méthyle, 301 mg de 6-(4-benzyl-5-oxo-2,5-dihydro-1H-pyrazol-1-yl)-N-{(1R,3S)-3-[(benzyloxy)méthyl]cyclopentyl}pyridine-3-sulfonamide sous forme d'une poudre .
Rendement= 44%

### 29.8. 6-(4-benzyl-5-oxo-2,5-dihydro-1H-pyrazol-1-yl)-N-[(1R,3S)-3-(hydroxyméthyl) cyclopentyl]-N-méthylpyridine-3-sulfonamide

A une solution de 0.3 g (0.57 mmoles de 6-(4-benzyl-5-oxo-2,5-dihydro-1H-pyrazol-1-yl)-N-{(1R,3S)-3-[(benzyloxy)methyl]cyclopentyl}pyridine-3-sulfonamide dans 2mL de DCM refroidie à -78°C, on ajoute 1.7 m L de tribromure de bore . On laisse ensuite remonter la température à 0°C et l'agitation est poursuivie 1h à 0°C. On ajoute ensuite 10 mL de MeOH à -10°C puis le milieu est concentré sous pression réduite. Le milieu est repris par 100 mL de DCM, lavé successivement avec une solution saturée en NaHCO₃ (2x20mL) et de la saumure (20 mL), séché sur Na₂SO₄, concentré sous pression réduite puis purifié par chromatographie sur colonne de gel de silice en éluant avec un mélange DCM / MeOH 90/10 . On obtient 126 mg de 6-(4-benzyl-5-oxo-2,5-dihydro-1H-pyrazol-1-yl)-N-[(1R,3S)-3-(hydroxyméthyl)cyclopentyl]-N-méthylpyridine-3-sulfonamide sous forme s'une poudre..
Rendement= 50%
α²⁰_{D} = : -12° (c=0,1 ; MEOH)
PF(°C) =108
M = C₂₂H₂₆N₄O₄S = 442 ; M+H = 443; Méthode 2: Tr= 1,09 min.
RMN ¹H, d6-DMSO, 400MHz, δ (ppm): 8,8 (s,1H); 8,7 (sl,1H); 8,4 (d,1H); 7,8 (sl,1H); 7,4-7,3 (m,5H); 7,1 (m, 1H); 4,5 (sl,1 H); 4,4(m, 1H); 3,6 (sl,2H); 3,4 (m,2H); 2,8 (s, 3H); 1,9 (m, 1H); 1,6-1,1 (m,6H)

### Exemple 30: 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide (Composé n°147 du tableau I)

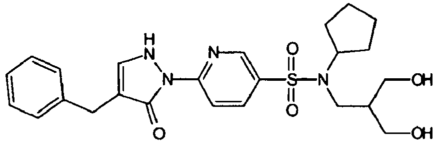

### 30.1. (2,2-diméthyl-1,3-dioxan-5-yl) méthyl 4-méthylbenzènesulfonate

A une solution de 1.5g (10.26 mmoles) de (2,2-diméthyl-1,3-dioxan-5-yl)méthanol, de 1.71 mL de NEt3 dans 15 mL de DCM, refroidie à 0°C, on ajoute 2.15 g(11.3 mmoles) de chlorure de tosyle . On laisse revenir le milieu à T.A, et l'agitation est poursuivie 1h. Le milieu est repris par 100 mL de de DCM, lavé successivement avec de l' HCl 0.1N (2x20mL) et de la saumure (20 mL), séché sur Na₂SO₄ , concentré sous pression réduite puis purifié par chromatographie sur colonne de gel de silice en éluant avec un mélange DCM / MeOH 99:1. On obtient 3 g de (2,2-diméthyl-1,3-dioxan-5-yl) méthyl 4-méthylbenzènesulfonate sous forme d'une huile incolore
Rendement= 97%
RMN ¹H, CDCl₃, 400MHz, δ (ppm) : 7.8 (d, 2H); 7,3 (d,2H); 4,1 ( d, 2H); 3,9 (dd,2H); 3,6(dd,2H) ;2,4 (s, 3H); 1,9 (m,1H); 1,4 (s, 3H); 1,2 (s,3H)

### 30.2. N-[(2,2-diméthyl-1,3-dioxan-5-yl)méthyl]cyclopentanamine

Un mélange de 1.5 g (5 mmoles) de (2,2-diméthyl-1,3-dioxan-5-yl) méthyl 4-méthylbenzènesulfonate et de 10 mL (101 mmoles) de cyclopentylamine est chauffé 12h à 80°C. Le milieu est concentré sous pression réduite puis repris par 200 mL d'Et₂O, lavé avec de l'eau (2x50mL)), puis séché sur Na₂SO₄ , concentré sous pression réduite On obtient 1 g de N-[(2,2-diméthyl-1,3-dioxan-5-yl)méthyl]cyclopentanamine sous forme d'une huile qui est engagée tel quel dans l'étape suivante.
Rendement= 100%

### 30.3 N-cyclopentyl-N-[(2,2-dimethyl-1,3-dioxan-5-yl)méthyl]-6-hydrazinylpyridine-3-sulfonamide

Le composé est préparé selon les procédés 14.1 et 14.3 à partir N-[(2,2-diméthyl-1,3-dioxan-5-yl)méthyl]cyclopentanamine, du chlorure de 6-chloropyridine-3-sulfonyle et d'hydrazine hydrate.
Rendement :70%
RMN ¹H, d6-DMSO, 400MHz, δ (ppm): 8,5 (s,1H); 8,25 (s,1H); 8,0 (s,1H); 7,8(d, 1H); 7,7 (s,1H); 7,2 (d,1H); 6,8 (d, 1H); 4,4 (s,1H); 4,2 (m, 1H); 3,9 (dd, 2H); 3,6 (dd, 2H); 3,0 (dd,2H); 2 ,0 (m, 1H); 1,9 (d,2H); 1,6-1,,4 (m, 4H); 1,3 (s, 3H); 1,2 (s, 3H)

### 30.4. 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide

Selon le procédé décrit dans l'exemple 11, on obtient à partir de 0.485 g de N-cyclopentyl-N-[(2,2-diméthyl-1,3-dioxan-5-yl)méthyl]-6-hydrazinylpyridine-3-sulfonamide et de 0.242 g de 2-benzyl-3-oxopropanoate de méthyle, 79 mg de 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide sous forme d'une poudre .
Rendement= 14%
PF(°C) >260
M = C₂₄H₃₀N₄O₅S = 486 ; M+H = 487; Méthode 2: Tr= 2,09 min .
RMN ¹H, d6-DMSO, 400MHz, δ (ppm): 12 (sl,1H); 8,8 (s,1H); 8,6 (t,2H); 8,25 (d,1H); 7,7 (s,1H); 7,4-7,2 (m,5H); 4,3 (q, 2H); 3,9 (m ,2H); 3,0 (m,1H);2,8 (m, 1H); 2,7 (s, 3H); 1,6-1,3 (m,8H)

### Exemple 31: 6-(4-benzyl-5-oxo-2,5-dihydro-1H-pyrazol-1-yl)-N-cyclopentyl-N-[(2R)-2,3-dihydroxypropyl]pyridine-3-sulfonamide (Composé n°142 du tableau I)

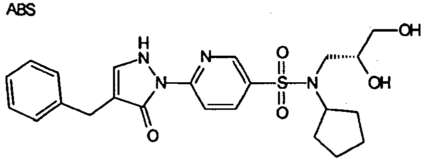

Selon les procédés décrits dans l'exemple 30, on obtient le composé à partir du [(4R)-2,2-diméthyl-1,3-dioxolan-4-yl]méthanol, de la cyclopentylamine, du chlorure de 6-chloropyridine-3-sulfonyle et du 2-benzyl-3-oxopropanoate de méthyle.
PF(°C) =184
α²⁰_{D} = : -24° (c=0,1 DMSO)
M = C₂₃H₂₈N₄O₅S = 472 ; M+H = 473; Méthode 2: Tr= 1.39 min .
RMN ¹H, d6-DMSO, 400MHz, δ (ppm): 12 (sl,1H); 8,8 (s,1H); 8,6 (sl,1H); 8,4 (d,1H); 7,8 (s,1H); 7,3-7,1 (m,5H); 4,8 (s, 1H); 4,6 (s, 1H); ,4,2 (m, 1H); 3,8 (s ,1H); 3,6 (s,2H); 3,3 (m, 2H); 2,9 (m, 1H); 1,6-1,3 (m, 9H)

### Exemple 32: 6-(4-benzyl-5-oxo-2,5-dihydro-1H-pyrazol-1-yl)-N-cyclopentyl-N-[(2S)-2,3-dihydroxypropyl]pyridine-3-sulfonamide (Composé n°148 du tableau I)

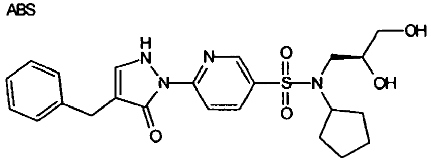

Selon les procédés décrits dans l'exemple 30, on obtient le composé à partir de [(4S)-2,2-diméthyl-1,3-dioxolan-4-yl]méthanol, de la cyclopentylamine, du chlorure de 6-chloropyridine-3-sulfonyle et du 2-benzyl-3-oxopropanoate de méthyle.
PF(°C) =184
a²⁰_{D} = : -+33° (c=0,15 ; DMSO)
M = C₂₃H₂₈N₄O₅S = 472 ; M+H = 473; Méthode 2: Tr= 1.39 min .
RMN ¹H, d6-DMSO, 400MHz, δ (ppm): 12 (sl,1H); 8,8 (s,1H); 8,6 (sl,1H); 8,4 (d,1H); 7,8 (s,1H); 7,3-7,1 (m,5H); 4,8 (s, 1H); 4,6 (s, 1H); ,4,2 (m, 1H); 3,8 (s ,1H); 3,6 (s,2H); 3,3 (m, 2H); 2,9 (m, 1H); 1,6-1,3 (m, 9H)

### Exemple 33: 6-(4-benzyl-5-oxo-2,5-dihydro-1H-pyrazol-1-yl)-N-(2,3-dihydroxypropyl) -N-phénylpyridine-3-sulfonamide (Composé n°143 du tableau I)

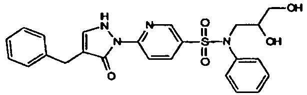

### 32.1. 6-chloro-N-phénylpyridine-3-sulfonamide

Selon le procédé 14.1 , on obtient à partir de 1g de chlorure de 6-chloropyridine-3-sulfonyle et de 0.86 mL d'aniline , 1.15 g de 6-chloro-N-phénylpyridine-3-sulfonamide sous forme d'un solide jaune.
Rendement= 91%
RMN ¹H, d6-DMSO, 400MHz, δ (ppm): : 10,5 (sl,1H); 8,7 (s,1H); 8,1 (dd,1H); 7,75 (dd,1H); 7,3 (m, 2H); 7,2 (m, 3H)

### 32.2. 6-chloro-N-phényl-N-(prop-2-èn-1-yl)pyridine-3-sulfonamide

Selon le procédé 23.2, on obtient à partir de 1.15 g de 6-chloro-N-phénylpyridine-3-sulfonamide et de 0.37 mL de bromure d'allyle, 1.29 g de 6-chloro-N-phényl-N-(prop-2-èn-1-yl)pyridine-3-sulfonamide sous forme d'un solide jaune.
Rendement= 97%
RMN ¹H, d6-DMSO, 400MHz, δ (ppm): : 8,6 (s,1H); 8,0 (dd,1H); 7,9 (dd,1H); 7,4 (m, 3H); 7,2 (m, 2H); 5,7 ( m, 1H); 5,2 (dd, 1 H); 5,1 (dd,1 H); 4,3 (d, 2H)

### 32.3. 6-chloro-N-(2,3-dihydroxypropyl)-N-phénylpyridine-3-sulfonamide

A une solution de 1,.3 g (4.2 mmoles) de 6-chloro-N-phényl-N-(prop-2-èn-1-yl)pyridine-3-sulfonamide dans 17 mL d'un mélange (1/1) de tBuOH et d'eau , on ajoute, à T.A, 1.37 g (11.7 mmoles) de NMO et 0,52 mL(0.04 mmoles) d'OsO4 2.5% dans le tBuOH. L'agitation est maintenue 12h . Le milieu est alors dilué avec 200mL d'eau et extrait avec de l'Et₂O (2X 100mL), séché sur Na₂SO₄, filtré et concentré sous pression réduite. On obtient 1,14g de 6-chloro-N-(2,3-dihydroxypropyl)-N-phénylpyridine-3-sulfonamide sous forme d'huile utilisé tel quel à l'étape suivante.
Rendement= 80%.
RMN ¹H, CDCl₃,400MHz, δ (ppm) : 8,5 (s, 1H); 7,8 (d,1H); 7,4-7,3 ( m, 4H); 7,1 (d, 2H); 3,,7-3,5 (m,3H); 2,5 (sl, 1 H); 2,0 (sl, 1 H); (m,2H)

### 32.4. 6-chloro-N-[(2,2-diméthyl-1,3-dioxolan-4-yl)méthyl]-N-phenylpyridine-3-sulfonamide

Un mélange de 0.67g (1.96 mmoles) de 6-chloro-N-(2,3-dihydroxypropyl)-N-phénylpyridine-3-sulfonamide, de 0.53mL(4.3 mmoles) de 2,2 diméthoxypropane,et de 37 mg de pTsOH dans 4 mL de DMF est agité 3h à T.A. Le milieu est repris par 100 mL d'AcOEt, lavé avec 50 mL d'une solution saturée en NaHCO₃, 50mL d'eau puis séché sur Na₂SO₄, filtré et concentré sous pression réduite. On obtient 0.54 g 6-chloro-N-[(2,2-diméthyl-1,3-dioxolan-4-yl)méthyl]-N-phenylpyridine-3-sulfonamide sous forme d'un solide marron utilisé tel quel à l'étape suivante.
Rendement= 73%.
RMN ¹H, CDCl₃, 400MHz, δ (ppm) : 8,5 (s, 1H); 7,7 (d,1H); 7,4 ( d, 1H); 7,3 (m, 3H); 7,0 (d, 2H) ; 4,1 (m, 1H); 3,9 (m, 1H), 3,8 (dd,2H); 3,5 (m,1H); 1,3 (s, 3H); 1,2 (s, 3H)

### 32.5. N-[(2,2-diméthyl-1,3-dioxolan-4-yl)méthyl]-6-hydrazinyl-N-phenylyridine-3-sulfonamide

Selon le procédé 5..2, on obtient à partir de 0.54 g de 6-chloro-N-[(2,2-diméthyl-1,3-dioxolan-4-yl)méthyl]-N-phénylpyridine-3-sulfonamide et de 30µL d'hydrazine hydrate, 0.53 g de N-[(2,2-diméthyl-1,3-dioxolan-4-yl)méthyl]-6-hydrazinyl-N-phénylpyridine-3-sulfonamide sous forme d'un solide blanc.
Rendement= 99%
RMN ¹H, d6-DMSO, 400MHz, δ (ppm): : 8,6 (s,1H); 8.1 (s, 1H); 7,5 (d, 1H); 7,4 (m, 3H); 7,2 (d, 2H); 6,8 (sl, 1H); 1,3 (s,3H); 1,2 (s, 3H)

### 32.6 6-(4-benzyl-5-oxo-2,5-dihydro-1H-pyrazol-1-yl)-N-(2,3-dihydroxypropyl) -N-phénylpyridine-3-sulfonamide

Selon le procédé 1.3, on obtient, à partir de 0.2 g (0.53 mmoles) de N-[(2,2-diméthyl-1,3-dioxolan-4-yl)méthyl]-6-hydrazinyl-N-phénylpyridine-3-sulfonamide et 0.1g de 2-benzyl-3-oxopropanoate de méthyle, 127 mg de 6-(4-benzyl-5-oxo-2,5-dihydro-1H-pyrazol-1-yl)-N-(2,3-dihydroxypropyl) -N-phénylpyridine-3-sulfonamide sous forme d'un solide blanc.
Rendement= 50%
PF(°C) =174
M = C₂₄H₂₄N₄O₅S = 480 ; M+H = 481; Méthode 2: Tr= 1.39 min .
RMN ¹H, d6-DMSO, 400MHz, δ (ppm): 12 (sl,1H); 8,5 (sl,1H); 8,4 (s, 1H) ;8,1 (d,2H); 7,8 (s,1H); 7,4-7,2 (m,7H); 7,2-7,1 (m,3H); 4,7 (d, 1H); 4,5 (t ,1H); 3,6 (dd,2H);3,4 (m, ,4H)

### Exemple 33: 3-(2-{5-[cyclopentyl(méthyl)sulfamoyl]pyridin-2-yl}-3-oxo-2,3-dihydro-1H-pyrazol-4-yl)-3-phényl-N-(2,2,2-trifluoroéthyl)propanamide (Composé n°146 du tableau I)

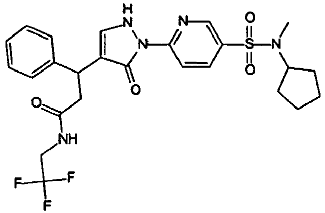

### 33.1 3-(2-{5-[cyclopentyl(méthyl)sulfamoyl]pyridin-2-yl}-3-oxo-2,3-dihydro-1H-pyrazol-4-yl)-3-phényl-N-(2,2,2-trifluoroéthyl)propanamide

A une solution de 200 mg (0.43 mmoles) de 3-(2-{5-[cyclopentyl(méthyl)sulfamoyl] pyridin-2-yl}-3-oxo-2,3-dihydro-1H-pyrazol-4-yl)-3-phénylpropanoate de 0.3 mL (1.7 mmoles) de DIEA et 30µL (0.43 mmoles) de 2,2,2-trifluoroéthylamine dans 1 mL de DCM, on ajoute à 0°C , 207 mg (0.64 mmoles) de TBTU. Le milieu est agité 12 h à T.A. On ajoute ensuite 0.3mL supplémentaires de 2,2,2-trifluoroéthylamine et le milieu est chauffé à 40°C pendant 6h . Le milieu est repris par 20 mL de de DCM, lavé successivement avec de l'eau (2x10mL) et de la saumure (20 mL), séché sur Na₂SO₄ puis concentré sous pression réduite puis purifié par chromatographie sur colonne de gel de silice en éluant avec un gradient cyclohexane / AcOEt de 0 à 10 % d'AcOEt . On obtient 32 mg de 3-(2-{5-[cyclopentyl(méthyl) sulfamoyl]pyridin-2-yl}-3-oxo-2,3-dihydro-1 H-pyrazol-4-yl)-3-phényl-N-(2,2,2-trifluoroéthyl) propanamide sous forme de poudre .
Rendement= 14%
PF(°C) =80
M = C₂₅H₂₈F₃N₅O₄S = 551 ; M+H = 552; Méthode 2: Tr= 1.,21min.
RMN ¹H, d6-DMSO, 400MHz, δ (ppm): 12 (sl,1H); 8,8 (s,1H); 8,6 (t,2H); 8,25 (d,1H); 7,7 (s,1H); 7,4-7,2 (m,5H); 4,3 (q, 2H); 3,9 (m ,2H); 3,0 (m,1H);2,8 (m, 1H); 2,7 (s, 3H); 1,6-1,3 (m,8H)

### Exemple 34: 6-(4-benzyl-5-oxo-2,5-dihydro-1H-pyrazol-1-yl)-N-cyclopentyl-N-[3-hydroxy-2-(hydroxyméthyl)propyl]pyridine-3-sulfonamide (Composé n°147 du tableau I)

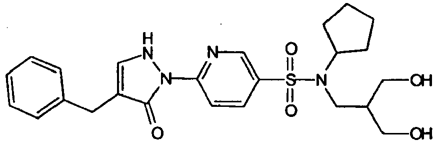

### 34.1. (2,2-diméthyl-1,3-dioxan-5-yl) méthyl 4-méthylbenzènesulfonate

A une solution de 1.5g (10.26 mmoles) de (2,2-diméthyl-1,3-dioxan-5-yl)méthanol, de 1.71 mL de NEt3 dans 15 mL de DCM, refroidie à 0°c, on ajoute 2.15 g(11.3 mmoles) de chlorure de tosyle .On laisse revenir le milieu à T.A, et l'agitation est poursuivie 1h. Le milieu est repris par 100 mL de de DCM, lavé successivement avec HCl 0.11 N (2x20mL) et de la saumure (20 mL), séché sur Na₂SO₄ , concentré sous pression réduite puis purifié par chromatographie sur colonne de gel de silice en éluant avec un mélange DCM / MeOH 99:1.. On obtient 3 g de (2,2-dimethyl-1,3-dioxan-5-yl) méthyl 4-méthylbenzènesulfonate sous forme d'une huile incolore
Rendement= 97%
RMN ¹H, CDCl₃, 400MHz, δ (ppm) : 7.8 (d, 2H); 7,3 (d,2H); 4,1 ( d, 2H); 3,9 (dd,2H); 3,6(dd,2H) ;2,4 (s, 3H); 1,9 (m,1H); 1,4 (s, 3H); 1,2 (s,3H)

### 34.2. N-[(2,2-diméthyl-1,3-dioxan-5-yl)méthyl]cyclopentanamine

Un mélange de 1.5 g (5 mmoles) de (2,2-diméhyl-1,3-dioxan-5-yl) méthyl 4-méthylbenzènesulfonate et de 10 mL (101 mmoles) de cyclopentylamine est chauffé 12h à 80°C. Le milieu est concentré sous pression réduite puis repris par 200 mL d'Et₂O, lavé avec de l'eau (2x50mL)), puis séché sur Na₂SO₄ , concentré sous pression réduite On obtient 1 g de N-[(2,2-diméthyl-1,3-dioxan-5-yl)méthyl]cyclopentanamine sous forme d'une huile qui est engagé tel quel dans l'étape suivante.
Rendement= 100%

### 34.3. N-cyclopentyl-N-[(2,2-diméthyl-1,3-dioxan-5-yl)méthyl]-6-hydrazinylpyridine-3-sulfonamide

Le composé est préparé selon les procédés 14.1 et 5.2 à partir N-[(2,2-diméthyl-1,3-dioxan-5-yl)méthyl]cyclopentanamine , du chlorure de 6-chloropyridine-3-sulfonyle et d'hydrazine hydrate.
Rendement :70%
RMN ¹H, d6-DMSO, 400MHz, δ (ppm): 8,5 (s,1H); 8,25 (s,1H); 8,0 (s,1H); 7,8(d, 1H) ; 7,7 (s,1H); 7,2 (d,1H); 6,8 (d, 1H); 4,4 (s,1H); 4,2 (m, 1H); 3,9 (dd, 2H); 3,6 (dd, 2H); 3,0 (dd,2H); 2 ,0 (m, 1H); 1,9 (d,2H); 1,6-1,,4 (m, 4H); 1,3 (s, 3H); 1,2 (s, 3H)

### 34.4. 6-(4-benzyl-5-oxo-2,5-dihydro-1H-pyrazol-1-yl)-N-cyclopentyl-N-[3-hydroxy-2-(hydroxyméthyl)propyl]pyridine-3-sulfonamide

Selon le procédé décrit dans l'exemple 11, on obtient à partir de 0.485 g de N-cyclopentyl-N-[(2,2-diméthyl-1,3-dioxan-5-yl)méthyl]-6-hydrazinylpyridine-3-sulfonamide et de 0.242 g 2-benzyl-3-oxopropanoate de méthyle, 79 mg de 6-(4-benzyl-5-oxo-2,5-dihydro-1H-pyrazol-1-yl)-N-cyclopentyl-N-[3-hydroxy-2-(hydroxyméthyl)propyl]pyridine-3-sulfonamide sous forme d'une poudre .
Rendement= 14%
PF(°C) >260
M = C₂₄H₃₀N₄O₅S = 486 ; M+H = 487; Méthode 2: Tr= 2,09 min .
RMN ¹H, d6-DMSO, 400MHz, δ (ppm): 12 (sl,1H); 8,8 (s,1H); 8,6 (t,2H); 8,25 (d,1H); 7,7 (s,1H); 7,4-7,2 (m,5H); 4,3 (q, 2H); 3,9 (m ,2H); 3,0 (m,1H);2,8 (m, 1H); 2,7 (s, 3H); 1,6-1,3 (m,8H)

### Exemple 35: 3-{2-[5-(tert-butylsulfamoyl)pyridin-2-yl]-3-oxo-2,3-dihydro-1H-pyrazol-4-yl}-3-(pyridin-3-yl)propanoate de méthyle (Composé n°154 du tableau I)

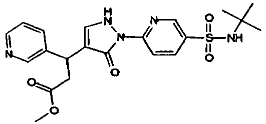

### 35.1. (2Z)-3-(pyridin-3-yl)pent-2-ènedioate de diéthyle

A une solution de 15 mL (85.6 mmoles) de (2E)-pent-2-ènedioate de diéthyle dans 5 mL de DMF anh., on ajoute, sous flux d'argon, 0.62g (2.8 mmoles) de Pd(OAc)₂, 1.7g (5.56 mmoles) de P(OTol)₃ et 5.5 mL (39.7 mmoles) de NEt3 , puis le milieu est chauffé à 40°C et 4 mL (39.75 mmoles) de 3-bromopyridine sont ajoutés. Le milieu est chauffé ensuite 24h à 90°C puis le milieu est repris par 100 mL de d'AcOEt , lavé avec 100 mL d'eau , séché sur Na₂SO₄ , concentré sous pression réduite et purifié par chromatographie sur colonne de gel de silice en éluant avec un gradient cyclohexane / AcOEt de 0 à 10 % d'AcOEt.. On obtient 9.12 g de de (2Z)-3-(pyridin-3-yl)pent-2-ènedioate de diéthyle sous forme d'une cire jaune
Rendement= 87%
RMN ¹H, CDCl₃, 400MHz, δ (ppm) 8,8 (s, 1H); 8,6 (d,1H); 7,8 ( dd, 1H); 7,4 (dd, 1H); 6,3 (s, 1H); 4,2 (q,2H);4,15 (s, 2H); 4,1 (q, 2H); 1,3 (t, 3H); 1,2 (t,3H)

### 35.2. 2-formyl-3-(pyridin-3-yl)pentanedioate de diéthyle

Le composé est préparé selon les procédés 21.2 et 14.2 à partir du (2Z)-3-(pyridin-3-yl)pent-2-ènedioate de diéthyle
Rendement 18%

### 35.3. 3-{2-[5-(tert-butylsulfamoyl)pyridin-2-yl]-3-oxo-2.3-dihidro-1H-pyrazol-4-yl}-3-(pyridin-3-yl)propanoate de méthyle

Selon les procédés 22.5 à 22.7, on obtient, à partir de 88 mg de 2-formyl-3-(pyridin-3-yl)pentanedioate de diéthyle et de 74 mg de N-tert-butyl-6-hydrazinylpyridine-3-sulfonamide, puis lyophilisation du composé obtenu en présence d'1 eq d'HCl 0.1N , 17 mg de chlorhydrate de 3-{2-[5-(tert-butylsulfamoyl)pyridin-2-yl]-3-oxo-2,3-dihydro-1H-pyrazol-4-yl}-3-(pyridin-3-yl)propanoate de méthyle sous forme d'un lyophilisat vert.
Rendement= 17%
PF(°C) =146
M = C₂₁H₂₅N₅O₅S = 459; M+H = 460; Méthode 2: Tr= 1,19 min .
RMN ¹H, d6-DMSO, 400MHz, δ (ppm): 12,5 (sl,1H); 8,8 (s,1H); 8,7 (sz, 1H); 8,6 (d, 1H); 8,4 (sl,1H); 8,3 (d, 1H);8,2 (d, 1H); 7,9 (s,1H); 7,8 (t, 1H); 7,6 (s, 1 H); 4,3 (t, 1H); 3,5 (s, 3H); 3,2 (m ,2H);1,0 (s, 9H)

Les **tableaux I** et **II** qui suivent illustrent les structures chimiques et les propriétés physiques de quelques exemples de composés selon l'invention.

Le **tableau I** illustre des composés de formule (I), selon l'invention, dans laquelle R représente -SO₂-NR3R4. Ces composés sont appelés ci-après composés de formule (I').

Le **tableau Il** illustre des composés de formule (I), selon l'invention, dans laquelle R est tel que défini dans ledit tableau. Ces composés sont appelés ci-après composés de formule (I").

**Les tableaux I & II** qui suivent illustrent les structures chimiques et les propriétés physiques de quelques exemples de composés selon l'invention.

Dans ces tableaux :
- dans la colonne « sel », « - » représente un composé sous forme de base libre, alors que « CF₃COOH », « HCl » et « Na » représentent respectivement un composé sous forme de sel d'acide trifluoroacétique, sous forme de sels de chlorhydrate et sous forme de sels de sodium;
- dans les autres colonnes, « - » signifie que le substituant en question n'est pas présent sur la molécule ;
- Me, Et, n-Pr, i-Pr, n-Bu et i-Bu représentent respectivement les groupes méthyle, éthyle, n-propyle, isopropyle, n-butyle et isobutyle ;
- Ph et Bn représentent respectivement les groupes phényle et benzyle ;
- la colonne « PF » indique le point de fusion, en °C, du composé concerné ;
- dans la colonne « LC/MS » et la colonne « Méthode » sont indiqués respectivement, le pic MH⁺ identifié par spectrométrie de masse et la méthode analytique de chromatographie liquide haute performance utilisée et détaillée précédemment.

**TABLEAU I**

| | | | | | | | | |
|---|---|---|---|---|---|---|---|---|
| 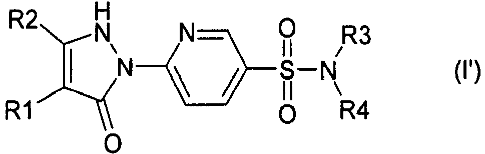 | | | | | | | | |

| *N*° | ***R1*** | ***R2*** | ***R3*** | ***R4*** | ***Sel*** | ***PF*** | ***LC-MS MH*+** | ***méthode*** |
|---|---|---|---|---|---|---|---|---|
| *1* | 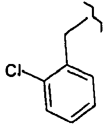 | ***Me*** | 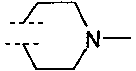 | | ***CF₃CO₂H*** | ***202*** | ***463*** | ***1*** |
| *2* | 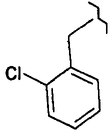 | ***Me*** | 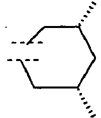 | | **-** | ***170*** | ***476*** | ***1*** |
| *3* | 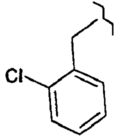 | ***Me*** | ***Et*** | ***Et*** | **-** | ***58*** | ***436*** | ***1*** |
| *4* | 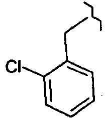 | ***Me*** | ***Et*** | 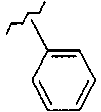 | - | ***260*** | ***484*** | ***1*** |
| *5* | 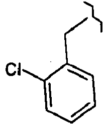 | ***Me*** | ***iPr*** | ***iPr*** | **-** | ***212*** | ***464*** | ***1*** |
| *6* | 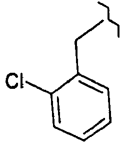 | ***Me*** | 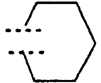 | | **-** | ***190*** | ***448*** | ***1*** |
| *7* | 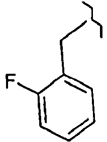 | ***Me*** | ***Et*** | ***Et*** | **-** | ***158*** | ***420*** | ***1*** |
| *8* | 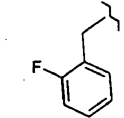 | ***Me*** | 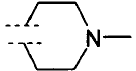 | | ***CF₃CO₂H*** | ***210*** | ***447*** | ***1*** |
| *9* | 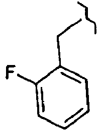 | ***Me*** | 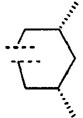 | | - | ***230*** | ***460*** | ***1*** |
| *10* | 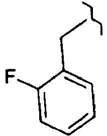 | ***Me*** | ***Et*** | 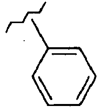 | - | ***215*** | ***468*** | ***1*** |
| *11* | 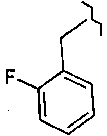 | ***Me*** | 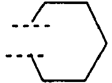 | | **-** | ***198*** | ***432*** | ***1*** |
| *12* | 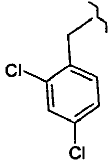 | ***Me*** | 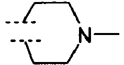 | | ***CF₃CO₂H*** | ***176*** | ***496*** | ***1*** |
| *13* | 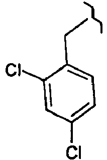 | ***Me*** | 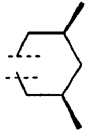 | | **-** | ***212*** | ***509*** | ***1*** |
| *14* | 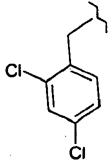 | ***Me*** | ***Et*** | 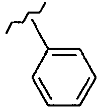 | **-** | ***178*** | ***517*** | ***1*** |
| *15* | 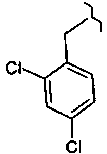 | ***Me*** | ***iPr*** | ***iPr*** | - | ***226*** | ***497*** | ***1*** |
| *16* | 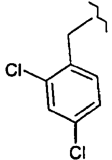 | ***Me*** | 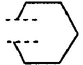 | | - | ***212*** | ***481*** | ***1*** |
| *17* | 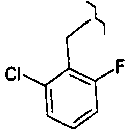 | ***Me*** | 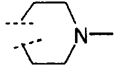 | | ***CF₃CO₂H*** | ***172*** | ***481*** | ***1*** |
| *18* | 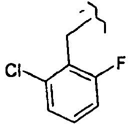 | ***Me*** | 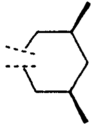 | | - | ***240*** | ***494*** | ***1*** |
| *19* | 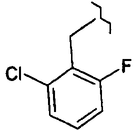 | ***Me*** | ***Et*** | ***Et*** | - | ***170*** | ***454*** | ***1*** |
| *20* | 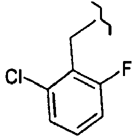 | ***Me*** | 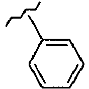 | ***Et*** | **-** | ***206*** | ***502*** | ***1*** |
| *21* | 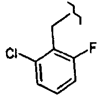 | ***Me*** | ***iPr*** | ***iPr*** | **-** | ***80*** | ***482*** | ***1*** |
| *22* | 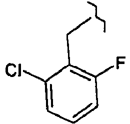 | ***Me*** | 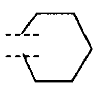 | | - | ***60*** | ***466*** | ***1*** |
| *23* | 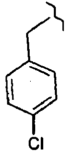 | ***Me*** | 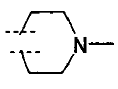 | | ***CF₃CO₂H*** | ***158*** | ***463*** | ***1*** |
| *24* | 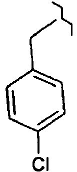 | ***Me*** | 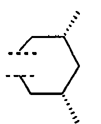 | | - | ***252*** | ***476*** | ***1*** |
| *25* | 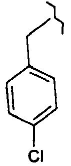 | ***Me*** | ***Et*** | ***Et*** | **-** | ***196*** | ***436*** | ***1*** |
| *26* | 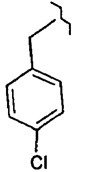 | ***Me*** | ***Et*** | 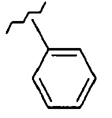 | **-** | ***202*** | ***484*** | ***1*** |
| *27* | 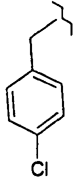 | ***Me*** | ***iPr*** | ***iPr*** | - | ***220*** | ***464*** | ***1*** |
| *28* | 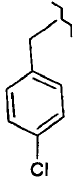 | ***Me*** | 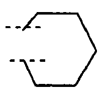 | | - | ***228*** | ***448*** | ***1*** |
| *29* | 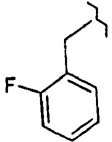 | ***Me*** | ***iPr*** | ***iPr*** | - | ***82*** | ***448*** | ***1*** |
| *30* | 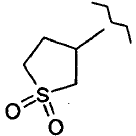 | ***Me*** | 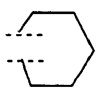 | | - | ***250*** | ***442*** | ***1*** |
| *31* | 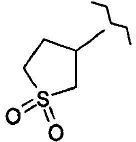 | ***Me*** | ***Et*** | 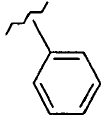 | - | ***264*** | ***478*** | ***1*** |
| *32* | 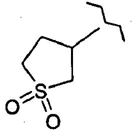 | ***Me*** | ***iPr*** | ***iPr*** | - | ***200*** | ***458*** | ***1*** |
| *33* | 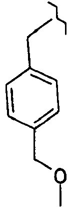 | ***Me*** | ***Et*** | 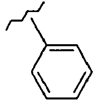 | - | ***177*** | ***494*** | ***2*** |
| *34* | 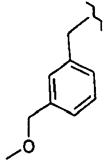 | ***Me*** | ***Et*** | 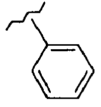 | - | ***144*** | ***494*** | ***2*** |
| *35* | 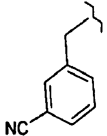 | ***Me*** | ***Et*** | 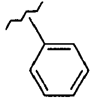 | - | ***198*** | ***475*** | ***2*** |
| *36* | 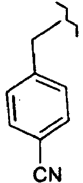 | ***Me*** | ***Et*** | 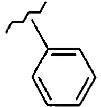 | - | ***218*** | ***475*** | ***2*** |
| *37* | 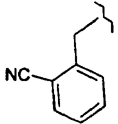 | ***Me*** | ***Et*** | 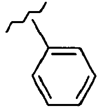 | - | ***112*** | ***475*** | ***2*** |
| *38* | 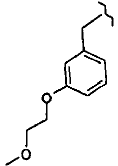 | ***Me*** | ***Et*** | 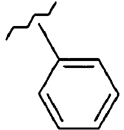 | - | ***126*** | ***523*** | ***2*** |
| *39* | 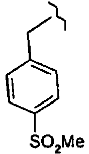 | ***Me*** | ***Et*** | 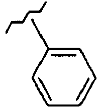 | - | ***224*** | ***527*** | ***2*** |
| *40* | 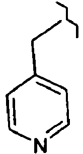 | ***Me*** | ***Et*** | 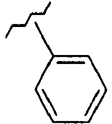 | ***HCl*** | ***192*** | ***451*** | ***2*** |
| *41* | 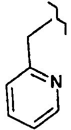 | ***Me*** | ***Et*** | 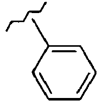 | ***HCl-*** | ***150*** | ***451*** | ***2*** |
| *42* | 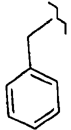 | ***Me*** | ***Et*** | 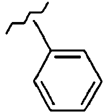 | ***-*** | ***212*** | ***449*** | ***2*** |
| *43* | 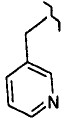 | ***Me*** | ***Et*** | 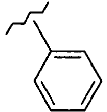 | ***HCl*** | ***128*** | ***451*** | ***2*** |
| *44* | 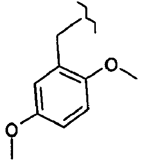 | ***Me*** | ***Et*** | 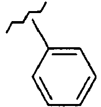 | ***-*** | ***140*** | ***509*** | ***2*** |
| *45* | 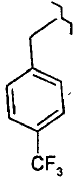 | ***Me*** | ***Et*** | 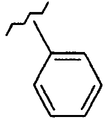 | ***-*** | ***222*** | ***518*** | ***2*** |
| *46* | 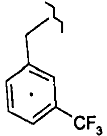 | ***Me*** | ***Et*** | 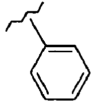 | ***-*** | ***178*** | ***518*** | ***2*** |
| *47* | 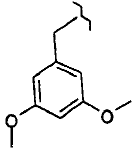 | ***Me*** | ***Et*** | 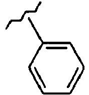 | ***-*** | ***162*** | ***509*** | ***2*** |
| *48* | 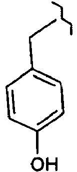 | ***Me*** | ***Et*** | 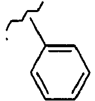 | ***-*** | ***208*** | ***465*** | ***2*** |
| *49* | 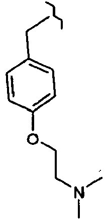 | ***Me*** | ***Et*** | 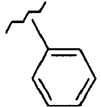 | ***-*** | ***178*** | ***536*** | ***2*** |
| *50* | 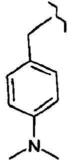 | ***Me*** | ***Et*** | 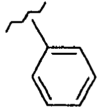 | ***-*** | ***182*** | ***492*** | ***2*** |
| *51* | 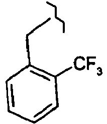 | ***Me*** | ***Et*** | 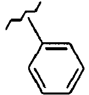 | ***-*** | ***172*** | ***480*** | ***2*** |
| *52* | 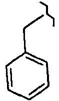 | ***Me*** | ***H*** | ***-(CH₂)₂CH(CH₃)₂*** | ***HCl*** | ***100*** | ***416*** | ***2*** |
| *53* | 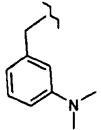 | ***Me*** | ***Et*** | 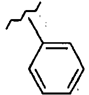 | ***-*** | ***122*** | ***491*** | ***2*** |
| *54* | 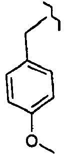 | ***Me*** | ***Et*** | 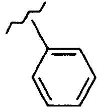 | ***-*** | ***188*** | ***479*** | ***2*** |
| *55* | 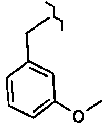 | ***Me*** | ***Et*** | 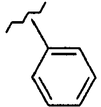 | ***-*** | ***146*** | ***479*** | ***2*** |
| *56* | 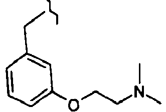 | ***Me*** | ***Et*** | 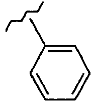 | ***HCl*** | ***124*** | ***537*** | ***2*** |
| *57* | 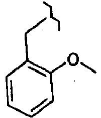 | ***Me*** | ***Et*** | 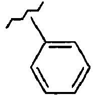 | ***-*** | ***108*** | ***479*** | ***2*** |
| *58* | 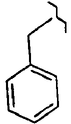 | ***Me*** | 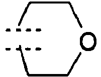 | | ***-*** | ***196*** | ***416*** | |
| *59* | 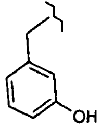 | ***Me*** | ***Et*** | 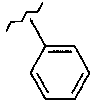 | ***-*** | ***202*** | ***466*** | ***2*** |
| *60* | 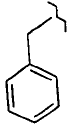 | ***Et*** | ***Et*** | 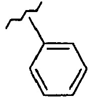 | ***-*** | ***194*** | ***464*** | ***2*** |
| *61* | 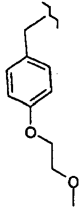 | ***Me*** | ***Et*** | 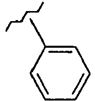 | ***-*** | ***176*** | ***523*** | ***2*** |
| *62* | 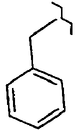 | ***Me*** | 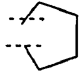 | | ***HCl*** | ***195*** | ***400*** | ***2*** |
| *63* | 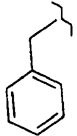 | ***Me*** | 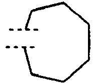 | | ***HCl*** | ***182*** | ***428*** | ***2*** |
| *64* | 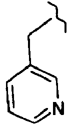 | ***Me*** | ***Et*** | ***Et*** | ***-*** | ***166*** | ***403*** | ***2*** |
| *65* | 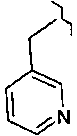 | ***Me*** | ***Et*** | ***Et*** | ***HCl*** | ***154*** | ***403*** | ***2*** |
| *66* | 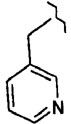 | ***Me*** | ***Me*** | ***Me*** | ***-*** | ***212*** | ***375*** | ***2*** |
| *67* | 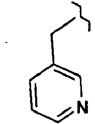 | ***Me*** | ***Me*** | ***Me*** | ***HCl*** | ***170*** | ***375*** | ***2*** |
| *68* | 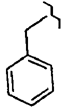 | ***nPr*** | ***Et*** | 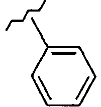 | ***-*** | ***180*** | ***477*** | ***2*** |
| *69* | 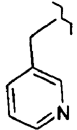 | ***Me*** | ***H*** | ***iPr*** | ***HCl*** | ***228*** | ***389*** | ***2*** |
| *70* | 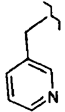 | ***Me*** | 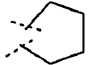 | | ***HCl*** | ***180*** | ***401*** | ***2*** |
| *71* | 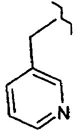 | ***Me*** | ***H*** | ***t-Bu*** | ***HCl*** | ***230*** | ***403*** | ***2*** |
| *72* | 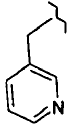 | ***Me*** | ***H*** | 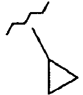 | ***HCl*** | ***206*** | ***387*** | ***2*** |
| *73* | 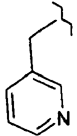 | ***Me*** | ***H*** | 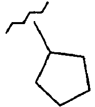 | ***HCl*** | ***242*** | ***415*** | ***2*** |
| *74* | 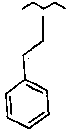 | ***Me*** | ***Et*** | 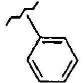 | ***-*** | ***158*** | ***463*** | ***2*** |
| *75* | 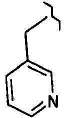 | ***Me*** | ***Me*** | 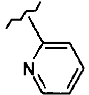 | ***HCl*** | ***106*** | ***438*** | ***2*** |
| *76* | 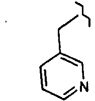 | ***Me*** | 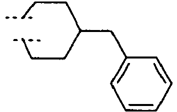 | | ***HCl*** | ***160*** | ***505*** | ***2*** |
| *77* | 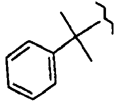 | ***Me*** | ***Et*** | 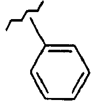 | ***-*** | ***122*** | ***477*** | ***2*** |
| *78* | 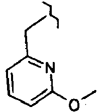 | ***Me*** | ***Et*** | 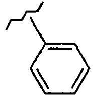 | ***-*** | ***174*** | ***480*** | ***2*** |
| *79* | 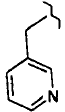 | ***Me*** | ***H*** | 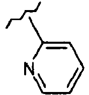 | ***HCl*** | ***174*** | ***424*** | ***2*** |
| *80* | 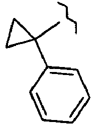 | ***Me*** | ***Et*** | 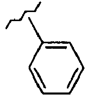 | ***-*** | ***146*** | ***475*** | ***2*** |
| *81* | 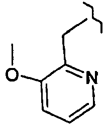 | ***Me*** | ***Et*** | 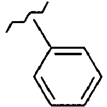 | ***-*** | ***130*** | ***480*** | ***2*** |
| *82* | 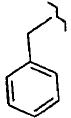 | ***-CH₂-OMe*** | ***Et*** | 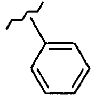 | ***-*** | ***158*** | ***479*** | ***2*** |
| *83* | 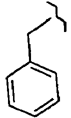 | ***-CF₃*** | ***Et*** | 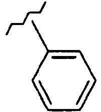 | ***-*** | ***186*** | ***503*** | ***3*** |
| *85* | 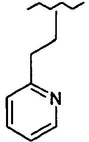 | ***Me*** | ***Et*** | 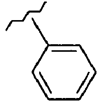 | ***-*** | ***188*** | ***464*** | ***2*** |
| *86* | 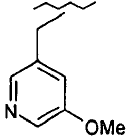 | ***Me*** | ***Et*** | 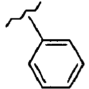 | ***-*** | ***138*** | ***480*** | ***2*** |
| *87* | 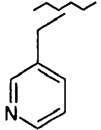 | ***Me*** | ***Me*** | ***-(CH₂)₂-OMe*** | ***-*** | ***136*** | ***418*** | ***2*** |
| *88* | 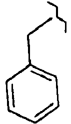 | ***i-Bu*** | ***Et*** | 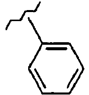 | ***-*** | ***138*** | ***491*** | ***2*** |
| *89* | 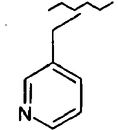 | ***Me*** | ***H*** | ***Et*** | ***HCl*** | ***196*** | ***374*** | ***2*** |
| *90* | 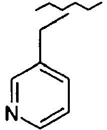 | ***Me*** | ***H*** | 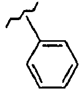 | ***HCl*** | ***154*** | ***422*** | ***2*** |
| *92* | 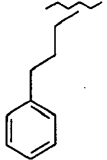 | ***Me*** | ***Et*** | 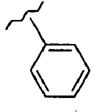 | ***-*** | ***158*** | ***477*** | ***2*** |
| *93* | 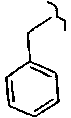 | ***H*** | ***Et*** | 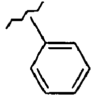 | ***-*** | ***180*** | ***435*** | ***2*** |
| *94* | 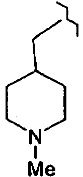 | ***Me*** | ***Et*** | 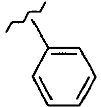 | ***HCl*** | ***150*** | ***470*** | ***2*** |
| *95* | 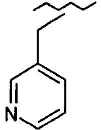 | ***Me*** | ***Me*** | 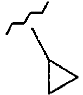 | ***HCl*** | ***138*** | ***400*** | ***2*** |
| *96* | 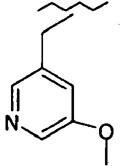 | ***Me*** | ***H*** | ***tBu*** | ***HCl*** | ***150*** | ***432*** | ***2*** |
| *97* | 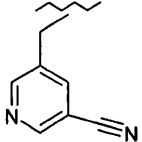 | ***Me*** | ***Et*** | 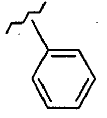 | ***-*** | ***164*** | ***475*** | ***2*** |
| *98A* | 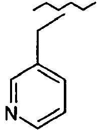 | ***H*** | ***H*** | ***tBu*** | ***-*** | ***220*** | ***388*** | ***2*** |
| *98B* | 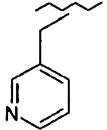 | ***H*** | ***H*** | ***tBu*** | ***HCl*** | ***140*** | ***388*** | ***2*** |
| *99* | 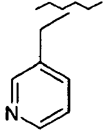 | ***H*** | ***Et*** | 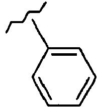 | ***HCl*** | ***142*** | ***436*** | ***2*** |
| *100* | 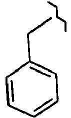 | ***H*** | ***Me*** | ***tBu*** | ***-*** | ***186*** | ***401*** | ***2*** |
| *101* | 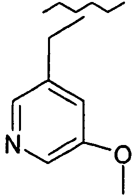 | ***H*** | ***Et*** | 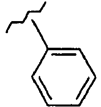 | ***HCl*** | ***136*** | ***466*** | ***2*** |
| *102* | 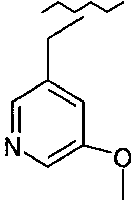 | ***Me*** | ***Me*** | ***tBu*** | ***-*** | ***84*** | ***446*** | ***2*** |
| *103* | 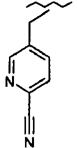 | ***Me*** | ***Et*** | 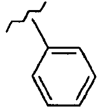 | ***-*** | ***120*** | ***475*** | ***2*** |
| *104* | 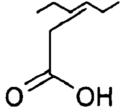 | ***H*** | ***Et*** | 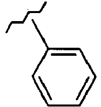 | ***-*** | ***174*** | ***403*** | ***2*** |
| *105* | 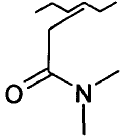 | ***H*** | ***Et*** | 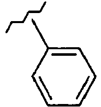 | ***-*** | ***168*** | ***430*** | ***2*** |
| *106* | 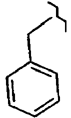 | 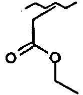 | ***Et*** | 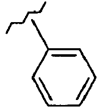 | ***-*** | ***156*** | ***521*** | ***2*** |
| *107* | 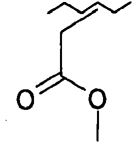 | ***H*** | ***Et*** | 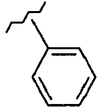 | ***-*** | ***140*** | ***417*** | ***2*** |
| *108* | 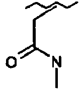 | ***H*** | ***Et*** | 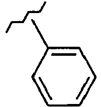 | ***-*** | ***194*** | ***416*** | ***2*** |
| *109* | 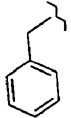 | 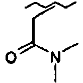 | ***Et*** | 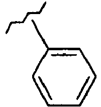 | ***-*** | ***163*** | ***520*** | ***2*** |
| *110* | 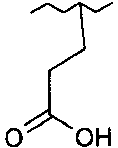 | ***H*** | ***Et*** | 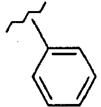 | ***-*** | ***214*** | ***417*** | ***2*** |
| *111* | 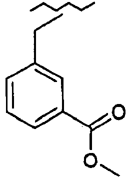 | ***Me*** | ***Et*** | 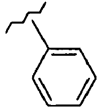 | ***-*** | ***172*** | ***507*** | ***2*** |
| *112* | 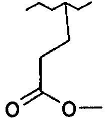 | ***H*** | ***Et*** | 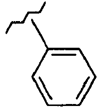 | ***-*** | ***128*** | ***431*** | ***2*** |
| *113* | 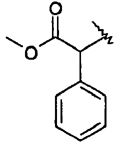 | ***H*** | ***H*** | ***tBu*** | ***-*** | ***66*** | ***445*** | ***2*** |
| *114* | 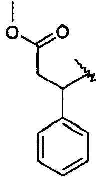 | ***H*** | ***Me*** | 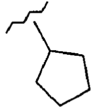 | ***-*** | ***80*** | ***485*** | ***3*** |
| *115* | 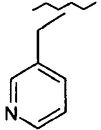 | ***H*** | ***Et*** | 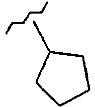 | ***HCl*** | ***110*** | ***428*** | ***2*** |
| *116* | 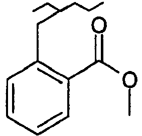 | ***Me*** | ***Et*** | 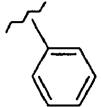 | ***-*** | ***146*** | ***507*** | ***2*** |
| *117* | 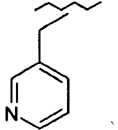 | ***H*** | ***Me*** | 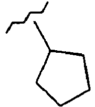 | ***HCl*** | ***124*** | ***414*** | ***2*** |
| *118* | 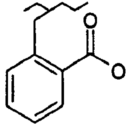 | ***Me*** | ***Et*** | 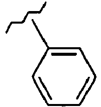 | ***2 Na*** | ***>260*** | ***491*** | ***2*** |
| *119* | 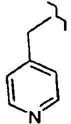 | ***H*** | ***Et*** | 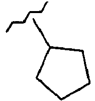 | ***HCl*** | ***115*** | ***428*** | ***2*** |
| *120* | 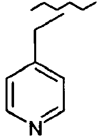 | ***H*** | ***Me*** | 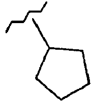 | ***HCl*** | ***130*** | ***414*** | ***2*** |
| *122* | 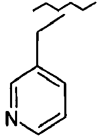 | ***H*** | ***Me*** | ***-CH₂-tBu*** | ***-*** | ***175*** | ***416*** | ***2*** |
| *123* | 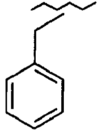 | ***H*** | ***-(CH₂)₃***-***N(Me)₂*** | 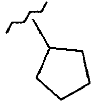 | ***HCl*** | ***230*** | ***484*** | ***2*** |
| *124* | 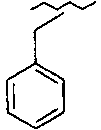 | ***H*** | ***-(CH₂)₂-OH*** | 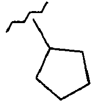 | ***-*** | ***152*** | ***443*** | ***2*** |
| *125* | 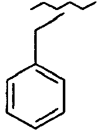 | ***H*** | ***-(CH₂)₃-OH*** | 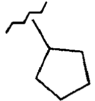 | ***-*** | ***138*** | ***457*** | ***2*** |
| *126* | 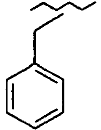 | ***H*** | ***-(CH₂)₃-O-CH₂-Ph*** | 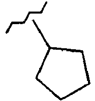 | ***-*** | ***116*** | ***547*** | ***2*** |
| *127* | 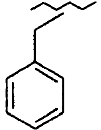 | ***H*** | ***-(CH₂)₂-O-CH₂-Ph*** | 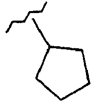 | ***-*** | ***100*** | ***533*** | ***2*** |
| *128* | 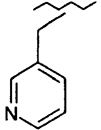 | ***H*** | ***-CH₂CO₂Me*** | 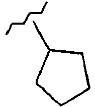 | ***HCl*** | ***110*** | ***472*** | ***2*** |
| *129* | 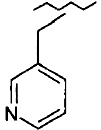 | ***H*** | 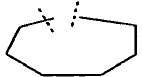 | | ***-*** | ***190*** | ***414*** | ***2*** |
| *131* | 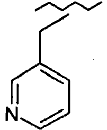 | ***H*** | ***Me*** | 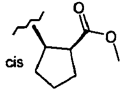 | ***HCl*** | ***156*** | ***472*** | ***2*** |
| *132* | 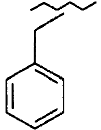 | ***H*** | ***-CH₂-CH₂***-***NMe₂*** | 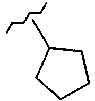 | ***HCl*** | ***192*** | ***470*** | ***2*** |
| *133* | 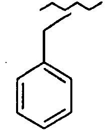 | ***H*** | 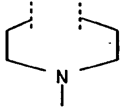 | | ***HCl*** | ***200*** | ***414*** | ***2*** |
| *134* | 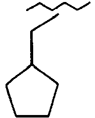 | ***H*** | ***Et*** | 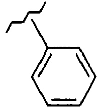 | ***-*** | ***170*** | ***427*** | ***2*** |
| *135* | 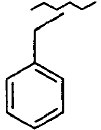 | ***H*** | 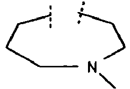 | | ***HCl*** | ***200*** | ***428*** | ***2*** |
| *136* | 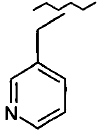 | ***H*** | ***H*** | 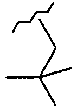 | ***HCl*** | ***123*** | ***402*** | ***2*** |
| *137* | 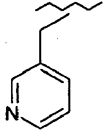 | ***H*** | ***H*** | 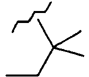 | ***HCl*** | ***126*** | ***402*** | ***2*** |
| *138* | 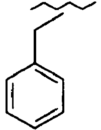 | ***H*** | ***Me*** | 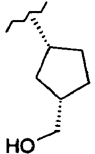 | ***-*** | ***108*** | ***443*** | ***2*** |
| *139* | 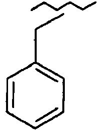 | ***H*** | ***Me*** | 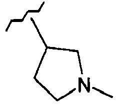 | ***HCl*** | ***130*** | ***428*** | ***2*** |
| *140* | 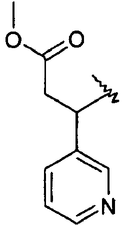 | ***H*** | ***Me*** | 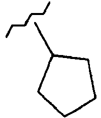 | ***HCl*** | ***124*** | ***485*** | ***2*** |
| *141* | 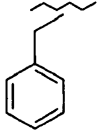 | ***H*** | ***H*** | 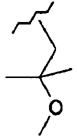 | ***HCl*** | ***194*** | ***417*** | ***2*** |
| *142* | 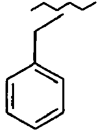 | ***H*** | 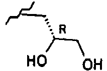 | 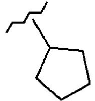 | ***-*** | ***184*** | ***473*** | ***2*** |
| *143* | 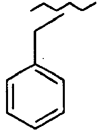 | ***H*** | ***-CH₂-CHOH-CH₂OH*** | 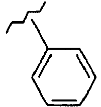 | ***-*** | ***174*** | ***481*** | ***2*** |
| *144* | 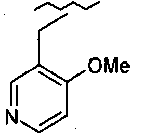 | ***H*** | ***Me*** | 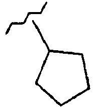 | ***HCl*** | ***40*** | ***444*** | ***2*** |
| *145* | 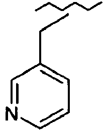 | ***H*** | ***Me*** | 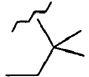 | ***-*** | ***162*** | ***416*** | ***2*** |
| *146* | 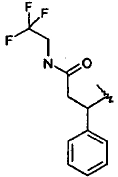 | ***H*** | ***Me*** | 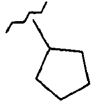 | ***-*** | ***80*** | ***552*** | ***2*** |
| *147* | 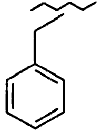 | ***H*** | 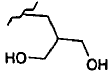 | 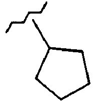 | ***-*** | **>*260*** | ***487*** | ***2*** |
| *148* | 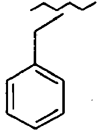 | ***H*** | 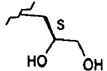 | 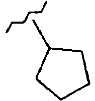 | ***-*** | ***184*** | ***473*** | ***2*** |
| *149* | 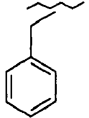 | ***H*** | ***H*** | 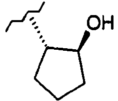 | ***-*** | ***176*** | ***415*** | ***2*** |
| *150* | 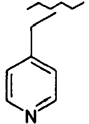 | ***H*** | ***H*** | 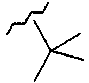 | ***-*** | ***226*** | ***388*** | ***2*** |
| *151* | 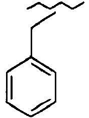 | ***H*** | 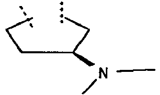 | | ***HCl*** | ***198*** | ***428*** | ***2*** |
| *152* | 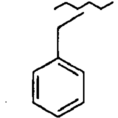 | ***H*** | ***H*** | 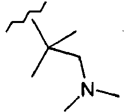 | ***HCl*** | ***230*** | ***430*** | ***2*** |
| *153* | 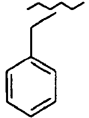 | ***H*** | ***Me*** | 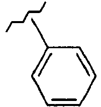 | ***-*** | ***198*** | ***421*** | ***2*** |
| *154* | 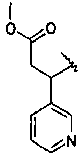 | ***H*** | ***H*** | 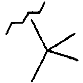 | ***HCl*** | ***146*** | ***460*** | ***2*** |
| *155* | 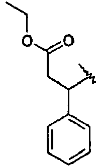 | ***H*** | ***H*** | 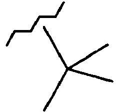 | ***-*** | ***130*** | ***473*** | ***2*** |
| *156* | 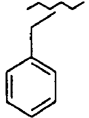 | ***H*** | ***-CH₂-CHOH-CH₂OH*** | 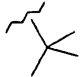 | ***-*** | ***>260*** | ***461*** | ***2*** |
| *157* | 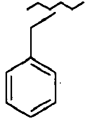 | ***H*** | ***H*** | 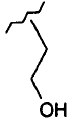 | ***-*** | ***180*** | ***375*** | ***2*** |
| *158* | 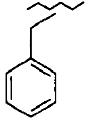 | ***H*** | ***-CH₂-CHOH-CH₂OH*** | 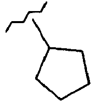 | ***-*** | ***168*** | ***473*** | ***2*** |
| *159* | 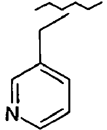 | ***Me*** | 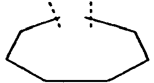 | | ***Na*** | **>*220*** | ***428*** | ***2*** |
| *160* | 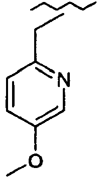 | ***Me*** | ***Me*** | 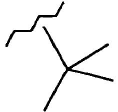 | ***-*** | ***165*** | ***446*** | ***2*** |
| *161* | 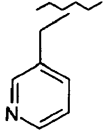 | ***H*** | ***CH₂-CHOH- CH₂OH*** | 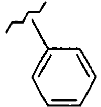 | ***HCl*** | ***148*** | ***482*** | ***2*** |

**Tableau II**

| | | | | | | | |
|---|---|---|---|---|---|---|---|
| 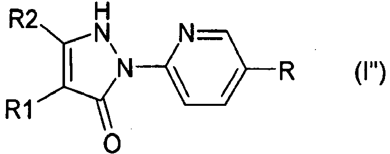 | | | | | | | |

| **N°** | ***R1*** | ***R2*** | ***R*** | ***Sel*** | ***PF*** | ***LC-MS MH+*** | ***méthode*** |
|---|---|---|---|---|---|---|---|
| 162 | 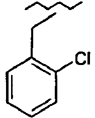 | ***Me*** | ***-CF₃*** | ***-*** | | ***368*** | ***1*** |
| 163 | 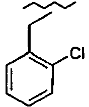 | ***Me*** | ***-C(O)OH*** | | ***246*** | ***381*** | ***2*** |
| 164 | 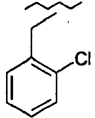 | ***Me*** | ***H*** | ***-*** | | ***300*** | ***1*** |
| 165 | 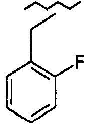 | ***Me*** | ***H*** | ***-*** | | ***284*** | ***1*** |
| 166 | 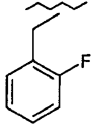 | ***Me*** | ***-CF₃*** | *-* | | ***352*** | ***1*** |
| 167 | 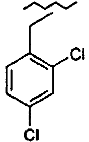 | ***Me*** | ***H*** | *-* | | ***334*** | ***1*** |
| 168 | 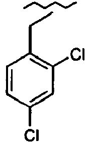 | ***Me*** | ***-CF₃*** | *-* | | ***402*** | ***1*** |
| 169 | 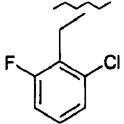 | ***Me*** | ***-CF₃*** | *-* | | ***386*** | ***1*** |
| 170 | 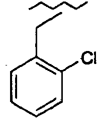 | ***Me*** | ***-C(O)OMe*** | ***HCl*** | ***258*** | ***358*** | ***2*** |
| 171 | 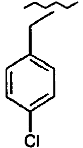 | ***Me*** | ***H*** | *-* | | ***300*** | ***1*** |
| 172 | 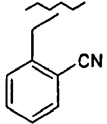 | ***Me*** | ***H*** | - | ***157*** | ***291*** | ***2*** |
| 173 | 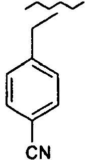 | ***Me*** | ***H*** | - | ***154*** | ***291*** | ***2*** |
| 174 | 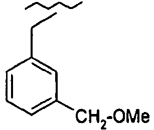 | ***Me*** | ***H*** | - | ***55*** | ***493*** | ***2*** |
| 175 | 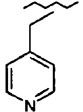 | ***Me*** | ***H*** | - | ***162*** | ***267*** | ***2*** |
| 176 | 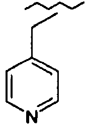 | ***Me*** | ***H*** | ***HCl*** | ***208*** | ***267*** | ***2*** |
| 177 | 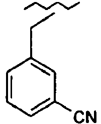 | ***Me*** | ***H*** | - | ***90*** | ***291*** | ***2*** |
| 178 | 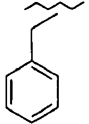 | ***Me*** | ***-Br*** | - | ***164*** | ***345*** | ***2*** |
| 179 | 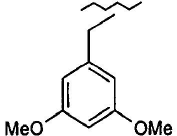 | ***Me*** | ***H*** | - | ***96*** | ***326*** | ***2*** |
| 180 | 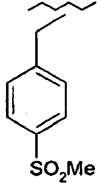 | ***Me*** | ***H*** | - | ***144*** | ***344*** | ***2*** |
| 181 | 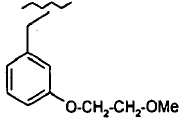 | ***Me*** | ***H*** | - | ***68*** | ***340*** | ***2*** |
| 182 | 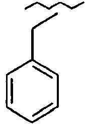 | ***Me*** | ***H*** | - | ***110*** | ***266*** | ***2*** |
| 183 | 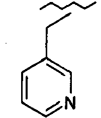 | ***Me*** | ***H*** | - | ***80*** | ***267*** | ***2*** |
| 184 | 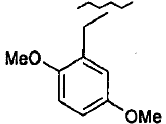 | ***Me*** | ***H*** | - | ***62*** | ***326*** | ***2*** |
| 185 | 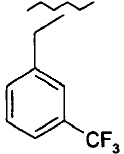 | ***Me*** | ***H*** | - | ***78*** | ***334*** | ***2*** |
| 186 | 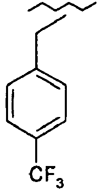 | ***Me*** | ***H*** | - | ***94*** | ***334*** | ***2*** |
| 187 | 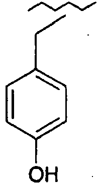 | ***Me*** | ***H*** | - | ***212*** | ***465*** | ***2*** |
| 188 | 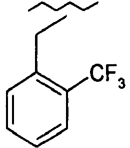 | ***Me*** | ***H*** | - | ***106*** | ***334*** | ***2*** |
| 189 | 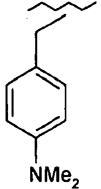 | ***Me*** | ***H*** | - | ***110*** | ***309*** | ***2*** |
| 190 | 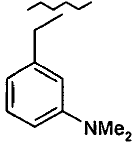 | ***Me*** | ***H*** | ***HCl*** | ***132*** | ***309*** | ***2*** |
| 191 | 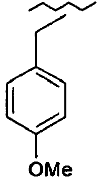 | ***Me*** | ***H*** | - | ***96*** | ***296*** | ***2*** |
| 192 | 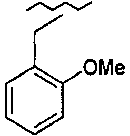 | ***Me*** | ***H*** | - | ***254*** | ***296*** | ***2*** |
| 193 | 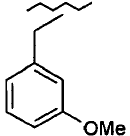 | ***Me*** | ***H*** | - | ***120*** | ***296*** | ***2*** |
| 194 | 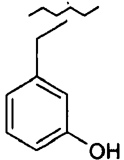 | ***Me*** | ***H*** | - | ***150*** | ***281*** | ***2*** |
| 195 | 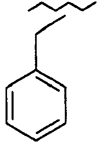 | ***Et*** | ***H*** | - | ***76*** | ***280*** | ***2*** |
| 196 | 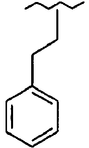 | ***Me*** | ***H*** | - | ***128*** | ***280*** | ***2*** |
| 197 | 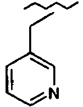 | ***Me*** | 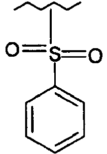 | - | ***176*** | ***407*** | ***2*** |
| 198 | 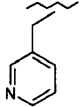 | ***H*** | ***-CO2-CH2-tBu*** | - | ***160*** | ***367*** | ***2*** |
| 199 | 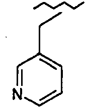 | ***Me*** | ***COO t-Bu*** | ***HCl*** | ***156*** | ***367*** | ***2*** |
| 200 | 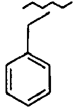 | ***H*** | ***-CO2-CH2-tBu*** | - | ***130*** | ***366*** | ***2*** |
| 201 | 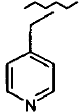 | ***H*** | ***-CO2-CH2-tBu*** | - | ***132*** | ***367*** | ***2*** |
| 202 | 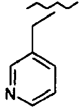 | ***H*** | ***CO2tBu*** | ***HCl*** | ***160*** | ***353*** | ***2*** |

Les composés selon l'invention ont fait l'objet d'essais pharmacologiques en vue de déterminer leurs propriétés, avec en particulier :
- Un test *in vitro* de mesure directe de stabilisation de la protéine HIF1-alpha, facteur de transcription exprimé de façon constitutive dans les cellules mais dégradé dans les conditions normales en oxygène par le système ubiquitin/protéasome.
- Un test fonctionnel permettant la mesure dans des cellules He3pB de la sécrétion de VEGF et d'EPO qui sont deux marqueurs de l'activation de HIF1-alpha dans les hépatocytes.

Ces deux tests sont décrits ci-dessous.

### 1. Mesure de la stabilisation de HIF1alpha dans les cellules HEKEA

### 1.1 Objectif

HIF est un facteur de transcription impliqué dans l'adaptation des cellules à l'hypoxie. Ce facteur de transcription est a minima un hétéro-dimère constitué de 2 protéines, ARNT et HIF1alpha. ARNT est exprimé de façon constitutive par les cellules et l'essentiel de la régulation du complexe s'effectue via la stabilisation de la protéine HIF1-alpha. En effet cette protéine, dans les conditions normales en oxygène (20% équivalents approximativement à la valeur de l'oxygène ambiant), est hydroxylée spécifiquement sur 2 prolines (proline 402 et 564 pour la protéine humaine) par des HIF prolyl-hydroxylases entrainant la liaison de la protéine de von Hippel Lindau (VHL). Cette liaison de VHL sur HIF1-alpha provoque alors la dégradation de HIF1alpha par le système ubiquitine/protéasome. En hypoxie (O₂ < 5% dans les tests cellulaires) les HIF prolyl-hydroxylases sont inhibées ce qui se traduit par une augmentation de la quantité de la protéine HIF1alpha dans les cellules. Ce dernier peut alors s'associer à ARNT pour basculer dans le noyau et activer ses gènes cibles.

Les gènes activés par HIF étant impliqués dans la réponse adaptative des cellules à l'hypoxie et des tissus à l'ischémie, l'objectif est d'identifier et de caractériser des composés qui stabilisent HIF1alpha dans les cellules afin d'amplifier ou de mimer son activité bénéfique.

Il existe de nombreux tests décrivant la mesure indirecte de l'activité de HIF via des systèmes de gènes rapporteur (HRE_luciférase) ou via la mesure de protéines induites par HIF (exemple VEGF ou EPO). De plus les seuls tests permettant de mesurer directement la quantité de la protéine HIF1alpha dans les cellules sont des tests utilisant des anticorps comme le Western Blot comprenant des phases d'extraction des cellules (lysats totaux ou extraits nucléaires) qui sont consommatrices en termes de cellules, de temps en limitant ainsi la capacité de screening de composés. L'objectif était donc de développer un test de screening sensible adaptable en plaques 384 puits permettant de mesurer directement la quantité de la protéine HIF1alpha dans le noyau des cellules. Ce test a été établi dans des cellules HEK (Cellules humaines épithéliales issues d'adénocarcinome rénal).

### 1.2 Principe du Test

Le test est un test cellulaire basé sur le principe de complémentation d'enzyme, l'enzyme utilisée ici étant la beta galactosidase. Les cellules HEKEA sont des cellules HEK exprimant de façon stable et restreinte dans leur noyau, la beta galactosidase mutante (fragment omega aussi appelé EA) (lignée vendue par DiscoverX). Cette construction permet d'avoir une activité beta galactosidase uniquement quand la protéine comportant le fragment de complémentation Prolabel a migré dans le noyau.

La protéine d'intérêt comportant le fragment Prolabel est ici un HIF1alpha ou HIF1alpha muté sur les 2 Prolines 402 et 564 remplacées par des Alanines, est fusionné coté C_terminal par biologie moléculaire (vecteur DiscoverX vendu par Clontech) avec le petit fragment peptidique de complémentation (Prolabel ou ED, 4 kDa environ). Le vecteur codant ensuite pour la protéine chimère HIF1alpha_Prolabel est ensuite transfecté dans des cellules HEKEA pour l'obtention de clones stables (HEKEA_HIF1alphaPLBL)

La quantité de protéine HIF1alpha « taggué »Prolabel en position C-terminale obtenue après traitement des cellules à l'hypoxie ou des composés potentiellement activateurs de HIF est mesurée par ajout aux cellules d'un tampon de lyse contenant un substrat chemiluminescent de la beta galactosidase.

La mesure de l'activité beta galactosidase sera proportionnelle à la quantité de Prolabel donc de HIF1 alpha ayant migré dans le noyau des cellules.

Des expériences ont été réalisées en interne en parallèle pour valider que le fragment Prolabel seul n'était pas stable dans les cellules et ne permettait donc pas la mesure d'une activité.

### 1.3 Protocole

### 1.3. 1 Plan de l'expérience

1) Ensemencement des cellules à J 0
2) Adhérence 24 heures en normoxie
3) Préparation et ajout des produits (Biomek 2000 et FX) J+1
4) Incubation en Normoxie pendant 6h
5) Lecture des plaques (en luminescence)

### 1.3.2 Ensemencement des cellules

Les cellules sont ensemencées avec le Multidrop en plaques 384 puits blanches à fond opaque (Greiner ref 3704), dans 30µl de milieu de culture (1% SVF) à 10.000 cellules/puits (plaque cellules).

### 1.3.3 Traitement

### • Préparation de la plaque de dilution (plaque DL)

Les produits à tester sont préparés à 3 x 10⁻² M dans 100% DMSO puis dilués à 3x10⁻⁴M dans le milieu à 0.1% SVF (10µl dans 990µl MEM). Ils sont ensuite déposés à la main dans la colonne 12 d'une plaque 96 puits à fond rond (200 µl de chaque composé) appelée plaque de dilution (dl). La plaque dl complète de 3x 10⁻⁴M à 10⁻⁹M est alors réalisée par le Biomek 2000 (programme : Gamme 10 points en série). Pour les références et contrôles, 100µl de DMEM 0.1% SVF sont ajoutés en colonne 1, 100µl de Deferoxamine 10⁻³M en colonne 2, puits A B C D et 100 µl de Deferoxamine 5x10⁻³ M colonne 2, puits E F G H.

### • Distribution plaque DL dans plaques cellules

3.3 µL sont prélevés de la plaque DL par pipetage avec le Biomek FX 96 pour être déposés en duplicate horizontaux (colonne 1 à 24).dans chaque plaque cellules 384 puits (plaque cellules HEKEA_HIF1alphaPLBL)

Les cellules sont alors déposées pendant 6 h dans un incubateur à 37°C (O₂ ambiant, 6% CO₂).

### 1.3.4 Mesure de l'activité beta galactosidase.

Le Kit utilisé est le Kit chemiluminescent PROLABEL(Ref 93-0001 DiscoverX)

Après les 6 h d'incubation à 37°C les cellules sont lysées avec ajout de15 µl de tampon de lyse contenant le substrat de la beta galactosidase (19 volumes de Path hunter cell assay buffer + 5 volumes de Emarald II solution +1 volume de Galacton star) directement rajouté au 30 µl de milieu dans la plaque.Les plaques sont incubées 60 minutes à l'abri de la lumière avant lecture de la luminescence au Top Count. Les EC50 des composés sont ensuite calculés avec un logiciel de fitting approprié et reportés dans le **tableau III** ci-dessous.

L'activité activatrice d'un composé vis à vis de HIF est donnée par la concentration molaire qui produit 50% de la réponse maximale de ce même composé.

**TABLEAU III**

| **N°composé** | **EC50 (M)** |
|---|---|
| **14** | **3,8E-06** |
| **31** | **3,0E-06** |
| **37** | **3,2E-06** |
| **38** | **4,7E-06** |
| **39** | **1,8E-06** |
| **42** | **4,8E-06** |
| **43** | **3,1E-06** |
| **44** | **3,1E-06** |
| **46** | **9,6E-06** |
| **47** | **1,1E-06** |
| **48** | **4,3E-06** |
| **51** | **4,4E-06** |
| **53** | **2,6E-06** |
| **54** | **6,4E-06** |
| **56** | **2,1E-06** |
| **58** | **5,7E-06** |
| **59** | **1,7E-06** |
| **62** | **1,6E-05** |
| **63** | **9,2E-06** |
| **64** | **4,3E-06** |
| **68** | **1,5E-06** |
| **70** | **3,5E-06** |
| **71** | **5,0E-06** |
| **72** | **7,5E-06** |
| **74** | **3,7E-06** |
| **76** | **2,0E-06** |
| **77** | **4,8E-06** |
| **80** | **3,3E-06** |
| **81** | **7,5E-06** |
| **82** | **1,5E-06** |
| **83** | **3,3E-06** |
| **84** | **9,9E-06** |
| **85** | **1,8E-06** |
| **87** | **9,8E-06** |
| **88** | **8,8E-07** |
| **92** | **8,6E-07** |
| **93** | **3,5E-07** |
| **98B** | **2.5E-06** |

### 1.4 Divers

### 1.4.1. Entretien des cellules HEKEA HIF1alpha PLBL.

Les cellules sont cultivées en milieu complet (cf ci dessous) en Flask T225. à 37°C dans un incubateur à CO₂

### 1.4.2. Milieu de culture des cellules HEKEA HIF1alpha PLBL

| | |
|---|---|
| DMEM | 500 mL |
| +SVF10%(GIBCO 10500-056) | 50 mL |
| +Glutamine (2mM final) | 5 mL |
| +Penicillline + streptomycine (200mg/mL) | 5 mL |
| +Hygromycine B (100µg/mL) | 1.1 mL |
| +Geneticine (400µg/mL final) | 4.4 mL |

### 2. Mesure de la sécrétion de VEGF et d'EPO par les hépatocytes Hep3B

### 2.1. Objectif

HIF est un facteur de transcription impliqué dans l'adaptation des cellules à l'hypoxie Les gènes activés par HIF étant impliqués dans la réponse adaptative des cellules à l'hypoxie et des tissus à l'ischémie, l'objectif est d'identifier et de caractériser des composés qui stabilisent HIF1alpha dans les cellules afin d'amplifier ou de mimer son activité bénéfique. HIF1alpha a été identifié suite à l'analyse du promoteur du gène de l'EPO, ce qui fait de cette protéine un des marqueurs princeps de l'activation de HIF1alpha. D'autre part, le VEGF est également identifié dans la littérature comme un des principaux marqueurs de l'activation de HIF. C'est la raison pour laquelle la mesure de ces deux protéines a été retenue pour caractériser les composés activateurs de HIF dans les Hep3B.

L'objectif était donc de développer un test de screening sensible adaptable en plaques 96 puits permettant de mesurer directement la quantité de VEGF et d'EPO dans le surnageant des Hep3B. (Cellules issues d'hepato-carcinome humain) en réponse aux potentiels activateurs de HIF.

### 2.2. Principe du Test

Le test est un test ELISA permettant la mesure du VEGF et de l'EPO dans le surnageant des cellules Hep3B traitées par l'hypoxie ou la deferoxamine en contrôles ou les potentiels activateurs de HIF. Le test a été adapté en 96 puits permettant une plus grande capacité de screening des composés.

### 2.3. Protocole

### 2.3.1 Plan de l'expérience

1) Ensemencement des cellules à J 0
2) Adhérence 6heures en normoxie
3) Préparation et ajout des produits (Biomek 2000 et FX)
4) Incubation en normoxie pendant 18h
5) Dosage EPO et VEGF dans le surnageant à J+1

### 2.3.2 Ensemencement des cellules

Les cellules sont repiquées dans 100 µl de milieu de culture (10% SVF) en plaques 96 puits noires à fond opaque (référence Costar 3916) à 30.000 cellules/puits, avec le multidrop.

### 2.3.3 Traitement des cellules

### • Préparation de la plaque de dilution (plaque DL)

Les produits à tester sont préparés à 10⁻² M dans 100% DMSO puis dilués à 310⁴M dans le milieu à 0.1 % SVF (6 µl dans 194 µl MEM) Déposer 200 µl de chaque composé dans la colonne 12 d'une plaque dilution 96 puits Les gammes de dilution de 3x10⁻⁴M à 3x10⁻⁸M sont réalisées par le Biomek 2000 (programme : Gamme 9 points en série).100µl de MEM 0.1% SVF et de Deferoxamine 5x10⁻³M sont ajoutés comme contrôles en colonne 3 respectivement puits A,B,C,D et puits E,F,G,H

### • Distribution plaque DL dans plaques cellules

Le milieu des cellules ensemencées la veille en plaques 96 puits est changé pour 90 µl de milieu 0.1 % SVF et 10 µl sont distribués avec le FX 96 à partir des plaques DL 96 vers les plaques cellules

Les plaques cellules ainsi traitées sont déposées pendant 18 h dans un incubateur à 37°C (O₂ ambiant, 6% CO₂).

### 2.3.4 Dosage EPO et VEGF

Les surnageants (80 µl) des Hep3B en plaques 96 puits traités avec les potentiels activateurs de HIF sont récupérés à la pipette multicanaux pour dosage simultané du VEGF et de l'EPO en ELISA selon les instructions du fournisseur (Kit EPO Mesoscale (ref K15122B-2)). Les EC50 pour EPO et VEGF des composés sont ensuite calculés avec un logiciel de fitting approprié et reportés dans le **tableau IV** ci-dessous.

### 2.4. Divers

### Milieu de culture des cellules Hep3B:

| | |
|---|---|
| MEM + Earles (GIBCO 310095) | 500mL |
| + 10% SVF (GIBCO 10500-056) | 50mL |
| + Glutamine 2mM final | 5mL |
| + Acides aminés non essentiels 1% | 5mL |

### 3. Résultats

L'activité activatrice d'un composé vis à vis de HIF est donnée par la concentration qui produit 50% de la réponse maximale de ce même compose dans le **tableau IV** ci-dessous.

**TABLEAU IV**

| **N°composé** | **EC50 EPO (M)** | **EC50 VEGF (M)** |
|---|---|---|
| **14** | **3,0E-07** | **-** |
| **20** | **1 E-06** | **1.1E-06** |
| **31** | **2,1E-06** | **-** |
| **34** | **9 E-07** | **-** |
| **37** | **1,0E-06** | - |
| **38** | **1,0E-06** | - |
| **39** | **2,0E-06** | - |
| **42** | **1,1E-06** | **1,3E-06** |
| **43** | **1,5E-06** | **1,7E-06** |
| **44** | **1,9E-06** | **2,1E-06** |
| **46** | **7,0E-07** | **1,2E-06** |
| **47** | **2,0E-06** | **2,8E-06** |
| **48** | **1,1E-06** | **1,9E-06** |
| **51** | **3,0E-07** | **4,0E-07** |
| **53** | **9,0E-07** | **3,2E-06** |
| **54** | **4,5E-07** | **3,0E-07** |
| **56** | **2E-06** | **2,5E-06** |
| **58** | **3,2E-06** | **3,6E-06** |
| **59** | **8,1E-07** | **6,8E-07** |
| **62** | **9,0E-07** | **1,0E-06** |
| **64** | **1,4E-06** | **2,4E-06** |
| **68** | **4,0E-07** | **5,0E-07** |
| **70** | **2,7E-06** | **2,9E-06** |
| **71** | **1,6E-06** | **1,6E-06** |
| **72** | **1,0E-06** | **1,7E-06** |
| **74** | **3E-07** | |
| **76** | **2,0E-07** | **2,0E-07** |
| **77** | **1,4E-06** | **1,3E-06** |
| **80** | **4,0E-07** | **9,0E-07** |
| **81** | **1,2E-06** | **1,9E-06** |
| **82** | **1E-06** | **1,2E-06** |
| **83** | **1,4E-06** | **1,3E-06** |
| **84** | **2,9E-06** | **3,3E-06** |
| **85** | **2,5E-06** | **2,5E-06** |
| **88** | **3,0E-07** | **4,0E-07** |
| **92** | **5,0E-07** | **5,0E-07** |
| **93** | **4,0E-07** | **4,0E-07** |

Les composés selon l'invention peuvent donc être utilisés pour la préparation de médicaments, en particulier de médicaments activateurs du facteur de transcription HIF. Ainsi, selon un autre de ses aspects, l'invention a pour objet des médicaments qui comprennent un composé de formule (I), ou un sel d'addition de ce dernier à un acide pharmaceutiquement acceptable du composé de formule (I).

L'invention concerne également une composition pharmaceutique comprenant un composé de formule (I) selon la présente invention, ou un sel pharmaceutiquement acceptable de ce composé, ainsi qu'au moins un excipient pharmaceutiquement acceptable.

Ces médicaments trouvent leur emploi en thérapeutique, notamment dans le traitement/la prophylaxie en particulier de maladies cardiovasculaires, l'ischémie des membres inférieurs, l'insuffisance cardiaque, les maladies coronariennes d'origine ischémique comme l'angine de poitrine ou l'infarctus du myocarde, l'arterosclérose, les accidents vasculaires cérébraux d'origine ischémique, l'hypertension pulmonaire et toutes les pathologies provoquée par une occlusion vasculaire partielle ou totale chez l'homme et l'animal.

Ces médicaments trouvent également leur emploi en thérapeutique dans le traitement/la prophylaxie des glaucomes, des maladies rénales ou dans les maladies cérébrales d'origine neurodégénératives ou pas, des anémies, ou d'un médicament destiné à favoriser la cicatrisation ou agents ou à raccourcir la période de reconvalescence post-opératoire ou d'un médicament destiné au traitement des états de fatigue générale ou encore d'un médicament utilisé dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique.

Ces composés trouvent leur emploi en thérapeutique, notamment dans le traitement/la prophylaxie des anémies.

Ces composés sont également utilisables chez l'homme et l'animal dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique

Ces composés sont potentiellement utilisables chez l'homme et l'animal en tant qu'agents favorisant la cicatrisation ou agents permettant de racourcir la période de reconvalescence post opératoire.

Ces composés sont potentiellement utilisables chez l'homme et l'animal dans le traitement des états de fatigue générale allant jusqu'à la cachexie apparaissant en particulier chez les sujets agés.

Ces composés sont potentiellement utilisables chez l'homme et l'animal dans le traitement des glaucones, des maladies rénales ou dans les maladies cérébrales d'origine neurodégénératives ou pas.

Enfin les composés décrits dans l'invention sont potentiellement utilisables chez l'homme et l'animal pour le traitement de maladies cardiaques ou périphériques d'origine ischémique via une médecine régénérative dans des approches autologues et hétérologues utilisant des cellules souches non embryonnaires ou de cellules myoblastiques à des fins thérapeutiques que ce soit en traitement de ces cellules avant administration ou en traitement simultané à l'administration locale de ces cellules.

D'autre part, les composés décrits dans l'invention peuvent être utilisés seuls ou si nécessaire en combinaison avec un ou des autres composé(s) actif(s) utiles dans le traitement de l'hypertension, de l'insuffisance cardiaque, du diabète et de l'anémie.

Par exemple, on peut citer l'association d'un composé selon l'invention avec un ou plusieurs composés choisis parmi des inhibiteurs de l'enzyme de conversion, des antagonistes du récepteur de l'angiotensine II, des beta blockants, des antagonistes du récepteur aux mineralocorticoides, des diurétiques, des antagonistes calciques, des statines et des dérivés de la digitaline.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également des composés de formule (I) pour utilisation dans une méthode de traitement des pathologies ci-dessus indiquées qui comprend l'administration, à un patient, d'une dose efficace d'un composé selon l'invention, ou un de ses sels pharmaceutiquement acceptables.

## Revendications

1. composé répondant à la formule (I) suivante: 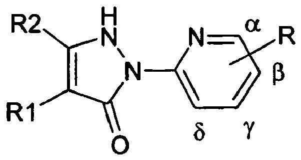 dans laquelle
**R** représente un groupe -SO₂-NR3R4, ou un groupe -SO₂-R4 ; **R3, R4** et **R5** étant tels que définis ci-dessous ;
**R1** représente un groupe hétérocycloalkyle ne contenant pas d'atome d'azote, un groupe -W-(C3-C6)cycloalkyle, un groupe W-aryle, un groupe -W-hétéroaryle, un groupe -W-hétérocycloalkyle, un groupe -W-COOR5 ou un groupe -W-CONR5R6,
(i) lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes-(C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkylène-O-(C1-C5)alkyle, les groupes -O-(C1-C5)alkylène-NR5R6, les groupes -SO₂-(C1-C5)alkyle, les groupes -NR5R6 et les groupes - CO₂R5, et
(ii) étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
**R2** représente un atome d'hydrogène, un groupe -(C1-C5)alkyle, un groupe -(C1-C5)alkylène-O-(C1-C5)alkyle, un groupe -halogéno(C1-C5)alkyle, un groupe -W-COOR5, un groupe -W-C(O)NHR5 ou un groupe -W-C(O)-NR5R6 ; **W, R5** et **R6** étant tels que définis ci-dessous;
étant entendu que :
o **n** représente 0, 1 ou 2 ;
o **W** est
(i) un groupe -(C1-C5)alkylène-, éventuellement substitué par un groupe choisi parmi les groupes -(CH₂)n-CO₂R5 et les groupes -(CH₂)n-(CO)NR5R6, avec n tel que défini ci-dessus et **R5** et **R6** tels que définis ci-dessous ;ou
(ii) un groupe -(C3-C6)cycloalkylène-,
o **R3** et **R4**
(i) Identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe -W-CHOH-CH₂OH, un groupe -W-CO₂R5, un groupe -W-NR5R6 ou un groupe -W-O-(CH₂)n-aryle;
lesdits groupes -(C3-C6)cycloalkyle et hétérocycloalkyle étant éventuellement substitués
o sur au moins un atome de carbone par au moins un groupe choisi parmi -(C1-C5)alkyle, un groupe -(C1-C5)alcoxy, une fonction hydroxy, un groupe -W-NR5R6 et un groupe -W-CO₂R5, dans le cas des groupes -(C3-C6)cycloalkyle et hétérocycloalkyle et/ou
o sur au moins un hétéroatome choisi parmi l'azote par au moins un groupe choisi parmi -(C1-C5)alkyle dans le cas d'un groupe hétérocycloalkyle,
avec **W** et n tels que définis précédemment et **R5** et **R6** tels que définis ci-dessous et étant entendu que lorsque **R3** et **R4** sont identiques, ils ne peuvent pas être un atome d'hydrogène;
(ii) ou bien **R3** et **R4** forment ensemble avec l'atome d'azote qui les porte un groupe hétérocycloalkyle, éventuellement substitué sur au moins un atome de carbone et/ou, le cas échéant, sur au moins un hétéroatome, par au moins un substituant choisi parmi les groupes -(C1-C5)alkyle et les groupes -CH₂-aryle ;
○ **R5** et **R6,** identiques ou différents, représentent, indépendamment l'un de l'autre, un atome d'hydrogène, un groupe -(C1-C5)alkyle ou un groupe (C1-C5) halogénoalkyle,
à l'état de base ou de sel d'addition à un acide.

2. Composé répondant à la formule (I) selon la revendication 1, **caractérisé en ce que** :
**R** représente un groupe -SO₂-NR3R4, ou un groupe -SO₂-R4 ; **R3, R4** et **R5** étant tels que définis ci-dessous ;
et/ou
**R1** représente un groupe hétérocycloalkyle ne contenant pas d'atome d'azote, un groupe -W-(C3-C6)cycloalkyle, un groupe -W-aryle, un groupe -W-hétéroaryle, un groupe -W-hétérocycloalkyle, un groupe -W-COOR5 ou un groupe -W-CONR5R6,
(i) lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes -(C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkyléne-O-(C1-C5)alkyle, les groupes -O-(C1-C5)alkylène-NR5R6, les groupes -SO₂-(C1-C5)alkyle, les groupes -NR5R6 et les groupes - CO₂R5, et
(ii) étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
et/ou
**R2** représente un atome d'hydrogène, un groupe -(C1-C5)alkyle, un groupe -(C1-C5)alkylène-O-(C1-C5)alkyle, un groupe -halogéno(C1-C5)alkyle, un groupe -W-COOR5, un groupe -W-C(O)NHR5 ou un groupe W-C(O)-NR5R6 ; **W, R5** et **R6** étant tels que définis ci-dessous;
et/ou
**n** représente 0, 1 ou 2 ;
et/ou
**W** est
(i) un groupe -(C1-C5)alkylène-, éventuellement substitué par un groupe choisi parmi les groupes -(CH₂)n-CO2R5 et les groupes -(CH₂)n-(CO)NR5R6, avec n tel que défini ci-dessus et **R5** et **R6** tels que définis ci-dessous ;ou
(ii) un groupe -(C3-C6)cycloalkylène-,
et/ou
**R3** et **R4**
(i) Identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe -W-CHOH-CH₂OH, un groupe -W-CO₂R5, un groupe -W-NR5R6 ou un groupe -W-O-(CH₂)n-aryle;
lesdits groupes -(C3-C6)cycloalkyle et hétérocycloalkyle étant éventuellement substitués
o sur au moins un atome de carbone par au moins un groupe choisi parmi -(C1-C5)alkyle, un groupe -(C1-C5)alcoxy, une fonction hydroxy, un groupe -W-NR5R6 et un groupe -W-CO₂R5, dans le cas des groupes -(C3-C6)cycloalkyle et hétérocycloalkyle et/ou
o sur au moins un hétéroatome choisi parmi l'azote par au moins un groupe choisi parmi -(C1-C5)alkyle, dans le cas d'un groupe hétérocycloalkyle,
avec **W** et **n** tels que définis précédemment et **R5** et **R6** tels que définis ci-dessous et étant entendu que lorsque **R3** et **R4** sont identiques, ils ne peuvent pas être un atome d'hydrogène;
(ii) ou bien **R3** et **R4** forment ensemble avec l'atome d'azote qui les porte un groupe hétérocycloalkyle, éventuellement substitué sur au moins un atome de carbone et/ou, le cas échéant, sur au moins un hétéroatome, par au moins un substituant choisi parmi les groupes -(C1-C5)alkyle et les groupes -CH₂-aryle ;
et/ou
**R5** et **R6,** identiques ou différents, représentent, indépendamment l'un de l'autre, un atome d'hydrogène, un groupe -(C1-C5)alkyle ou un groupe (C1-C5) halogénoalkyle.

3. Composé de formule (I) selon l'une quelconque des revendications 1 à 2, **caractérisé en ce que R** représente un groupe -SO₂-NR3R4 avec **R3** et **R4** tel que définis dans la revendication 1.

4. Composé de formule (I) selon l'une quelconque des revendications 1 à 2, **caractérisé en ce que R** représente un groupe -SO₂-R4 avec **R4** et **R5** tels que définis dans la revendication 1.

5. Composé de formule (I) selon l'une quelconque des revendications 1 à 4, **caractérisé en ce que** R est un substituant de l'atome situé en position β de la pyridine.

6. Composé de formule (I) selon l'une quelconque des revendications 1 à 5, **caractérisé en ce que R1** représente un groupe hétérocycloalkyle ne contenant pas d'atome d'azote, un groupe -W-(C3-C6)cycloalkyle, un groupe W-aryle, un groupe W-hétéroaryle, un groupe -W-hétérocycloalkyle, un groupe -W-COOR5 ou un groupe -W-CONR5R6,
lesdits groupes aryle, hétéroaryle et hétérocycloalkyle étant éventuellement substitués sur au moins un atome de carbone par au moins un substituant choisi parmi les atomes d'halogène, les groupes (C1-C5)alkyle, les groupes -(C1-C5)alkylène-O-(C1-C5)alkyle, les groupes -(C1-C5)alcoxy, une fonction hydroxy, les groupes -halogéno(C1-C5)alkyle, une fonction cyano, les groupes -O(C1-C5)alkylène-O-(C1-C5)alkyle, les groupes -O-(C1-C5)alkylène-NR5R6, les groupes -SO₂-(C1-C5)alkyle, les groupes - NR5R6 et les groupes - CO₂R5, et
étant entendu que lorsqu'il s'agit d'un groupe hétérocycloalkyle, ledit groupe comprenant au moins un atome d'azote, ce dernier peut éventuellement porter un substituant choisi parmi les groupes (C1-C5)alkyle,
où **W** est un groupe (C1-C5)alkylène ou un groupe (C3-C6)cycloalkylène et où **R5** et **R6,** identiques ou différents, représentent, indépendamment l'un de l'autre, un atome d'hydrogène, un groupe -(C1-C5)alkyle ou un groupe (C1-C5) halogénoalkyle.

7. Composé de formule (I) selon la revendication 6, **caractérisé en ce que** ledit groupe hétérocycloalkyle représente un groupe pipéridinyle, ledit groupe aryle représente un groupe phényle et ledit groupe hétéroaryle représente un groupe pyridine.

8. Composé de formule (I) selon l'une quelconque des revendications 1 à 7, **caractérisé en ce que R2** représente un atome d'hydrogène, un groupe -(C1-C5)alkyle, un groupe - (C1-C5)alkylène-O-(C1-C5)alkyle, un groupe -halogéno(C1-C5)alkyle, un groupe -W-COOR5, un groupe -W-C(O)NHR5 ou un groupe -W-C(O)-NR5R6 où **W, R5** et **R6** sont tels que définis dans la revendication 1.

9. Composé de formule (I) selon l'une quelconque des revendications 1 à 3 et 5 à 7, **caractérisé en ce que R** représente un groupe -SO₂-NR3R4, avantageusement situé en position β de la pyridine, et dans lequelle **R3** et **R4,** identiques ou différents, représentent, indépendamment l'un de l'autre, un atome d'hydrogène, un groupe -(C1-C5)alkyle, un groupe -(C3-C6)cycloalkyle, un groupe -(C1-C5)alkylène-O-(C1-C5)alkyle, un aryle, un groupe -CH₂-aryle, un hétéroaryle, un hétérocycloalkyle, un groupe -W-OH, un groupe - W-CHOH-CH₂OH, un groupe -W-CO₂R5, un groupe -W-NR5R6 ou un groupe -W-O-(CH₂)n-aryle;
étant entendu que :
lorsque **R3** et **R4** sont identiques, ils ne peuvent pas être un atome d'hydrogène;
et que
lorsque **R3** et/ou **R4** sont choisis parmi lesdits groupes -(C3-C6)cycloalkyle et hétérocycloalkyle, ces derniers peuvent éventuellement être substitués
o sur au moins un atome de carbone, par au moins un groupe choisi parmi -(C1-C5)alkyle, un groupe -(C1-C5)alcoxy, une fonction hydroxy, un groupe -W-NR5R6 et un groupe -W-CO₂R5, dans le cas des groupes -(C3-C6)cycloalkyle et hétérocycloalkyle, et/ou
o sur au moins un hétéroatome choisi parmi l'azote par au moins un groupe choisi parmi -(C1-C5)alkyle, dans le cas d'un groupe hétérocycloalkyle,
où **W, R5** et **R6** sont tels que définis selon la revendication 1 et où n représente 0, 1 ou 2.

10. Composé de formule (I) selon la revendication 9, **caractérisé en ce que** ledit groupe hétérocycloalkyle représente un groupe pipéridinyle, ledit groupe aryle représente un groupe phényle et ledit groupe hétéroaryle représente un groupe pyridine.

11. Composé de formule (I) selon l'une quelconque des revendications 1 à 3 et 5 à 7, **caractérisé en ce que** R représente un groupe -SO₂-NR3R4, avantageusement situé en position β de la pyridine, et dans lequelle **R3** et **R4** forment ensemble avec l'atome d'azote qui les porte un groupe hétérocycloalkyle, éventuellement substitué sur au moins un atome de carbone et/ou, le cas échéant, sur au moins un hétéroatome, par au moins un substituant choisi parmi les groupes -(C1-C5)alkyle et les groupes -CH₂-aryle.

12. Composé de formule (I) selon la revendication 11, **caractérisé en ce que** ledit groupe hétérocycloalkyle représente un groupe choisi parmi un groupe pipéridinyle, pipérazinyle, morpholinyle, pyrrolidinyle, hexaméthylèneimino, et ledit groupe aryle représente un groupe phényle.

13. Composé de formule (I) selon l'une quelconque des revendications 1 à 12, **caractérisé en ce que** le ou le(s) groupe(s) **R, R1** et/ou **R2** comprend ou comprennent le ou les groupe(s) **R5** et/ou **R6,**
**R5** ou **R6** est un atome d'hydrogène, un groupe -(C1-C5)alkyle ou un groupe (C1-C5) halogénoalkyle,ou
**R5** et **R6,** identiques ou différents, représentent, indépendamment l'un de l'autre, un atome d'hydrogène, un groupe -(C1-C5)alkyle ou un groupe (C1-C5) halogénoalkyle.

14. Composé de formule (I) selon la revendication 13, **caractérisé en ce que R5** et/ou **R6** sont choisis parmi les groupes (C1-C5)alkyle.

15. Composé de formule (I) selon l'une quelconque des revendications 1 à 13, **caractérisé en ce que R** représente un groupe -SO₂-NR3R4 ou un atome d'hydrogène avec R3 et R4 tels que définis dans la revendication 1 et/ou **R2** représente un atome d'hydrogène ou un groupe -(C1-C5)alkyle, avantageusement un méthyle, et/ou **R1** représente un groupe -W-aryle ou un groupe -W-hétéroaryle, avec avantageusement ledit W représentant un -CH₂-, ledit aryle représentant un phényle et ledit hétéroaryle représentant une pyridine.

16. Composé de formule (I) selon l'une quelconque des revendications 1 à 15, **caractérisé en ce qu'**il s'agit du :
• 4-(2-chlorobenzyl)-5-methyl-2-[5-(trifluoromethyl)pyridin-2-yl]-1,2-dihydro-3H-pyrazol-3-one ;
• Acide 6-[4-(2-chlorobenzyl)-3-methyl-5-oxo-2,5-dihydro-1H-pyrazol-1-yl]pyridine-3-carboxylique ;
• 4-(2-chlorobenzyl)-5-methyl-2-(pyridin-2-yl)-1,2-dihydro-3H-pyrazol-3-one ;
• Trifluoroacétate de 4-(2-chlorobenzyl)-5-methyl-2-{5-[(4-methylpiperazin-1-yl)sulfonyl]pyridin-2-yl}-1,2-dihydro-3H-pyrazol-3-one ;
• (+/-)4-(2-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
• 6-[4-(2-chlorobenzyl)-3-methyl-5-oxo-2,5-dihydro-1H-pyrazol-1-yl]-N,N-diethylpyridine-3-sulfonamide ;
• 6-[4-(2-chlorobenzyl)-3-methyl-5-oxo-2,5-dihydro-1H-pyrazol-1-yl]-N-ethyl-N-phenylpyridine-3-sulfonamide ;
• 6-[4-(2-chlorobenzyl)-3-methyl-5-oxo-2,5-dihydro-1H-pyrazol-1-yl]-N,N-di(propan-2-yl)pyridine-3-sulfonamide ;
• 4-(2-chlorobenzyl)-5-methyl-2-[5-(piperidin-1-ylsulfonyl)pyridin-2-yl]-1,2-dihydro-3H-pyrazol-3-one ;
• N,N-diethyl-6-[4-(2-fluorobenzyl)-3-methyl-5-oxo-2,5-dihydro-1H-pyrazol-1-yl]pyridine-3-sulfonamide ;
• 4-(2-fluorobenzyl)-5-methyl-2-(pyridin-2-yl)-1,2-dihydro-3H-pyrazol-3-one ;
• 4-(2-fluorobenzyl)-5-methyl-2-[5-(trifluoromethyl)pyridin-2-yl]-1,2-dihydro-3H-pyrazol-3-one ;
• Trifluoroacétate de 4-(2-fluorobenzyl)-5-methyl-2-{5-[(4-methylpiperazin-1-yl)sulfonyl]pyridin-2-yl}-1,2-dihydro-3H-pyrazol-3-one ;
• 2-(5-{[(3R,5S)-3,5-dimethylpiperidin-1-yl]sulfonyl}pyridin-2-yl)-4-(2-fluorobenzyl)-5-methyl-1,2-dihydro-3H-pyrazol-3-one ;
• N-ethyl-6-[4-(2-fluorobenzyl)-3-methyl-5-oxo-2,5-dihydro-1H-pyrazol-1-yl]-N-phenylpyridine-3-sulfonamide ;
• 4-(2-fluorobenzyl)-5-methyl-2-[5-(piperidin-1-ylsulfonyl)pyridin-2-yl]-1,2-dihydro-3H-pyrazol-3-one ;
• 4-(2,4-dichlorobenzyl)-5-methyl-2-(pyridin-2-yl)-1,2-dihydro-3H-pyrazol-3-one ;
• 4-(2,4-dichlorobenzyl)-5-methyl-2-[5-(trifluoromethyl)pyridin-2-yl]-1,2-dihydro-3H-pyrazol-3-one ;
• Trifiuoroacétate de 4-(2,4-dichlorobenzyl)-5-methyl-2-{5-[(4-methylpiperazin-1-yl)sulfonyl]pyridin-2-yl}-1,2-dihydro-3H-pyrazol-3-one ;
• 4-(2,4-dichlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
• 6-[4-(2,4-dichlorobenzyl)-3-methyl-5-oxo-2,5-dihydro-1H-pyrazol-1-yl]-N-ethyl-N-phenylpyridine-3-sulfonamide ;
• 6-[4-(2,4-dichlorobenzyl)-3-methyl-5-oxo-2,5-dihydro-1H-pyrazol-1-yl]-N,N-di(propan-2-yl)pyridine-3-sulfonamide ;
• 4-(2,4-dichlorobenzyl)-5-methyl-2-[5-(piperidin-1-ylsulfonyl)pyridin-2-yl]-1,2-dihydro-3H-pyrazol-3-one ;
• 4-(2-chloro-6-fluorobenzyl)-5-methyl-2-[5-(trifluoromethyl)pyridin-2-yl]-1,2-dihydro -3H-pyrazol-3-one ;
• Trifluoroacétate de 4-(2-chloro-6-fluorobenzyl)-5-methyl-2-{5-[(4-methylpiperazin-1-yl)sulfonyl]pyridin-2-yl}-1,2-dihydro-3H-pyrazol-3-one ;
• 4-(2-chloro-6-fluorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
• 6-[4-(2-chloro-6-fluorobenzyl)-3-methyl-5-oxo-2,5-dihydro-1H-pyrazol-1-yl]-N,N-diethylpyridine-3-sulfonamide ;
• 6-[4-(2-chloro-6-fluorobenzyl)-3-methyl-5-oxo-2,5-dihydro-1H-pyrazol-1-yl]-N-ethyl-N-phenylpyridine-3-sulfonamide ;
• 6-[4-(2-chloro-6-fluorobenzyl)-3-methyl-5-oxo-2,5-dihydro-1H-pyrazol-1-yl]-N,N-di(propan-2-yl)pyridine-3-sulfonamide ;
• 4-(2-chloro-6-fluorobenzyl)-5-methyl-2-[5-(piperidin-1-ylsulfonyl)pyridin-2-yl]-1,2-dihydro-3H-pyrazol-3-one ;
• methyl 6-[4-(2-chlorobenzyl)-3-methyl-5-oxo-2,5-dihydro-1H-pyrazol-1-yl]pyridine-3-carboxylate ;
• 4-(4-chlorobenzyl)-5-methyl-2-(pyridin-2-yl)-1,2-dihydro-3H-pyrazol-3-one ;
• 4-(4-chlorobenzyl)-5-methyl-2-{5-[(4-methylpiperazin-1-yl)sulfonyl]pyridin-2-yl}-1,2-dihydro-3H-pyrazol-3-one ;
• 4-(4-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one ;
• 6-[4-(4-chlorobenzyl)-3-methyl-5-oxo-2,5-dihydro-1H-pyrazol-1-yl]-N,N-diethylpyridine-3-sulfonamide ;
• 6-[4-(4-chlorobenzyl)-3-methyl-5-oxo-2,5-dihydro-1H-pyrazol-1-yl]-N-ethyl-N-phenylpyridine-3-sulfonamide ;
• 6-[4-(4-chlorobenzyl)-3-methyl-5-oxo-2,5-dihydro-1H-pyrazol-1-yl]-N,N-di(propan-2-yl)pyridine-3-sulfonamide ;
• 4-(4-chlorobenzyl)-5-methyl-2-[5-(piperidin-1-ylsulfonyl)pyridin-2-yl]-1,2-dihydro-3H-pyrazol-3-one ;
• 6-[4-(2-fluorobenzyl)-3-methyl-5-oxo-2,5-dihydro-1H-pyrazol-1-yl]-N,N-di(propan-2-yl)pyridine-3-sulfonamide ;
• 4-(1,1-dioxidotetrahydrothiophen-3-yl)-5-methyl-2-[5-(piperidin-1-ylsulfonyl)pyridin-2-yl]-1,2-dihydro-3H-pyrazol-3-one ;
• 6-[4-(1,1-dioxidotetrahydrothiophen-3-yl)-3-methyl-5-oxo-2,5-dihydro-1H-pyrazol-1-yl]-N-ethyl-N-phenylpyridine-3-sulfonamide ;
• 6-[4-(1,1-dioxidotetrahydrothiophen-3-yl)-3-methyl-5-oxo-2,5-dihydro-1H-pyrazol-1-yl]-N,N-di(propan-2-yl)pyridine-3-sulfonamide ;
• 2-{[5-methyl-3-oxo-2-(pyridin-2-yl)-2,3-dihydro-1H-pyrazol-4-yl]methyl}benzonitrile ;
• 4-{[5-methyl-3-oxo-2-(pyridin-2-yl)-2,3-dihydro-1H-pyrazol-4-yl]methyl}benzonitrile;
• N-ethyl-6-{4-[4-(methoxymethyl)benzyl]-3-methyl-5-oxo-2,5-dihydro-1H-pyrazol-1-yl}-N-phenylpyridine-3-sulfonamide ;
• 4-[3-(methoxymethyl)benzyl]-5-methyl-2-(pyridin-2-yl)-1,2-dihydro-3H-pyrazol-3-one ;
• N-ethyl-6-{4-[3-(methoxymethyl)benzyl]-3-methyl-5-oxo-2,5-dihydro-1H-pyrazol-1-yl}-N-phenylpyridine-3-sulfonamide ;
• 6-[4-(3-cyanobenzyl)-3-methyl-5-oxo-2,5-dihydro-1H-pyrazol-1-yl]-N-ethyl-N-phenylpyridine-3-sulfonamide ;
• 6-[4-(4-cyanobenzyl)-3-methyl-5-oxo-2,5-dihydro-1H-pyrazol-1-yl]-N-ethyl-N-phenylpyridine-3-sulfonamide ;
• 5-methyl-2-(pyridin-2-yl)-4-(pyridin-4-ylmethyl)-1,2-dihydro-3H-pyrazol-3-one ;
• 3-{[5-methyl-3-oxo-2-(pyridin-2-yl)-2,3-dihydro-1H-pyrazol-4-yl]methyl}benzonitrile;
• 4-benzyl-2-(5-bromopyridin-2-yl)-5-methyl-1,2-dihydro-3H-pyrazol-3-one ;
• 6-[4-(2-cyanobenzyl)-3-methyl-5-oxo-2,5-dihydro-1H-pyrazol-1-yl]-N-ethyl-N-phenylpyridine-3-sulfonamide ;
• N-ethyl-6-{4-[3-(2-methoxyethoxy)benzyl]-3-methyl-5-oxo-2,5-dihydro-1H-pyrazol-1-yl}-N-phenylpyridine-3-sulfonamide ;
• 4-(3,5-dimethoxybenzyl)-5-methyl-2-(pyridin-2-yl)-1,2-dihydro-3H-pyrazol-3-one ;
• 5-methyl-4-[4-(methylsulfonyl)benzyl]-2-(pyridin-2-yl)-1,2-dihydro-3H-pyrazol-3-one ;
• N-ethyl-6-{3-methyl-4-[4-(methylsulfonyl)benzyl]-5-oxo-2,5-dihydro-1H-pyrazol-1-yl}-N-phenylpyridine-3-sulfonamide ;
• 4-[3-(2-methoxyethoxy)benzyl]-5-methyl-2-(pyridin-2-yl)-1,2-dihydro-3H-pyrazol-3-one ;
• 4-benzyl-5-methyl-2-(pyridin-2-yl)-1,2-dihydro-3H-pyrazol-3-one ;
• N-ethyl-6-[3-methyl-5-oxo-4-(pyridin-4-ylmethyl)-2,5-dihydro-1H-pyrazol-1-yl]-N-phenylpyridine-3-sulfonamide ;
• N-ethyl-6-[3-methyl-5-oxo-4-(pyridin-2-ylmethyl)-2,5-dihydro-1H-pyrazol-1-yl]-N-phenylpyridine-3-sulfonamide ;
• 5-methyl-2-(pyridin-2-yl)-4-(pyridin-3-ylmethyl)-1,2-dihydro-3H-pyrazol-3-one ;
• 6-(4-benzyl-3-methyl-5-oxo-2,5-dihydro-1H-pyrazol-1-yl)-N-ethyl-N-phenylpyridine-3-sulfonamide ;
• N-ethyl-6-[3-methyl-5-oxo-4-(pyridin-3-ylmethyl)-2,5-dihydro-1H-pyrazol-1-yl]-N-phenylpyridine-3-sulfonamide ;
• 4-(2,5-dimethoxybenzyl)-5-methyl-2-(pyridin-2-yl)-1,2-dihydro-3H-pyrazol-3-one ;
• 6-[4-(2,5-dimethoxybenzyl)-3-methyl-5-oxo-2,5-dihydro-1H-pyrazol-1-yl]-N-ethyl-N-phenylpyridine-3-sulfonamide ;
• N-ethyl-6-{3-methyl-4-[(1-methylpiperidin-4-yl)methyl]-5-oxo-2,5-dihydro-1H-pyrazol-1-yl}-N-phenylpyridine-3-sulfonamide ;
• N-ethyl-6-{3-methyl-5-oxo-4-[4-(trifluoromethyl)benzyl]-2,5-dihydro-1H-pyrazol-1-yl}-N-phenylpyridine-3-sulfonamide ;
• N-ethyl-6-{3-methyl-5-oxo-4-[3-(trifluoromethyl)benzyl]-2,5-dihydro-1H-pyrazol-1-yl}-N-phenylpyridine-3-sulfonamide ;
• 5-methyl-2-(pyridin-2-yl)-4-[3-(trifluoromethyl)benzyl]-1,2-dihydro-3H-pyrazol-3-one ;
• 5-methyl-2-(pyridin-2-yl)-4-[4-(trifluoromethyl)benzyl]-1,2-dihydro-3H-pyrazol-3-one ;
• 6-[4-(3,5-dimethoxybenzyl)-3-methyl-5-oxo-2,5-dihydro-1H-pyrazol-1-yl]-N-ethyl-N-phenylpyridine-3-sulfonamide ;
• 4-(4-hydroxybenzyl)-5-methyl-2-(pyridin-2-yl)-1,2-dihydro-3H-pyrazol-3-one ;
• N-ethyl-6-[4-(4-hydroxybenzyl)-3-methyl-5-oxo-2,5-dihydro-1H-pyrazol-1-yl]-N-phenylpyridine-3-sulfonamide ;
• 6-(4-{4-[2-(dimethylamino)ethoxy]benzyl}-3-methyl-5-oxo-2,5-dihydro-1H-pyrazol-1-yl)-N-ethyl-N-phenylpyridine-3-sulfonamide ;
• 6-{4-[4-(dimethylamino)benzyl]-3-methyl-5-oxo-2,5-dihydro-1H-pyrazol-1-yl}-N-ethyl-N-phenylpyridine-3-sulfonamide ;
• 5-methyl-2-(pyridin-2-yl)-4-[2-(trifluoromethyl)benzyl]-1,2-dihydro-3H-pyrazol-3-one ;
• 4-[4-(dimethylamino)benzyl]-5-methyl-2-(pyridin-2-yl)-1,2-dihydro-3H-pyrazol-3-one ;
• N-ethyl-6-{3-methyl-5-oxo-4-[2-(trifluoromethyl)benzyl]-2,5-dihydro-1H-pyrazol-1-yl}-N-phenylpyridine-3-sulfonamide ;
• 6-(4-benzyl-3-methyl-5-oxo-2,5-dihydro-1H-pyrazol-1-yl)-N-(3-methylbutyl)pyridine-3-sulfonamide ;
• 4-[3-(dimethylamino)benzyl]-5-methyl-2-(pyridin-2-yl)-1,2-dihydro-3H-pyrazol-3-one ;
• 6-{4-[3-(dimethylamino)benzyl]-3-methyl-5-oxo-2,5-dihydro-1H-pyrazol-1-yl}-N-ethyl-N-phenylpyridine-3-sulfonamide ;
• 4-(4-methoxybenzyl)-5-methyl-2-(pyridin-2-yl)-1,2-dihydro-3H-pyrazol-3-one ;
• N-ethyl-6-[4-(4-methoxybenzyl)-3-methyl-5-oxo-2,5-dihydro-1H-pyrazol-1-yl]-N-phenylpyridine-3-sulfonamide ;
• 4-(2-methoxybenzyl)-5-methyl-2-(pyridin-2-yl)-1,2-dihydro-3H-pyrazol-3-one ;
• 4-(3-methoxybenzyl)-5-methyl-2-(pyridin-2-yl)-1,2-dihydro-3H-pyrazol-3-one ;
• N-ethyl-6-[4-(3-methoxybenzyl)-3-methyl-5-oxo-2,5-dihydro-1H-pyrazol-1-yl]-N-phenylpyridine-3-sulfonamide ;
• 6-(4-{3-[2-(dimethylamino)ethoxy]benzyl}-3-methyl-5-oxo-2,5-dihydro-1H-pyrazol-1-yl)-N-ethyl-N-phenylpyridine-3-sulfonamide ;
• N-ethyl-6-[4-(2-methoxybenzyl)-3-methyl-5-oxo-2,5-dihydro-1H-pyrazol-1-yl]-N-phenylpyridine-3-sulfonamide ;
• 4-benzyl-5-methyl-2-[5-(morpholin-4-ylsulfonyl)pyridin-2-yl]-1,2-dihydro-3H-pyrazol-3-one ;
• N-ethyl-6-[4-(3-hydroxybenzyl)-3-methyl-5-oxo-2,5-dihydro-1H-pyrazol-1-yl]-N-phenylpyridine-3-sulfonamide ;
• 6-(4-benzyl-3-ethyl-5-oxo-2,5-dihydro-1H-pyrazol-1-yl)-N-ethyl-N-phenylpyridine-3-sulfonamide ;
• 4-(3-hydroxybenzyl)-5-methyl-2-(pyridin-2-yl)-1,2-dihydro-3H-pyrazol-3-one ;
• N-ethyl-6-{4-[4-(2-methoxyethoxy)benzyl]-3-methyl-5-oxo-2,5-dihydro-1H-pyrazol-1-yl}-N-phenylpyridine-3-sulfonamide ;
• 4-benzyl-5-ethyl-2-(pyridin-2-yl)-1,2-dihydro-3H-pyrazol-3-one ;
• 4-benzyl-5-methyl-2-[5-(pyrrolidin-1-ylsulfonyl)pyridin-2-yl]-1,2-dihydro-3H-pyrazol-3-one ;
• 2-[5-(azepan-1-ylsulfonyl)pyridin-2-yl]-4-benzyl-5-methyl-1,2-dihydro-3H-pyrazol-3-one ;
• N,N-diethyl-6-[3-methyl-5-oxo-4-(pyridin-3-ylmethyl)-2,5-dihydro-1H-pyrazol-1-yl]pyridine-3-sulfonamide ;
• chlorhydrate de N,N-diethyl-6-[3-methyl-5-oxo-4-(pyridin-3-ylmethyl)-2,5-dihydro-1 H-pyrazol-1-yl]pyridine-3-sulfonamide ;
• N,N-dimethyl-6-[3-methyl-5-oxo-4-(pyridin-3-ylmethyl)-2,5-dihydro-1H-pyrazol-1-yl]pyridine-3-sulfonamide ;
• Chlorhydrate de N,N-dimethyl-6-[3-methyl-5-oxo-4-(pyridin-3-ylmethyl)-2,5-dihydro-1 H-pyrazol-1-yl]pyridine-3-sulfonamide ;
• Chlorhydrate de 6-(4-benzyl-5-oxo-3-propyl-2,5-dihydro-1H-pyrazol-1-yl)-N-ethyl-N-phenylpyridine-3-sulfonamide ;
• Chlorhydrate de 6-[3-methyl-5-oxo-4-(pyridin-3-ylmethyl)-2,5-dihydro-1H-pyrazol-1-yl]-N-(propan-2-yl)pyridine-3-sulfonamide ;
• Chlorhydrate de 5-methyl-4-(pyridin-3-ylmethyl)-2-[5-(pyrrolidin-1-ylsulfonyl)pyridin-2-yl]-1,2-dihydro-3H-pyrazol-3-one ;
• Chlorhydrate de N-tert-butyl-6-[3-methyl-5-oxo-4-(pyridin-3-ylmethyl)-2,5-dihydro-1 H-pyrazol-1-yl]pyridine-3-sulfonamide ;
• Chlorhydrate de N-cyclopropyl-6-[3-methyl-5-oxo-4-(pyridin-3-ylmethyl)-2,5-dihydro-1H-pyrazol-1-yl]pyridine-3-sulfonamide ;
• Chlorhydrate de N-cyclopentyl-6-[3-methyl-5-oxo-4-(pyridin-3-ylmethyl)-2,5-dihydro-1H-pyrazol-1-yl]pyridine-3-sulfonamide ;
• N-ethyl-6-[3-methyl-5-oxo-4-(2-phenylethyl)-2,5-dihydro-1H-pyrazol-1-yl]-N-phenylpyridine-3-sulfonamide ;
• 5-methyl-4-(2-phenylethyl)-2-(pyridin-2-yl)-1,2-dihydro-3H-pyrazol-3-one ;
• Chlorhydrate de N-methyl-6-[3-methyl-5-oxo-4-(pyridin-3-ylmethyl)-2,5-dihydro-1 H-pyrazol-1-yl]-N-(pyridin-2-yl)pyridine-3-sulfonamide ;
• 2-{5-[(4-benzylpiperidin-1-yl)sulfonyl]pyridin-2-yl}-5-methyl-4-(pyridin-3-ylmethyl)-1,2-dihydro-3H-pyrazol-3-one ;
• N-ethyl-6-[3-methyl-5-oxo-4-(2-phenylpropan-2-yl)-2,5-dihydro-1H-pyrazol-1-yl]-N-phenylpyridine-3-sulfonamide ;
• N-ethyl-6-{4-[(6-methoxypyridin-2-yl)methyl]-3-methyl-5-oxo-2,5-dihydro-1H-pyrazol-1-yl}-N-phenylpyridine-3-sulfonamide ;
• Chlorhydrate de 6-[3-methyl-5-oxo-4-(pyridin-3-ylmethyl)-2,5-dihydro-1H-pyrazol-1-yl]-N-(pyridin-2-yl)pyridine-3-sulfonamide ;
• N-ethyl-6-[3-methyl-5-oxo-4-(1-phenylcyclopropyl)-2,5-dihydro-1H-pyrazol-1-yl]-N-phenylpyridine-3-sulfonamide ;
• Chlorhydrate de N-ethyl-6-{4-[(3-methoxypyridin-2-yl)methyl]-3-methyl-5-oxo-2,5-dihydro-1H-pyrazol-1-yl}-N-phenylpyridine-3-sulfonamide ;
• 6-[4-benzyl-3-(methoxymethyl)-5-oxo-2,5-dihydro-1H-pyrazol-1-yl]-N-ethyl-N-phenylpyridine-3-sulfonamide ;
• 6-[4-benzyl-5-oxo-3-(trifluoromethyl)-2,5-dihydro-1H-pyrazol-1-yl]-N-ethyl-N-phenylpyridine-3-sulfonamide ;
• 1-[5-(azepan-1-ylsulfonyl)pyridin-2-yl]-3-methyl-4-(pyridin-3-ylmethyl)-1H-pyrazol-5-olate ;
• N-ethyl-6{3-methyl-5-oxo-4-[2-(pyridin-2-yl)ethyl]-2,5-dihydro-1H-pyrazol-1-yl}-N-phenylpyridine-3-suffonamide ;
• N-ethyl-6-{4-[(5-methoxypyridin-3-yl)methyl]-3-methyl-5-oxo-2,5-dihydro-1H-pyrazol-1-yl}-N-phenylpyridine-3-sulfonamide ;
• N-(2-methoxyethyl)-N-methyl-6-[3-methyl-5-oxo-4-(pyridin-3-ylmethyl)-2,5-dihydro-1 H-pyrazol-1-yl]pyridine-3-sulfonamide;
• 6-[4-benzyl-3-(2-methylpropyl)-5-oxo-2,5-dihydro-1H-pyrazol-1-yl]-N-ethyl-N-phenylpyridine-3-sulfonamide ;
• N-ethyl-6-[3-methyl-5-oxo-4-(pyridin-3-ylmethyl)-2,5-dihydro-1H-pyrazol-1-yl]pyridine-3-sulfonamide ;
• 6-[3-methyl-5-oxo-4-(pyridin-3-ylmethyl)-2,5-dihydro-1H-pyrazol-1-yl]-N-phenylpyridine-3-sulfonamide ;
• N-ethyl-6-[3-methyl-5-oxo-4-(3-phenylpropyl)-2,5-dihydro-1H-pyrazol-1-yl]-N-phenylpyridine-3-sulfonamide ;
• 6-(4-benzyl-5-oxo-2,5-dihydro-1H-pyrazol-1-yl)-N-ethyl-N-phenylpyridine-3-sulfonamide ;
• N-cyclopropyl-N-methyl-6-[3-methyl-5-oxo-4-(pyridin-3-ylmethyl)-2,5-dihydro-1H-pyrazol-1-yl]pyridine-3-sulfonamide ;
• N-tert-butyl-6-{4-[(5-methoxypyridin-3-yl)methyl]-3-methyl-5-oxo-2,5-dihydro-1H-pyrazol-1-yl}pyridine-3-sulfonamide ;
• 6-{4-[(5-cyanopyridin-3-yl)methyl]-3-methyl-5-oxo-2,5-dihydro-1H-pyrazol-1-yl}-N-ethyl-N-phenylpyridine-3-sulfonamide ;
• tert-butyl 6-[5-oxo-4-(pyridin-3-ylmethyl)-2,5-dihydro-1H-pyrazol-1-yl]pyridine-3-carboxylate ;
• tert-butyl 6-[3-methyl-5-oxo-4-(pyridin-3-ylmethyl)-2,5-dihydro-1H-pyrazol-1-yl]pyridine-3-carboxylate ;
• N-tert-butyl-6-[5-oxo-4-(pyridin-3-ylmethyl)-2,5-dihydro-1H-pyrazol-1-yl]pyridine-3-sulfonamide ;
• chlorhydrate de N-tert-butyl-6-[5-oxo-4-(pyridin-3-ylmethyl)-2,5-dihydro-1H-pyrazol-1-yl]pyridine-3-sulfonamide ;
• N-ethyl-6-[5-oxo-4-(pyridin-3-ylmethyl)-2,5-dihydro-1H-pyrazol-1-yl]-N-phenylpyridine-3-sulfonamide ;
• 6-(4-benzyl-5-oxo-2,5-dihydro-1H-pyrazol-1-yl)-N-tert-butyl-N-methylpyridine-3-sulfonamide ;
• N-ethyl-6-{4-[(5-methoxypyridin-3-yl)methyl]-5-oxo-2,5-dihydro-1H-pyrazol-1-yl}-N-phenylpyridine-3-sulfonamide ;
• 5-methyl-2-[5-(phenylsulfonyl)pyridin-2-yl]-4-(pyridin-3-ylmethyl)-1,2-dihydro-3H-pyrazol-3-one ;
• N-tert-butyl-6-{4-[(5-methoxypyridin-3-yl)methyl]-3-methyl-5-oxo-2,5-dihydro-1H-pyrazol-1-yl}-N-methylpyridine-3-sulfonamide ;
• 6-{4-[(6-cyanopyridin-3-yl)methyl]-3-methyl-5-oxo-2,5-dihydro-1H-pyrazol-1-yl}-N-ethyl-N-phenylpyridine-3-sulfonamide ;
• Acide (2-{5-[ethyl(phenyl)sulfamoyl]pyridin-2-yl}-3-oxo-2,3-dihydro-1H-pyrazol-4-yl)acetique;
• 2-(2-(5-[ethyl(phenyl)sulfamoyl]pyridin-2-yl)-3-oxo-2,3-dihydro-1H-pyrazol-4-yl)-N,N-dimethylacetamide ;
• methyl (2-{5-[ethyl(phenyl)sulfamoyl]pyridin-2-yl}-3-oxo-2,3-dihydro-1H-pyrazol-4-yl)acetate ;
• ethyl (4-benzyl-1-{5-[ethyl(phenyl)sulfamoyl]pyridin-2-yl}-5-oxo-2,5-dihydro-1H-pyrazol-3-yl)acetate ;
• 2-(2-{5-[ethyl(phenyl)sulfamoyl]pyridin-2-yl}-3-oxo-2,3-dihydro-1H-pyrazol-4-yl)-N-methylacetamide ;
• N-tert-butyl-6-{4-[(5-methoxypyridin-2-yl)methyl]-3-methyl-5-oxo-2,5-dihydro-1H-pyrazol-1-yl}-N-methylpyridine-3-sulfonamide ;
• 2-(4-benzyl-1-{5-[ethyl(phenyl)sulfamoyl]pyridin-2-yl}-5-oxo-2,5-dihydro-1H-pyrazol-3-yl)-N,N-dimethylacetamide ;
• Acide 3-(2-{5-[ethyl(phenyl)sulfamoyl]pyridin-2-yl}-3-oxo-2,3-dihydro-1H-pyrazol-4-yl)propanoique ;
• methyl3-[(2-{5-[ethyl(phenyl)sulfamoyl]pyridin-2-yl}-5-methyl-3-oxo-2,3-dihydro-1H-pyrazol-4-yl)methyl]benzoate ;
• methyl 3-(2-{5-[ethyl(phenyl)sulfamoyl]pyridin-2-yl}-3-oxo-2,3-dihydro-1H-pyrazol-4-yl)propanoate ;
• methyl {2-[5-(tert-butylsulfamoyl)pyridin-2-yl]-3-oxo-2,3-dihydro-1H-pyrazol-4-yl}(phenyl)acetate ;
• methyl 2-[(2-{5-[ethyl(phenyl)sulfamoyl]pyridin-2-yl}-5-methyl-3-oxo-2,3-dihydro-1H-pyrazol-4-yl)methyl]benzoate ;
• N-cyclopentyl-N-ethyl-6-[5-oxo-4-(pyridin-3-ylmethyl)-2,5-dihydro-1H-pyrazol-1-yl]pyridine-3-sulfonamide ;
• N-cyclopentyl-N-methyl-6-[5-oxo--4-(pyridin-3-ylmethyl)-2,5-dihydro-1H-pyrazol-1-yl]pyridine-3-sulfonamide ;
• 2-[(1-{5-[ethyl(phenyl)sulfamoyl]pyridin-2-yl}-3-methyl-5-oxido-1H-pyrazol-4-yl)methyl]benzoate ;
• 2-[5-(azepan-1-ylsulfonyl)pyridin-2-yl]-4-(pyridin-3-ylmethyl)-1,2-dihydro-3H-pyrazol-3-one ;
• N-cyclopentyl-N-methyl-6-[5-oxo-4-(pyridin-4-ylmethyl)-2,5-dihydro-1H-pyrazol-1-yl]pyridine-3-sulfonamide ;
• N-cyclopentyl-N-ethyl-6-[5-oxo-4-(pyridin-4-ylmethyl)-2,5-dihydro-1H-pyrazol-1-yl]pyridine-3-sulfonamide ;
• methyl 3-(2-{5-[cyclopentyl(methyl)sulfamoyl]pyridin-2-yl}-3-oxo-2,3-dihydro-1H-pyrazol-4-yl)-3-phenylpropanoate ;
• N-(2,2-dimethylpropyl)-N-methyl-6-[5-oxo-4-(pyridin-3-ylmethyl)-2,5-dihydro-1H-pyrazol-1-yl]pyridine-3-sulfonamide ;
• 2,2-dimethylpropyl 6-[5-oxo-4-(pyridin-3-ylmethyl)-2,5-dihydro-1H-pyrazol-1-yl]pyridine-3-carboxylate ;
• methyl N-cyclopentyl-N-({6-[oxo-4-(pyridin-3-ylmethyl)-2,5-dihydro-1H-pyrazol-1-yl]pyridin-3-yl}sulfonyl)glycinate ;
• 6-(4-benzyl-5-oxo-2,5-dihydro-1H-pyrazol-1-yl)-N-[2-(benzyloxy)ethyl]-N-cyclopentylpyridine-3-sulfonamide ;
• 6-(4-benzyl-5-oxo-2,5-dihydro-1H-pyrazol-1-yl)-N-[3-(benzyloxy)propyl]-N-cyclopentylpyridine-3-sulfonamide ;
• 6-(4-benzyl-5-oxo-2,5-dihydro-1H-pyrazol-1-yl)-N-cyclopentyl-N-(3-hydroxypropyl)pyridine-3-sulfonamide ;
• 6-(4-benzyl-5-oxo-2,5-dihydro-1H-pyrazol-1-yl)-N-cyclopentyl-N-(2-hydroxyethyl)pyridine-3-sulfonamide ;
• methyl (1S,2R)-2-[methyl({6-[5-oxo-4-(pyridin-3-ylmethyl)-2,5-dihydro-1H-pyrazol-1-yl]pyridin-3-yl}sulfonyl)amino]cyclopentanecarboxylate ;
• 6-(4-benzyl-5-cxo-2,5-dihydro-1H-pyrazol-1-yl)-N-cyclopentyl-N-(2,3-dihydroxypropyl)pyridine-3-sulfonamide ;
• 6-(4-benzyl-5-oxo-2,5-dihydro-1H-pyrazol-1-yl)-N-cyclopentyl-N-[2-(dimethylamino)ethyl]pyridine-3-sulfonamide ;
• 4-benzyl-2-{5-[(4-methylpiperazin-1-yl)sulfonyl]pyridin-2-yl}-1,2-dihydro-3H-pyrazol-3-one ;
• 6-[4-(cyclopentylmethyl)-5-oxo-2,5-dihydro-1H-pyrazol-1-yl]-N-ethyl-N-phenylpyridine-3-sulfonamide ;
• 4-benzyl-2-{5-[(4-methyl-1,4-diazepan-1-yl)sulfonyl]pyridin-2-yl}-1,2-dihydro-3H-pyrazol-3-one ;
• N-(2,2-dimethylpropyl)-6-[5-oxo-4-(pyridin-3-ylmethyl)-2,5-dihydro-1H-pyrazol-1-yl]pyridine-3-sulfonamide ;
• N-(2-methylbutan-2-yl)-6-[5-oxo-4-(pyridin-3-ylmethyl)-2,5-dihydro-1H-pyrazol-1-yl]pyridine-3-sulfonamide ;
• 6-(4-benzyl-5-oxo-2,5-dihydro-1H-pyrazol-1-yl)-N-[(1R,3S)-3-(hydroxymethyl)cyclopentyl]-N-methylpyridine-3-sulfonamide ;
• 6-(4-benzyl-5-oxo-2,5-dihydro-1H-pyrazol-1-yl)-N-methyl-N-(1-methylpyrrolidin-3-yl)pyridine-3-sulfonamide ;
• 6-(4-benzyl-5-oxo-2,5-dihydro-1H-pyrazol-1-yl)-N-(2-methoxy-2-methylpropyl)pyridine-3-sulfonamide ;
• 2,2-dimethylpropyl 6-(4-benzyl-5-oxo-2,5-dihydro-1H-pyrazol-1-yl)pyridine-3-carboxylate ;
• 6-(4-benzyl-5-oxo-2,5-dihydro-1H-pyrazol-1-yl)-N-cyclopentyl-N-[(2R)-2,3-dihydroxypropyl]pyridine-3-sulfonamide ;
• 6-(4-benzyl-5-oxo-2,5-dihydro-1H-pyrazol-1-yl)-N-(2,3-dihydroxypropyl)-N-phenylpyridine-3-sulfonamide ;
• N-cyclopentyl-6-{4-[(4-methoxypyridin-3-yl)methyl]-5-oxo-2,5-dihydro-1H-pyrazol-1-yl}-N-methylpyridine-3-sulfonamide ;
• N-methyl-N-(2-methylbutan-2-yl)-6-[5-oxo-4-(pyridin-3-ylmethyl)-2,5-dihydro-1H-pyrazol-1-yl]pyridine-3-sulfonamide ;
• 3-(2-{5-[cyclopentyl(methyl)sulfamoyl]pyridin-2-yl}-3-oxo-2,3-dihydro-1H-pyrazol-4-yl)-3-phenyl-N-(2,2,2-trifluoroethyl)propanamide ;
• 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide ;
• 6-(4-benzyl-5-oxo-2,5-dihydro-1H-pyrazo)-1-yl)-N-cyclopentyl-N-[(2S)-2,3-dihydroxypropyl]pyridine-3-sulfonamide ;
• 2,2-dimethylpropyl 6-[5-oxo-4-(pyridin-4-ylmethyl)-2,5-dihydro-1H-pyrazol-1-yl]pyridine-3-carboxylate ;
• 6-(4-benzyl-5-oxo-2,5-dihydro-1H-pyrazol-1-yl)-N-[(1S,2S)-2-hydroxycyclopentyl]pyridine-3-sulfonamide ;
• N-tert-butyl-6-[5-oxo-4-(pyridin-4-ylmethyl)-2,5-dihydro-1H-pyrazol-1-yl]pyridine-3-sulfonamide ;
• 4-benzyl-2-(5-{[(3R)-3-(dimethylamino)pyrrolidin-1-yl]sulfonyl}pyridin-2-yl)-1,2-dihydro-3H-pyrazol-3-one ;
• 6-(4-benzyl-5-oxo-2,5-dihydro-1H-pyrazol-1-yl)-N-[1-(dimethylamino)-2-methylpropan-2-yl]pyridine-3-sulfonamide ;
• 6-(4-benzyl-5-oxo-2,5-dihydro-1H-pyrazol-1-yl)-N-methyl-N-phenylpyridine-3-sulfonamide ;
• 3-{2-[5-(tert-butylsulfamoyl)pyridin-2-yl]-3-oxo-2,3-dihydro-1H-pyrazol-4-yl}-3-(pyridin-3-yl)propanoate de méthyle ;
• 3-{2-[5-(tert-butylsulfamoyl)pyridin-2-yl]-3-oxo-2,3-dihydro-1H-pyrazol-4-yl}-3-phenylpropanoate d'éthyle ;
• 6-(4-benzyl-5-oxo-2,5-dihydro-1H-pyrazol-1-yl)-N-tert-butyl-N-(2,3-dihydroxypropyl)pyridine-3-sulfonamide ;
• 6-(4-benzyl-5-oxo-2,5-dihydro-1H-pyrazol-1-yl)-N-(2-hydroxyethyl)pyridine-3-sulfonamide ;
• Chlorhydrate de N-(2,3-dihydroxypropyl)-6-[5-oxo-4-(pyridin-3-ylmethyl)-2,5-dihydro-1H-pyrazol-1-yl]-N-phenylpyridine-3-sulfonamide ;
• Chlorhydrate de 3-(2-{5-[cyclopentyl (methyl)sulfamoyl]pyridin-2-yl}-3-oxo-2,3-dihydro-1H-pyrazol-4-yl)-3-(pyridin-3-yl) propanoate de méthyle ;
• Chlorhydrate de 6-(4-benzyl-5-oxo-2,5-dihydro-1H-pyrazol-1-yl)-N-cyclopentyl-N-[3-(dimethylamino) propyl]pyridine-3-sulfonamide;
• Chlorhydrate de 6-(4-benzyl-5-oxo-2,5-dihydro-1H-pyrazol-1-yl)-N-cyclopentyl-N-[3-(diméthylamino)propyl]pyridine-3-sulfonamide.

17. Procédé de préparation d'un composé de formule (I) selon l'une quelconque des revendications 1 à 16, **caractérisé en ce que** l'on fait réagir un composé de formule (II) 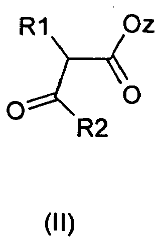 dans laquelle R1, R2 sont tels que définis dans la revendication 1 et z représente un groupe alkyle,
avec un composé de formule (III) 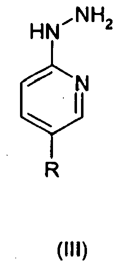 dans laquelle **R** est tel que défini dans la revendication 1.

18. Médicament, **caractérisé en ce qu'**il comprend un composé de formule (I) selon l'une quelconque des revendications 1 à 16, ou un sel d'addition de ce composé à un acide pharmaceutiquement acceptable du composé de formule (I).

19. Composition pharmaceutique, **caractérisée en ce qu'**elle comprend un composé de formule (I) selon l'une quelconque des revendications 1 à 16, ou un sel pharmaceutiquement acceptable de ce composé, ainsi qu'au moins un excipient pharmaceutiquement acceptable.

20. Utilisation d'un composé de formule (I) selon l'une quelconque des revendications 1 à 16 pour la préparation d'un médicament destiné au traitement/la prophylaxie de maladies cardiovasculaires.

21. Utilisation d'un composé de formule (I) selon l'une quelconque des revendications 1 à 16 pour la préparation d'un médicament destiné au traitement/la prophylaxie de l'ischémie des membres inférieurs, l'insuffisance cardiaque, les maladies coronariennes d'origine ischémique comme l'angine de poitrine ou l'infarctus du myocarde, l'arterosclérose, les accidents vasculaires cérébraux d'origine ischémique, l'hypertension pulmonaire et toutes les pathologies provoquées par une occlusion vasculaire partielle ou totale.

22. Utilisation d'un composé de formule (I) selon l'une quelconque des revendications 1 à 16 pour la préparation d'un médicament destiné au traitement/Ia prophylaxie des glaucomes, des maladies rénales ou dans les maladies cérébrales d'origine neurodégénératives ou pas, des anémies ou d'un médicament destiné à favoriser la cicatrisation ou agents ou à raccourcir la période de reconvalescence post-opératoire ou d'un médicament destiné au traitement des états de fatigue générale ou encore d'un médicament utilisé dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique.

23. Utilisation d'un composé de formule (I) selon l'une quelconque des revendications 1 à 16 pour la préparation d'un médicament destiné au traitement/Ia prophylaxie de maladies cardiaques ou périphériques d'origine ischémique via une médecine régénérative utilisant des cellules souches.

24. Association d'un composé de formule (I) selon l'une quelconque des revendications 1 à 16 avec un ou des autres composé(s) actif(s) utiles dans le traitement de l'hypertension, de l'insuffisance cardiaque, du diabète et de l'anémie.

## Patentansprüche

1. Verbindung der folgenden Formel (I): 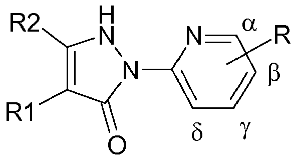 worin
**R für eine** -SO₂-NR3R4-Gruppe oder eine -SO₂-R4-Gruppe steht; wobei **R3, R4** und **R5** die nachstehend angegebene Bedeutung besitzen;
**R1** für eine kein Stickstoffatom enthaltende Heterocycloalkylgruppe, eine -W-(C3-C6)-Cycloalkylgruppe, eine -W-Arylgruppe, eine -W-Heteroarylgruppe, eine -W-Heterocycloalkylgruppe, eine -W-COOR5-Gruppe oder eine -W-CONR5R6-Gruppe steht,
(i) wobei die Aryl-, Heteroaryl- und Heterocycloalkylgruppen gegebenenfalls an mindestens einem Kohlenstoffatom durch mindestens einen Substituenten, der aus Halogenatomen, (C1-C5)-Alkylgruppen, -(C1-C5)-Alkylen-O-(C1-C5)-alkylgruppen, -(C1-C5)-Alkoxygruppen, einer Hydroxylfunktion, -Halogen-(C1-C5)-alkyl-gruppen, einer Cyanofunktion, -O-(C1-C5)-Alkylen-O-(C1-C5)-alkylgruppen, -O-(C1-C5)-Alkylen-NR5R6-Gruppen, -SO₂-(C1-C5)-Alkylgruppen, -NR5R6-Gruppen und -CO₂R5-Gruppen ausgewählt ist, substituiert sind, und
(ii) mit der Maßgabe, dass dann, wenn es sich um eine Heterocycloalkylgruppe mit mindestens einem Stickstoffatom handelt, dieses Atom gegebenenfalls einen aus (C1-C5)-Alkylgruppen ausgewählten Substituenten tragen kann,
**R2** für ein Wasserstoffatom, eine -(C1-C5)-Alkylgruppe, eine -(C1-C5)-Alkylen-O-(C1-C5)-alkylgruppe, eine -Halogen-(C1-C5)-alkylgruppe, eine -W-COOR5-Gruppe, eine -W-C(O)NHR5-Gruppe oder eine -W-C(O)-NR5R6-Gruppe steht; wobei **W, R5** und **R6** die nachstehend angegebene Bedeutung besitzen;
mit den Maßgaben, dass:
o **n** für 0, 1 oder 2 steht;
o **W** für
(i) eine -(C1-C5)-Alkylen-Gruppe, die gegebenenfalls durch eine aus -(CH₂)n-CO₂R5-Gruppen und -(CH₂)n-(CO)NR5R6-Gruppen ausgewählte Gruppen substituiert ist, wobei **n** die vorstehend angegebene Bedeutung besitzt und **R5** und **R6** die nachstehend angegebene Bedeutung besitzen; oder
(ii) eine - (C3-C6) -Cycloalkylen-Gruppe steht;
o **R3** und **R4**
(i) gleich oder verschieden sind und unabhängig voneinander für ein Wasserstoffatom, eine -(C1-C5)-Alkyl-gruppe, eine -(C3-C6)-Cycloalkylgruppe, eine -(C1-C5)-Alkylen-O-(C1-C5)-alkylgruppe, ein Aryl, eine -CH₂-Arylgruppe, ein Heteroaryl, ein Heterocycloalkyl, eine -W-OH-Gruppe, eine -W-CHOH-CH₂OH-Gruppe, eine -W-CO₂R5-Gruppe, eine -W-NR5R6-Gruppe oder eine -W-O-(CH₂)n-Arylgruppe stehen;
wobei die -(C3-C6)-Cycloalkyl- und Heterocycloalkylgruppen gegebenenfalls
o im Fall von - (C3-C6) -Cycloalkyl- und Heterocycloalkylgruppen an mindestens einem Kohlenstoffatom durch mindestens eine Gruppe, die aus -(C1-C5)-Alkyl, einer -(C1-C5)-Alkoxygruppe, einer Hydroxylfunktion, einer -W-NR5R6-Gruppe und einer -W-CO₂R5-Gruppe ausgewählt ist, und/oder
o im Fall einer Heterocycloalkylgruppe an mindestens einem aus Stickstoff ausgewählten Heteroatom durch mindestens eine Gruppe, die aus -(C1-C5)-Alkyl ausgewählt ist,
substituiert sind,
wobei **W** und **n** die vorstehend angegebene Bedeutung besitzen und **R5** und **R6** die nachstehend angegebene Bedeutung besitzen, und mit der Maßgabe, dass **R3** und **R4** dann, wenn sie gleich sind, nicht für ein Wasserstoffatom stehen können;
(ii) oder **R3** und **R4** zusammen mit dem Stickstoffatom, das sie trägt, eine Heterocycloalkylgruppe bilden, die gegebenenfalls an mindestens einem Kohlenstoffatom und/oder gegebenenfalls an mindestens einem Heteratom durch mindestens einen Substituenten, der aus - (C1-C5) -Alkylgruppen und -CH₂-Arylgruppen ausgewählt ist, substituiert ist;
o **R5** und **R6** gleich oder verschieden sind und unabhängig voneinander für ein Wasserstoffatom, eine-(C1-C5)-Alkylgruppe oder eine -(C1-C5)-Halogenalkylgruppe stehen,
in Basen- oder Säureadditionssalzform.

2. Verbindung der Formel (I) nach Anspruch 1, **dadurch gekennzeichnet, dass**:
**R** für eine -SO₂-NR3R4-Gruppe oder eine -SO₂-R4-Gruppe steht; wobei **R3, R4** und **R5** die nachstehend angegebene Bedeutung besitzen;
und/oder
**R1** für eine kein Stickstoffatom enthaltende Heterocycloalkylgruppe, eine -W-(C3-C6)-Cycloalkylgruppe, eine -W-Arylgruppe, eine -W-Heteroarylgruppe, eine -W-Heterocycloalkylgruppe, eine -W-COOR5-Gruppe oder eine -W-CONR5R6-Gruppe steht,
(i) wobei die Aryl-, Heteroaryl- und Heterocycloalkylgruppen gegebenenfalls an mindestens einem Kohlenstoffatom durch mindestens einen Substituenten, der aus Halogenatomen, (C1-C5)-Alkylgruppen, -(C1-C5)-Alkylen-O-(C1-C5)-alkylgruppen, -(C1-C5)-Alkoxygruppen, einer Hydroxylfunktion, -Halogen-(C1-C5)-alkyl-gruppen, einer Cyanofunktion, -0-(C1-C5)-Alkylen-O-(C1-C5)-alkylgruppen, -0-(C1-C5)-Alkylen-NR5R6-Gruppen, -SO₂-(C1-C5)-Alkylgruppen, -NR5R6-Gruppen und -CO₂R5-Gruppen ausgewählt ist, substituiert sind, und
(ii) mit der Maßgabe, dass dann, wenn es sich um eine Heterocycloalkylgruppe mit mindestens einem Stickstoffatom handelt, dieses Atom gegebenenfalls einen aus (C1-C5)-Alkylgruppen ausgewählten Substituenten tragen kann, und/oder
**R2** für ein Wasserstoffatom, eine -(C1-C5)-Alkylgruppe, eine -(C1-C5)-Alkylen-O-(C1-C5)-alkylgruppe, eine -Halogen-(C1-C5)-alkylgruppe, eine -W-COOR5-Gruppe, eine -W-C(O)NHR5-Gruppe oder eine -W-C(0)-NR5R6-Gruppe steht; wobei **W, R5** und
**R6** die nachstehend angegebene Bedeutung besitzen;
und/oder
**n** für 0, 1 oder 2 steht;
und/oder
**W** für
(i) eine -(C1-C5)-Alkylen-Gruppe, die gegebenenfalls durch eine aus -(CH₂)n-CO₂R5-Gruppen und -(CH₂)n-(CO)NR5R6-Gruppen ausgewählte Gruppen substituiert ist, wobei **n** die vorstehend angegebene Bedeutung besitzt und **R5** und **R6** die nachstehend angegebene Bedeutung besitzen; oder
(ii) eine - (C3-C6) -Cycloalkylen-Gruppe
steht;
und/oder
o **R3** und **R4**
(i) gleich oder verschieden sind und unabhängig voneinander für ein Wasserstoffatom, eine -(C1-C5)-Alkyl-gruppe, eine -(C3-C6)-Cycloalkylgruppe, eine -(C1-C5)-Alkylen-O-(C1-C5)-alkylgruppe, ein Aryl, eine -CH₂-Arylgruppe, ein Heteroaryl, ein Heterocycloalkyl, eine -W-OH-Gruppe, eine -W-CHOH-CH₂OH-Gruppe, eine -W-CO₂R5-Gruppe, eine -W-NR5R6-Gruppe oder eine -W-O-(CH₂)n-Aryl-gruppe stehen;
wobei die -(C3-C6)-Cycloalkyl- und Heterocycloalkylgruppen gegebenenfalls
o im Fall von - (C3-C6) -Cycloalkyl- und Heterocycloalkylgruppen an mindestens einem Kohlenstoffatom durch mindestens eine Gruppe, die aus -(C1-C5)-Alkyl, einer -(C1-C5)-Alkoxygruppe, einer Hydroxylfunktion, einer -W-NR5R6-Gruppe und einer -W-CO₂R5-Gruppe ausgewählt ist, und/oder
o im Fall einer Heterocycloalkylgruppe an mindestens einem aus Stickstoff ausgewählten Heteroatom durch mindestens eine Gruppe, die aus -(C1-C5)-Alkyl ausgewählt ist,
substituiert sind,
wobei **W** und **n** die vorstehend angegebene Bedeutung besitzen und **R5** und **R6** die nachstehend angegebene Bedeutung besitzen, und mit der Maßgabe, dass **R3** und **R4** dann, wenn sie gleich sind, nicht für ein Wasserstoffatom stehen können;
(ii) oder **R3** und **R4** zusammen mit dem Stickstoffatom, das sie trägt, eine Heterocycloalkylgruppe bilden, die gegebenenfalls an mindestens einem Kohlenstoffatom und/oder gegebenenfalls an mindestens einem Heteratom durch mindestens einen Substituenten, der aus - (C1-C5) -Alkylgruppen und -CH₂-Arylgruppen ausgewählt ist, substituiert ist;
und/oder
o **R5** und **R6** gleich oder verschieden sind und unabhängig voneinander für ein Wasserstoffatom, eine-(C1-C5)-Alkylgruppe oder eine -(C1-C5)-Halogenalkylgruppe stehen.

3. Verbindung der Formel (I) nach einem der Ansprüche 1 bis 2, **dadurch gekennzeichnet, dass R** für eine -SO₂-NR3R4-Gruppe steht, wobei **R3** und **R4** die in Anspruch 1 angegebene Bedeutung besitzen.

4. Verbindung der Formel (I) nach einem der Ansprüche 1 bis 2, **dadurch gekennzeichnet, dass** R für eine -SO₂-R4-Gruppe steht, wobei **R4** und **R5** die in Anspruch 1 angegebene Bedeutung besitzen.

5. Verbindung der Formel (I) nach einem der Ansprüche 1 bis 4, **dadurch gekennzeichnet, dass** R ein Substituent des Atoms in der β-Position des Pyridins ist.

6. Verbindung der Formel (I) nach einem der Ansprüche 1 bis 5, **dadurch gekennzeichnet, dass R1** für eine kein Stickstoffatom enthaltende Heterocycloalkylgruppe, eine -W-(C3-C6)-Cycloalkylgruppe, eine -W-Arylgruppe, eine -W-Heteroarylgruppe, eine -W-Heterocycloalkylgruppe, eine -W-COOR5-Gruppe oder eine -W-CONR5R6-Gruppe steht,
wobei die Aryl-, Heteroaryl- und Heterocycloalkylgruppen gegebenenfalls an mindestens einem Kohlenstoffatom durch mindestens einen Substituenten, der aus Halogenatomen, (C1-C5)-Alkylgruppen, -(C1-C5)-Alkylen-O-(C1-C5)-alkylgruppen, -(C1-C5)-Alkoxygruppen, einer Hydroxylfunktion, -Halogen-(C1-C5)-alkylgruppen, einer Cyanofunktion, -O-(C1-C5)-Alkylen-O-(C1-C5)-alkylgruppen, -O-(C1-C5)-Alkylen-NR5R6-Gruppen, -SO₂-(C1-C5)-Alkylgruppen, -NR5R6-Gruppen und -CO₂R5-Gruppen ausgewählt ist, substituiert sind, und
mit der Maßgabe, dass dann, wenn es sich um eine Heterocycloalkylgruppe mit mindestens einem Stickstoffatom handelt, dieses Atom gegebenenfalls einen aus (C1-C5)-Alkylgruppen ausgewählten Substituenten tragen kann,
wobei **W** für eine (C1-C5)-Alkylengruppe oder eine (C3-C6)-Cycloalkylengruppe steht und **R5** und **R6** gleich oder verschieden sind und unabhängig voneinander für ein Wasserstoffatom, eine -(C1-C5)-Alkylgruppe oder eine -(C1-C5)-Halogenalkylgruppe stehen.

7. Verbindung der Formel (I) nach Anspruch 6, **dadurch gekennzeichnet, dass** die Heterocycloalkylgruppe für eine Piperidinylgruppe steht, die Arylgruppe für eine Phenylgruppe steht und die Heteroarylgruppe für eine Pyridingruppe steht.

8. Verbindung der Formel (I) nach einem der Ansprüche 1 bis 7, **dadurch gekennzeichnet, dass R2** für ein Wasserstoffatom, eine -(C1-C5)-Alkylgruppe, eine -(C1-C5)-Alkylen-O-(C1-C5)-alkylgruppe, eine -Halogen-(C1-C5)-alkylgruppe, eine -W-COOR5-Gruppe, eine -W-C(O)NHR5-Gruppe oder eine -W-C(O)-NR5R6-Gruppe steht, wobei **W, R5** und **R6** die in Anspruch 1 angegebene Bedeutung besitzen.

9. Verbindung der Formel (I) nach einem der Ansprüche 1 bis 3 und 5 bis 7, **dadurch gekennzeichnet, dass**
**R** für eine -SO₂-NR3R4-Gruppe steht, die vorteilhafterweise in der β-position des Pyridins steht und worin **R3** und **R4,** gleich oder verschieden sind und unabhängig voneinander für ein Wasserstoffatom, eine -(C1-C5)-Alkylgruppe, eine -(C3-C6)-Cycloalkylgruppe, eine -(C1-C5)-Alkylen-O-(C1-C5)-alkylgruppe, ein Aryl, eine -CH₂-Arylgruppe, eine Heteroaryl, ein Heterocycloalkyl, eine -W-OH-Gruppe, eine -W-CHOH-CH₂OH-Gruppe, eine -W-CO₂R5-Gruppe, eine -W-NR5R6-Gruppe oder eine -W-O-(CH₂)n-Arylgruppe stehen;
mit den Maßgaben, dass:
**R3** und **R4** dann, wenn sie gleich sind, nicht für ein Wasserstoffatom stehen können; und dass dann, wenn **R3** und/oder **R4** aus den -(C3-C6)-Cycloalkyl- und Heterocycloalkylgruppen ausgewählt sind, diese Gruppen gegebenenfalls
o im Fall von - (C3-C6) -Cycloalkyl- und Heterocycloalkylgruppen an mindestens einem Kohlenstoffatom durch mindestens eine Gruppe, die aus -(C1-C5)-Alkyl, einer -(C1-C5)-Alkoxygruppe, einer Hydroxylfunktion, einer -W-NR5R6-Gruppe und einer -W-CO₂R5-Gruppe ausgewählt ist, und/oder
o im Fall einer Heterocycloalkylgruppe an mindestens einem aus Stickstoff ausgewählten Heteroatom durch mindestens eine Gruppe, die aus -(C1-C5)-Alkyl ausgewählt ist,
substituiert sind,
wobei **W, R5** und **R6** die in Anspruch 1 angegebene Bedeutung besitzen und **n** für 0, 1 oder 2 steht.

10. Verbindung der Formel (I) nach Anspruch 9, **dadurch gekennzeichnet, dass** die Heterocycloalkylgruppe für eine Piperidinylgruppe steht, die Arylgruppe für eine Phenylgruppe steht und die Heteroarylgruppe für eine Pyridingruppe steht.

11. Verbindung der Formel (I) nach einem der Ansprüche 1 bis 3 und 5 bis 7, **dadurch gekennzeichnet, dass R** für eine -SO₂-NR3R4-Gruppe steht, die vorteilhafterweise in der β-position des Pyridins steht und worin **R3** und **R4** zusammen mit dem Stickstoffatom, das sie trägt, eine Heterocycloalkylgruppe bilden, die gegebenenfalls an einem Kohlenstoffatom und/oder gegebenenfalls an mindestens einem Heteratom durch mindestens einen Substituenten, der aus -(C1-C5)-Alkylgruppen und -CH₂-Arylgruppen ausgewählt ist, substituiert ist.

12. Verbindung der Formel (I) nach Anspruch 11, **dadurch gekennzeichnet, dass** die Heterocycloalkylgruppe für eine aus einer Piperidinyl-, Piperazinyl-, Morpholinyl-, Pyrrolidinyl- und Hexamethyleniminogruppe ausgewählte Gruppe steht und die Arylgruppe für eine Phenylgruppe steht.

13. Verbindung der Formel (I) nach einem der Ansprüche 1 bis 12, **dadurch gekennzeichnet, dass** die Gruppe bzw. Gruppen **R, R1** und/oder **R2** die Gruppe bzw. die Gruppen **R5** und/oder **R6** umfasst bzw. umfassen,
**R5** oder **R6** für ein Wasserstoffatom, eine -(C1-C5)-Alkylgruppe oder eine -(C1-C5)-Halogenalkylgruppe steht bzw.
**R5** und **R6** gleich oder verschieden sind und unabhängig voneinander für ein Wasserstoffatom, eine -(C1-C5)-Alkylgruppe oder eine -(C1-C5)-Halogenalkylgruppe stehen.

14. Verbindung der Formel (I) nach Anspruch 13, **dadurch gekennzeichnet, dass R5** und/oder **R6** aus (C1-C5)-Alkylgruppen ausgewählt sind.

15. Verbindung der Formel (I) nach einem der Ansprüche 1 bis 13, **dadurch gekennzeichnet, dass R** für eine -SO₂-NR3R4-Gruppe oder ein Wasserstoffatom steht, wobei R3 und R4 die in Anspruch 1 angegebene Bedeutung besitzen, und/oder **R2** für ein Wasserstoffatom oder eine -(C1-C5)-Alkylgruppe, vorzugsweise ein Methyl, steht und/oder **R1** für eine -W-Arylgruppe oder eine -W-Heteroarylgruppe steht, wobei vorteilhafterweise das W für ein -CH₂-steht, das Aryl für ein Phenyl steht und das Heteroaryl für ein Pyridin steht.

16. Verbindung der Formel (I) nach einem der Ansprüche 1 bis 15, **dadurch gekennzeichnet, dass** es sich um
• 4-(2-Chlorbenzyl)-5-methyl-2-[5-(trifluormethyl)pyridin-2-yl]-1,2-dihydro-3H-pyrazol-3-on;
• 6-[4-(2-Chlorbenzyl)-3-methyl-5-oxo-2,5-dihydro-1H-pyrazol-1-yl]pyridin-3-carbonsäure;
• 4-(2-Chlorbenzyl)-5-methyl-2-(pyridin-2-yl)-1,2-dihydro-3H-pyrazol-3-on;
• 4-(2-Chlorbenzyl)-5-methyl-2-{5-[(4-methylpiperazin-1-yl)sulfonyl]pyridin-2-yl}-1,2-dihydro-3H-pyrazol-3-on-trifluoracetat;
• (±) 4-(2-Chlorbenzyl)-2-(5-{[(3R, 5S)-3,5-dimethylpiperidin-1-yl]sulfonyl}pyridin-2-yl)-5-methyl-1,2-dihydro-3H-pyrazol-3-on;
• 6-[4-(2-Chlorbenzyl)-3-methyl-5-oxo-2,5-dihydro-1H-pyrazol-1-yl]-N,N-diethylpyridin-3-sulfonamid;
• 6-[4-(2-Chlorbenzyl)-3-methyl-5-oxo-2,5-dihydro-1H-pyrazol-1-yl]-N-ethyl-N-phenylpyridin-3-sulfonamid;
• 6-[4-(2-Chlorbenzyl)-3-methyl-5-oxo-2,5-dihydro-1H-pyrazol-1-yl]-N,N-di(propan-2-yl)pyridin-3-sulfonamid;
• 4-(2-Chlorbenzyl)-5-methyl-2-[5-(piperidin-1-ylsulfonyl)pyridin-2-yl]-1,2-dihydro-3H-pyrazol-3-on;
• N,N-Diethyl-6-[4-(2-fluorbenzyl)-3-methyl-5-oxo-2,5-dihydro-1H-pyrazol-1-yl]pyridin-3-sulfonamid;
• 4-(2-Fluorbenzyl)-5-methyl-2-(pyridin-2-yl)-1,2-dihydro-3H-pyrazol-3-on;
• 4-(2-Fluorbenzyl)-5-methyl-2-[5-(trifluormethyl)pyridin-2-yl]-1,2-dihydro-3H-pyrazol-3-on;
• 4-(2-Fluorbenzyl)-5-methyl-2-{5-[(4-methylpiperazin-1-yl)sulfonyl]pyridin-2-yl}-1,2-dihydro-3H-pyrazol-3-on-trifluoracetat;
• 2-(5-{[(3R,5S)-3,5-Dimethylpiperidin-1-yl]sulfonyl}pyridin-2-yl)-4-(2-fluorbenzyl)-5-methyl-1,2-dihydro-3H-pyrazol-3-on;
• N-Ethyl-6-[4-(2-fluorbenzyl)-3-methyl-5-oxo-2,5-dihydro-1H-pyrazol-1-yl]-N-phenylpyridin-3-sulfonamid;
• 4-(2-Fluorbenzyl)-5-methyl-2-[5-(piperidin-1-ylsulfonyl)pyridin-2-yl]-1,2-dihydro-3H-pyrazol-3-on;
• 4-(2,4-Dichlorbenzyl)-5-methyl-2-(pyridin-2-yl)-1,2-dihydro-3H-pyrazol-3-on;
• 4-(2,4-Dichlorbenzyl)-5-methyl-2-[5-(trifluormethyl)pyridin-2-yl]-1,2-dihydro-3H-pyrazol-3-on;
• 4-(2,4-Dichlorbenzyl)-5-methyl-2-{5-[(4-methylpiperazin-1-yl)sulfonyl]pyridin-2-yl}-1,2-dihydro-3H-pyrazol-3-on-trifluoracetat;
• 4-(2,4-Dichlorbenzyl)-2-(5-{[(3R, 5S)-3,5-dimethylpiperidin-1-yl]sulfonyl}pyridin-2-yl)-5-methyl-1,2-dihydro-3H-pyrazol-3-on;
• 6-[4-(2,4-Dichlorbenzyl)-3-methyl-5-oxo-2,5-dihydro-1H-pyrazol-1-yl]-N-ethyl -N-phenylpyridin-3-sulfonamid;
• 6-[4-(2,4-Dichlorbenzyl)-3-methyl-5-oxo-2,5-dihydro-1H-pyrazol-1-yl]-N,N-di(propan-2-yl)pyridin-3-sulfonamid;
• 4-(2,4-Dichlorbenzyl)-5-methyl-2-[5-(piperidin-1-ylsulfonyl)pyridin-2-yl]-1,2-dihydro-3H-pyrazol-3-on;
• 4-(2-Chlor-6-fluorbenzyl)-5-methyl-2-[5-(trifluormethyl)pyridin-2-yl]-1,2-dihydro-3H-pyrazol-3-on;
• 4-(2-Chlor-6-fluorbenzyl)-5-methyl-2-{5-[(4-methylpiperazin-1-yl)sulfonyl]pyridin-2-yl}-1,2-dihydro-3H-pyrazol-3-on-trifluoracetat;
• 4-(2-Chlor-6-fluorbenzyl)-2-(5-{[(3R, 5S)-3,5-dimethylpiperidin-1-yl]sulfonyl}pyridin-2-yl)-5-methyl-1,2-dihydro-3H-pyrazol-3-on;
• 6-[4-(2-Chlor-6-fluorbenzyl)-3-methyl-5-oxo-2,5-dihydro-1H-pyrazol-1-yl]-N,N-diethylpyridin-3-sulfonamid;
• 6-[4-(2-Chlor-6-fluorbenzyl)-3-methyl-5-oxo-2,5-dihydro-1H-pyrazol-1-yl]-N-ethyl-N-phenylpyridin-3-sulfonamid;
• 6-[4-(2-Chlor-6-fluorbenzyl)-3-methyl-5-oxo-2,5-dihydro-1H-pyrazol-1-yl]-N,N-di(propan-2-yl)pyridin-3-sulfonamid;
• 4-(2-Chlor-6-fluorbenzyl)-5-methyl-2-[5-(piperidin-1-ylsulfonyl)pyridin-2-yl]-1,2-dihydro-3H-pyrazol-3-on;
• 6-[4-(2-Chlorbenzyl)-3-methyl-5-oxo-2,5-dihydro-1H-pyrazol-1-yl]pyridin-3-carbonsäuremethylester;
• 4-(4-Chlorbenzyl)-5-methyl-2-(pyridin-2-yl)-1,2-dihydro-3H-pyrazol-3-on;
• 4-(4-Chlorbenzyl)-5-methyl-2-{5-[(4-methylpiperazin-1-yl)sulfonyl]pyridin-2-yl}-1,2-dihydro-3H-pyrazol-3-on;
• 4-(4-Chlorbenzyl)-2-(5-{[(3R, 5S)-3,5-dimethylpiperidin-1-yl]sulfonyl}pyridin-2-yl)-5-methyl-1,2-dihydro-3H-pyrazol-3-on;
• 6-[4-(4-Chlorbenzyl)-3-methyl-5-oxo-2,5-dihydro-1H-pyrazol-1-yl]-N,N-diethylpyridin-3-sulfonamid;
• 6-[4-(4-Chlorbenzyl)-3-methyl-5-oxo-2,5-dihydro-1H-pyrazol-1-yl]-N-ethyl-N-phenylpyridin-3-sulfonamid;
• 6-[4-(4-Chlorbenzyl)-3-methyl-5-oxo-2,5-dihydro-1H-pyrazol-1-yl]-N,N-di(propan-2-yl)pyridin-3-sulfonamid;
• 4-(4-Chlorbenzyl)-5-methyl-2-[5-(piperidin-1-ylsulfonyl)pyridin-2-yl]-1,2-dihydro-3H-pyrazol-3-on;
• 6-[4-(2-Fluorbenzyl)-3-methyl-5-oxo-2,5-dihydro-1H-pyrazol-1-yl]-N,N-di(propan-2-yl)pyridin-3-sulfonamid;
• 4-(1,1-Dioxidotetrahydrothiophen-3-yl)-5-methyl-2-[5-(piperidin-1-ylsulfonyl)pyridin-2-yl]-1,2-dihydro-3H-pyrazol-3-on;
• 6-[4-(1,1-Dioxidotetrahydrothiophen-3-yl)-3-methyl-5-oxo-2,5-dihydro-1H-pyrazol-1-yl]-N-ethyl-N-phenylpyridin-3-sulfonamid;
• 6-[4-(1,1-Dioxidotetrahydrothiophen-3-yl)-3-methyl-5-oxo-2,5-dihydro-1H-pyrazol-1-yl]-N,N-di(propan-2-yl)pyridin-3-sulfonamid;
• 2-{[5-Methyl-3-oxo-2-(pyridin-2-yl)-2,3-dihydro-1H-pyrazol-4-yl]methyl}benzonitril;
• 4-{[5-Methyl-3-oxo-2-(pyridin-2-yl)-2,3-dihydro-1H-pyrazol-4-yl]methyl}benzonitril;
• N-Ethyl-6-{4-[4-(methoxymethyl)benzyl]-3-methyl-5-oxo-2,5-dihydro-1H-pyrazol-1-yl}-N-phenylpyridin-3-sulfonamid;
• 4-[3-(Methoxymethyl)benzyl]-5-methyl-2-(pyridin-2-yl)-1,2-dihydro-3H-pyrazol-3-on;
• N-Ethyl-6-{4-[3-(methoxymethyl)benzyl]-3-methyl-5-oxo-2,5-dihydro-1H-pyrazol-1-yl}-N-phenylpyridin-3-sulfonamid;
• 6-[4-(3-Cyanobenzyl)-3-methyl-5-oxo-2,5-dihydro-1H-pyrazol-1-yl]-N-ethyl-N-phenylpyridin-3-sulfonamid;
• 6-[4-(4-Cyanobenzyl)-3-methyl-5-oxo-2,5-dihydro-1H-pyrazol-1-yl]-N-ethyl-N-phenylpyridin-3-sulfonamid;
• 5-Methyl-2-(pyridin-2-yl)-4-(pyridin-4-ylmethyl)-1,2-dihydro-3H-pyrazol-3-on;
• 3-{[5-Methyl-3-oxo-2-(pyridin-2-yl)-2,3-dihydro-1H-pyrazol-4-yl]methyl}benzonitril;
• 4-Benzyl-2-(5-brompyridin-2-yl)-5-methyl-1,2-dihydro-3H-pyrazol-3-on;
• 6-[4-(2-Cyanobenzyl)-3-methyl-5-oxo-2,5-dihydro-1H-pyrazol-1-yl]-N-ethyl-N-phenylpyridin-3-sulfonamid;
• N-Ethyl-6-{4-[3-(2-methoxyethoxy)benzyl]-3-methyl-5-oxo-2,5-dihydro-1H-pyrazol-1-yl}-N-phenylpyridin-3-sulfonamid;
• 4-(3,5-Dimethoxybenzyl)-5-methyl-2-(pyridin-2-yl)-1,2-dihydro-3H-pyrazol-3-on;
• 5-Methyl-4-[4-(methylsulfonyl)benzyl]-2-(pyridin-2-yl)-1,2-dihydro-3H-pyrazol-3-on;
• N-Ethyl-6-{3-methyl-4-[4-(methylsulfonyl)benzyl]-5-oxo-2,5-dihydro-1H-pyrazol-1-yl}-N-phenylpyridin-3-sulfonamid;
• 4-[3-(2-Methoxyethoxy)benzyl]-5-methyl-2-(pyridin-2-yl)-1,2-dihydro-3H-pyrazol-3-on;
• 4-Benzyl-5-methyl-2-(pyridin-2-yl)-1,2-dihydro-3H-pyrazol-3-on;
• N-Ethyl-6-[3-methyl-5-oxo-4-(pyridin-4-ylmethyl)-2,5-dihydro-1H-pyrazol-1-yl]-N-phenyl-pyridin-3-sulfonamid;
• N-Ethyl-6-[3-methyl-5-oxo-4-(pyridin-2-ylmethyl)-2,5-dihydro-1H-pyrazol-1-yl]-N-phenyl-pyridin-3-sulfonamid;
• 5-Methyl-2-(pyridin-2-yl)-4-(pyridin-3-ylmethyl)-1,2-dihydro-3H-pyrazol-3-on;
• 6-(4-Benzyl-3-methyl-5-oxo-2,5-dihydro-1H-pyrazol-1-yl)-N-ethyl-N-phenylpyridin-3-sulfonamid;
• N-Ethyl-6-[3-methyl-5-oxo-4-(pyridin-3-ylmethyl)-2,5-dihydro-1H-pyrazol-1-yl]-N-phenyl-pyridin-3-sulfonamid;
• 4-(2,5-Dimethoxybenzyl)-5-methyl-2-(pyridin-2-yl)-1,2-dihydro-3H-pyrazol-3-on;
• 6-[4-(2,5-Dimethoxybenzyl)-3-methyl-5-oxo-2,5-dihydro-1H-pyrazol-1-yl]-N-ethyl-N-phenyl-pyridin-3-sulfonamid;
• N-Ethyl-6-{3-methyl-4-[(1-methylpiperidin-4-yl)methyl]-5-oxo-2,5-dihydro-1H-pyrazol-1-yl}-N-phenylpyridin-3-sulfonamid;
• N-Ethyl-6-{3-methyl-5-oxo-4-[4-(trifluormethyl)benzyl]-2,5-dihydro-1H-pyrazol-1-yl}-N-phenylpyridin-3-sulfonamid;
• N-Ethyl-6-{3-methyl-5-oxo-4-[3-(trifluormethyl)benzyl]-2,5-dihydro-1H-pyrazol-1-yl}-N-phenylpyridin-3-sulfonamid;
• 5-Methyl-2-(pyridin-2-yl)-4-[3-(trifluormethyl)benzyl]-1,2-dihydro-3H-pyrazol-3-on;
• 5-Methyl-2-(pyridin-2-yl)-4-[4-(trifluormethyl)benzyl]-1,2-dihydro-3H-pyrazol-3-on;
• 6-[4-(3,5-Dimethoxybenzyl)-3-methyl-5-oxo-2,5-dihydro-1H-pyrazol-1-yl]-N-ethyl-N-phenyl-pyridin-3-sulfonamid;
• 4-(4-Hydroxybenzyl)-5-methyl-2-(pyridin-2-yl)-1,2-dihydro-3H-pyrazol-3-on;
• N-Ethyl-6-[4-(4-hydroxybenzyl)-3-methyl-5-oxo-2,5-dihydro-1H-pyrazol-1-yl]-N-phenylpyridin-3-sulfonamid;
• 6-(4-{4-[2-(Dimethylamino)ethoxy]benzyl}-3-methyl-5-oxo-2,5-dihydro-1H-pyrazol-1-yl)-N-ethyl-N-phenylpyridin-3-sulfonamid;
• 6-{4-[4-(Dimethylamino)benzyl]-3-methyl-5-oxo-2,5-dihydro-1H-pyrazol-1-yl}-N-ethyl-N-phenylpyridin-3-sulfonamid;
• 5-Methyl-2-(pyridin-2-yl)-4-[2-(trifluormethyl)benzyl]-1,2-dihydro-3H-pyrazol-3-on;
• 4-[4-(Dimethylamino)benzyl]-5-methyl-2-(pyridin-2-yl)-1,2-dihydro-3H-pyrazol-3-on;
• N-Ethyl-6-{3-methyl-5-oxo-4-[2-(trifluormethyl)benzyl]-2,5-dihydro-1H-pyrazol-1-yl}-N-phenylpyridin-3-sulfonamid;
• 6-(4-Benzyl-3-methyl-5-oxo-2,5-dihydro-1H-pyrazol-1-yl)-N-(3-methylbutyl)pyridin-3-sulfonamid;
• 4-[3-(Dimethylamino)benzyl]-5-methyl-2-(pyridin-2-yl)-1,2-dihydro-3H-pyrazol-3-on;
• 6-{4-[3-(Dimethylamino)benzyl]-3-methyl-5-oxo-2,5-dihydro-1H-pyrazol-1-yl}-N-ethyl-N-phenylpyridin-3-sulfonamid;
• 4-(4-Methoxybenzyl)-5-methyl-2-(pyridin-2-yl)-1,2-dihydro-3H-pyrazol-3-on;
• N-Ethyl-6-[4-(4-methoxybenzyl)-3-methyl-5-oxo-2,5-dihydro-1H-pyrazol-1-yl]-N-phenylpyridin-3-sulfonamid;
• 4-(2-Methoxybenzyl)-5-methyl-2-(pyridin-2-yl)-1,2-dihydro-3H-pyrazol-3-on;
• 4-(3-Methoxybenzyl)-5-methyl-2-(pyridin-2-yl)-1,2-dihydro-3H-pyrazol-3-on;
• N-Ethyl-6-[4-(3-methoxybenzyl)-3-methyl-5-oxo-2,5-dihydro-1H-pyrazol-1-yl]-N-phenylpyridin-3-sulfonamid;
• 6-(4-{3-[2-(Dimethylamino)ethoxy]benzyl}-3-methyl-5-oxo-2,5-dihydro-1H-pyrazol-1-yl)-N-ethyl-N-phenylpyridin-3-sulfonamid;
• N-Ethyl-6-[4-(2-methoxybenzyl)-3-methyl-5-oxo-2,5-dihydro-1H-pyrazol-1-yl]-N-phenylpyridin-3-sulfonamid;
• 4-Benzyl-5-methyl-2-[5-(morpholin-4-ylsulfonyl)pyridin-2-yl]-1,2-dihydro-3H-pyrazol-3-on;
• N-Ethyl-6-[4-(3-hydroxybenzyl)-3-methyl-5-oxo-2,5-dihydro-1H-pyrazol-1-yl]-N-phenylpyridin-3-sulfonamid;
• 6-(4-Benzyl-3-ethyl-5-oxo-2,5-dihydro-1H-pyrazol-1-yl)-N-ethyl-N-phenylpyridin-3-sulfonamid;
• 4-(3-Hydroxybenzyl)-5-methyl-2-(pyridin-2-yl)-1,2-dihydro-3H-pyrazol-3-on;
• N-Ethyl-6-{4-[4-(2-methoxyethoxy)benzyl]-3-methyl-5-oxo-2,5-dihydro-1H-pyrazol-1-yl}-N-phenylpyridin-3-sulfonamid;
• 4-Benzyl-5-ethyl-2-(pyridin-2-yl)-1,2-dihydro-3H-pyrazol-3-on;
• 4-Benzyl-5-methyl-2-[5-(pyrrolidin-1-ylsulfonyl)pyridin-2-yl]-1,2-dihydro-3H-pyrazol-3-on;
• 2-[5-(Azepan-1-ylsulfonyl)pyridin-2-yl]-4-benzyl-5-methyl-1,2-dihydro-3H-pyrazol-3-on;
• N,N-Diethyl-6-[3-methyl-5-oxo-4-(pyridin-3-ylmethyl)-2,5-dihydro-1H-pyrazol-1-yl]pyridin-3-sulfonamid;
• N,N-Diethyl-6-[3-methyl-5-oxo-4-(pyridin-3-ylmethyl)-2,5-dihydro-1H-pyrazol-1-yl]pyridin-3-sulfonamid-hydrochlorid;
• N,N-Dimethyl-6-[3-methyl-5-oxo-4-(pyridin-3-ylmethyl)-2,5-dihydro-1H-pyrazol-1-yl]pyridin-3-sulfonamid;
• N,N-Dimethyl-6-[3-methyl-5-oxo-4-(pyridin-3-ylmethyl)-2,5-dihydro-1H-pyrazol-1-yl]pyridin-3-sulfonamid-hydrochlorid;
• 6-(4-Benzyl-5-oxo-3-propyl-2,5-dihydro-1H-pyrazol-1-yl)-N-ethyl-N-phenylpyridin-3-sulfonamid-hydrochlorid;
• 6-[3-Methyl-5-oxo-4-(pyridin-3-ylmethyl)-2,5-dihydro-1H-pyrazol-1-yl]-N-(propan-2-yl)pyridin-3-sulfonamid-hydrochlorid;
• 5-Methyl-4-(pyridin-3-ylmethyl)-2-[5-(pyrrolidin-1-ylsulfonyl)pyridin-2-yl]-1,2-dihydro-3H-pyrazol-3-on-hydrochlorid;
• N-tert.-Butyl-6-[3-methyl-5-oxo-4-(pyridin-3-ylmethyl)-2,5-dihydro-1H-pyrazol-1-yl]pyridin-3-sulfonamid-hydrochlorid;
• N-Cyclopropyl-6-[3-methyl-5-oxo-4-(pyridin-3-ylmethyl)-2,5-dihydro-1H-pyrazol-1-yl]pyridin-3-sulfonamid-hydrochlorid;
• N-Cyclopentyl-6-[3-methyl-5-oxo-4-(pyridin-3-ylmethyl)-2,5-dihydro-1H-pyrazol-1-yl]pyridin-3-sulfonamid-hydrochlorid;
• N-Ethyl-6-[3-methyl-5-oxo-4-(2-phenylethyl)-2,5-dihydro-1H-pyrazol-1-yl]-N-phenylpyridin-3-sulfonamid;
• 5-Methyl-4-(2-phenylethyl)-2-(pyridin-2-yl)-1,2-dihydro-3H-pyrazol-3-on;
• N-Methyl-6-[3-methyl-5-oxo-4-(pyridin-3-ylmethyl)-2,5-dihydro-1H-pyrazol-1-yl]-N-(pyridin-2-yl)pyridin-3-sulfonamid-hydrochlorid;
• 2-{5-[(4-Benzylpiperidin-1-yl)sulfonyl]pyridin-2-yl}-5-methyl-4-(pyridin-3-ylmethyl)-1,2-dihydro-3H-pyrazol-3-on;
• N-Ethyl-6-[3-methyl-5-oxo-4-(2-phenylpropan-2-yl)-2,5-dihydro-1H-pyrazol-1-yl]-N-phenyl-pyridin-3-sulfonamid;
• N-Ethyl-6-{4-[(6-methoxypyridin-2-yl)methyl]-3-methyl-5-oxo-2,5-dihydro-1H-pyrazol-1-yl}-N-phenylpyridin-3-sulfonamid;
• 6-[3-Methyl-5-oxo-4-(pyridin-3-ylmethyl)-2,5-dihydro-1H-pyrazol-1-yl]-N-(pyridin-2-yl)pyridin-3-sulfonamid-hydrochlorid;
• N-Ethyl-6-[3-methyl-5-oxo-4-(1-phenylcyclopropyl)-2,5-dihydro-1H-pyrazol-1-yl]-N-phenylpyridin-3-sulfonamid;
• N-Ethyl-6-{4-[(3-methoxypyridin-2-yl)methyl]-3-methyl-5-oxo-2,5-dihydro-1H-pyrazol-1-yl}-N-phenylpyridin-3-sulfonamid-hydrochlorid;
• 6-[4-Benzyl-3-(methoxymethyl)-5-oxo-2,5-dihydro-1H-pyrazol-1-yl]-N-ethyl-N-phenylpyridin-3-sulfonamid;
• 6-[4-Benzyl-5-oxo-3-(trifluormethyl)-2,5-dihydro-1H-pyrazol-1-yl]-N-ethyl-N-phenylpyridin-3-sulfonamid;
• 1-[5-(Azepan-1-ylsulfonyl)pyridin-2-yl]-3-methyl-4-(pyridin-3-ylmethyl)-1H-pyrazol-5-olat;
• N-Ethyl-6-{3-methyl-5-oxo-4-[2-(pyridin-2-yl)ethyl]-2,5-dihydro-1H-pyrazol-1-yl}-N-phenyl-pyridin-3-sulfonamid;
• N-Ethyl-6-{4-[(5-methoxypyridin-3-yl)methyl]-3-methyl-5-oxo-2,5-dihydro-1H-pyrazol-1-yl}-N-phenylpyridin-3-sulfonamid;
• N-(2-Methoxyethyl)-N-methyl-6-[3-methyl-5-oxo-4-(pyridin-3-ylmethyl)-2,5-dihydro-1H-pyrazol-1-yl]pyridin-3-sulfonamid;
• 6-[4-Benzyl-3-(2-methylpropyl)-5-oxo-2,5-dihydro-1H-pyrazol-1-yl]-N-ethyl-N-phenylpyridin-3-sulfonamid;
• N-Ethyl-6-[3-methyl-5-oxo-4-(pyridin-3-ylmethyl)-2,5-dihydro-1H-pyrazol-1-yl]pyridin-3-sulfonamid;
• 6-[3-Methyl-5-oxo-4-(pyridin-3-ylmethyl)-2,5-dihydro-1H-pyrazol-1-yl]-N-phenylpyridin-3-sulfonamid;
• N-Ethyl-6-[3-methyl-5-oxo-4-(3-phenylpropyl)-2,5-dihydro-1H-pyrazol-1-yl]-N-phenylpyridin-3-sulfonamid;
• 6-(4-Benzyl-5-oxo-2,5-dihydro-1H-pyrazol-1-yl)-N-ethyl-N-phenylpyridin-3-sulfonamid;
• N-Cyclopropyl-N-methyl-6-[3-methyl-5-oxo-4-(pyridin-3-ylmethyl)-2,5-dihydro-1H-pyrazol-1-yl]pyridin-3-sulfonamid;
• N-tert.-Butyl-6-{4-[(5-methoxypyridin-3-yl)methyl]-3-methyl-5-oxo-2,5-dihydro-1H-pyrazol-1-yl}pyridin-3-sulfonamid;
• 6-{4-[(5-Cyanopyridin-3-yl)methyl]-3-methyl-5-oxo-2,5-dihydro-1H-pyrazol-1-yl}-N-ethyl-N-phenylpyridin-3-sulfonamid;
• 6-[5-Oxo-4-(pyridin-3-ylmethyl)-2,5-dihydro-1H-pyrazol-1-yl]pyridin-3-carbonsäure-tert.-butylester;
• 6-[3-methyl-5-oxo-4-(pyridin-3-ylmethyl)-2,5-dihydro-1H-pyrazol-1-yl]pyridin-3-carbonsäure-tert.-butylester;
• N-tert.-Butyl-6-[5-oxo-4-(pyridin-3-ylmethyl)-2,5-dihydro-1H-pyrazol-1-yl]pyridin-3-sulfonamid;
• N-tert.-Butyl-6-[5-oxo-4-(pyridin-3-ylmethyl)-2,5-dihydro-1H-pyrazol-1-yl]pyridin-3-sulfonamid-hydrochlorid;
• N-Ethyl-6-[5-oxo-4-(pyridin-3-ylmethyl)-2,5-dihydro-1H-pyrazol-1-yl]-N-phenylpyridin-3-sulfonamid;
• 6-(4-Benzyl-5-oxo-2,5-dihydro-1H-pyrazol-1-yl)-N-tert.-butyl-N-methylpyridin-3-sulfonamid;
• N-Ethyl-6-{4-[(5-methoxypyridin-3-yl)methyl]-5-oxo-2,5-dihydro-1H-pyrazol-1-yl}-N-phenyl-pyridin-3-sulfonamid;
• 5-Methyl-2-[5-(phenylsulfonyl)pyridin-2-yl]-4-(pyridin-3-ylmethyl)-1,2-dihydro-3H-pyrazol-3-on;
• N-tert.-Butyl-6-{4-[(5-methoxypyridin-3-yl)methyl]-3-methyl-5-oxo-2,5-dihydro-1H-pyrazol-1-yl}-N-methylpyridin-3-sulfonamid;
• 6-{4-[(6-Cyanopyridin-3-yl)methyl]-3-methyl-5-oxo-2,5-dihydro-1H-pyrazol-1-yl}-N-ethyl-N-phenylpyridin-3-sulfonamid;
• (2-{5-[Ethyl(phenyl)sulfamoyl]pyridin-2-yl}-3-oxo-2,3-dihydro-1H-pyrazol-4-yl)essigsäure;
• 2-(2-{5-[Ethyl(phenyl)sulfamoyl]pyridin-2-yl}-3-oxo-2,3-dihydro-1H-pyrazol-4-yl)-N,N-dimethyl-acetamid;
• (2-{5-[Ethyl(phenyl)sulfamoyl]pyridin-2-yl}-3-oxo-2,3-dihydro-1H-pyrazol-4-yl)essigsäure-methylester;
• (4-Benzyl-1-{5-[ethyl(phenyl)sulfamoyl]pyridin-2-yl}-5-oxo-2,5-dihydro-1H-pyrazol-3-yl)-essigsäureethylester;
• 2-(2-{5-[Ethyl(phenyl)sulfamoyl]pyridin-2-yl}-3-oxo-2,3-dihydro-1H-pyrazol-4-yl)-N-methyl-acetamid;
• N-tert.-Butyl-6-{4-[(5-methoxypyridin-2-yl)methyl]-3-methyl-5-oxo-2,5-dihydro-1H-pyrazol-l-yl}-N-methylpyridin-3-sulfonamid;
• 2-(4-Benzyl-1-{5-[ethyl(phenyl) sulfamoyl]-pyridin-2-yl}-5-oxo-2,5-dihydro-1H-pyrazol-3-yl)-N,N-dimethylacetamid;
• 3-(2-{5-[Ethyl(phenyl)sulfamoyl]pyridin-2-yl}-3-oxo-2,3-dihydro-1H-pyrazol-4-yl)propansäure;
• 3-[(2-{5-[Ethyl(phenyl)sulfamoyl]pyridin-2-yl}-5-methyl-3-oxo-2,3-dihydro-1H-pyrazol-4-yl)methyl]benzoesäuremethylester;
• 3-(2-{5-[Ethyl(phenyl)sulfamoyl]pyridin-2-yl}-3-oxo-2,3-dihydro-1H-pyrazol-4-yl)propansäure-methylester;
• {2-[5-(tert.-Butylsulfamoyl)pyridin-2-yl]-3-oxo-2,3-dihydro-1H-pyrazol-4-yl}(phenyl)essigsäure-methylester;
• 2-[(2-{5-[Ethyl(phenyl)sulfamoyl]pyridin-2-yl}-5-methyl-3-oxo-2,3-dihydro-1H-pyrazol-4-yl)methyl]benzoesäuremethylester;
• N-Cyclopentyl-N-ethyl-6-[5-oxo-4-(pyridin-3-ylmethyl)-2,5-dihydro-1H-pyrazol-1-yl]pyridin-3-sulfonamid;
• N-Cyclopentyl-N-methyl-6-[5-oxo-4-(pyridin-3-ylmethyl)-2,5-dihydro-1H-pyrazol-1-yl]pyridin-3-sulfonamid;
• 2-[(1-{5-[Ethyl(phenyl)sulfamoyl]pyridin-2-yl}-3-methyl-5-oxido-1H-pyrazol-4-yl)methyl]benzoat;
• 2-[5-(Azepan-1-ylsulfonyl)pyridin-2-yl]-4-(pyridin-3-ylmethyl)-1,2-dihydro-3H-pyrazol-3-on;
• N-Cyclopentyl-N-methyl-6-[5-oxo-4-(pyridin-4-ylmethyl)-2,5-dihydro-1H-pyrazol-1-yl]pyridin-3-sulfonamid;
• N-Cyclopentyl-N-ethyl-6-[5-oxo-4-(pyridin-4-ylmethyl)-2,5-dihydro-1H-pyrazol-1-yl]pyridin-3-sulfonamid;
• 3-(2-{5-[Cyclopentyl(methyl)sulfamoyl]pyridin-2-yl}-3-oxo-2,3-dihydro-1H-pyrazol-4-yl)-3-phenylpropansäuremethylester;
• N-(2,2-Dimethylpropyl)-N-methyl-6-[5-oxo-4-(pyridin-3-ylmethyl)-2,5-dihydro-1H-pyrazol-1-yl]pyridin-3-sulfonamid;
• 6-[5-Oxo-4-(pyridin-3-ylmethyl)-2,5-dihydro-1H-pyrazol-1-yl]pyridin-3-carbonsäure-2,2-dimethylpropylester;
• N-Cyclopentyl-N-({6-[5-oxo-4-(pyridin-3-ylmethyl)-2,5-dihydro-1H-pyrazol-1-yl]pyridin-3-yl}sulfonyl)glycinmethylester;
• 6-(4-Benzyl-5-oxo-2,5-dihydro-1H-pyrazol-1-yl)-N-[2-(benzyloxy)ethyl]-N-Cyclopentylpyridin-3-sulfonamid;
• 6-(4-Benzyl-5-oxo-2,5-dihydro-1H-pyrazol-1-yl)-N-[3-(benzyloxy)propyl]-N-Cyclopentylpyridin-3-sulfonamid;
• 6-(4-Benzyl-5-oxo-2,5-dihydro-1H-pyrazol-1-yl)-N-cyclopentyl-N-(3-hydroxypropyl)pyridin-3-sulfonamid;
• 6-(4-Benzyl-5-oxo-2,5-dihydro-1H-pyrazol-1-yl)-N-cyclopentyl-N-(2-hydroxyethyl)pyridin-3-sulfonamid;
• (1S,2R)-2-[Methyl({6-[5-oxo-4-(pyridin-3-yl-methyl)-2,5-dihydro-1H-pyrazol-1-yl]pyridin-3-yl}sulfonyl)amino]cyclopentancarbonsäure-methylester;
• 6-(4-Benzyl-5-oxo-2,5-dihydro-1H-pyrazol-1-yl)-N-cyclopentyl-N-(2,3-dihydroxypropyl)pyridin-3-sulfonamid;
• 6-(4-Benzyl-5-oxo-2,5-dihydro-1H-pyrazol-1-yl)-N-cyclopentyl-N-[2-(dimethylamino)ethyl]pyridin-3-sulfonamid;
• 4-Benzyl-2-{5-[(4-methylpiperazin-1-yl)sulfonyl]pyridin-2-yl}-1,2-dihydro-3H-pyrazol-3-on;
• 6-[4-(Cyclopentylmethyl)-5-oxo-2,5-dihydro-1H-pyrazol-1-yl]-N-ethyl-N-phenylpyridin-3-sulfonamid;
• 4-Benzyl-2-{5-[(4-methyl-1,4-diazepan-1-yl)sulfonyl]pyridin-2-yl}-1,2-dihydro-3H-pyrazol-3-on;
• N-(2,2-Dimethylpropyl)-6-[5-oxo-4-(pyridin-3-ylmethyl)-2,5-dihydro-1H-pyrazol-1-yl]pyridin-3-sulfonamid;
• N-(2-Methylbutan-2-yl)-6-[5-oxo-4-(pyridin-3-ylmethyl)-2,5-dihydro-1H-pyrazol-1-yl]pyridin-3-sulfonamid;
• 6-(4-Benzyl-5-oxo-2,5-dihydro-1H-pyrazol-1-yl)-N-[(1R,3S)-3-(hydroxymethyl)cyclopentyl]-N-methylpyridin-3-sulfonamid;
• 6-(4-Benzyl-5-oxo-2,5-dihydro-1H-pyrazol-1-yl)-N-methyl-N-(1-methylpyrrolidin-3-yl)pyridin-3-sulfonamid;
• 6-(4-Benzyl-5-oxo-2,5-dihydro-1H-pyrazol-1-yl)-N-(2-methoxy-2-methylpropyl)pyridin-3-sulfonamid;
• 6-(4-Benzyl-5-oxo-2,5-dihydro-1H-pyrazol-1-yl)-pyridin-3-carbonsäure-2,2-dimethylpropylester;
• 6-(4-Benzyl-5-oxo-2,5-dihydro-1H-pyrazol-1-yl)-N-cyclopentyl-N-[(2R)-2,3-dihydroxypropyl]-pyridin-3-sulfonamid;
• 6-(4-Benzyl-5-oxo-2,5-dihydro-1H-pyrazol-1-yl)-N-(2,3-dihydroxypropyl)-N-phenylpyridin-3-sulfonamid;
• N-Cyclopentyl-6-{4-[(4-methoxypyridin-3-yl)methyl]-5-oxo-2,5-dihydro-1H-pyrazol-1-yl}-N-methylpyridin-3-sulfonamid;
• N-Methyl-N-(2-methylbutan-2-yl)-6-[5-oxo-4-(pyridin-3-ylmethyl)-2,5-dihydro-1H-pyrazol-1-yl]pyridin-3-sulfonamid;
• 3-(2-{5-[Cyclopentyl(methyl)sulfamoyl]pyridin-2-yl}-3-oxo-2,3-dihydro-1H-pyrazol-4-yl)-3-phenyl-N-(2,2,2-trifluorethyl)propanamide;
• 6-(4-Benzyl-5-oxo-2,5-dihydro-1H-pyrazol-1-yl)-N-cyclopentyl-N-[3-hydroxy-2-(hydroxymethyl)-propyl]pyridin-3-sulfonamid;
• 6-(4-Benzyl-5-oxo-2,5-dihydro-1H-pyrazol-1-yl)-N-cyclopentyl-N-[(2S)-2,3-dihydroxypropyl]-pyridin-3-sulfonamid;
• 6-[5-Oxo-4-(pyridin-4-ylmethyl)-2,5-dihydro-1H-pyrazol-1-yl]pyridin-3-carbonsäure-2,2-dimethylpropylester;
• 6-(4-Benzyl-5-oxo-2,5-dihydro-1H-pyrazol-1-yl)-N-[(1S,2S)-2-hydroxycyclopentyl]pyridin-3-sulfonamid;
• N-tert.-Butyl-6-[5-oxo-4-(pyridin-4-ylmethyl)-2,5-dihydro-1H-pyrazol-1-yl]pyridin-3-sulfonamid;
• 4-Benzyl-2-(5-{[(3R)-3-(dimethylamino)-pyrrolidin-1-yl]sulfonyl}pyridin-2-yl)-1,2-dihydro-3H-pyrazol-3-on;
• 6-(4-Benzyl-5-oxo-2,5-dihydro-1H-pyrazol-1-yl)-N-[1-(dimethylamino)-2-methylpropan-2-yl]pyridin-3-sulfonamid;
• 6-(4-Benzyl-5-oxo-2,5-dihydro-1H-pyrazol-1-yl)-N-methyl-N-phenylpyridin-3-sulfonamid;
• 3-{2-[5-(tert.-Butylsulfamoyl)pyridin-2-yl]-3-oxo-2,3-dihydro-1H-pyrazol-4-yl}-3-(pyridin-3-yl)propansäuremethylester;
• 3-{2-[5-(tert.-Butylsulfamoyl)pyridin-2-yl]-3-oxo-2,3-dihydro-1H-pyrazol-4-yl}-3-phenyl-propansäureethylester;
• 6-(4-Benzyl-5-oxo-2,5-dihydro-1H-pyrazol-1-yl)-N-tert.-butyl-N-(2,3-dihydroxypropyl)pyridin-3-sulfonamid;
• 6-(4-Benzyl-5-oxo-2,5-dihydro-1H-pyrazol-1-yl)-N-(2-hydroxyethyl)pyridin-3-sulfonamid;
• N-(2,3-Dihydroxypropyl)-6-[5-oxo-4-(pyridin-3-ylmethyl)-2,5-dihydro-1H-pyrazol-1-yl]-N-phenyl-pyridin-3-sulfonamid-hydrochlorid;
• 3-(2-{5-[Cyclopentyl(methyl)sulfamoyl]pyridin-2-yl}-3-oxo-2,3-dihydro-1H-pyrazol-4-yl)-3-(pyridin-3-yl)propansäuremethylester-hydrochlorid;
• 6-(4-Benzyl-5-oxo-2,5-dihydro-1H-pyrazol-1-yl)-N-cyclopentyl-N-[3-(dimethylamino)propyl]-pyridin-3-sulfonamid-hydrochlorid;
• 6-(4-Benzyl-5-oxo-2,5-dihydro-1H-pyrazol-1-yl)-N-cyclopentyl-N-[3-(dimethylamino)propyl]-pyridin-3-sulfonamid-hydrochlorid handelt.

17. Verfahren zur Herstellung einer Verbindung der Formel (I) nach einem der Ansprüche 1 bis 16, **dadurch gekennzeichnet, dass** man eine Verbindung der Formel (II) 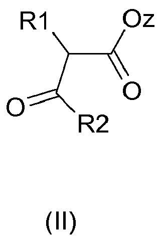 worin R1 und R2 die in Anspruch 1 angegebene Bedeutung besitzen und z für eine Alkylgruppe steht,
mit einer Verbindung der Formel (III) 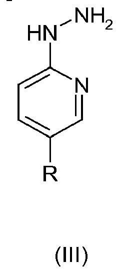 worin R die Anspruch 1 angegebene Bedeutung besitzt, umsetzt.

18. Medikament, **dadurch gekennzeichnet, dass** es eine Verbindung der Formel (I) nach einem der Ansprüche 1 bis 16 oder ein Additionssalz dieser Verbindung mit einer pharmazeutisch unbedenklichen Säure der Verbindung der Formel (I) umfasst.

19. Pharmazeutische Zusammensetzung, **dadurch gekennzeichnet, dass** sie eine Verbindung der Formel (I) nach einem der Ansprüche 1 bis 16 oder ein pharmazeutisch unbedenkliches Salz dieser Verbindung sowie mindestens einen pharmazeutisch unbedenklichen Hilfsstoff umfasst.

20. Verwendung einer Verbindung der Formel (I) nach einem der Ansprüche 1 bis 16 zur Herstellung eines Medikaments zur Behandlung/Prophylaxe von Herz-Kreislauf-Erkrankungen.

21. Verwendung einer Verbindung der Formel (I) nach einem der Ansprüche 1 bis 16 zur Herstellung eines Medikaments zur Behandlung/Prophylaxe von Ischämie der unteren Gliedmaßen, Herzinsuffizienz, Koronarerkrankungen ischämischen Ursprungs, beispielsweise Angina pectoris oder Myokardinfarkt, Arteriosklerose, Schlaganfällen ischämischen Ursprungs, pulmonaler Hypertonie und allen durch teilweisen oder vollständigen Gefäßverschluss verursachten Pathologien.

22. Verwendung einer Verbindung der Formel (I) nach einem der Ansprüche 1 bis 16 zur Herstellung eines Medikaments zur Behandlung/Prophylaxe von Glaukomen, Nierenerkrankungen oder Hirnerkrankungen neurodegenerativen oder anderen Ursprungs, Anämien, oder eines Medikaments zur Förderung der Vernarbung oder Mitteln zur Verkürzung des Zeitraums der postoperativen Genesung oder eines Medikaments zur Behandlung von allgemeinen Erschöpfungszuständen oder auch eines Medikaments zum Erhalt von Blut im Rahmen von Autotransfusionen, die nach schweren chrirurgischen Eingriffen wie Schädel- oder Thoraxchirurgie oder bei Herz-, Karotis- oder Aortaoperationen notwendig sind.

23. Verwendung einer Verbindung der Formel (I) nach einem der Ansprüche 1 bis 16 zur Herstellung eines Medikaments zur Behandlung/Prophylaxe von Herzerkrankungen oder peripheren Erkrankungen ischämischen Ursprungs mit einer regenerativen Medizin unter Verwendung von Stammzellen.

24. Kombination einer Verbindung der Formel (I) nach einem der Ansprüche 1 bis 16 mit einem oder mehreren anderen Wirkstoffen, der bzw. die bei der Behandlung von Hypertonie, Herzinsuffizienz Diabetes und Anämie verwendbar ist bzw. sind.

## Claims

1. Compound corresponding to formula (I) below: 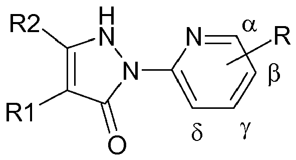 in which
R represents a group -SO₂-NR3R4 or a group -SO₂-R4; R3, R4 and R5 being as defined below;
R1 represents a heterocycloalkyl group not containing a nitrogen atom, a group -W-(C3-C6)cycloalkyl, a group -W-aryl, a group -W-heteroaryl, a group -W-heterocycloalkyl, a group -W-COOR5 or a group -W-CONR5R6,
(i) the said aryl, heteroaryl and heterocycloalkyl groups being optionally substituted on at least one carbon atom with at least one substituent chosen from halogen atoms, groups (C1-C5)alkyl, groups -(C1-C5)alkylene-O-(C1-C5)alkyl, groups -(C1-C5)alkoxy, a hydroxyl function, groups -halo(C1-C5)alkyl, a cyano function, groups -O(C1-C5)alkylene-O-(C1-C5)alkyl, groups -O-(C1-C5)alkylene-NR5R6, groups -SO₂-(C1-C5)alkyl, groups -NR5R6 and groups -CO₂R5, and
(ii) it being understood that when it is a heterocycloalkyl group, the said group comprising at least one nitrogen atom, this atom may optionally bear a substituent chosen from groups (C1-C5)alkyl,
R2 represents a hydrogen atom, a group -(C1-C5)alkyl, a group -(C1-C5)alkylene-O-(C1-C5)alkyl, a group -halo(C1-C5)alkyl, a group -W-COOR5, a group -W-C(O)NHR5 or a group -W-C(O)-NR5R6; W, R5 and R6 being as defined below;
it being understood that:
○ **n** represents 0, 1 or 2;
○ **W** is
(i) a group -(C1-C5)alkylene-, optionally substituted with a group chosen from groups -(CH₂)n-CO₂R5 and groups -(CH₂)n-(CO)NR5R6, with n as defined above and **R5** and **R6** as defined below; or
(ii) a group -(C3-C6)cycloalkylene-,
○ **R3** and **R4**
(i) which may be identical or different, represent, independently of each other, a hydrogen atom, a group -(C1-C5)alkyl, a group -(C3-C6)cycloalkyl, a group -(C1-C5)alkylene-O-(C1-C5)alkyl, an aryl, a group -CH₂-aryl, a heteroaryl, a heterocycloalkyl, a group -W-OH, a group -W-CHOH-CH₂OH, a group -W-CO₂R5, a group -W-NR5R6 or a group -W-O-(CH₂)n-aryl;
the said groups -(C3-C6)cycloalkyl and heterocycloalkyl being optionally substituted
○ on at least one carbon atom with at least one group chosen from -(C1-C5)alkyl, a group -(C1-C5)alkoxy, a hydroxyl function, a group -W-NR5R6 and a group -W-CO₂R5, in the case of the groups -(C3-C6)cycloalkyl and heterocycloalkyl and/or
○ on at least one heteroatom chosen from nitrogen with at least one group chosen from -(C1-C5)alkyl in the case of a heterocycloalkyl group,
with **W** and **n** as defined previously and **R5** and **R6** as defined below and it being understood that when **R3** and **R4** are identical, they cannot be a hydrogen atom;
(ii) or alternatively **R3** and **R4** form, together with the nitrogen atom that bears them, a heterocycloalkyl group, optionally substituted on at least one carbon atom and/or, where appropriate, on at least one heteroatom, with at least one substituent chosen from groups -(C1-C5)alkyl and groups -CH₂-aryl;
○ **R5** and **R6,** which may be identical or different, represent, independently of each other, a hydrogen atom, a group -(C1-C5)alkyl or a group -(C1-C5)haloalkyl,
in the form of the base or an acid-addition salt.

2. Compound corresponding to formula (I) according to Claim 1, **characterized in that**:
**R** represents a group -SO₂-NR3R4 or a group -SO₂-R4; **R3, R4** and **R5** being as defined below;
and/or
**R1** represents a heterocycloalkyl group not containing a nitrogen atom, a group -W-(C3-C6)cycloalkyl, a group -W-aryl, a group -W-heteroaryl, a group -W-heterocycloalkyl, a group -W-COOR5 or a group -W-CONR5R6,
(i) the said aryl, heteroaryl and heterocycloalkyl groups being optionally substituted on at least one carbon atom with at least one substituent chosen from halogen atoms, groups (C1-C5)alkyl, groups -(C1-C5)alkylene-O-(C1-C5)alkyl, groups -(C1-C5)alkoxy, a hydroxyl function, groups -halo(C1-C5)alkyl, a cyano function, groups -O(C1-C5)alkylene-O-(C1-C5)alkyl, groups -O-(C1-C5)alkylene-NR5R6, groups -SO₂-(C1-C5)alkyl, groups -NR5R6 and groups -CO₂R5, and
(ii) it being understood that when it is a heterocycloalkyl group, the said group comprising at least one nitrogen atom, this atom may optionally bear a substituent chosen from groups (C1-C5)alkyl,
and/or
**R2** represents a hydrogen atom, a group -(C1-C5)alkyl, a group -(C1-C5)alkylene-O-(C1-C5)alkyl, a group -halo(C1-C5)alkyl, a group -W-COOR5, a group -W-C(O)NHR5 or a group -W-C(O)-NR5R6; **W, R5** and **R6** being as defined below;
and/or
**n** represents 0, 1 or 2;
and/or
**W** is
(i) a group -(C1-C5)alkylene-, optionally substituted with a group chosen from groups -(CH₂)n-CO₂R5 and groups -(CH₂)n-(CO)NR5R6, with n as defined above and **R5** and **R6** as defined below; or
(ii) a group -(C3-C6)cycloalkylene-,
and/or
**R3** and **R4**
(i) which may be identical or different, represent, independently of each other, a hydrogen atom, a group -(C1-C5)alkyl, a group -(C3-C6)cycloalkyl, a group -(C1-C5)alkylene-O-(C1-C5)alkyl, an aryl, a group -CH₂-aryl, a heteroaryl, a heterocycloalkyl, a group -W-OH, a group -W-CHOH-CH₂OH, a group -W-CO₂R5, a group -W-NR5R6 or a group -W-O-(CH₂)n-aryl;
the said groups -(C3-C6)cycloalkyl and heterocycloalkyl being optionally substituted
○ on at least one carbon atom with at least one group chosen from -(C1-C5)alkyl, a group -(C1-C5)alkoxy, a hydroxyl function, a group -W-NR5R6 and a group -W-CO₂R5, in the case of the groups -(C3-C6)cycloalkyl and heterocycloalkyl and/or
○ on at least one heteroatom chosen from nitrogen with at least one group chosen from -(C1-C5)alkyl, in the case of a heterocycloalkyl group,
with **W** and **n** as defined previously and **R5** and **R6** as defined below and it being understood that when **R3** and **R4** are identical, they cannot be a hydrogen atom;
(ii) or alternatively **R3** and **R4** form, together with the nitrogen atom that bears them, a heterocycloalkyl group, optionally substituted on at least one carbon atom and/or, where appropriate, on at least one heteroatom, with at least one substituent chosen from groups -(C1-C5)alkyl and groups -CH₂-aryl;
and/or
**R5** and **R6,** which may be identical or different, represent, independently of each other, a hydrogen atom, a group -(C1-C5)alkyl or a group -(C1-C5)haloalkyl.

3. Compound of formula (I) according to either of Claims 1 and 2, **characterized in that R** represents a group -SO₂-NR3R4 with **R3** and **R4** as defined in Claim 1.

4. Compound of formula (I) according to either of Claims 1 and 2, **characterized in that R** represents a group -SO₂-R4 with **R4** and **R5** as defined in Claim 1.

5. Compound of formula (I) according to any one of Claims 1 to 4, **characterized in that** R is a substituent of the atom in the β position of pyridine.

6. Compound of formula (I) according to any one of Claims 1 to 5, **characterized in that R1** represents a heterocycloalkyl group not containing a nitrogen atom, a group -W-(C3-C6)cycloalkyl, a group -W-aryl, a group -W-heteroaryl, a group -W-heterocycloalkyl, a group -W-COOR5 or a group -W-CONR5R6,
the said aryl, heteroaryl and heterocycloalkyl groups being optionally substituted on at least one carbon atom with at least one substituent chosen from halogen atoms, groups (C1-C5)alkyl, groups -(C1-C5)alkylene-O-(C1-C5)alkyl, groups -(C1-C5)alkoxy, a hydroxyl function, groups -halo(C1-C5)alkyl, a cyano function, groups -O(C1-C5)alkylene-O-(C1-C5)alkyl, groups -O-(C1-C5)alkylene-NR5R6, groups -SO₂-(C1-C5)alkyl, groups -NR5R6 and groups -CO₂R5, and
it being understood that when it is a heterocycloalkyl group, the said group comprising at least one nitrogen atom, this atom may optionally bear a substituent chosen from groups (C1-C5)alkyl,
in which **W** is a group (C1-C5)alkylene or a group (C3-C6)cycloalkylene and in which **R5** and **R6,** which may be identical or different, represent, independently of each other, a hydrogen atom, a group -(C1-C5)alkyl or a group -(C1-C5)haloalkyl.

7. Compound of formula (I) according to Claim 6, **characterized in that** the said heterocycloalkyl group represents a piperidyl group, the said aryl group represents a phenyl group and the said heteroaryl group represents a pyridyl group.

8. Compound of formula (I) according to any one of Claims 1 to 7, **characterized in that R2** represents a hydrogen atom, a group -(C1-C5)alkyl, a group -(C1-C5)alkylene-O-(C1-C5)alkyl, a group -halo(C1-C5)alkyl, a group -W-COOR5, a group -W-C(O)NHR5 or a group -W-C(O)-NR5R6 in which **W, R5** and **R6** are as defined in Claim 1.

9. Compound of formula (I) according to any one of Claims 1 to 3 and 5 to 7, **characterized in that R** represents a group -SO₂-NR3R4, advantageously in the β position of pyridine, and in which **R3** and **R4,** which may be identical or different, represent, independently of each other, a hydrogen atom, a group -(C1-C5)alkyl, a group -(C3-C6)cycloalkyl, a group -(C1-C5)alkylene-O-(C1-C5)alkyl, an aryl, a group -CH₂-aryl, a heteroaryl, a heterocycloalkyl, a group -W-OH, a group -W-CHOH-CH₂OH, a group -W-CO₂R5, a group -W-NR5R6 or a group -W-O-(CH₂)n-aryl;
it being understood that:
when **R3** and **R4** are identical, they cannot be a hydrogen atom; and that
when **R3** and/or **R4** are chosen from the said groups -(C3-C6)cycloalkyl and heterocycloalkyl, these groups may be optionally substituted
○ on at least one carbon atom, with at least one group chosen from -(C1-C5)alkyl, a group -(C1-C5)alkoxy, a hydroxyl function, a group -W-NR5R6 and a group -W-CO₂R5, in the case of the groups -(C3-C6)cycloalkyl and heterocycloalkyl, and/or
○ on at least one heteroatom chosen from nitrogen with at least one group chosen from -(C1-C5)alkyl, in the case of a heterocycloalkyl group,
in which **W, R5** and **R6** are as defined according to Claim 1 and in which n represents 0, 1 or 2.

10. Compound of formula (I) according to Claim 9, **characterized in that** the said heterocycloalkyl group represents a piperidyl group, the said aryl group represents a phenyl group and the said heteroaryl group represents a pyridyl group.

11. Compound of formula (I) according to any one of Claims 1 to 3 and 5 to 7, **characterized in that R** represents a group -SO₂-NR3R4, advantageously in the β position of pyridine, and in which **R3** and **R4** form, together with the nitrogen atom that bears them, a heterocycloalkyl group, optionally substituted on at least one carbon atom and/or, where appropriate, on at least one heteroatom, with at least one substituent chosen from groups -(C1-C5)alkyl and groups -CH₂-aryl.

12. Compound of formula (I) according to Claim 11, **characterized in that** the said heterocycloalkyl group represents a group chosen from piperidyl, piperazinyl, morpholinyl, pyrrolidinyl and hexamethyleneimino groups, and the said aryl group represents a phenyl group.

13. Compound of formula (I) according to any one of Claims 1 to 12, **characterized in that** the group(s) **R, R1** and/or **R2** comprise the group(s) **R5** and/or **R6,**
**R5** or **R6** is a hydrogen atom, a group -(C1-C5)alkyl or a group -(C1-C5)haloalkyl,
or
**R5** and **R6,** which may be identical or different, represent, independently of each other, a hydrogen atom, a group -(C1-C5)alkyl or a group -(C1-C5)haloalkyl.

14. Compound of formula (I) according to Claim 13, **characterized in that R5** and/or **R6** are chosen from groups (C1-C5)alkyl.

15. Compound of formula (I) according to any one of Claims 1 to 13, **characterized in that R** represents a group -SO₂-NR3R4 or a hydrogen atom with R3 and R4 as defined in Claim 1 and/or **R2** represents a hydrogen atom or a group -(C1-C5)alkyl, advantageously a methyl, and/or **R1** represents a group -W-aryl or a group -W-heteroaryl, advantageously with the said W representing a -CH₂-, the said aryl representing a phenyl and the said heteroaryl representing a pyridine.

16. Compound of formula (I) according to any one of Claims 1 to 15, **characterized in that** it is:
• 4-(2-chlorobenzyl)-5-methyl-2-[5-(trifluoromethyl)pyridin-2-yl]-1,2-dihydro-3H-pyrazol-3-one;
• 6-[4-(2-chlorobenzyl)-3-methyl-5-oxo-2,5-dihydro-1H-pyrazol-1-yl]pyridine-3-carboxylic acid;
• 4-(2-chlorobenzyl)-5-methyl-2-(pyridin-2-yl)-1,2-dihydro-3H-pyrazol-3-one;
• 4-(2-chlorobenzyl)-5-methyl-2-{5-[(4-methylpiperazin-1-yl)sulfonyl]pyridin-2-yl}-1,2-dihydro-3H-pyrazol-3-one trifluoroacetate;
• (±)4-(2-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one;
• 6-[4-(2-chlorobenzyl)-3-methyl-5-oxo-2,5-dihydro-1H-pyrazol-1-yl]-N,N-diethylpyridine-3-sulfonamide;
• 6-[4-(2-chlorobenzyl)-3-methyl-5-oxo-2,5-dihydro-1H-pyrazol-1-yl]-N-ethyl-N-phenylpyridine-3-sulfonamide;
• 6-[4-(2-chlorobenzyl)-3-methyl-5-oxo-2,5-dihydro-1H-pyrazol-1-yl]-N,N-di(propan-2-yl)-pyridine-3-sulfonamide;
• 4-(2-chlorobenzyl)-5-methyl-2-[5-(piperidin-1-ylsulfonyl)pyridin-2-yl]-1,2-dihydro-3H-pyrazol-3-one;
• N,N-diethyl-6-[4-(2-fluorobenzyl)-3-methyl-5-oxo-2,5-dihydro-1H-pyrazol-1-yl]pyridine-3-sulfonamide;
• 4-(2-fluorobenzyl)-5-methyl-2-(pyridin-2-yl)-1,2-dihydro-3H-pyrazol-3-one;
• 4-(2-fluorobenzyl)-5-methyl-2-[5-(trifluoromethyl)pyridin-2-yl]-1,2-dihydro-3H-pyrazol-3-one;
• 4-(2-fluorobenzyl)-5-methyl-2-{5-[(4-methylpiperazin-1-yl)sulfonyl]pyridin-2-yl}-1,2-dihydro-3H-pyrazol-3-one trifluoroacetate;
• 2-(5-{[(3R,5S)-3,5-dimethylpiperidin-1-yl]sulfonyl}pyridin-2-yl)-4-(2-fluorobenzyl)-5-methyl-1,2-dihydro-3H-pyrazol-3-one;
• N-ethyl-6-[4-(2-fluorobenzyl)-3-methyl-5-oxo-2,5-dihydro-1H-pyrazol-1-yl]-N-phenylpyridine-3-sulfonamide;
• 4-(2-fluorobenzyl)-5-methyl-2-[5-(piperidin-1-ylsulfonyl)pyridin-2-yl]-1,2-dihydro-3H-pyrazol-3-one;
• 4-(2,4-dichlorobenzyl)-5-methyl-2-(pyridin-2-yl)-1,2-dihydro-3H-pyrazol-3-one;
• 4-(2,4-dichlorobenzyl)-5-methyl-2-[5-(trifluoromethyl)pyridin-2-yl]-1,2-dihydro-3H-pyrazol-3-one;
• 4-(2,4-dichlorobenzyl)-5-methyl-2-{5-[(4-methylpiperazin-1-yl)sulfonyl]pyridin-2-yl}-1,2-dihydro-3H-pyrazol-3-one trifluoroacetate;
• 4-(2,4-dichlorobenzyl)-2-(5-{[(3R,5S)-3,5-dimethylpiperidin-1-yl]sulfonyl}pyridin-2-yl)-5-methyl-1,2-dihydro-3H-pyrazol-3-one;
• 6-[4-(2,4-dichlorobenzyl)-3-methyl-5-oxo-2,5-dihydro-1H-pyrazol-1-yl]-N-ethyl -N-phenylpyridine-3-sulfonamide;
• 6-[4-(2,4-dichlorobenzyl)-3-methyl-5-oxo-2,5-dihydro-1H-pyrazol-1-yl]-N,N-di(propan-2-yl)pyridine-3-sulfonamide;
• 4-(2,4-dichlorobenzyl)-5-methyl-2-[5-(piperidin-1-ylsulfonyl)pyridin-2-yl]-1,2-dihydro-3H-pyrazol-3-one;
• 4-(2-chloro-6-fluorobenzyl)-5-methyl-2-[5-(trifluoromethyl)pyridin-2-yl]-1,2-dihydro-3H-pyrazol-3-one;
• 4-(2-chloro-6-fluorobenzyl)-5-methyl-2-{5-[(4-methylpiperazin-1-yl)sulfonyl]pyridin-2-yl}-1,2-dihydro-3H-pyrazol-3-one trifluoroacetate;
• 4-(2-chloro-6-fluorobenzyl)-2-(5-{[(3R,5S)-3,5-dimethylpiperidin-1-yl]sulfonyl}pyridin-2-yl)-5-methyl-1,2-dihydro-3H-pyrazol-3-one;
• 6-[4-(2-chloro-6-fluorobenzyl)-3-methyl-5-oxo-2,5-dihydro-1H-pyrazol-1-yl]-N,N-diethyl-pyridine-3-sulfonamide;
• 6-[4-(2-chloro-6-fluorobenzyl)-3-methyl-5-oxo-2,5-dihydro-1H-pyrazol-1-yl]-N-ethyl-N-phenylpyridine-3-sulfonamide;
• 6-[4-(2-chloro-6-fluorobenzyl)-3-methyl-5-oxo-2,5-dihydro-1H-pyrazol-1-yl]-N,N-di(propan-2-yl)pyridine-3-sulfonamide;
• 4-(2-chloro-6-fluorobenzyl)-5-methyl-2-[5-(piperidin-1-ylsulfonyl)pyridin-2-yl]-1,2-dihydro-3H-pyrazol-3-one;
• methyl 6-[4-(2-chlorobenzyl)-3-methyl-5-oxo-2,5-dihydro-1H-pyrazol-1-yl]pyridine-3-carboxylate;
• 4-(4-chlorobenzyl)-5-methyl-2-(pyridin-2-yl)-1,2-dihydro-3H-pyrazol-3-one;
• 4-(4-chlorobenzyl)-5-methyl-2-{5-[(4-methylpiperazin-1-yl)sulfonyl]pyridin-2-yl}-1,2-dihydro-3H-pyrazol-3-one;
• 4-(4-chlorobenzyl)-2-(5-{[(3R,5S)-3,5-dimethylpiperidin-1-yl]sulfonyl}pyridin-2-yl)-5-methyl-1,2-dihydro-3H-pyrazol-3-one;
• 6-[4-(4-chlorobenzyl)-3-methyl-5-oxo-2,5-dihydro-1H-pyrazol-1-yl]-N,N-diethylpyridine-3-sulfonamide;
• 6-[4-(4-chlorobenzyl)-3-methyl-5-oxo-2,5-dihydro-1H-pyrazol-1-yl]-N-ethyl-N-phenylpyridine-3-sulfonamide;
• 6-[4-(4-chlorobenzyl)-3-methyl-5-oxo-2,5-dihydro-1H-pyrazol-1-yl]-N,N-di(propan-2-yl)pyridine-3-sulfonamide;
• 4-(4-chlorobenzyl)-5-methyl-2-[5-(piperidin-1-ylsulfonyl)pyridin-2-yl]-1,2-dihydro-3H-pyrazol-3-one;
• 6-[4-(2-fluorobenzyl)-3-methyl-5-oxo-2,5-dihydro-1H-pyrazol-1-yl]-N,N-di(propan-2-yl)pyridine-3-sulfonamide;
• 4-(1,1-dioxidotetrahydrothiophen-3-yl)-5-methyl-2-[5-(piperidin-1-ylsulfonyl)pyridin-2-yl]-1,2-dihydro-3H-pyrazol-3-one;
• 6-[4-(1,1-dioxidotetrahydrothiophen-3-yl)-3-methyl-5-oxo-2,5-dihydro-1H-pyrazol-1-yl]-N-ethyl-N-phenylpyridine-3-sulfonamide;
• 6-[4-(1,1-dioxidotetrahydrothiophen-3-yl)-3-methyl-5-oxo-2,5-dihydro-1H-pyrazol-1-yl]-N,N-di(propan-2-yl)pyridine-3-sulfonamide;
• 2-{[5-methyl-3-oxo-2-(pyridin-2-yl)-2,3-dihydro-1H-pyrazol-4-yl]methyl}benzonitrile;
• 4-{[5-methyl-3-oxo-2-(pyridin-2-yl)-2,3-dihydro-1H-pyrazol-4-yl]methyl}benzonitrile;
• N-ethyl-6-{4-[4-(methoxymethyl)benzyl]-3-methyl-5-oxo-2,5-dihydro-1H-pyrazol-1-yl}-N-phenylpyridine-3-sulfonamide;
• 4-[3-(methoxymethyl)benzyl]-5-methyl-2-(pyridin-2-yl)-1,2-dihydro-3H-pyrazol-3-one;
• N-ethyl-6-{4-[3-(methoxymethyl)benzyl]-3-methyl-5-oxo-2,5-dihydro-1H-pyrazol-1-yl}-N-phenylpyridine-3-sulfonamide;
• 6-[4-(3-cyanobenzyl)-3-methyl-5-oxo-2,5-dihydro-1H-pyrazol-1-yl]-N-ethyl-N-phenylpyridine-3-sulfonamide;
• 6-[4-(4-cyanobenzyl)-3-methyl-5-oxo-2,5-dihydro-1H-pyrazol-1-yl]-N-ethyl-N-phenylpyridine-3-sulfonamide;
• 5-methyl-2-(pyridin-2-yl)-4-(pyridin-4-ylmethyl)-1,2-dihydro-3H-pyrazol-3-one;
• 3-{[5-methyl-3-oxo-2-(pyridin-2-yl)-2,3-dihydro-1H-pyrazol-4-yl]methyl}benzonitrile;
• 4-benzyl-2-(5-bromopyridin-2-yl)-5-methyl-1,2-dihydro-3H-pyrazol-3-one;
• 6-[4-(2-cyanobenzyl)-3-methyl-5-oxo-2,5-dihydro-1H-pyrazol-1-yl]-N-ethyl-N-phenylpyridine-3-sulfonamide;
• N-ethyl-6-{4-[3-(2-methoxyethoxy)benzyl]-3-methyl-5-oxo-2,5-dihydro-1H-pyrazol-1-yl}-N-phenylpyridine-3-sulfonamide;
• 4-(3,5-dimethoxybenzyl)-5-methyl-2-(pyridin-2-yl)-1,2-dihydro-3H-pyrazol-3-one;
• 5-methyl-4-[4-(methylsulfonyl)benzyl]-2-(pyridin-2-yl)-1,2-dihydro-3H-pyrazol-3-one;
• N-ethyl-6-{3-methyl-4-[4-(methylsulfonyl)benzyl]-5-oxo-2,5-dihydro-1H-pyrazol-1-yl}-N-phenylpyridine-3-sulfonamide;
• 4-[3-(2-methoxyethoxy)benzyl]-5-methyl-2-(pyridin-2-yl)-1,2-dihydro-3H-pyrazol-3-one;
• 4-benzyl-5-methyl-2-(pyridin-2-yl)-1,2-dihydro-3H-pyrazol-3-one;
• N-ethyl-6-[3-methyl-5-oxo-4-(pyridin-4-ylmethyl)-2,5-dihydro-1H-pyrazol-1-yl]-N-phenyl-pyridine-3-sulfonamide;
• N-ethyl-6-[3-methyl-5-oxo-4-(pyridin-2-ylmethyl)-2,5-dihydro-1H-pyrazol-1-yl]-N-phenyl-pyridine-3-sulfonamide;
• 5-methyl-2-(pyridin-2-yl)-4-(pyridin-3-ylmethyl)-1,2-dihydro-3H-pyrazol-3-one;
• 6-(4-benzyl-3-methyl-5-oxo-2,5-dihydro-1H-pyrazol-1-yl)-N-ethyl-N-phenylpyridine-3-sulfonamide;
• N-ethyl-6-[3-methyl-5-oxo-4-(pyridin-3-ylmethyl)-2,5-dihydro-1H-pyrazol-1-yl]-N-phenyl-pyridine-3-sulfonamide;
• 4-(2,5-dimethoxybenzyl)-5-methyl-2-(pyridin-2-yl)-1,2-dihydro-3H-pyrazol-3-one;
• 6-[4-(2,5-dimethoxybenzyl)-3-methyl-5-oxo-2,5-dihydro-1H-pyrazol-1-yl]-N-ethyl-N-phenyl-pyridine-3-sulfonamide;
• N-ethyl-6-{3-methyl-4-[(1-methylpiperidin-4-yl)methyl]-5-oxo-2,5-dihydro-1H-pyrazol-1-yl}-N-phenylpyridine-3-sulfonamide;
• N-ethyl-6-{3-methyl-5-oxo-4-[4-(trifluoromethyl)benzyl]-2,5-dihydro-1H-pyrazol-1-yl}-N-phenylpyridine-3-sulfonamide;
• N-ethyl-6-{3-methyl-5-oxo-4-[3-(trifluoromethyl)benzyl]-2,5-dihydro-1H-pyrazol-1-yl}-N-phenylpyridine-3-sulfonamide;
• 5-methyl-2-(pyridin-2-yl)-4-[3-(trifluoromethyl)benzyl]-1,2-dihydro-3H-pyrazol-3-one;
• 5-methyl-2-(pyridin-2-yl)-4-[4-(trifluoromethyl)benzyl]-1,2-dihydro-3H-pyrazol-3-one;
• 6-[4-(3,5-dimethoxybenzyl)-3-methyl-5-oxo-2,5-dihydro-1H-pyrazol-1-yl]-N-ethyl-N-phenyl-pyridine-3-sulfonamide;
• 4-(4-hydroxybenzyl)-5-methyl-2-(pyridin-2-yl)-1,2-dihydro-3H-pyrazol-3-one;
• N-ethyl-6-[4-(4-hydroxybenzyl)-3-methyl-5-oxo-2,5-dihydro-1H-pyrazol-1-yl]-N-phenylpyridine-3-sulfonamide;
• 6-(4-{4-[2-(dimethylamino)ethoxy]benzyl}-3-methyl-5-oxo-2,5-dihydro-1H-pyrazol-1-yl}-N-ethyl-N-phenylpyridine-3-sulfonamide;
• 6-{4-[4-(dimethylamino)benzyl]-3-methyl-5-oxo-2,5-dihydro-1H-pyrazol-1-yl}-N-ethyl-N-phenylpyridine-3-sulfonamide;
• 5-methyl-2-(pyridin-2-yl)-4-[2-(trifluoromethyl)benzyl]-1,2-dihydro-3H-pyrazol-3-one;
• 4-[4-(dimethylamino)benzyl]-5-methyl-2-(pyridin-2-yl)-1,2-dihydro-3H-pyrazol-3-one;
• N-ethyl-6-{3-methyl-5-oxo-4-[2-(trifluoromethyl)benzyl]-2,5-dihydro-1H-pyrazol-1-yl}-N-phenylpyridine-3-sulfonamide;
• 6-(4-benzyl-3-methyl-5-oxo-2,5-dihydro-1H-pyrazol-1-yl)-N-(3-methylbutyl)pyridine-3-sulfonamide;
• 4-[3-(dimethylamino)benzyl]-5-methyl-2-(pyridin-2-yl)-1,2-dihydro-3H-pyrazol-3-one;
• 6-{4-[3-(dimethylamino)benzyl]-3-methyl-5-oxo-2,5-dihydro-1H-pyrazol-1-yl}-N-ethyl-N-phenylpyridine-3-sulfonamide;
• 4-(4-methoxybenzyl)-5-methyl-2-(pyridin-2-yl)-1,2-dihydro-3H-pyrazol-3-one;
• N-ethyl-6-[4-(4-methoxybenzyl)-3-methyl-5-oxo-2,5-dihydro-1H-pyrazol-1-yl]-N-phenyl-pyridine-3-sulfonamide;
• 4-(2-methoxybenzyl)-5-methyl-2-(pyridin-2-yl)-1,2-dihydro-3H-pyrazol-3-one;
• 4-(3-methoxybenzyl)-5-methyl-2-(pyridin-2-yl)-1,2-dihydro-3H-pyrazol-3-one;
• N-ethyl-6-[4-(3-methoxybenzyl)-3-methyl-5-oxo-2,5-dihydro-1H-pyrazol-1-yl]-N-phenyl-pyridine-3-sulfonamide;
• 6-(4-{3-[2-(dimethylamino)ethoxy]benzyl}-3-methyl-5-oxo-2,5-dihydro-1H-pyrazol-1-yl)-N-ethyl-N-phenylpyridine-3-sulfonamide;
• N-ethyl-6-[4-(2-methoxybenzyl)-3-methyl-5-oxo-2,5-dihydro-1H-pyrazol-1-yl]-N-phenyl-pyridine-3-sulfonamide;
• 4-benzyl-5-methyl-2-[5-(morpholin-4-ylsulfonyl)pyridin-2-yl]-1,2-dihydro-3H-pyrazol-3-one;
• N-ethyl-6-[4-(3-hydroxybenzyl)-3-methyl-5-oxo-2,5-dihydro-1H-pyrazol-1-yl]-N-phenylpyridine-3-sulfonamide;
• 6-(4-benzyl-3-ethyl-5-oxo-2,5-dihydro-1H-pyrazol-1-yl)-N-ethyl-N-phenylpyridine-3-sulfonamide;
• 4-(3-hydroxybenzyl)-5-methyl-2-(pyridin-2-yl)-1,2-dihydro-3H-pyrazol-3-one;
• N-ethyl-6-{4-[4-(2-methoxyethoxy)benzyl]-3-methyl-5-oxo-2,5-dihydro-1H-pyrazol-1-yl}-N-phenylpyridine-3-sulfonamide;
• 4-benzyl-5-ethyl-2-(pyridin-2-yl)-1,2-dihydro-3H-pyrazol-3-one;
• 4-benzyl-5-methyl-2-[5-(pyrrolidin-1-ylsulfonyl)pyridin-2-yl]-1,2-dihydro-3H-pyrazol-3-one;
• 2-[5-(azepan-1-ylsulfonyl)pyridin-2-yl]-4-benzyl-5-methyl-1,2-dihydro-3H-pyrazol-3-one;
• N,N-diethyl-6-[3-methyl-5-oxo-4-(pyridin-3-ylmethyl)-2,5-dihydro-1H-pyrazol-1-yl]pyridine-3-sulfonamide;
• N,N-diethyl-6-[3-methyl-5-oxo-4-(pyridin-3-ylmethyl)-2,5-dihydro-1H-pyrazol-1-yl]pyridine-3-sulfonamide hydrochloride;
• N,N-dimethyl-6-[3-methyl-5-oxo-4-(pyridin-3-ylmethyl)-2,5-dihydro-1H-pyrazol-1-yl]pyridine-3-sulfonamide;
• N,N-dimethyl-6-[3-methyl-5-oxo-4-(pyridin-3-ylmethyl)-2,5-dihydro-1H-pyrazol-1-yl]pyridine-3-sulfonamide hydrochloride;
• 6-(4-benzyl-5-oxo-3-propyl-2,5-dihydro-1H-pyrazol-1-yl)-N-ethyl-N-phenylpyridine-3-sulfonamide hydrochloride;
• 6-[3-methyl-5-oxo-4-(pyridin-3-ylmethyl)-2,5-dihydro-1H-pyrazol-1-yl]-N-(propan-2-yl)pyridine-3-sulfonamide hydrochloride;
• 5-methyl-4-(pyridin-3-ylmethyl)-2-[5-(pyrrolidin-1-ylsulfonyl)pyridin-2-yl]-1,2-dihydro-3H-pyrazol-3-one hydrochloride;
• N-tert-butyl-6-[3-methyl-5-oxo-4-(pyridin-3-ylmethyl)-2,5-dihydro-1H-pyrazol-1-yl]pyridine-3-sulfonamide hydrochloride;
• N-cyclopropyl-6-[3-methyl-5-oxo-4-(pyridin-3-ylmethyl)-2,5-dihydro-1H-pyrazol-1-yl]pyridine-3-sulfonamide hydrochloride;
• N-cyclopentyl-6-[3-methyl-5-oxo-4-(pyridin-3-ylmethyl)-2,5-dihydro-1H-pyrazol-1-yl]pyridine-3-sulfonamide hydrochloride;
• N-ethyl-6-[3-methyl-5-oxo-4-(2-phenylethyl)-2,5-dihydro-1H-pyrazol-1-yl]-N-phenylpyridine-3-sulfonamide;
• 5-methyl-4-(2-phenylethyl)-2-(pyridin-2-yl)-1,2-dihydro-3H-pyrazol-3-one;
• N-methyl-6-[3-methyl-5-oxo-4-(pyridin-3-ylmethyl)-2,5-dihydro-1H-pyrazol-1-yl]-N-(pyridin-2-yl)pyridine-3-sulfonamide hydrochloride;
• 2-{5-[(4-benzylpiperidin-1-yl)sulfonyl]pyridin-2-yl}-5-methyl-4-(pyridin-3-ylmethyl)-1,2-dihydro-3H-pyrazol-3-one;
• N-ethyl-6-[3-methyl-5-oxo-4-(2-phenylpropan-2-yl)-2,5-dihydro-1H-pyrazol-1-yl]-N-phenyl-pyridine-3-sulfonamide;
• N-ethyl-6-{4-[(6-methoxypyridin-2-yl)methyl]-3-methyl-5-oxo-2,5-dihydro-1H-pyrazol-1-yl}-N-phenylpyridine-3-sulfonamide;
• 6-[3-methyl-5-oxo-4-(pyridin-3-ylmethyl)-2,5-dihydro-1H-pyrazol-1-yl]-N-(pyridin-2-yl)pyridine-3-sulfonamide hydrochloride;
• N-ethyl-6-[3-methyl-5-oxo-4-(l-phenylcyclopropyl)-2,5-dihydro-1H-pyrazol-1-yl]-N-phenyl-pyridine-3-sulfonamide;
• N-ethyl-6-{4-[(3-methoxypyridin-2-yl)methyl]-3-methyl-5-oxo-2,5-dihydro-1H-pyrazol-1-yl}-N-phenylpyridine-3-sulfonamide hydrochloride;
• 6-[4-benzyl-3-(methoxymethyl)-5-oxo-2,5-dihydro-1H-pyrazol-1-yl]-N-ethyl-N-phenylpyridine-3-sulfonamide;
• 6-[4-benzyl-5-oxo-3-(trifluoromethyl)-2,5-dihydro-1H-pyrazol-1-yl]-N-ethyl-N-phenylpyridine-3-sulfonamide;
• 1-[5-(azepan-1-ylsulfonyl)pyridin-2-yl]-3-methyl-4-(pyridin-3-ylmethyl)-1H-pyrazol-5-olate;
• N-ethyl-6-{3-methyl-5-oxo-4-[2-(pyridin-2-yl)ethyl]-2,5-dihydro-1H-pyrazol-1-yl}-N-phenyl-pyridine-3-sulfonamide;
• N-ethyl-6-{4-[(5-methoxypyridin-3-yl)methyl]-3-methyl-5-oxo-2,5-dihydro-1H-pyrazol-1-yl}-N-phenylpyridine-3-sulfonamide;
• N-(2-methoxyethyl)-N-methyl-6-[3-methyl-5-oxo-4-(pyridin-3-ylmethyl)-2,5-dihydro-1H-pyrazol-1-yl]pyridine-3-sulfonamide;
• 6-[4-benzyl-3-(2-methylpropyl)-5-oxo-2,5-dihydro-1H-pyrazol-1-yl]-N-ethyl-N-phenylpyridine-3-sulfonamide;
• N-ethyl-6-[3-methyl-5-oxo-4-(pyridin-3-ylmethyl)-2,5-dihydro-1H-pyrazol-1-yl]pyridine-3-sulfonamide;
• 6-[3-methyl-5-oxo-4-(pyridin-3-ylmethyl)-2,5-dihydro-1H-pyrazol-1-yl]-N-phenylpyridine-3-sulfonamide;
• N-ethyl-6-[3-methyl-5-oxo-4-(3-phenylpropyl)-2,5-dihydro-1H-pyrazol-1-yl]-N-phenylpyridine-3-sulfonamide;
• 6-(4-benzyl-5-oxo-2,5-dihydro-1H-pyrazol-1-yl)-N-ethyl-N-phenylpyridine-3-sulfonamide;
• N-cyclopropyl-N-methyl-6-[3-methyl-5-oxo-4-(pyridin-3-ylmethyl)-2,5-dihydro-1H-pyrazol-1-yl]pyridine-3-sulfonamide;
• N-tert-butyl-6-{4-[(5-methoxypyridin-3-yl)methyl]-3-methyl-5-oxo-2,5-dihydro-1H-pyrazol-1-yl}pyridine-3-sulfonamide;
• 6-{4-[(5-cyanopyridin-3-yl)methyl]-3-methyl-5-oxo-2,5-dihydro-1H-pyrazol-1-yl}-N-ethyl-N-phenylpyridine-3-sulfonamide;
• tert-butyl 6-[5-oxo-4-(pyridin-3-ylmethyl)-2,5-dihydro-1H-pyrazol-1-yl]pyridine-3-carboxylate;
• tert-butyl 6-[3-methyl-5-oxo-4-(pyridin-3-ylmethyl)-2,5-dihydro-1H-pyrazol-1-yl]pyridine-3-carboxylate;
• N-tert-butyl-6-[5-oxo-4-(pyridin-3-ylmethyl)-2,5-dihydro-1H-pyrazol-1-yl]pyridine-3-sulfonamide;
• N-tert-butyl-6-[5-oxo-4-(pyridin-3-ylmethyl)-2,5-dihydro-1H-pyrazol-1-yl]pyridine-3-sulfonamide hydrochloride;
• N-ethyl-6-[5-oxo-4-(pyridin-3-ylmethyl)-2,5-dihydro-1H-pyrazol-1-yl]-N-phenylpyridine-3-sulfonamide;
• 6-(4-benzyl-5-oxo-2,5-dihydro-1H-pyrazol-1-yl)-N-tert-butyl-N-methylpyridine-3-sulfonamide;
• N-ethyl-6-{4-[(5-methoxypyridin-3-yl)methyl]-5-oxo-2,5-dihydro-1H-pyrazol-1-yl}-N-phenyl-pyridine-3-sulfonamide;
• 5-methyl-2-[5-(phenylsulfonyl)pyridin-2-yl]-4-(pyridin-3-ylmethyl)-1,2-dihydro-3H-pyrazol-3-one;
• N-tert-butyl-6-{4-[(5-methoxypyridin-3-yl)methyl]-3-methyl-5-oxo-2,5-dihydro-1H-pyrazol-1-yl}-N-methylpyridine-3-sulfonamide;
• 6-{4-[(6-cyanopyridin-3-yl)methyl]-3-methyl-5-oxo-2,5-dihydro-1H-pyrazol-1-yl}-N-ethyl-N-phenylpyridine-3-sulfonamide;
• (2-{5-[ethyl(phenyl)sulfamoyl]pyridin-2-yl}-3-oxo-2,3-dihydro-1H-pyrazol-4-yl)acetic acid;
• 2-(2-{5-[ethyl(phenyl)sulfamoyl]pyridin-2-yl}-3-oxo-2,3-dihydro-1H-pyrazol-4-yl)-N,N-dimethyl-acetamide;
• methyl (2-{5-[ethyl(phenyl)sulfamoyl]pyridin-2-yl}-3-oxo-2,3-dihydro-1H-pyrazol-4-yl)acetate;
• ethyl (4-benzyl-1-{5-[ethyl(phenyl)sulfamoyl]pyridin-2-yl}-5-oxo-2,5-dihydro-1H-pyrazol-3-yl)acetate;
• 2-(2-{5-[ethyl(phenyl)sulfamoyl]pyridin-2-yl}-3-oxo-2,3-dihydro-1H-pyrazol-4-yl)-N-methyl-acetamide;
• N-tert-butyl-6-{4-[(5-methoxypyridin-2-yl)methyl]-3-methyl-5-oxo-2,5-dihydro-1H-pyrazol-1-yl}-N-methylpyridine-3-sulfonamide;
• 2-(4-benzyl-1-{5-[ethyl(phenyl)sulfamoyl]pyridin-2-yl}-5-oxo-2,5-dihydro-1H-pyrazol-3-yl)-N,N-dimethylacetamide;
• 3-(2-{5-[ethyl(phenyl)sulfamoyl]pyridin-2-yl}-3-oxo-2,3-dihydro-1H-pyrazol-4-yl)propanoic acid;
• methyl 3-[(2-{5-[ethyl(phenyl)sulfamoyl]pyridin-2-yl}-5-methyl-3-oxo-2,3-dihydro-1H-pyrazol-4-yl)methyl]benzoate;
• methyl 3-(2-{5-[ethyl(phenyl)sulfamoyl]pyridin-2-yl}-3-oxo-2,3-dihydro-1H-pyrazol-4-yl)-propanoate;
• methyl {2-[5-(tert-butylsulfamoyl)pyridin-2-yl]-3-oxo-2,3-dihydro-1H-pyrazol-4-yl}(phenyl)-acetate;
• methyl 2-[(2-{5-[ethyl(phenyl)sulfamoyl]pyridin-2-yl}-5-methyl-3-oxo-2,3-dihydro-1H-pyrazol-4-yl)methyl]benzoate;
• N-cyclopentyl-N-ethyl-6-[5-oxo-4-(pyridin-3-ylmethyl)-2,5-dihydro-1H-pyrazol-1-yl]pyridine-3-sulfonamide;
• N-cyclopentyl-N-methyl-6-[5-oxo-4-(pyridin-3-ylmethyl)-2,5-dihydro-1H-pyrazol-1-yl]pyridine-3-sulfonamide;
• 2-[(1-{5-[ethyl(phenyl)sulfamoyl]pyridin-2-yl}-3-methyl-5-oxido-1H-pyrazol-4-yl)methyl]-benzoate;
• 2-[5-(azepan-1-ylsulfonyl)pyridin-2-yl]-4-(pyridin-3-ylmethyl)-1,2-dihydro-3H-pyrazol-3-one;
• N-cyclopentyl-N-methyl-6-[5-oxo-4-(pyridin-4-ylmethyl)-2,5-dihydro-1H-pyrazol-1-yl]pyridine-3-sulfonamide;
• N-cyclopentyl-N-ethyl-6-[5-oxo-4-(pyridin-4-ylmethyl)-2,5-dihydro-1H-pyrazol-1-yl]pyridine-3-sulfonamide;
• methyl 3-(2-{5-[cyclopentyl(methyl)sulfamoyl]pyridin-2-yl}-3-oxo-2,3-dihydro-1H-pyrazol-4-yl)-3-phenylpropanoate;
• N-(2,2-dimethylpropyl)-N-methyl-6-[5-oxo-4-(pyridin-3-ylmethyl)-2,5-dihydro-1H-pyrazol-1-yl]pyridine-3-sulfonamide;
• 2,2-dimethylpropyl 6-[5-oxo-4-(pyridin-3-ylmethyl)-2,5-dihydro-1H-pyrazol-1-yl]pyridine-3-carboxylate;
• methyl N-cyclopentyl-N-({6-[5-oxo-4-(pyridin-3-ylmethyl)-2,5-dihydro-1H-pyrazol-1-yl]pyridin-3-yl}sulfonyl)glycinate;
• 6-(4-benzyl-5-oxo-2,5-dihydro-1H-pyrazol-1-yl)-N-[2-(benzyloxy)ethyl]-N-cyclopentylpyridine-3-sulfonamide;
• 6-(4-benzyl-5-oxo-2,5-dihydro-1H-pyrazol-1-yl)-N-[3-(benzyloxy)propyl]-N-cyclopentylpyridine-3-sulfonamide;
• 6-(4-benzyl-5-oxo-2,5-dihydro-1H-pyrazol-1-yl)-N-cyclopentyl-N-(3-hydroxypropyl)pyridine-3-sulfonamide;
• 6-(4-benzyl-5-oxo-2,5-dihydro-1H-pyrazol-1-yl)-N-cyclopentyl-N-(2-hydroxyethyl)pyridine-3-sulfonamide;
• methyl (1S,2R)-2-[methyl({6-[5-oxo-4-(pyridin-3-ylmethyl)-2,5-dihydro-1H-pyrazol-1-yl]pyridin-3-yl}sulfonyl)amino]cyclopentanecarboxylate;
• 6-(4-benzyl-5-oxo-2,5-dihydro-1H-pyrazol-1-yl)-N-cyclopentyl-N-(2,3-dihydroxypropyl)pyridine-3-sulfonamide;
• 6-(4-benzyl-5-oxo-2,5-dihydro-1 H-pyrazol-1-yl)-N-cyclopentyl-N-[2-(dimethylamino)ethyl]-pyridine-3-sulfonamide;
• 4-benzyl-2-{5-[(4-methylpiperazin-1-yl)sulfonyl]pyridin-2-yl}-1,2-dihydro-3H-pyrazol-3-one;
• 6-[4-(cyclopentylmethyl)-5-oxo-2,5-dihydro-1H-pyrazol-1-yl]-N-ethyl-N-phenylpyridine-3-sulfonamide;
• 4-benzyl-2-{5-[(4-methyl-1,4-diazepan-1-yl)sulfonyl]pyridin-2-yl}-1,2-dihydro-3H-pyrazol-3-one;
• N-(2,2-dimethylpropyl)-6-[5-oxo-4-(pyridin-3-ylmethyl)-2,5-dihydro-1H-pyrazol-1-yl]pyridine-3-sulfonamide;
• N-(2-methylbutan-2-yl)-6-[5-oxo-4-(pyridin-3-ylmethyl)-2,5-dihydro-1H-pyrazol-1-yl]pyridine-3-sulfonamide;
• 6-(4-benzyl-5-oxo-2,5-dihydro-1H-pyrazol-1-yl)-N-[(1R,3S)-3-(hydroxymethyl)yclopentyl]-N-methylpyridine-3-sulfonamide;
• 6-(4-benzyl-5-oxo-2,5-dihydro-1H-pyrazol-1-yl)-N-methyl-N-(1-methylpyrrolidin-3-yl)pyridine-3-sulfonamide;
• 6-(4-benzyl-5-oxo-2,5-dihydro-1H-pyrazol-1-yl)-N-(2-methoxy-2-methylpropyl)pyridine-3-sulfonamide;
• 2,2-dimethylpropyl 6-(4-benzyl-5-oxo-2,5-dihydro-1H-pyrazol-1-yl)pyridine-3-carboxylate;
• 6-(4-benzyl-5-oxo-2,5-dihydro-1H-pyrazol-1-yl)-N-cyclopentyl-N-[(2R)-2,3-dihydroxypropyl]-pyridine-3-sulfonamide;
• 6-(4-benzyl-5-oxo-2,5-dihydro-1H-pyrazol-1-yl)-N-(2,3-dihydroxypropylyN-phenylpyridine-3-sulfonamide;
• N-cyclopentyl-6-{4-[(4-methoxypyridin-3-yl)methyl]-5-oxo-2,5-dihydro-1H-pyrazol-1-yl}-N-methylpyridine-3-sulfonamide;
• N-methyl-N-(2-methylbutan-2-yl)-6-[5-oxo-4-(pyridin-3-ylmethyl)-2,5-dihydro-1H-pyrazol-1-yl]pyridine-3-sulfonamide;
• 3-(2-{5-[cyclopentyl(methyl)sulfamoyl]pyridin-2-yl}-3-oxo-2,3-dihydro-1H-pyrazol-4-yl)-3-phenyl-N-(2,2,2-trifluoroethyl)propanamide;
• 6-(4-benzyl-5-oxo-2,5-dihydro-1H-pyrazol-1-yl)-N-cyclopentyl-N-[3-hydroxy-2-(hydroxymethyl)propyl]pyridine-3-sulfonamide;
• 6-(4-benzyl-5-oxo-2,5-dihydro-1H-pyrazol-1-yl)-N-cyclopentyl-N-[(2S)-2,3-dihydroxypropyl]-pyridine-3-sulfonamide;
• 2,2-dimethylpropyl 6-[5-oxo-4-(pyridin-4-ylmethyl)-2,5-dihydro-1H-pyrazol-1-yl]pyridine-3-carboxylate;
• 6-(4-benzyl-5-oxo-2,5-dihydro-1H-pyrazol-1-yl)-N-[(1S,2S)-2-hydroxycyclopentyl]pyridine-3-sulfonamide;
• N-tert-butyl-6-[5-oxo-4-(pyridin-4-ylmethyl)-2,5-dihydro-1H-pyrazol-1-yl]pyridine-3-sulfonamide;
• 4-benzyl-2-(5-{[(3R)-3-(dimethylamino)pyrrolidin-1-yl]sulfonyl}pyridin-2-yl)-1,2-dihydro-3H-pyrazol-3-one;
• 6-(4-benzyl-5-oxo-2,5-dihydro-1H-pyrazol-1-yl)-N-[1-(dimethylamino)-2-methylpropan-2-yl]pyridine-3-sulfonamide;
• 6-(4-benzyl-5-oxo-2,5-dihydro-1H-pyrazol-1-yl)-N-methyl-N-phenylpyridine-3-sulfonamide;
• methyl 3-{2-[5-(tert-butylsulfamoyl)pyridin-2-yl]-3-oxo-2,3-dihydro-1H-pyrazol-4-yl}-3-(pyridin-3-yl)propanoate;
• ethyl 3-{2-[5-(tert-butylsulfamoyl)pyridin-2-yl]-3-oxo-2,3-dihydro-1H-pyrazol-4-yl}-3-phenyl-propanoate;
• 6-(4-benzyl-5-oxo-2,5-dihydro-1H-pyrazol-1-yl)-N-tert-butyl-N-(2,3-dihydroxypropyl)pyridine-3-sulfonamide;
• 6-(4-benzyl-5-oxo-2,5-dihydro-1H-pyrazol-1-yl)-N-(2-hydroxyethyl)pyridine-3-sulfonamide;
• N-(2,3-dihydroxypropyl)-6-[5-oxo-4-(pyridin-3-ylmethyl)-2,5-dihydro-1H-pyrazol-1-yl]-N-phenyl-pyridine-3-sulfonamide hydrochloride;
• methyl 3-(2-{5-[cyclopentyl(methyl)sulfamoyl]pyridin-2-yl}-3-oxo-2,3-dihydro-1H-pyrazol-4-yl)-3-(pyridin-3-yl)propanoate hydrochloride;
• 6-(4-benzyl-5-oxo-2,5-dihydro-1H-pyrazol-1-yl)-N-cyclopentyl-N-[3-(dimethylamino)propyl]-pyridine-3-sulfonamide hydrochloride;
• 6-(4-benzyl-5-oxo-2,5-dihydro-1H-pyrazol-1-yl)-N-cyclopentyl-N-[3-(dimethylamino)propyl]-pyridine-3-sulfonamide hydrochloride.

17. Process for preparing a compound of formula (I) according to any one of Claims 1 to 16, **characterized in that** a compound of formula (II) 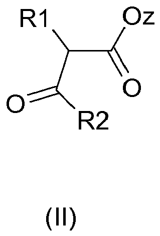 in which R1 and R2 are as defined in Claim 1 and z represents an alkyl group, is reacted with a compound of formula (III) 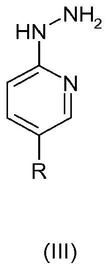 in which R is as defined in Claim 1.

18. Medicament, **characterized in that** it comprises a compound of formula (I) according to any one of Claims 1 to 16, or an addition salt of this compound with a pharmaceutically acceptable acid of the compound of formula (I).

19. Pharmaceutical composition, **characterized in that** it comprises a compound of formula (I) according to any one of Claims 1 to 16, or a pharmaceutically acceptable salt of this compound, and also at least one pharmaceutically acceptable excipient.

20. Use of a compound of formula (I) according to any one of Claims 1 to 16, for the preparation of a medicament for the treatment/prophylaxis of cardiovascular diseases.

21. Use of a compound of formula (I) according to any one of Claims 1 to 16, for the preparation of a medicament for the treatment/prophylaxis of ischaemia of the lower limbs, cardiac insufficiency, coronary diseases of ischaemic origin, for instance angina pectoris or myocardial infarction, arteriosclerosis, strokes of ischaemic origin, pulmonary hypertension and any pathology caused by partial or total vascular occlusion.

22. Use of a compound of formula (I) according to any one of Claims 1 to 16, for the preparation of a medicament for the treatment/prophylaxis of glaucoma, renal diseases or cerebral diseases of neurodegenerative origin or otherwise, anaemia, or a medicament for promoting cicatrization, or agents for shortening the post-operative convalescence period, or a medicament for treating general fatigue conditions, or alternatively a medicament used for the purpose of obtaining blood in the context of autotransfusions necessary following major surgical interventions such as cranial or chest surgery, or heart, carotid or aortic operations.

23. Use of a compound of formula (I) according to any one of Claims 1 to 16, for the preparation of a medicament for the treatment/prophylaxis of cardiac or peripheral diseases of ischaemic origin via regenerative medicine using stem cells.

24. Combination of a compound of formula (I) according to any one of Claims 1 to 16 with one or more active compound(s) that is (are) useful in the treatment of hypertension, cardiac insufficiency, diabetes and anaemia.
